# EUROPEAN PATENT APPLICATION

(11) **EP 2 136 209 A1**
(43) Date of publication of application: **23.12.2009**
(21) Application number: 08721397.1
(22) Date of filing: 05.03.2008
(51) Int. Cl.: G01N 33/15, G01N 33/50

(54) **METHOD FOR EXAMINATION OF ACTION OF ANTI-CANCER AGENT UTILIZING SPLICING DEFECT AS MEASURE**

(30) Priority: 05.03.2007 US 904774 P; 28.09.2007 US 960403 P
(71) Applicant: Eisai R&D Management Co., Ltd., Tokyo 112-8088 (JP)
(72) Inventor: MIZUI, Yoshiharu, Tsukuba-shi Ibaraki 300-2635 (JP); HATA, Naoko, Tsukuba-shi Ibaraki 300-2635 (JP); IWATA, Masao, Tsukuba-shi Ibaraki 300-2635 (JP); SAGANE, Koji, Tsukuba-shi Ibaraki 300-2635 (JP); UESUGI, Mai, Tsukuba-shi Ibaraki 300-2635 (JP); KADOWAKI, Tadashi, Tsukuba-shi Ibaraki 300-2635 (JP); YOSHIDA, Taku, Tsukuba-shi Ibaraki 300-2635 (JP)
(74) Representative: Woods, Geoffrey Corlett
(86) International application number: PCT/JP2008/053977
(87) International publication number: WO 2008/111464

(57) **Abstract**

An object of the present invention is to provide a method, a probe, a primer, an antibody, a reagent, and a kit for assaying an action of a pladienolide derivative to a living subject. According to the present invention, there is provided a method for assaying an action of the pladienolide derivative using splicing defect as an index.

## Description

### BACKGROUND OF THE INVENTION

### Field of the Invention

The present invention relates to a method for assaying an action of an antitumor agent using splicing defect as an index, more particularly, a method for assaying an action of an antitumor agent using an increase in the expression level of pre-mRNA or abnormal protein as an index.

### Background Art

Pladienolide derivatives are derivatives of natural pladienolide. Since pladienolide exhibits an excellent antitumor activity (Mizui et al., 2004, J. Antibiotics 577:188-196), search for a compound with higher antitumor activities has been performed to find the pladienolide derivatives (WO2002/060890 and WO2003/099813).

### SUMMARY OF THE INVENTION

The present inventors have found that pre-mRNA of a certain group of genes and abnormal proteins increase when a pladienolide derivative is contacted with a sample obtained from living cells including cancerous cells, and peripheral blood (PBMC) and whole blood (PBC) of a subject. Without wishing to be bound by any theory, administration of the pladienolide derivative may cause an introduction of a mutation in pre-mRNA of a certain group of genes thereby inducing splicing defect. The present invention was made based on such findings.

It is an object of the present invention to provide a method, a probe, a primer, an antibody, a reagent and a kit for assaying an action of the pladienolide derivative on a living subject.

According to the present invention, there are provided inventions (1) to (19) as follows.

(1) A method for assaying an action of a compound represented by formula (I), a pharmaceutically acceptable salt thereof, or a solvate of them to a mammal, which comprises detecting splicing defect caused by the compound represented by formula (I), the pharmaceutically acceptable salt thereof, or the solvate of them:

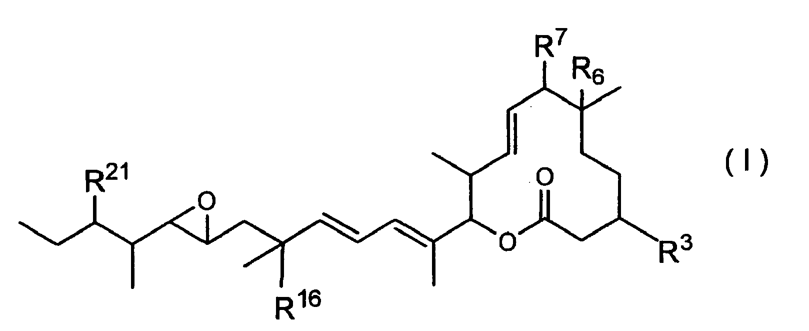

wherein R³, R⁶, R⁷ and R²¹, the same or different, each represents
1) a hydroxyl group or an oxo group formed together with the carbon atom to which it is bound, provided that R⁶ is limited to a hydroxyl group,
2) an optionally substituted C₁₋₂₂ alkoxy group,
3) an optionally substituted unsaturated C₂₋₂₂ alkoxy group,
4) an optionally substituted C₇₋₂₂ aralkyloxy group,
5) an optionally substituted 5- to 14-membered heteroaralkyloxy group,
6) RCO-O- wherein R represents
   a) a hydrogen atom,
   b) an optionally substituted C₁₋₂₂ alkyl group,
   c) an optionally substituted unsaturated C₂₋₂₂ alkyl group,
   d) an optionally substituted C₆₋₁₄ aryl group,
   e) an optionally substituted 5- to 14-membered heteroaryl group,
   f) an optionally substituted C₇₋₂₂ aralkyl group,
   g) an optionally substituted 5- to 14-membered heteroaralkyl group,
   h) an optionally substituted C₁₋₂₂ alkoxy group,
   i) an optionally substituted unsaturated C₂₋₂₂ alkoxy group,
   j) an optionally substituted C₆₋₁₄ aryloxy group or
   k) an optionally substituted 5- to 14-membered heteroaryloxy group,
7) R^{S1} R^{S2}R^{S3}SiO- wherein R^{S1}, R^{S2}, and R^{S3}, the same or different, each represents
   a) a C₁₋₆ alkyl group or
   b) a C₆₋₁₄ aryl group,
8) a halogen atom,
9) R^{N1}R^{N2}N-R^{M}- wherein R^{M} represents
   a) a single bond,
   b) -CO-O-,
   c) -SO₂-O-,
   d) -CS-O-or
   e) -CO-NR^{N3}- wherein R ^{N3} represents a hydrogen atom or an optionally substituted C₁₋₆ alkyl group, provided that each of the leftmost bond in b) to e)is bound to the nitrogen atom; and R^{N1} and R^{N2}, the same or different from each other and each represents
      a) a hydrogen atom,
      b) an optionally substituted C₁₋₂₂ alkyl group,
      c) an optionally substituted unsaturated C₂₋₂₂ alkyl group,
      d) an optionally substituted aliphatic C₂₋₂₂ acyl group,
      e) an optionally substituted aromatic C₇₋₁₅ acyl group,
      f) an optionally substituted C₆₋₁₄ aryl group,
      g) an optionally substituted 5- to 14-membered heteroaryl group,
      h) an optionally substituted C₇₋₂₂ aralkyl group,
      i) an optionally substituted C₁₋₂₂ alkylsulfonyl group,
      j) an optionally substituted C₆₋₁₄ arylsulfonyl group,
      k) an optionally substituted 3- to 14-membered non-aromatic heterocyclic group formed by R^{N1} and R^{N2} together with the nitrogen atom to which R^{N1} and R^{N2} are bound, and the non-aromatic heterocyclic group optionally has substituent(s),
      l) an optionally substituted 5- to 14-membered heteroaralkyl group,
      m) an optionally substituted C₃₋₁₄ cycloalkyl group or
      n) an optionally substituted 3- to 14-membered non-aromatic heterocyclic group,
10) R^{N4}SO₂-O- wherein R^{N4} represents
   a) an optionally substituted C₁₋₂₂ alkyl group,
   b) an optionally substituted C₆₋₁₄ aryl group,
   c) an optionally substituted C₁₋₂₂ alkoxy group,
   d) an optionally substituted unsaturated C₂₋₂₂ alkoxy group,
   e) an optionally substituted C₆₋₁₄ aryloxy group,
   f) an optionally substituted 5- to 14-membered heteroaryloxy group,
   g) an optionally substituted C₇₋₂₂ aralkyloxy group or
   h) an optionally substituted 5- to 14-membered heteroaralkyloxy group,
11) (R^{N5}O)₂PO-O- wherein R^{N5} represents
   a) an optionally substituted C₁₋₂₂ alkyl group,
   b) an optionally substituted unsaturated C₂₋₂₂ alkyl group,
   c) an optionally substituted C₆₋₁₄ aryl group,
   d) an optionally substituted 5- to 14-membered heteroaryl group,
   e) an optionally substituted C₇₋₂₂ aralkyl group or
   f) an optionally substituted 5- to 14-membered heteroaralkyl group,
12) (R^{N1}R^{N2}N)₂PO-O- wherein R^{N1} and R^{N2} have the same meanings as defined above or
13) (R^{N1}R^{N2}N)(R^{N5}O)PO-O- wherein R^{N1}, R^{N2} and R^{N5} have the same meanings as defined above, provided that a compound in which R³, R⁶, R⁷ and R²¹ are all hydroxyl groups, and a compound in which R³, R⁶ and R²¹ are all hydroxyl groups and R⁷ is an acetoxy group are excluded,
   R¹⁶ represents a hydrogen atom or hydroxyl group.
   (2) The method according to (1), wherein the compound represented by formula (I) is selected from the group consisting of:
   (8E,12E,14E)-7-(N-(2-(N',N'-Dimethylamino)ethyl)-N-methylcarbamoyloxy)-3,6,16,21-tetrahydroxy-6,10,12,16,20-pentamethyl18,19-epoxytricosa-8,12,14-trien-11-olide;
   (8E,12E,14E)-3,6,16,21-Tetrahydroxy-6,10,12,16,20-pentamethyl-7-((4-methylhomopiperazin-1-yl)carbonyl)oxy-18,19-epoxytricosa-8,12,14-trien-11-olide;
   (8E,12E,14E)-3,6,16,21-Tetrahydroxy-6,10,12,16,20-pentamethyl-7-((4-methyl piperazin-1-yl)carbonyl)oxy-18,19-epoxytricosa-8,12,14-trien-11-olide;
   (8E,12E,14E)-7-((4-Butylpiperazin-1-yl)carbonyl)oxy-3,6,16,21-tetrahydroxy-6,10,12,16,20-pentamethyl-18,19-epoxytricosa-8,12,14-trien-11-olide;
   (8E,12E,14E)-7-((4-Ethylpiperazin-1-yl)carbonyl)oxy-3,6,16,21-tetrahydroxy-6,10,12,16,20-pentamethyl-18,19-epoxytricosa-8,12,14-trien-11-olide;
   (8E,12E,14E)-3,6,16,21-Tetrahydroxy-6,10,12,16,20-pentamethyl-7-((4-propylpiperazin-1-yl)carbonyl)oxy-18,19-epoxytricosa-8,12,14-trien-11-olide;
   (8E,12E,14E)-7-((4-Cyclohexylpiperazin-1-yl)carbonyl)oxy-3,6,16,21-tetrahydroxy-6,10,12,16,20-pentamethyl-18,19-epoxytricosa-8,12,14-trien-11-olide;
   (8E,12E,14E)-7-((4-(Cyclopropylmethyl)piperazin-1-yl)carbonyl)oxy-3,6,16,
   21-tetrahydroxy-6,10,12,16,20-pentamethyl-18,19-epoxytricosa-8,12,14-trien-11-olide;
   (8E,12E,14E)-3,6,16,21-Tetrahydroxy-6,10,12,16,20-pentamethyl-7-((4-propylhomopiperazin-1-yl)carbonyl)oxy-18,19-epoxytricosa-8,12,14-trien-11-olide;
   (8E,12E,14E)-7-((4-(Cyclopropylmethyl)homopiperazin-1-yl)carbonyl)oxy-3,6, 16,21-tetrahydroxy-6,10,12,16,20-pentamethyl-18,19-epoxytricosa-8,12,14-trien-11-olide;
   (8E,12E,14E)-7-((4-Cyclopentylpiperazin-1-yl)carbonyl)oxy-3,6,16,21-tetrahydroxy-6,10,12,16,20-pentamethyl-18,19-epoxytricosa-8,12,14-trien-11-olide;
   (8E,12E,14E)-3,6,16,21-Tetrahydroxy-7-((4-isopropylpiperazin-1-yl)carbonyl)oxy-6,10,12,16,20-pentamethyl-18,19-epoxytricosa-8,12,14-trien-11-olide;
   (8E,12E,14E)-7-((4-Cycloheptylpiperazin-1-yl)carbonyl)oxy-3,6,16,21-Tetrahydroxy-6,10,12,16,20-pentamethyl-18,19-epoxytricosa-8,12,14-trien-11-olide;
   (8E,12E,14E)-7-(N-(2-(N',N'-Diethylamino)ethyl)-N-methylcarbamoyloxy)-3,6,16,21-tetrahydroxy-6,10,12,16,20-pentamethyl-18,19-epoxytricosa-8,12,14-trien-11-olide;
   (8E,12E,14E)-3,6,16,21-Tetrahydroxy-7-((4-isobutylhomopiperazin-1-yl)carbonyl)oxy-6,10,12,16,20-pentamethyl-18,19-epoxytricosa-8,12,14-trien-11-olide;
   (8E,12E,14E)-7-((4-Ethylhomopiperazin-1-yl)carbonyl)oxy-3,6,16,21-tetrahydroxy-6,10,12,16,20-pentamethyl-18,19-epoxytricosa-8,12,14-trien-11-olide;
   (8E,12E,14E)-7-((4-Butylhomopiperazin-1-yl)carbonyl)oxy-3,6,16,21-tetrahydroxy-6,10,12,16,20-pentamethyl-18,19-epoxytricosa-8,12,14-trien-11-olide;
   (8E,12E,14E)-3,16,21-Trihydroxy-6-methoxy-6,10,12,16,20-pentamethyl-7-((4-methylpiperazin-1-yl)carbonyl)oxy-18,19-epoxytricosa-8,12,14-trien-11-olide;
   (8E,12E,14E)-3,16,21-Trihydroxy-6-methoxy-6,10,12,16,20-pentamethyl-7-((4-(piperidin-1-yl)piperidin-1-yl)carbonyl)oxy-18,19-epoxytricosa-8,12,14-trien-11-olide;
   (8E,12E,14E)-3,6,7,21-Tetrahydroxy-6,10,12,16,20-pentamethyl-18,19-epoxytricosa-8,12,14-trien-11-olide;
   (8E,12E,14E)-7-((4-(2,2-Dimethylpropyl)homopiperazin-1-yl)carbonyl)oxy-3,6,16,21-tetrahydroxy-6,10,12,16,20-pentamethyl-1-8,19-epoxytricosa-8,12,14-trien-11-olide; and
   (8E,12E,14E)-3,6,16-Trihydroxy-21-methoxy-6,10,12,16,20-pentamethyl-7-((4-methylpiperazin-1-yl)carbonyl)oxy-18,19-epoxytricosa-8,12,14-trien-11-olide.

(3) The method according to (1), wherein the detection of splicing defect comprises the steps of:
(a) measuring the expression level of pre-mRNA before and after administration of the compound represented by formula (I), the pharmaceutically acceptable salt thereof ,or the solvate of them to a mammal;
(b) comparing, based on the expression level measured in (a), the expression level of pre-mRNA before and after administration of the compound represented by formula (I), the pharmaceutically acceptable salt thereof, or the solvate of them, to determine that the compound represented by formula (I), the pharmaceutically acceptable salt thereof, or the solvate of them exerts an action to the mammal when the expression level of pre-mRNA after the administration increases.

(4) The method according to (3), wherein the pre-mRNA of which expression level is measured is pre-mRNA of at least one gene selected from the genes listed in Table 1, Table 2, Table 3, Table 4, and Table 5, or a homologous gene thereof.

(5) The method according to (4), wherein the gene(s) are selected from DNAJB1, BZW1, NUP54, RIOK3, CDKN1B, STK17B, EIF4A1, and ID1.

(6) The method according to (3), wherein in step (a), the expression level of pre-mRNA in samples obtained from a subject before and after administration of the compound represented by formula (I), the pharmaceutically acceptable salt thereof, or the solvate of them, is measured.

(7) The method according to (6), wherein the samples obtained from the subject are selected from hemocytes in peripheral blood, plasma, and serum.

(8) A probe or primer for assaying an action of a compound represented by formula (I), a pharmaceutically acceptable salt thereof, or a solvate of them to a mammal, which consists of a polynucleotide capable of hybridizing with a polynucleotide consisting of a nucleotide sequence of at least one gene selected from the genes listed in Table 1, Table 2, Table 3, Table 4, and Table 5, or a homologous gene thereof, or a complementary sequence thereof.

(9) The probe or primer according to (8), which is capable of detecting a genomic intron region or a part thereof in a gene listed in Table 1, Table 2, Table 3, Table 4 and Table 5, or which is capable of detecting a polynucleotide lacking a part of a genomic exon region in a gene listed in Table 1, Table 2, Table 3, Table 4 and Table 5.

(10) A reagent or kit for assaying an action of a compound represented by formula (I), a pharmaceutically acceptable salt thereof, or a solvate of them to a mammal, which comprises the probe or the primer according to (8).

(11) The method according to (1), wherein the detection of splicing defect comprises the steps of:
(f) measuring the expression level of an abnormal protein before and after administration of the compound represented by formula (I), the pharmaceutically acceptable salt thereof, or the solvate of them to a mammal;
(g) comparing, based on the expression level measured in (f), the expression level of the abnormal protein before and after administration of the compound represented by formula (I), the pharmaceutically acceptable salt thereof or the solvate of them, to determine that the compound represented by formula (I), the pharmaceutically acceptable salt thereof, or the solvate of them exerts an action to the mammal when the expression level of the abnormal protein after administration increases.

(12) The method according to (11), wherein the abnormal protein of which expression level is measured is a protein consisting of amino acids encoded by a polynucleotide of at least one gene selected from the genes listed in Table 1 and Table 3, or a homologous gene thereof where splicing defect has been caused in the polynucleotide, or a protein consisting of amino acids encoded by a polynucleotide of at least one gene selected from the genes listed in Table 2, Table 4 and Table 5 or a homologous gene thereof where splicing defect has been caused in the polynucleotide.

(13) The method according to (11), wherein the abnormal protein of which expression level is measured is a protein consisting of amino acids encoded by a polynucleotide of at least one gene selected from DNAJB1, BZW1, NUP54, RIOK3, CDKN1B, STK17B, EIF4A1 and ID1 where splicing defect has been caused in the polynucleotide.

(14) The method according to (11), wherein in step (f), the expression level of the abnormal protein in the samples obtained from a subject before and after administration of the compound represented by formula (I) or the pharmaceutically acceptable salt thereof or the solvate of them, is measured.

(15) The method according to (14), wherein the samples obtained from the subject are selected from hemocytes in peripheral blood, plasma, and serum.

(16) An antibody against an abnormal protein consisting of amino acids encoded by a polynucleotide of at least one gene selected from the genes listed in Table 1, Table 2, Table 3, Table 4 and Table 5 where splicing defect has been caused in the polynucleotide, or a fragment thereof.

(17) A reagent or kit for assaying an action of a compound represented by formula (I), a pharmaceutically acceptable salt thereof, or a solvate of them to a mammal, comprising the antibody or the fragment thereof according to (16).
(18) Use of a probe or primer consisting of a polynucleotide capable of hybridizing with a polynucleotide consisting of a nucleotide sequence of at least one gene selected from the genes listed in Table 1, Table 2, Table 3, Table 4, and Table 5, and homologous genes thereof, or complementary sequences thereof, for assaying an action of a compound represented by formula (I), a pharmaceutically acceptable salt thereof, or a solvate of them to a mammal.
(19) Use of an antibody against a protein consisting of amino acids encoded by a polynucleotide of at least one gene selected from the genes listed in Table 1, Table 2, Table 3, Table 4 and Table 5, or a fragment of the antibody, for assaying an action of a compound represented by formula (I), a pharmaceutically acceptable salt thereof, or a solvate of them to a mammal.

According to the present invention, an action of the compound represented by formula (I) to a living body can be confirmed using splicing defect in a cancerous or normal tissue of a cancer patient or normal tissue of a healthy individual, more specifically, the expression level of pre-mRNA or an abnormal protein as an index. For instance, when splicing defect is found in the cancerous or normal tissue of the cancer patient, more specifically, the expression level of pre-mRNA or the abnormal protein increases, it can be determined that the compound represented by formula (I) exerts the action in the body and that thus administration of the drug is no longer required or less amount of the drug should be administrated. Further, when splicing defect is not found in the cancerous or normal tissue of the cancer patient, more specifically, the expression level of pre-mRNA or the abnormal protein exhibits the same amount as before the administration, it can be determined that the compound represented by formula (I) does not exert the action and further administration of the drug is required. Hence, according to the invention, by monitoring periodically the expression of pre-mRNA or the abnormal protein, the antitumor agent can be administered more effectively to the patient and a minimally required amount of the drug can be administered to the patient. In particular, since the action of the compound represented by formula (I) can be judged by monitoring the expression level of pre-mRNA or the abnormal protein in samples obtained from normal tissue such as blood of the patient, it is an advantage that the action of the compound represented by formula (I) to the living body can be readily and reliably assessed.

### BRIEF DESCRIPTION OF THE DRAWINGS

Figure 1 shows accumulation of pre-mRNA of CDKN1B (p27) and CDKN1A (p21) in HeLa cells.
Figure 2 shows accumulation of pre-mRNA of DNAJB1, BZW1, SPAG5, RIOK3, NUP54, and BRD2 in HeLa cells.
Figure 3 shows the expression of mRNA in whole blood (PBC) samples obtained from human peripheral blood. A: Results of samples obtained with the Tempus Blood RNA Tube. B: Results of samples obtained with the PAXgene Blood RNA Tube.
Figure 4 shows expression of a protein translated from the pre-mRNA.
Figure 5 shows results measured expression of precursor genes (DNAJB1 and EIF4A1) in blood of nude mice to which human colon cancer cells were subcutaneously transplanted and the pladienolide derivative was administered.
Figure 6 shows results measured expression of precursor genes (DNAJB1 and EIF4A1) in the tumor of nude mice to which human colon cancer cells were subcutaneously transplanted and the pladienolide derivative was administered.
Figure 7 shows a scatter diagram of the expression level of probe sets and genes of control cells, and standard deviation thereof. "A" represents the expression level and standard deviation of exons. "B" represents the expression level and standard deviation of genes.

### DETAILED DESCRIPTION OF THE INVENTION

All technical terms, scientific terms and terminologies used in the present specification have the same meanings as those that are generally understood by those ordinary skilled in the art in the technical fields to which the present invention belongs, and are used merely for the purpose of explaining a specific embodiment but are not intended to make limitation. The present invention can be carried out in various embodiments as long as not departing from the spirit thereof. All the prior art documents, published publications, patent publications and other patent documents cited in the present specification are incorporated into the present specification as references, and can be used for carrying out the present invention.

The "halogen atom" used in the specification of the present application means a fluorine atom, a chlorine atom, a bromine atom and an iodine atom. Among them, for example, a fluorine atom, a chlorine atom or a bromine atom is preferred, of which a fluorine atom or a chlorine atom is preferred.

The "C₁₋₂₂ alkyl group" used in the specification of the present application indicates a linear or branched alkyl group having 1 to 22 carbon atoms, such as methyl group, ethyl group, n-propyl group, iso-propyl group, n-butyl group, iso-butyl group, sec-butyl group, tert-butyl group, n-pentyl group, 1,1-dimethylpropyl group, 1,2-dimethylpropyl group, 2,2-dimethylpropyl group, 1-ethylpropyl group, n-hexyl group, 1-ethyl-2-methylpropyl group, 1,1,2-trimethylpropyl group, 1-propylpropyl group, 1-methylbutyl group, 2-methylbutyl group, 1,1-dimethylbutyl group, 1,2-dimethylbutyl group, 2,2-dimethylbutyl group, 1,3-dimethylbutyl group, 2,3-dimethylbutyl group, 2-ethylbutyl group, 2-methylpentyl group, 3-methylpentyl group, n-heptyl group, n-octyl group, n-nonyl group or n-decyl group; preferably a linear or branched alkyl group having 1 to 6 carbon atoms, such as methyl group, ethyl group, n-propyl group, iso-propyl group, n-butyl group, iso-butyl group, sec-butyl group, tert-butyl group or n-pentyl group; and more preferably, for example, methyl group, ethyl group, propyl group, iso-propyl group, n-butyl group, iso-butyl group or tert-butyl group.

The "unsaturated C₂₋₂₂ alkyl group" used in the specification of the present application indicates a linear or branched alkenyl group having 2 to 22 carbon atoms or a linear or branched alkynyl group having 2 to 22 carbon atoms, such as vinyl group, allyl group, 1-propenyl group, isopropenyl group, 2-methyl-1-propenyl group, 2-methyl-2-propenyl group, 1-butenyl group, 2-butenyl group, 3-butenyl group, 1-pentenyl group, 1-hexenyl group, 1,3-hexadienyl group, 1,5-hexadienyl group, ethynyl group, 1-propynyl group, 2-propynyl group, 1-butynyl group, 2-butynyl group, 3-butynyl group, 1-ethynyl-2-propynyl group, 2-methyl-3-butynyl group, 1-pentynyl group, 1-hexynyl group, 1,3-hexanediynyl group or 1,5-hexanediynyl group. It preferably indicates a linear or branched alkenyl group having 2 to 10 carbon atoms
or a linear or branched alkynyl group having 2 to 10 carbon atoms, such as vinyl group, allyl group, 1-propenyl group, 2-propenyl group, isopropenyl group, 3-methyl-2-butenyl group, 3,7-dimethyl-2,6-octadienyl group, ethynyl group, 1-propynyl group, 2-propynyl group, 1-butynyl group, 2-butynyl group, 3-butynyl group or 3-methyl-1-propynyl group.

The "C₆₋₁₄ aryl group" used in the specification of the present application means an aromatic cyclic hydrocarbon group having 6 to 14 carbon atoms, and a monocyclic group and condensed rings such as a bicyclic group and a tricyclic group are included. Examples thereof include phenyl group, indenyl group, 1-naphthyl group, 2-naphthyl group, azulenyl group, heptalenyl group, indacenyl group, acenaphthyl group, fluorenyl group, phenalenyl group, phenanthrenyl group and anthracenyl group; and preferred examples include phenyl group, 1-naphthyl group and 2-naphthyl group.

The "5- to 14-membered heteroaryl group" used in the specification of the present application means a monocyclic, bicyclic or tricyclic 5- to 14-membered aromatic heterocyclic group which contains one or more of hetero atoms selected from the group consisting of nitrogen atom, sulfur atom and oxygen atom. Preferred examples thereof include nitrogen-containing aromatic heterocyclic groups such as pyrrolyl group, pyridyl group, pyridazinyl group, pyrimidinyl group, pyrazinyl group, triazolyl group, tetrazolyl group, benzotriazolyl group, pyrazolyl group, imidazolyl group, benzimidazolyl group, indolyl group, isoindolyl group, indolizinyl group, purinyl group, indazolyl group, quinolyl group, isoquinolyl group, quinolizinyl group, phthalazinyl group, naphthyridinyl group, quinoxalinyl group, quinazolinyl group, cinnolinyl group, pteridinyl group, imidazotriazinyl group, pyrazinopyridazinyl group, acridinyl group, phenanthridinyl group, carbazolyl group, carbazolinyl group, perimidinyl group, phenanthrolinyl group, phenazinyl group, imidazopyridinyl group, imidazopyrimidinyl group, pyrazolopyridinyl group and pyrazolopyridinyl group; sulfur-containing aromatic heterocyclic groups such as thienyl group and benzothienyl group; and oxygen-containing aromatic heterocyclic groups such as furyl group, pyranyl group, cyclopentapyranyl group, benzofuryl group and isobenzofuryl group; aromatic heterocyclic groups containing two or more different hetero atoms, such as thiazolyl group, isothiazolyl group, benzothiazolyl group, benzthiadiazolyl group, phenothiazinyl group, isoxazolyl group, furazanyl group, phenoxazinyl group, oxazolyl group, isoxazoyl group, benzoxazolyl group, oxadiazolyl group, pyrazolooxazolyl group, imidazothiazolyl group, thienofuranyl group, furopyrrolyl group and pyridoxazinyl group; and preferred examples include thienyl group, furyl group, pyridyl group, pyridazyl group, pyrimidyl group and pyrazyl group.

The "3- to 14-membered non-aromatic heterocyclic group" used in the specification of the present application indicates a monocyclic, bicyclic or tricyclic 3- to 14-membered non-aromatic heterocyclic group which may contain one or more hetero atoms selected from the group consisting of nitrogen atom, sulfur atom and oxygen atom. Preferred examples thereof include aziridinyl group, azetidinyl group, pyrrolidinyl group, pyrrolyl group, piperidinyl group, piperazinyl group, homopiperidinyl group, homopiperazinyl group, imidazolyl group, pyrazolidyl group, imidazolidyl group, morpholyl group, thiomorpholyl group, imidazolinyl group, oxazolinyl group, 2,5-diazabicyclo[2.2.1]heptyl group, 2,5-diazabicyclo[2.2.2]octyl group, 3,8-diazabicyclo[3.2.1]octyl group, 1,4-diazabicyclo[4.3.0]nonyl group, quinuclidyl group, tetrahydrofuran-yl group and tetrahydrothiophen-yl group. The non-aromatic heterocyclic groups also include groups derived from pyridone ring, and non-aromatic fused rings (e.g., a group derived from, for example, phthalimide ring or succinimide ring).

The "C₇₋₂₂ aralkyl group" used in the specification of the present application means a group corresponding to the above-defined "C₁₋₂₂ alkyl group" of which substitutable moiety is replaced by the above-defined "C₆₋₁₄ aryl group". Specific examples thereof include benzyl group, phenethyl group, 3-phenylpropyl group, 4-phenylbutyl group, 1-naphthylmethyl group and 2-naphthylmethyl group; and preferred examples include aralkyl groups having 7 to 10 carbon atoms such as benzyl group and phenethyl group.

The "5- to 14-membered heteroaralkyl group" used in the specification of the present application means a group corresponding to the above-defined "C₁₋₂₂ alkyl group" of which substitutable moiety is replaced by the above-defined "5- to 14-membered heteroaryl group". Specific examples thereof include thienylmethyl group, furylmethyl group, pyridylmethyl group, pyridazylmethyl group, pyrimidylmethyl group and pyrazylmethyl group; and preferred examples include thienylmethyl group, furylmethyl group and pyridylmethyl group.

The "C₃₋₁₄ cycloalkyl group" used in the specification of the present application indicates a cycloalkyl group having 3 to 14 carbon atoms, and suitable examples thereof include cyclopropyl group, cyclobutyl group, cyclopentyl group, cyclohexyl group, cycloheptyl group and cyclooctyl group; and preferred examples include cyclopentyl group, cyclohexyl group, cycloheptyl group and cyclooctyl group.

The "C₄₋₉ cycloalkyl alkyl group" used in the specification of the present application means a group corresponding to the above-defined "C₁₋₂₂ alkyl group" of which substitutable moiety is replaced by the above-defined "C₃₋₁₄ cycloalkyl group". Specific examples thereof include cyclopropylmethyl group, cyclobutylmethyl group, cyclopentylmethyl group, cyclohexylmethyl group, cycloheptylmethyl group and cyclooctylmethyl group; and preferred example include cyclopropylmethyl group, cyclobutylmethyl group and cyclopentylmethyl group.

The "C₁₋₂₂ alkoxy group" used in the specification of the present application means a group corresponding to the above-defined "C₁₋₂₂ alkyl group" to which end an oxygen atom is bonded. Suitable examples thereof include methoxy group, ethoxy group, n-propoxy group, iso-propoxy group, n-butoxy group, iso-butoxy group, sec-butoxy group, tert-butoxy group, n-pentyloxy group, iso-pentyloxy group, sec-pentyloxy group, n-hexoxy group, iso-hexoxy group, 1,1-dimethylpropyloxy group, 1,2-dimethylpropyloxy group, 2,2-dimethylpropoxy group, 1-methyl-2-ethylpropoxy group, 1-ethyl-2-methylpropoxy group, 1,1,2-trimethylpropoxy group, 1,2,2-trimethylpropoxy group, 1,1-dimethylbutoxy group, 1,2-dimethylbutoxy group, 2,2-dimethylbutoxy group, 2,3-dimethylbutyloxy group, 1,3-dimethylbutoxy group, 2-ethylbutoxy group, 2-methylpentoxy group, 3-methylpentyloxy group and hexyloxy group; and preferred examples include methoxy group, ethoxy group, n-propoxy group, iso-propoxy group, iso-butoxy group and 2,2-dimethylpropyloxy group.

The "unsaturated C₂₋₂₂ alkoxy group" used in the specification of the present application means a group corresponding to the above-defined "unsaturated C₂₋₂₂ alkyl group" to which end an oxygen atom is bonded. Suitable examples thereof include vinyloxy group, allyloxy group, 1-propenyloxy group, 2-propenyloxy group, isopropenyloxy group, 2-methyl-1-propenyloxy group, 2-methyl-2-propenyloxy group, 1-butenyloxy group, 2-butenyloxy group, 3-butenyloxy group, 1-pentenyloxy group, 1-hexenyloxy group, 1,3-hexadienyloxy group, 1,5-hexadienyloxy group, propargyloxy group and 2-butynyloxy group; and preferred examples include allyloxy group, propargyloxy group and 2-butynyloxy group.

The "C₆₋₁₄ aryloxy group" used in the specification of the present application means a group corresponding to the above-defined "C₆₋₁₄ aryl group" to which end an oxygen atom is bonded. Specific examples thereof include phenyloxy group, indenyloxy group, 1-naphthyloxy group, 2-naphthyloxy group, azulenyloxy group, heptalenyloxy group, indacenyloxy group, acenaphthyloxy group, fluorenyloxy group, phenalenyloxy group, phenanthrenyloxy group and anthracenyloxy group; and preferred examples include phenyloxy group, 1-naphthyloxy group and 2-naphthyloxy group.

The "C₇₋₂₂ aralkyloxy group" used in the specification of the present application means a group corresponding to the above-defined "C₇₋₂₂ aralkyl group" to which end an oxygen atom is bonded. Specific examples thereof include benzyloxy group, phenethyloxy group, 3-phenylpropyloxy group, 4-phenylbutyloxy group, 1-naphthylmethyloxy group and 2-naphthylmethyloxy group; and preferred examples include benzyloxy group.

The "5- to 14-membered heteroaralkyloxy group" used in the specification of the present application means a group corresponding to the above-defined "5- to 14-membered heteroaralkyl group" to which end an oxygen atom is bonded. Specific examples thereof include thienylmethyloxy group, furylmethyloxy group, pyridylmethyloxy group, pyridazylmethyloxy group, pyrimidylmethyloxy group and pyrazylmethyloxy group; and preferred examples include thienylmethyloxy group, furylmethyloxy group and pyridinylmethyloxy group.

The "5- to 14-membered heteroaryloxy group" used in the specification of the present application means a group corresponding to the above-defined "5- to 14-membered heteroaryl group" to which end an oxygen atom is bonded. Specific examples thereof include pyrrolyloxy group, pyridyloxy group, pyridazinyloxy group, pyrimidinyloxy group, pyrazinyloxy group, triazolyloxy group, tetrazolyloxy group, benzotriazolyloxy group, pyrazolyloxy group, imidazolyloxy group, benzimidazolyloxy group, indolyloxy group, isoindolyloxy group, indolizinyloxy group, purinyloxy group, indazolyloxy group, quinolyloxy group, isoquinolyloxy group, quinolizyloxy group, phthalazyloxy group, naphthyridinyloxy
group, quinoxalyloxy group, quinazolinyloxy group, cinnolinyloxy group, pteridinyloxy group, imidazotriazinyloxy group, pyrazinopyridazinyloxy group, acridinyloxy group, phenanthridinyloxy group, carbazolyloxy group, carbazolinyloxy group, perimidinyloxy group, phenanthrolinyloxy group, phenazinyloxy group, imidazopyridinyloxy group, imidazopyrimidinyloxy group, pyrazolopyridinyloxy group, pyrazolopyridinyloxy group, thienyloxy group, benzothienyloxy group, furyloxy group, pyranyloxy group, cyclopentapyranyloxy group, benzofuryloxy group, isobenzofuryloxy group, thiazolyloxy group, isothiazolyloxy group, benzothiazolyloxy group, benzothiadiazolyloxy group, phenothiazinyloxy group, isoxazoyloxy group, furazanyloxy group, phenoxazinyloxy group, oxazolyloxy group, isoxazolyloxy group, benzoxazolyloxy group, oxadiazolyloxy group, pyrazolooxazolyloxy group, imidazothiazolyloxy group, thienofuranyloxy group, furopyrrolyloxy group and pyridoxazinyloxy group; and preferred examples include thienyloxy group, pyridyloxy group, pyrimidyloxy group and pyrazyloxy group.

The "aliphatic C₂₋₂₂ acyl group" used in the specification of the present application means a group corresponding to the above-defined "C₁₋₂₂ alkyl group" or "unsaturated C₂₋₂₂ alkyl group" to which end a carbonyl group is bonded. Examples thereof include acetyl group, propionyl group, butyryl group, iso-butyryl group, valeryl group, iso-valeryl group, pivaloyl group, caproyl group, decanoyl group, lauroyl group, myristoyl group, palmitoyl group, stearoyl group, arachidoyl group, acryloyl group, propiol group, crotonyl group, iso-crotonyl group, olenoyl group and linolenoyl group; and preferred examples include aliphatic acyl groups having 2 to 6 carbon atoms, such as acetyl group, propionyl group, butyryl group, iso-butyryl group and acryloyl group.

The "aromatic C₇₋₁₅ acyl group" used in the specification of the present application means a group corresponding to the above-defined "C₆₋₁₄ aryl group" or "5- to 14-membered heteroaryl group" to which end a carbonyl group is bonded. Examples thereof include benzoyl group, 1-naphthoyl group, 2-naphthoyl group, picolinoyl group, nicotinoyl group, isonicotinoyl group, furoyl group and thiophenecarbonyl group; and preferred examples include benzoyl group, picolinoyl group, nicotinoyl group and isonicotinoyl group.

The "C₁₋₂₂ alkylsulfonyl group" used in the specification of the present application means a group corresponding to the above-defined "C₁₋₂₂ alkyl group" to which a sulfonyl group is bound. Specific examples thereof include methylsulfonyl group, ethylsulfonyl group, n-propylsulfonyl group and iso-propylsulfonyl group; and preferred examples include methylsulfonyl group.

The "C₆₋₁₄ arylsulfonyl group" used in the specification of the present application means a group corresponding to the above-defined "C₆₋₁₄ aryl group" to which a sulfonyl group is bound. Specific examples thereof include benzenesulfonyl group, 1-naphthalenesulfonyl group and 2-naphthalenesulfonyl group; and preferred examples include benzenesulfonyl group.

The "aliphatic C₂₋₂₂ acyloxy group" used in the specification of the present application means a group corresponding to the above-defined "aliphatic C₂₋₂₂ acyl group" to which end an oxygen atom is bonded. Specific examples thereof include acetoxy group, propionyloxy group and acryloxy group; and preferred examples include acetoxy group and propionyloxy group.

The "C₂₋₂₂ alkoxycarbonyl group" used in the specification of the present application means a group corresponding to the above-defined "C₁₋₂₂ alkoxy group" to which end a carbonyl group is bonded. Examples thereof include methoxycarbonyl group, ethoxycarbonyl group, n-propoxycarbonyl group, iso-propoxycarbonyl group, n-butoxycarbonyl group, iso-butoxycarbonyl group, sec-butoxycarbonyl group and tert-butoxycarbonyl group; and preferred examples include ethoxycarbonyl group, iso-propoxycarbonyl group and tert-butoxycarbonyl group.

The "unsaturated C₃₋₂₂ alkoxycarbonyl group" used in the specification of the present application means a group corresponding to the above-defined "unsaturated C₂₋₂₂ alkoxy group" to which end a carbonyl group is bonded. Examples thereof include vinyloxycarbonyl group, allyloxycarbonyl group, 1-propenyloxycarbonyl group, isopropenyloxycarbonyl group, propargyloxycarbonyl group and 2-butynyloxycarbonyl group; and preferred examples include allyloxycarbonyl group.

The "C₁₋₂₂ alkylthio group" used in the specification of the present application means a group corresponding to the above-defined "C₁₋₂₂ alkyl group" to which end a sulfur atom is bonded. Examples thereof include methylthio group, ethylthio group, n-propylthio group and iso-propylthio group; and preferred examples include methylthio group or ethylthio group.

The "C₁₋₂₂ alkylsulfinyl group" used in the specification of the present application means a group corresponding to the above-defined "C₁₋₂₂ alkyl group" to which end a sulfinyl group is bonded. Examples thereof include methylsulfinyl group, ethylsulfinyl group, n-propylsulfinyl group and iso-propylsulfinyl group; and preferred examples include methanesulfinyl group or ethanesulfinyl group.

The "C₁₋₂₂ alkylsulfonyloxy group" used in the specification of the present application means a group corresponding to the above-defined "C₁₋₂₂ alkylsulfonyl group" to which end an oxygen atom is bonded. Examples thereof include methylsulfonyloxy group, ethylsulfonyloxy group, n-propylsulfonyloxy group and iso-propylsulfonyloxy group; and preferred examples include methylsulfonyloxy group.

The substituent of the phrase "an optionally substituted" used in the specification of the present application may be one or more groups selected from:
(1) a halogen atom,
(2) a hydroxyl group,
(3) a thiol group,
(4) a nitro group,
(5) a nitroso group,
(6) a cyano group,
(7) a carboxyl group,
(8) a hydroxysulfonyl group,
(9) a amino group,
(10) a C₁₋₂₂ alkyl group (for example, methyl group, ethyl group, n-propyl group, iso-propyl group, n-butyl group, iso-butyl group, sec-butyl group and tert-butyl group),
(11) an unsaturated C₂₋₂₂ alkyl group (for example, vinyl group, allyl group, 1-propenyl group, 2-propenyl group, isopropenyl group, ethynyl group, 1-propynyl group, 2-propynyl group, 1-butynyl group, 2-butynyl group and 3-butynyl group),
(12) a C₆₋₁₄ aryl group (for example, phenyl group, 1-naphthyl group and 2-naphthyl group),
(13) a 5- to 14-membered heteroaryl group (for example, thienyl group, furyl group, pyridyl group, pyridazyl group, pyrimidyl group and pyrazyl group),
(14) a 3- to 14-membered non-aromatic heterocyclic group (for example, aziridinyl group, azetidyl group, pyrrolidinyl group, pyrrolyl group, piperidinyl group, piperazinyl group, homopiperidinyl group, homopiperazinyl group, imidazolyl group, pyrazolidyl group, imidazolidyl group, morpholyl group, thiomorpholyl group, imidazolinyl group, oxazolinyl group and quinuclidyl group),
(15) a C₃₋₁₄ cycloalkyl group (for example, cyclopropyl group, cyclobutyl group, cyclopentyl group, cyclohexyl group, cycloheptyl group and cyclooctyl group),
(16) a C₁₋₂₂ alkoxy group (for example, methoxy group, ethoxy group, n-propoxy group, iso-propoxy group, sec-propoxy group, n-butoxy group, iso-butoxy group and tert-butoxy group),
(17) an unsaturated C₂₋₂₂ alkoxy group (for example, vinyloxy group, allyloxy group, 1-propenyloxy group, 2-propenyloxy group, isopropenyloxy group, ethynyloxy group, 1-propynyloxy group, 2-propynyloxy group, 1-butynyloxy group and 2-butynyloxy group),
(18) a C₆₋₁₄ aryloxy group (for example, phenyloxy group, 1-naphthyloxy group and 2-naphthyloxy group),
(19) a C₇₋₂₂ aralkyloxy group (for example, benzyloxy group, phenethyloxy group, 3-phenylpropyloxy group, 4-phenylbutyloxy group, 1-naphthylmethyloxy group and 2-naphthylmethyloxy group),
(20) a 5- to 14-membered heteroaralkyloxy group (for example, thienylmethyloxy group, furylmethyloxy group, pyridylmethyloxy group, pyridazylmethyloxy group, pyrimidylmethyloxy group and pyrazylmethyloxy group),
(21) a 5- to 14-membered heteroaryloxy group (for example, thienyloxy group, furyloxy group, pyridyloxy group, pyridazyloxy group, pyrimidyloxy group and pyrazyloxy group),
(22) an aliphatic C₂₋₂₂ acyl group (for example, acetyl group, propionyl group, butyryl group, iso-butyryl group, valeryl group, iso-valeryl group, pivalyl group, caproyl group, decanoyl group, lauroyl group, myristoyl group, palmitoyl group, stearoyl group, arachidoyl group, acryl group, propiol group, crotonyl group, iso-crotonyl group, olenoyl group and linolenoyl group),
(23) an aromatic C₇₋₁₅ acyl group (for example, benzoyl group, 1-naphthoyl group and 2-naphthoyl group),
(24) an aliphatic C₂₋₂₂ acyloxy group (for example, acetoxy group, propionyloxy group and acryloxy group),
(25) a C₂₋₂₂ alkoxycarbonyl group (for example, methoxycarbonyl group, ethoxycarbonyl group, n-propoxycarbonyl group, iso-propoxycarbonyl group, n-butoxycarbonyl group, iso-butoxycarbonyl group, sec-butoxycarbonyl group and tert-butoxycarbonyl group),
(26) an unsaturated C₃₋₂₂ alkoxycarbonyl group (for example, vinyloxycarbonyl group, allyloxycarbonyl group, 1-propenyloxycarbonyl group, 2-propenyloxycarbonyl group, isopropenyloxycarbonyl group, propargyloxycarbonyl group and 2-butynyloxycarbonyl group),
(27) a C₁₋₂₂ alkylthio group (for example, methylthio group, ethylthio group, n-propylthio group and iso-propylthio group),
(28) a C₁₋₂₂ alkylsulfinyl group (for example, methylsulfinyl group, ethylsulfinyl group, n-propylsulfinyl group and iso-propylsulfinyl group),
(29) a C₁₋₂₂ alkylsulfonyl group (for example, methylsulfonyl group, ethaylsulfonyl group, n-propanesulfonyl group and iso-propanesulfonyl group),
(30) a C₆₋₁₄ arylsulfonyl group (for example, benzenesulfonyl group, 1-naphthalenesulfonyl group and 2-naphthalenesulfonyl group),
(31) a C₁₋₂₂ alkylsulfonyloxy group (for example, methylsulfonyloxy group, ethylsulfonyloxy group, n-propylsulfonyloxy group and iso-propanesulfonyloxy group),
(32) carbamoyl group, and
(33) formyl group.
Among them, a preferred example is an amino group, a C₁₋₂₂ alkyl group, an unsaturated C₂₋₂₂ alkyl group, a C₆₋₁₄ aryl group, a 5- to 14-membered heteroaryl group, a 3- to 14-membered non-aromatic heterocyclic group and a C₃₋₁₄ cycloalkyl group, and a more preferred example is an amino group, a C₁₋₂₂ alkyl group, a 3- to 14-membered nonaromatic heterocyclic group and a C₃₋₁₄ cycloalkyl group. The above-mentioned (9) amino group and (31) carbamoyl group as the substituent in "an optionally substituted" may each be further substituted with one or two of a C₁₋₂₂ alkyl group, an unsaturated C₂₋₂₂ alkyl group or a C₆₋₁₄ aryl group.

In the present specification, the chemical formula of the compound according to the present invention is illustrated as a planimetric chemical formula for convenience but the compound can include certain isomers drawn from the chemical formula. The present invention can include all isomers and mixtures of the isomers such as a geometric isomer which is generated from the configuration of the compound, an optical isomer based on an asymmetric carbon, a rotamer, a stereoisomer and a tautomer. The present invention is not limited to the expediential description of the chemical formula, and can include either of isomers or a mixture thereof. Accordingly, when the compound according to the present invention has an asymmetric carbon in the molecule, and its optically active substance and racemate exist, any one is included. Further, when polymorphic crystals exist, the crystal form according to the present invention is not specifically limited to one form, and any one of the crystal forms may be single or a mixture of the crystal forms.

The "pharmaceutically acceptable salt" used in the present invention is not particularly restricted as long as it can form a salt with the compound represented by formula (I), and is pharmaceutically acceptable. Preferred examples thereof include halide hydroacid salt such as hydrochloric acid salt, hydrobromic acid salt, hydroiodic acid salt; inorganic acid salt such as sulphic acid salt, nitric acid salt, perchloric acid salt, phosphoric acid salt, carbonic acid salt, bicarbonic acid salt; organic carboxylic acid salt such as acetic acid salt, trifluoroacetic acid salt, maleic acid salt, tartaric acid salt, fumaric acid salt, citric acid salt; organic sulfonic acid salt such as methanesulfonic acid salt, trifluoro methanesulfonic acid salt, ethanesulfonic acid salt, benzenesulfonic acid salt, toluenesulfonic acid salt, camphorsulfonic acid salt; amino acid salt such as aspartic acid salt, glutamic acid salt; quaternary amine salt; alkaline metal salt such as sodium salt, potassium salt; and alkaline earth metal salt such as magnesium salt, calcium salt.

The "solvate" used in the present invention is not particularly restricted as long as it can form a solvate with the compound represented by formula (I) or the salt thereof, and is pharmaceutically acceptable. Preferred examples include hydrate, alcoholate such as ethanolate, and etherate.

The present invention also includes a metabolite generated by degradation of the compound represented by formula (I) within a living body, as well as a prodrug of the compound represented by formula (I) and the salt thereof. The "prodrug" used herein means an inert substance to which "an active moiety of a drug" (meaning "drug" corresponding to a prodrug) has been chemically modified, for the purpose of improvement of bioavailability and reduction of a side effect. After absorbed, it is metabolized into the active moiety *in vivo* and exerts an action. Accordingly, the term "prodrug" refers to any compound having a lower intrinsic activity than the corresponding "drug", which is, once administrated to a biological system, converted into the "drug" substance via a spontaneous chemical reaction, enzyme catalyzed reaction or metabolic reaction. Examples of such prodrugs include various prodrugs, for example, compounds produced by acylation, alkylation, phosphorylation, boration, carbonation, esterification, amidation, or urethanation of a functional group such as an amino, hydroxyl, or carboxyl group in the compound represented by formula (I). However, it should be noted that the exemplified prodrugs are not comprehensive but are merely typical, and other conventional various prodrugs can be prepared by a conventional method by a person having ordinary skill in the art from the compound represented by formula (I).

When the compound represented by formula (I) is administrated to a mammal in the method according to the present invention, the compound represented by formula (I) may be formulated by known methods. Conventional carriers are used for formulation, and the pharmaceutical products are prepared by conventional methods. Namely, when a solid formulation for oral use is prepared, a filler is added to the main drug, and if necessary, a binder, a disintegrant, a lubricant, a colorant, a flavoring agent and the like are added thereto, and then tablets, coated tablets, granules, powders, capsules and the like are prepared by conventional methods. It is needless to say that sugar coating, gelatin coating or suitable coating may be conducted on the tablet and granule, if necessary. When the compounds are formulated as an injection, a pH regulator, a buffer, a stabilizer, a solubilizer and the like are added to the main drug, if necessary, to prepare an subcutaneous, intramuscular, intra-articular or intravenous injection according to conventional procedures. When the compound represented by formula (I) is administered as a therapeutic or preventive agent for various diseases, it may be orally administered as tablets, powders, granules, capsules, syrups and the like, and may be parenterally administered as a spray, a suppository, an injection, a topical preparation or an infusion. Although the dose remarkably varies according to the severity of symptom, age, the kind of disease etc., approximately 1 mg to 100 mg per day for an adult is administered in general at one time or several times per day.

When the expression level of pre-mRNA of the genes listed in Table 1, Table 2, Table 3, Table 4, and Table 5 or homologous genes thereof, preferably the genes listed in Table 1 and Table2 or homologous genes thereof as well as an abnormal protein generated by splicing defect of the gene is monitored in the method according to the present invention, the expression level may be preferably monitored before administration of the compound represented by formula (I), followed by another monitoring at 3, 6, 8, 24, or 48 hours after the administration. In a preferred embodiment, follow-up monitoring of the expression level is carried out at the earliest three hours after the administration of the compound represented by formula (I).

Upon implementing the method according to the present invention the splicing defect can be detected using an increase in the expression level of pre-mRNA or the abnormal protein caused by the splicing defect as an index.

According to a first aspect of the invention, a method for assaying the action of the compound represented by formula (I), using the increase in the expression level of pre-mRNA as an index (invention (3)) is provided.

In step (a), cancer tissue or normal tissue such as hemocytes in peripheral blood, platelets, and serum can be taken from the mammal subjected to the assay and pre-mRNA samples can be prepared from the obtained samples to quantify the expression level of pre-mRNA. Preparation of pre-mRNA is well known (for example, "Molecular Cloning, A Laboratory Manual 3d ed. "(Cold Spring Harbor Press (2001)), and required devices, instruments, and reagents therefor are commercially available. Hence those skilled in the art may prepare pre-mRNA with no difficulties using the devices, apparatuses, and reagents as needed.

Measurement of the expression level of pre-mRNA in step (a) can be performed with any method selected from a Northern blot method, a dot blot method, an RT-PCR method, and a microarray (preferably, Human Exon 1.0 ST Array). The principles of these methods and how to carry out these methods are well-known, and the required devices and apparatuses therefor are commercially available. Moreover, in Examples below, an example in which the expression level of pre-mRNA is measured with these methods will be described. Those skilled in the art can measure the expression level of pre-mRNA with no difficulties using the Northern blot method, the dot blot method, the RT-PCR method, and the microarray. In the measurement of the expression level of pre-mRNA in step (a), preferably, the microarray can be used.

For the measurement of the expression level of pre-mRNA in step (a), a probe and a primer which consist of a polynucleotide capable of hybridizing with nucleotide sequences of genes listed in Table 1, Table 2, Table 3, Table 4, and Table 5 and homologous genes thereof, preferably genes listed in Table 1 and Table2 or homologous genes thereof, or their complementary sequences can be employed as a detection marker.

Any probe and primer according to the present invention may be employed as long as it can detect the expression of pre-mRNA (including a part thereof) of the genes listed in Table 1, Table 2, Table 3, Table 4, and Table 5 and homologous genes thereof, preferably the genes listed in Table 1 and Table2 or homologous genes thereof. The probe and the primer according to the present invention refers to a polymer consisting of bases or base pairs such as deoxyribonucleic acid (DNA) or ribonucleic acid (RNA). It has been known that double stranded cDNA can be used in tissue *in situ* hybridization, and the probe and the primer according to the present invention include such double stranded cDNA. Particularly preferred RNA probe and primer for detecting RNA in tissue include RNA probes (riboprobe).

The probe and the primer according to the present invention includes a probe or a primer which consists of a polynucleotide comprising a nucleotide sequence of at least 10, preferably at least 15, more preferably at least 20, further preferably at least 25 continuous nucleotides of the genes listed in Table 1, Table 2, Table 3, Table 4 and Table 5, and homologous genes thereof, preferably genes listed in Table 1 and Table2 or homologous genes thereof, or complementary sequences thereof as well as all mutated polynucleotide sequences thereof. The probe and the primer according to the present invention include a probe or a primer which consists of a polynucleotide comprising a nucleotide sequence of 10 to 50 or 10 to 30 continuous nucleotides, 15 to 50 or 15 to 30 continuous nucleotides, 20 to 50 or 20 to 30 continuous nucleotides, 25 to 50 or 25 to 30 continuous nucleotides, of the genes listed in Table 1, Table 2, Table 3, Table 4 and Table 5, and homologous genes thereof, preferably the genes listed in Table 1 and Table2 or homologous genes thereof or mutated polynucleotide sequences thereof.

The probe and the primer according to the present invention may be at least 10 bases in length, preferably at least 15 bases in length, more preferably at least 20 bases in length, further preferably at least 25 bases in length. The probe and the primer according to the present invention may also be 10 to 50 bases or 10 to 30 bases in length, 15 to 50 bases or 15 to 30 bases in length, 20 to 50 bases or 20 to 30 bases in length, 25 to 50 bases or 25 to 30 bases in length.

According to the preferred embodiment of the probe and the primer according to the present invention, there are provided the probe and the primer having 15 to 30 bases in length for assaying the action of the compound represented by formula (I) to the mammals, which consist of a polynucleotide comprising at least 10, preferably at least 15, more preferably at least 20, further preferably at least 25 continuous nucleotides of a polynucleotide sequence of the genes listed in Table 1, Table 2, Table 3, Table 4, and Table 5 or homologous genes thereof, preferably the genes listed in Table1 and Table2 or homologous genes thereof, as well as all mutated sequences thereof, and which are capable of hybridizing with a polynucleotide sequence of the genes listed in Table 1, Table 2, Table 3, Table 4, and Table 5 or homologous genes thereof, preferably the genes listed in Table 1 and Table2 or homologous genes thereof.

According to a preferred aspect of the probe and the primer according to the present invention, there are provided a probe and a primer which are capable of hybridizing with a more distinct region within a nucleotide sequence of the genes listed in Table 1, Table 2, Table 3, Table 4, and Table 5 or homologous genes thereof, preferably the genes listed in Table 1 and Table2 or homologous genes thereof. The above probe and primer allow more precise detection of the action of the compound represented by formula (I) to the mammal.

The probe according to the present invention can be chemically synthesized based on the nucleotide sequence of the gene subjected to be detected. Preparation of the probes is well known and can be carried out in accordance with, for example, "Molecular Cloning, A Laboratory Manual 2nd ed." (Cold Spring Harbor Press (1989)), "Current Protocols in Molecular Biology" (John Wiley & Sons (1987-1997)).

The primers according to the present invention can be used as a primer set comprising of two or more types of the primers.

The primer and the primer set according to the present invention can be used as a primer and a primer set in accordance with a conventional method in a known method for detecting the target gene using a nucleic acid amplification method such as a PCR method, a RT-PCR method, a real-time PCR method, and an *in situ* PCR method.

The primer set according to the present invention can be selected such that the nucleotide sequence of the target gene can be amplified with the nucleic acid amplification method such as the PCR method. The nucleic acid amplification method is well known and selection of the primer pair therein is obvious for those skilled in the art. For example, in the PCR method, the primers can be selected such that one of two primers (a primer pair) undergoes base pairing with the plus strand of the double stranded DNA of the gene subjected to be detected whereas the other of the primers undergoes base pairing with the minus strand of the double stranded DNA, as well as an extending strand extended with one primer can be paired with the other primer. In a LAMP method (WO00/28082), three regions from the 3' terminus termed F3c, F2c and F1c, and three regions from the 5' terminus termed B1, B2 and B3 are respectively defined for the gene subjected to be detected. Four types of primers can be designed using these six regions.

The primer according to the present invention may be chemically synthesized based on the nucleotide sequence of the gene subjected to be detected. Preparation of the primer is well known and can be carried out in accordance with, for example, "Molecular Cloning, A Laboratory Manual 2nd ed." (Cold Spring Harbor Press (1989)), "Current Protocols in Molecular Biology" (John Wiley & Sons (1987-1997)).

Table 1, Table 2, Table 3, Table 4, and Table 5 describe information specifying the genes and homologous genes thereof listed in these tables. Accordingly those skilled in the art can obtain information on the nucleotide sequence of the subject gene to be detected based on the information described in Table 1, Table 2, Table 3, Table 4, and Table 5 to design the probe and primer based thereon.

In addition, the genes listed in Table 1, Table 2, Table 3, Table 4, and Table 5 are known genes and probes and primers for detecting them are commercially available individually or as a detection kit or detection array.

The term "to hybridize" used in the specification of the present application means to hybridize with a target polynucleotide under stringent conditions. A specific example of the polynucleotide which hybridizes under stringent conditions includes a polynucleotide having at least 70% or more, preferably 80% or more, more preferably 85% or more, further preferably 90% or more, further more preferably 95% or more, particularly preferably 98% or more, and most preferably 99% or more homology to the target polynucleotide when the homology is calculated by a homology search software, such as FASTA, BLAST, Smith-Waterman (Meth. Enzym., 164, 765, (1988)), using default parameters. Further, hybridization "under stringent conditions" can be performed, for example, by a method of carrying out the reaction at a temperature of 40°C to 70°C, preferably at a temperature of 60°C to 65°C, in a hybridization buffer solution generally used by those skilled in the art, and carrying out washing in a washing solution at a salt concentration of 15 to 300 mmol/L, preferably at 15 to 60 mmol/L. The temperature and salt concentration can be appropriately adjusted depending on the length of the probe to be used. Further the hybridized product can be washed under conditions in 0.2x or 2x SSC and 0.1% SDS at a temperature of 20°C to 68°C. Whether stringent (high stringency) or mild (low stringency) conditions is used depends on a difference in salt concentrations and temperatures while the washing process. In cases where the difference in hybridizing depends on the salt concentration, the washing process can be carried out in 0.2x SSC and 0.1% SDS as a stringent washing buffer (high stringency wash buffer) and 2x SSC and 0.1% SDS as a mild washing buffer (low stringency wash buffer). Also, in cases where the difference in hybridizing depends on the temperature, the washing process may be carried out at 68°C for stringent conditions, at 42°C for medium (moderate stringency) conditions, or at room temperature (20-25°C) for mild conditions, but 0.2x SSC and 0.1% SDS are used in all the cases.

When pre-hybridization is carried out, it is carried out under the same condition as in hybridization, but pre (preliminary)-washing is not necessarily carried out under the same condition as in hybridization.

The "homologous gene" used in the specification of the present application means a gene encoding a protein functionally equivalent to a protein encoded by a certain gene. Whether it is "functionally equivalent" or not can be determined by evaluating if the protein has functions equivalent to biological phenomena or functions related to the expression of the gene. Such a gene encoding the functionally equivalent protein includes not only the so-called homologous gene but also a gene with polymorphism and a gene having a mutation without affecting the function.

Examples of the homologous gene include genes which have a nucleotide sequence of a certain gene wherein one or more (preferably one to several, or 1, 2, 3 or 4) nucleotides are inserted, substituted or deleted, or added to one or both termini, and which encode the functionally equivalent protein.

Examples of the homologous gene also include genes which encode an amino acid sequence encoded by a certain gene wherein one or more amino acids are inserted, substituted, or deleted, or added to one or both termini (modified amino acid sequence), and which encode the functionally equivalent protein.

"One or more amino acids are inserted, substituted, or deleted, or added to one or both termini" used in the specification of the present application means that a modification is made by a known technical method such as a site specific mutagenesis method or by substitution of several amino acids as in naturally occurring mutation.

The "modified amino acid sequence" used in the specification of the present application can be an amino acid sequence wherein, for example, 1 to 30 amino acids, preferably 1 to 20 amino acids, more preferably 1 to 9 amino acids, further preferably 1 to 5 amino acids, particularly preferably 1 to 2 amino acids have been inserted, substituted, or deleted, or added to one or both termini. Preferably, the modified amino acid sequence may be an amino acid sequence having one or more (preferably one or several or 1, 2, 3, or 4) conservative substitutions.

The term "conservative substitution" is used herein to mean that one or more amino acid residues are substituted with other chemically similar amino acid residues, so as not to substantially modify the functions of a protein. Examples of such conservative substitution include a case where a certain hydrophobic residue is substituted with another hydrophobic residue and a case where a certain polar residue is substituted with another polar residue having the same electric charge. Such functionally similar amino acids that can be used in such substitution are known as every amino acid types in the present technical field. Specific examples of a nonpolar (hydrophobic) amino acid include alanine, valine, isoleucine, leucine, proline, tryptophan, phenylalanine, and methionine. Examples of a polar (neutral) amino acid include glycine, serine, threonine, tyrosine, glutamine, asparagine, and cysteine. Examples of a (basic) amino acid having a positive charge include arginine, histidine, and lysine. Examples of an (acidic) amino acid having a negative charge include aspartic acid and glutamic acid.

In cases where the first aspect of the present invention is carried out using a cancer tissue and its surrounding tissue as assay samples, the action of the compound represented by formula (I) can be assayed preferably by using the expression level of pre-mRNA of the genes listed in Table 1 and Table 3 or homologous genes thereof as an index. Measurement of the expression level of the pre-mRNA in cases where the cancer tissue and the surrounding tissue are used as the samples, the microarray can be preferably used.

In cases where the first aspect of the present invention is carried out using a normal tissue, in particular peripheral blood and whole blood as the assay samples, the action of the compound represented by formula (I) can be assayed preferably by using the expression level of pre-mRNA of the genes listed in Table 2, Table 4, and Table 5, or homologous genes thereof as an index. Measurement of the expression level of the pre-mRNA in cases where the peripheral blood and whole blood are used as the samples, the microarray can be preferably used.

According to a second aspect of the present invention, a method for assaying the action of the compound represented by formula (I), using an increase in the expression level of an abnormal protein expressed resulting from splicing defect of pre mRNA (invention (11)) is provided.

The abnormal protein measured in step (f) is an abnormal protein resulting from splicing defect of the genes listed Table 1, Table 2, Table 3, Table 4, and Table 5, or homologous genes thereof. Table 1, Table 2, Table 3, Table 4, and Table 5 describe information specifying the genes listed in these tables and homologous genes thereof.

In step (f), samples are taken from a cancer tissue or normal tissue such as hemocytes in peripheral blood, plasma, and serum from a mammal subjected to the assay. Measurement of the expression level of the abnormal protein may be carried out with the collected samples as they are or a protein extracted therefrom. Extraction of the protein is well known (for example, Campa, M.J. et al. Cancer Res. 63, 1652-1656, 2003), and devices, instruments, and reagents necessary for carrying out the extraction are commercially available. Hence those skilled in the art may extract the protein with no difficulties using such the commercially available devices, apparatuses, and reagents as needed.

The measurement of the expression level of the abnormal protein in step (f) can be carried out with a method selected from a fluorescent antibody method, an enzyme immunoassay (ELISA) method, a radioimmunoassay (RIA) method, a Western blot method and an immunostaining (immunohistochemistry) method. The principle and implementation procedures of these methods are well known and devices and instruments necessary for carrying out the methods are commercially available. Furthermore, in Examples below, an example in which the expression level of pre-mRNA is measured with those methods will be described. Those skilled in the art may measure the expression level of pre-mRNA with no difficulties using the fluorescent antibody method, the enzyme immunoassay (ELISA) method, the radioimmunoassay (RIA) method, the Western blot method and the immunostaining (immunohistochemistry) method. In the measurement of the expression level of the abnormal protein in step (f), the enzyme immunoassay (ELISA) method, the Western blot method, the immunostaining (immunohistochemistry) method and a mass spectrometry method can be used.

In the measurement of the abnormal protein in step (f), an antibody against the abnormal protein generated from the splicing defect of the genes listed Table 1, Table 2, Table 3, Table 4, and Table 5 and a fragment thereof can be used as a detection marker.

Any abnormal protein may be employed for obtaining the antibody according to the present invention as long as it has antigenicity. A protein having an amino acid sequence of the abnormal protein wherein one or more amino acids are deleted, inserted, substituted or added can be used as the antigen for the abnormal protein. It is known that such a protein maintains the same biological activity as the original protein (Mark et al. (1984) Proc.Natl.Acad.Sci.USA 81:5662-6; Zoller and Smith (1982) Nucleic Acids Res. 10:6487-500; Wang et al. (1984) Science 224:1431-3; Dalbadie-McFarland et al. (1982) Proc.Natl.Acad.Sci. USA 79:6409-13). A technique to delete, insert, substitute or add one or more amino acids while maintaining the antigenicity of the original protein is known. For example, such a protein may be obtained by preparing and properly expressing a polynucleotide encoding an abnormal protein by site directed mutagenesis technique (Molecular Cloning, A Laboratory Manual 2nd ed., Cold Spring Harbor Press (1989); Current Protocols in Molecular Biology, John Wiley & Sons, (1987-1997), Section 8.1-8.5; Hashimoto-Goto et al. (1995) Gene 152:271-5; Kinkel (1985) Proc.Natl.Acad.Sci.USA 82:488-92; Kramer and Fritz (1987) Method.Enzymol. 154:350-67; Kunkel (1988) Method. Enzymol.85:2763-6).

The antibody according to the present invention includes an antibody having specificity against a part of the abnormal protein. That is, the abnormal protein for obtaining the antibody according to the present invention includes a polypeptide having the full length amino acid sequence of the abnormal protein as well as a fragment thereof having at least six amino acid residues (for example, not less than 6, 8, 10, 12 or 15 amino acid residues). A preferred fragment is a polypeptide fragment such as an amino terminus and a carboxyl terminus of the abnormal protein. An antigen determination site of the polypeptide can be predicted by a method analyzing the hydrophobicity/hydrophilicity of the amino acid sequence of the protein (Kyte-Doolittle (1982) J.Mol.Biol. 157:105-22), and a method analyzing a secondary structure (Chou-Fasman (1978) Ann.Rev.Biochem. 47:251-76) and further confirmed by a computer program (Anal.Biochem. 151:540-6 (1985)) or a technique such as PEPSCAN analysis (patent application publication JP60500684T) involving the synthesis of a short peptide to confirm the antigenicity.

Table 1, Table 2, Table 3, Table 4 and Table 5 describe information specifying the genes listed in these Tables and homologous genes thereof. Accordingly, those skilled in the art can obtain information on an amino acid encoded by the subject gene to be detected based on the information described in Table 1, Table 2, Table 3, Table 4 and Table 5, and can design and obtain an antibody based thereon.

In addition, the genes listed in Table 1, Table 2, Table 3, Table 4 and Table 5 are known genes and an antibody for detecting a protein encoded thereby is commercially available individually or as a detection kit or detection array.

The antibody according to the present invention may be obtained with a method known those skilled in the art (for example, "Current Protocols in Molecular Biology" (John Wiley & Sons (1987), Antibodies: A Laboratory Manual, Ed. Harlow and David Lane, Cold Spring Harbor Laboratory (1988)).

The antibody according to the present invention includes a polyclonal antibody, a monoclonal antibody, a chimeric antibody, a single chain antibody (scFv), a humanized antibody and a multispecific antibody. Also, the fragment of the antibody according to the present invention includes an antibody fragment such as Fab, Fab', F(ab')₂, Fc, and Fv.

For a polyclonal antibody, blood can be taken from a mammal sensitized with an antigen and blood serum can be isolated with known procedures from the blood to yield blood serum containing the polyclonal antibody. As needed, a fraction containing the polyclonal antibody can further be isolated from this blood serum.

For a monoclonal antibody, antibody-producing cells are taken from spleen or lympho node of a mammal sensitized with the above-mentioned antigen, and then undergo cell fusion with myeloma cell. The resultant hybridoma is subjected to cloning and the antibody was recovered from the culture thereof to yield the monoclonal antibody.

A fragment of the abnormal protein can be used as an immunogen. Alternatively, the synthesized one based on the amino acid sequence of the abnormal protein can be used. The antigen can be used as a complex with a carrier protein. A variety of condensing agents can be used for preparation of the complex between the antigen and the carrier protein, which condensing agents include glutaraldehyde, carbodiimide, and maleimide active ester. The carrier protein may be a usually used one such as bovine serum albumin, thyroglobulin, and hemocyanin. A procedure for coupling at a rate (volume) of 1 time to 5 times is usually employed.

Examples of the animal immunized include mice, rats, rabbits, guinea pigs, hamsters. An example of a method of inoculation is subcutaneous, intramuscular or intraperitoneal administration. The administration may be done in combination with Freund's complete adjuvant and Freund's incomplete adjuvant, and usually once every two to five weeks.

The antibody-producing cells obtained from the spleen or lymph node of the animal immunized undergo cell fusion with myeloma cells, and is isolated as hybridoma. As the myeloma cells, cells derived from mouse, rat, Homo sapiens and etc. are used. It is preferred that antibody-producing cell be derived from the same species. Yet there are cases where the cell fusion can be carried out between different species.

Procedures for the cell fusion may be carried out with a known method, in accordance with, for example, *Nature,* 256,495, 1975. Examples of fusion accelerator include polyethylene glycols and Sendai virus. The cell fusion can be usually carried out by using about 20 to 50% of concentration of polyethylene glycols (average molecular weight 1000 to 4000); at a temperature of 20 to 40°C, preferably 30 to 37°C; at a ratio in number of cells between antibody production cells and myeloma of usually about 1:1 to 10:1, and for about 1 to 10 minutes.

Various immunochemical methods can be employed for screening the antibody-producing hybridoma. Examples thereof include ELISA method using a microtiter plate coated with the abnormal protein, EIA method using a microtiter plate coated with an anti-immunoglobulin antibody, immune blot method using a nitrocellulose blotting membrane after electrophoresis of samples containing the abnormal protein.

Using such wells, cloning by, for example, a limiting dilution method can be further carried out to obtain a clone. Selection and breeding of the hybridoma is usually carried out culture medium for mammalian cells (such as RPMI1640) containing 10~20% bovine fetus serum and supplemented with HAT (hypoxanthine, aminopterin, and thymidine). The clone obtained in such a way is intraperitoneally transplanted into a SCID mouse previously administrated with pristine. Ten to fourteen days later, ascites containing the monoclonal antibody at a high concentration is obtained, which ascites can be used as a raw material for antibody purification. Also the clone may be cultured and the obtained culture may be used as a raw material for antibody purification

Any purification method may be used for purifying the monoclonal antibody as long as it is a known method for purifying an immunoglobulin. The purification can be readily accomplished by, for example, an ammonium sulfate fractionation method, a PEG fractionation method, an ethanol fractionation method, and use of an anion exchanger, as well as means such as affinity chromatography using the abnormal protein.

Purification of the polyclonal antibody from serum can be carried out in the same manner.

In cases where the procedure in the second aspect according to the present invention is carried out by using the cancer tissue and its surrounding tissue as assay samples, the action of the compound represented by formula (I) can preferably be assayed by using the expression level of the abnormal protein(s) expressed resulting from splicing defect of the genes listed in Table1 and Table 3 or homologous genes thereof as an index. In cases where the cancer tissue and the surrounding tissue are used as the samples, the measurement of the expression level of the abnormal proteins can preferably be employed with the enzyme immunoassay (ELISA) method, the Western blot technique, the immunostaining (immunohistochemistry) method and the mass spectrometry method.

In cases where the procedure in the second aspect of the present invention using peripheral blood or whole blood as assay samples, the action of the compound represented by formula (I) can preferably be assayed by using the expression level of the abnormal protein(s) expressed resulting from splicing defect of the genes listed in Table 2, Table 4, and Table 5 or homologous genes thereof as an index. In cases where peripheral blood or whole blood are used as the samples, the measurement of the expression level of the abnormal proteins can preferably be employed with the enzyme immunoassay (ELISA) method, the Western blot method, the immunostaining (immunohistochemistry) method and the mass spectrometry method.

The samples obtained from a subject refer to tissues, cells, body fluids, and the like which are obtained from the subject. Specific examples include biopsy, blood (including hemocytes, plasma, and serum), urine, tissue samples such as curettage tissue (buccal scrapes) of oral cavity, and tumor cells (cells from tumors of breast, lung, stomach, head and neck, colorectum, kidney, pancreas, uterus, liver, urinary bladder, endometrium, and prostate, as well as hemocytes of leukemia patients or of lymphocytes).

### EXAMPLES

The present invention is described in more detail by the examples below. The followings are illustrative of the invention and by no means intended to limit the invention to the embodiments described herein.

### Example 1: Analysis with Northern blotting

Gene expression that affects the cell cycle was examined by Northern blotting analysis using RNA from HeLa cells treated with pladienolide B. As a result, it was discovered that CDKN1B (p27) gene caused pre-mRNA accumulation.

HeLa cells (5x10⁵ cells/mL) were first seeded in a six-well plate and cultured overnight in RPMI1640 medium (containing 10% FCS, penicillin, and streptomycin). The cells were then treated with 10 µM of pladienolide B, 100 µM of DRB, or 1µg/mL of Actinomycin D for 0, 1, 2, and 4 hours. Subsequently, total RNA was obtained using RNeay mini kit (Qiagen) and absorbance at 260 nm was measured to quantify an amount of RNA. Each total RNA sample (10 µg) was, after combined with RNA SAMPLE LOADING BUFFER (SIGMA), subjected to electrophoresis in 1% denatured agarose gel containing formaldehyde, followed by blotting to nylon membrane and cross-linking with UV to make a Northern membrane.

In order to make a probe for CDKN1B(p27) and CDKN1A(p21), the entire length of gene was amplified by PCR with the following primers using cDNA reverse-transcribed from total RNA prepared from U251 cells as a template.

### Primers for CDKN1B

P27-1F: 5'-ATGTCAAACGTGCGAGTGTCTAAC-3' (SEQ ID NO: 1)
P27-2: 5'-TTACGTTTGACGTGTTGTGAGGCC-3' (SEQ ID NO: 2)

### Primers for CDKN1A

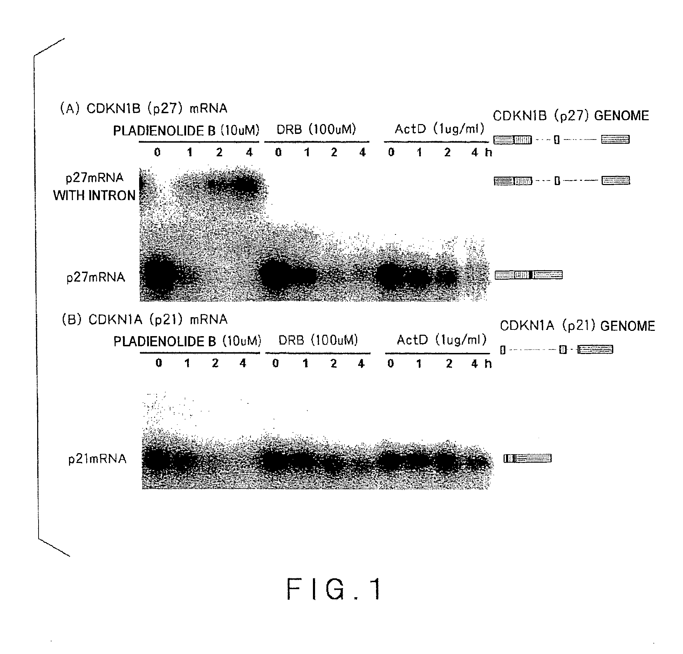

P21-1F: 5'-GCCATGTCAGAACCGGCTGGGGAT-3' (SEQ ID NO: 3)
P21-2: 5'-TTAGGGCTTCCTCTTGGAGAAGAT-3' (SEQ ID NO: 4)
The obtained fragment was separated by electrophoresis in agarose gel and purified. The fragment was labeled using Megaprime DNA labeling kit (Amersham) and Redivue ³²P (Amersham) and purified with ProbeQuant G-50 Micro Columns (Amersham). Using PerfectHyb hybridization solution (TOYOBO), the labeled probe was hybridized onto the blotting membrane. The membrane was washed with a buffer containing 2xSSC and 0.05% SDS, followed by a buffer containing 0.1×SSC and 0.1% SDS. The membrane was exposed to Imaging Plate (FUJIFILM) to determine intensity of photosensitivity with BAS2000 (FUJIFILM). Accumulation of CDKN1B pre-mRNA was observed only when the cells were treated with pladienolide (Figure 1A and B).

### Example 2: Analysis with cDNA library

In order to check if any other gene accumulated its pre-mRNA, a cDNA library was constructed from HeLa cells treated with pladienolide and by randomly sequencing 42 clones, genes containing an intron were screened. Primers were designed within the introns surrounding an exon of those genes. RT-PCR was performed for the total RNA of HeLa cells treated with pladienolide. DNAJB1, BZW1, SPAG5, RIOK3, NUP54, and BRD2 were identified as pre-mRNA-accumulating genes.

Semi-confluent HeLa cells cultured in a 10 cm dish in DMEM high glucose medium (containing 10%FCS, penicillin, and streptomycin) were treated with 10nM, 100nM, and 10µM of pladienolide B for 4 hours and 6 mL of TRIzol (Invitrogen) was added thereto so that the cells were dissolved. Total RNA was obtained in accordance with the protocol of TRIzol and absorbance at 260 nm was measured to quantify an amount of RNA.

Subsequently, mRNA was purified from total RNA collected from HeLa cells treated with 10nM, 100nM, 10µ of pladienolide B and without the treatment, using µMACS mRNA Isolation Kit (Miltenyi Biotec). mRNA was concentrated by ethanol precipitation and absorbance at 260 nm was measured to quantify an amount of RNA. For 1µg of mRNA, single stranded cDNA synthesis, double stranded cDNA synthesis, adaptor ligation in order was carried out using SuperScript Plasmid System for cDNA Synthesis and Cloning (Invitrogen), and the resultant DNA fragment was ligated to pCLex, which is a retovirus vector. The resultant vector construct was then transformed into XL10-Gold ultracompetent cells (STRATAGENE). The transformed cells were seeded on LB plates containing ampicillin and cultured overnight. From each plate of the sample treated with 10nM, 100nM, and 10µM of pladienolide B and without the treatment, 42 clones were separately picked up. A total of 42 clones was individually cultured and the plasmid was purified.

A nucleotide sequence of the genes inserted in the plasmid of the 42 clones extracted from the cDNA library of the sample treated with 10nM, 100nM, and 10µM of pladienolide B and without the treatment was obtained using BigDye Terminator(Applied Biosystems) and senseXIY primer (sequence: CCTCGATCCTCCCTTTATCCAGCCCTCACT) (SEQ ID NO: 5) with ABI PRISM3130 (Applied Biosystems). The obtained sequence was then compared with genome information with UCSC/BLAT to collect genes containing a sequence of an intron region. For the genes containing the sequence of the intron region, the following primers were designed within both-sided exon regions surrounding the intron region.

### DNAJB1-FW:

5'-GAACCAAAATCACTTTCCCCAAGGAAGGAG-3' (SEQ ID NO: 6)

### DNAJB1-RV:

5'-AATGAGGTCCCCACGTTTCTCGGGTGT-3' (SEQ ID NO: 7) BZW1-FW:
5'-GCCAATAAGCAAAGTGTTGAACACTTCAC-3' (SEQ ID NO: 8)

### BZW1-RV:

5'-AAGTGCTTGATGGCTTGCTCTGCTAC-3' (SEQ ID NO: 9)

### SPAG5-FW:

5'-ACATGGACAGCTTTGCTGAGTCGGTCC-3' (SEQ ID NO: 10)

### SPAG5-RV:

5'-TTGCTAGACGACTGTTTfCCAACTCCAG-3' (SEQ ID NO: 11)

### RIOK3-FW:

5'-GCTGAAGGACCATTTATTACTGGAG-3' (SEQ ID NO: 12) RIOK3-RV:
5'-TTCTTGCTGTGTTCTTTCTCCCACA-3' (SEQ ID NO: 13)

### NUP54-FW:

5'-CTAATCAAACAGGAAATTCAAAGGAAGAG-3' (SEQ ID NO: 14)

### NUP54-RV:

5'-CTTGATTTCTCGTAACAGATCTGCATC-3' (SEQ ID NO: 15)

### BRD2-FW:

5'-ACTCTCAGCAACAACACCAGAGCTCTA-3' (SEQ ID NO: 16)

### BRD2-RV:

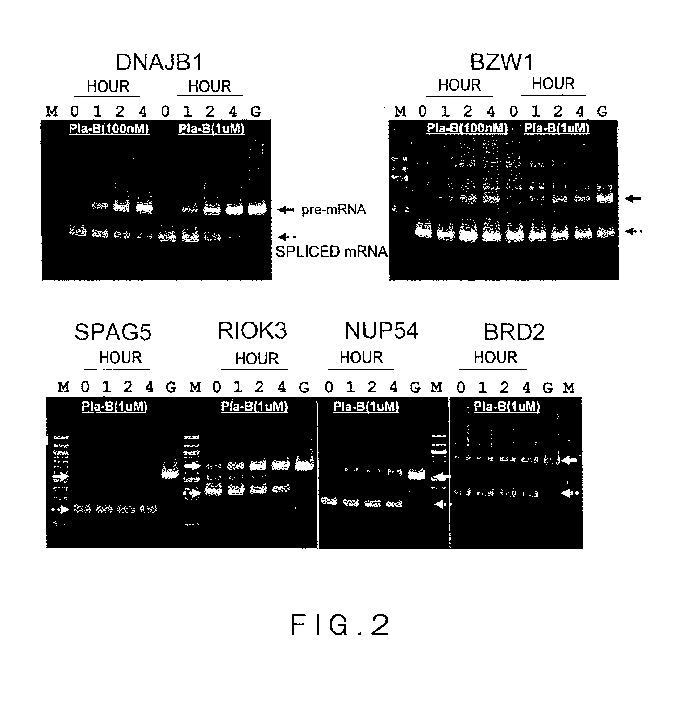

5'-TAGCTTTCGTGCCATTGCCACAACATC-3' (SEQ ID NO: 17)
Total RNA was collected from HeLa cells treated with 100nM or 1µM of Pladienolide B for 1, 2, or 4 hours or untreated. The total RNA was treated with DNase, and then subjected to reverse transcription reaction to yield cDNA. The obtained cDNA was then subjected to PCR using FastStart HiFi Polymerase (Roche Diagnostics) with the designed primers. Based on an electrophoresis pattern in agarose gel, a decrease in the expression of mature RNA and an increase in the expression of premature mRNA upon the Pladienolide B treatment was confirmed in DNAJB1, BZW1, SPAG5, RIOK3, NUP54, and BRD2 (Figure 2).

### Example 3: Analysis with microarray

Using RNA purified from human colon carcinoma cell strain WiDr treated with 14 nM of (8E,12E,14E)-7-((4-cycloheptylpiperazin-1-yl)carbonyl)oxy-3,6, 16,21-tetrahydroxy-6,10,12,16,20-pentamethyl-18,19-epoxytric osa-8,12,14-trien-11-olide (also referred to as "E7101") for six hours, microarray analysis (Human Exon 1.0 ST Array: Affymetrix) was carried out. Not only does this array comprehensively cover the exons for all genes, but it is also designed to have probes for regions estimated as potential exons by a computer prediction program, which enables detection of the expression of the exons and introns of all genes in theory. As a result, genes of which mature mRNA was sufficiently expressed when untreated and of which intron exhibited not less than ten times increase upon the treatment with (8E,12E,14E)-7-((4-cycloheptylpiperazin-1-yl)carbonyl)oxy-3,6, 16,21-tetrahydroxy-6,10,12,16,20-pentamethyl-18,19-epoxytric osa-8,12,14-trien-11-olide in at least two probe sets were identified.

WiDr cells were first suspended in RPMI1640 medium (containing 10% FBS, penicillin, and streptomycin) and seeded in a 10 cm dish (2x10⁶ cells/dish). After an overnight culture in an incubator with 5% carbon dioxide gas at 37°C, the medium was changed with medium containing 14 nM of (8E,12E,14E)-7-((4-cycloheptylpiperazin-1-yl)carbonyl)oxy-3,6, 16,21-tetrahydroxy-6,10,12,16,20-pentamethyl-18,19-epoxytric osa-8,12,14-trien-11-olide or containing vehicle alone. After cultured for additional six hours, TRI reagent (SIGMA) were added to the cells and the cells were harvested. RNA was purified in accordance with the protocol of TRI reagent, followed by further purification with RNeasy (QIAGEN). Absorbance at 260 nm was measured to quantify an amount of RNA. This experiment was repeated three times and RNA samples (n=3) were prepared.

Ribosomal RNA was removed from 1 µg of each total RNA of control cells (n=3) and cells with the treatment (n=3) by using RiboMinus Human/Mouse Transcriptome Isolation Kit (Invitrogen). Single stranded cDNA synthesis, double stranded cDNA synthesis, cRNA synthesis, second single stranded cDNA synthesis, cDNA fragmentation, and cDNA labeling in order were carried out for the total RNA from which ribosomal RNA was removed, by using GeneChip Whole Transcript Sense Target Labeling and Control Reagents (Affymetrix) to make a cDNA probe. The cDNA probe was used for hybridization with Human Exon 1.0 ST Array (Affymetrix). The array was washed and stained, and luminescence intensity was measured by a scanner.

The expression level of the probe set and the gene was quantified by using Expression Console Ver. 1.0 (Affymetrix) with Summarization Method and Normalization Method being set to "Median polish as used in RMA" and "None", respectively. The expression level of the probe was normalized with that of the gene. Welch's t-test was conducted for the treated group and the control group to determine a p value, which was converted into a q value by controlling False Discovery Ratio to pick out a significant probe set.

Probe sets showing that the q value was less than 1%; the normalized change in the level expression of the probe set in the group with the treatment was more than 10 times; the expression level of the probe set and the gene in the group with the treatment was more than 100; and probe set annotation was "extended, full, free", were picked out and further arranged by gene. A gene was, when more than one probe sets therefor were found, determined as a candidate gene with increased expression level of the intron regions (Table 1). In Table 1, for the gene evaluated, gene name (Gene Name), abbreviated name (Gene Symbol), accession number (Accession), alias name (Synonym), transcription number in Human Exon 1.0 ST Array (Human Exon 1.0 ST Array), chromosome number (Chromosome), type of strand (Strand), start region (Start), stop region (Stop), and change in expression level (Fold Change) were respectively shown.

**[Table 1]**

| Gene Name | Gene Symbol | Accession | Synonym | Human Exon 1.0 ST Array | Human Genome hg18 | | | | Fold Change |
|---|---|---|---|---|---|---|---|---|---|
| | | | | | Chromosome | Strand | Start | Stop | |
| sprouty-related, EVH1 domain containing 2 | SPRED2 | NM_181784 | FLJ21897 // FLJ31917 // MGC163164 // Spred-2 | 2556752 | chr2 | - | 65391279 | 65513247 | 31.56 |
| WD repeat domain 73 | WDR73 | NM_032856 | FLJ1488B // HSPC264 | 3636956 | chr15 | - | 82987018 | 82998719 | 30.61 |
| muscleblind-like 2 (Drosophila) | MBNL2 | NM_144778 // NM_207304 | DKFZp781H1296 // MBLL // MBLL39 // MGC120625 // MGC120828 // MGC120828 // PRO2032 | 3497586 | chr13 | + | 96672563 | 96644371 | 28.86 |
| brix domain containing 2 | BXDC2 | NM_018321 | BRIX // FLJ11100 | 2806231 | chr5 | + | 34951248 | 34962845 | 25.94 |
| development and differentiation enhancing factor 2 | DDEF2 | NM_003887 | AMAP2 // FLJ42910 // KIAA0400 // PAG3 // PAP // Pap-alpha // SHAG1 | 2468811 | chr2 | + | 9264345 | 9463243 | 22.92 |
| integrin, beta 1 (fibronectin receptor, beta polypeptide, antigen CD29 includes MDF2, MSK12) | ITGB1 | NM_002211 // NM_033867 // NM_033668 // NM_033669 | CD29 // FNRB // GPDA // MDF2 // MSK12 // VLAB | 3284188 | chr10 | - | 33211051 | 33321367 | 21.48 |
| eukaryotic translation initiation factor 2C, 2 | EIF2C2 | NM_012154 | AGO2 // MGC3183 // Q10 | 3156193 | chr8 | - | 141599440 | 141726037 | 21.33 |
| chromosome 10 open reading frame 18 | C10orf18 | XM_374765 | DKFZp781E1986 // FLJ20360 // bA318E3.2 | 3233322 | chr10 | + | 5766851 | 5846629 | 19.86 |
| K1AA0174 | KIAA0174 | NM_014761 | MGC117220 | 3667766 | chr16 | + | 70481983 | 70522196 | 19.65 |
| discoidin, CUB and LCCL domain containing 2 | DCBLD2 | NM_080927 | CLCP1 // ESDN | 2686023 | chr3 | - | 99997526 | 100124598 | 19.52 |
| ubiquitin protein ligase E3A (human papilloma virus E6-associated protein. Angelman syndrome) | UBE3A | NM_000482 // NM_130838 // NM_130839 | ANCR // AS // E6-AP // EPVE6AP // FLJ28981 // HPVE8A | 3614087 | chr15 | - | 23006655 | 23355108 | 19.35 |
| isopentenyl-diphosphate delta isomerase 1 | IDI1 | NM_004508 | IPP1 // IPPI1 | 3273601 | chr10 | - | 1075869 | 1092634 | 19.25 |
| chromosome 6 open reading frame 166 | C6orf166 | NM_018064 | FLJ10342 // dJ488L4.2 | 2963784 | chr6 | - | 88441297 | 88476721 | 19.23 |
| pumilio homolog 2 (Drosophila) | PUM2 | NM_015317 | FLJ38528 // KIAA0235 // MGC138251 // MGC1 38253 // PUMH2 // PUML2 | 2542816 | chr2 | - | 20291440 | 20450197 | 19.01 |
| suppressor of var1, 3-like 1 (S. cerevisiae) | SUPV3L1 | NM_003171 | SUV3 | 3250204 | chr10 | + | 70609946 | 70638996 | 18.81 |
| forkhead box K2 | FOXK2 | NM_004514 | ILF // ILF-1 // ILF1 | 3738969 | chr17 | + | 78070883 | 78195807 | 18.79 |
| WW domain containing adaptor with coiled-roil | WAC | NM_016828 // NM_100284 // NM_100486 | BM-016 // MGC10753 // PRO1741 // Wwp4 // bA48B24 // bA48B24.1 | 3240340 | chr10 | + | 28828043 | 29005095 | 18.53 |
| LTV1 homolog (S. cerevisiae) | LTV1 | NM_032860 | C6orf93 // FLJ14909 // dJ468K18.4 | 2929038 | chr6 | + | 144194149 | 144227146 | 18.37 |
| CDC20 cell division cycle 20 homolog (S. cerevisiae) | CDC20 | NM_001255 | CDC20A // MGC102824 // bA276H19.3 // p55CDC | 2333136 | chr1 | + | 43597180 | 43601431 | 18.22 |
| TAR DNA binding protein | TARDBP | NM_007375 | TDP-43 | 2320048 | chr1 | + | 10995025 | 11013170 | 18.13 |
| fetal Alzheimer antigen | BPTF | NM_004459 // NM_182641 | FAC1 // FALZ // NURF301 | 3732448 | chr17 | + | 63252111 | 63410954 | 17.61 |
| villin 2 (ezrin) | VIL2 | NM_003379 | CVIL // CVL // DKFZp762H157 // FW26216 // MGC1584 | 2981912 | chr6 | - | 159106770 | 159160432 | 17.50 |
| sorting nexin 19 | SNX19 | NM_014758 | CHET8 // DKFZp6671205 // KIAA0254 | 3398402 | chr11 | - | 130250320 | 130291815 | 17.12 |
| Cdk5 and Ab1 enzyme substrate 1 | CABLES1 | NM_138375 | FLJ35924 // HsT2563 | 3761531 | chr18 | + | 18940699 | 19101598 | 16.94 |
| SMAD specific E3 ubiquitin protein ligase 2 | SMURF2 | NM_022739 | DKFZp888F0270 // MGC138150 | 3786960 | chr17 | - | 59968891 | 60088896 | 16.60 |
| solute carrier family 2 (facilitated glucose transporter), member 1 | SLC2A1 | NM_006516 | GLUT // GLUT1 // MGC141885 // MGC141896 | 2409104 | chr1 | - | 43124794 | 43338329 | 16.44 |
| v-raf-1 murine leukemia viral oncogene homolog 1 | RAF1 | NM_022490 | CRAF // Raf-1 // c-Raf | 2663244 | chr3 | - | 12600117 | 12680654 | 16.41 |
| chromosome 16 open reading frame 80 | C16orf80 | NM_013242 | EVORF // GTL3 | 3693511 | chr16 | - | 56683698 | 56739201 | 16.25 |
| eukaryotic translation initiation factor 4A, isoform 1 | E1F4A1 | NM_001418 | DDX2A // EIF-4A // EIF4A | 3708826 | chr17 | + | 7416768 | 7423040 | 16.15 |
| testis derived transcript (3 LIM domains) | TES | NM_015641//NM_152829 | DKFZP586B2022 // MGC1146 // TESS II TESS-2 // TESTIN | 3020192 | chr7 | + | 115637811 | 115743800 | 15.95 |
| ring finger protein 40 | RNF40 | NM_014771 | BRE1B // DKFZp686K191 //KIAA0661 // MGC13051 // RBP95 // STARING | 3050535 | chr16 | + | 30081120 | 30695182 | 15.84 |
| squamous cell carcinoma antigen recognised by T coils 3 | START3 | NM_014706 | KIAA0156 // MGC138188 // RP11-13G14 // TIP110 // p110(nrb) | 3470253 | chr12 | - | 107440140 | 107479296 | 15.13 |
| heat shock 105kDa/110kDa proteins 1 | HSPH1 | NM_006644 | DKFZp688M05240 // HSP105 // HSP105A // HSP1058 // KIAA0201 // NY-CO-25 | 3508330 | chr13 | - | 30558258 | 30851892 | 15.12 |
| kelch-like 18 (Drosophila) | KLHL18 | NM_025010 | FLJ13703 // KIAA0795 | 2621275 | chr3 | + | 47298888 | 47387590 | 15.00 |
| myosin 1E | MYO1E | NM_004998 | HuncM-10 // MGC104638 // MYO1C | 3628826 | chr15 | | 57215720 | 57452363 | 14.49 |
| UM domain 7 | LMO7 | NM_005358 | FBX20 // FBXO20 // KIAA0858 // LOMP | 3494137 | chr13 | + | 75092571 | 75566974 | 14.34 |
| ring finger and SPRY domain containing 1 | RSPRY1 | NM_133388 | KIAA1972 | 3662612 | chr16 | + | 55777703 | 55831882 | 14.32 |
| KIAA1429 | KIAA1429 | NM_0154986// NM_183009 | DKFZP434I118 // DKFZp781B2117 // MGC138493 // MGC141940 // MSTP054 | 3145020 | chr8 | - | 95569109 | 95834851 | 14.19 |
| centromere protein L | CENPL | NM_033319 | C1 orf1 55 // CENP-L // FLJ31044 // RP3-383J4.1 // dJ383J4.3 | 2444451 | chr1 | - | 172035313 | 172104622 | 14.13 |
| Smcy homolog, X-linked (mouse) | JARID1C | NM_004187 | DXS1272E // MRXJ // MRXSJ // SMCX // XE169 | 4009062 | chrX | - | 53159032 | 53271329 | 14.09 |
| guanine nucleotide binding protein-like 3 (nucleolar) | GNL3 | NM_014388 // NM_206825 | C77032 // E2IG3 // MGC800 // NS | 2624074 | chr3 | + | 52694976 | 52705212 | 14.08 |
| RIO kinase 3 (yeast) | RIOK3 | NM_003831 // NM_145808 | DKFZp779L1370 // SUDD | 3781654 | chr18 | + | 19161035 | 19320545 | 13.87 |
| interleukin enhancer binding factor 2, 45kDa | ILF2 | NM_004515 | MGC8391 // NF45 // PRO3063 | 2436132 | chr1 | - | 151900372 | 151910103 | 13.79 |
| chromosome 9 open reading frame 86 // KIAA1984 | C9orf86 // KLAA1984 | NM_024718 // NM_001039374 | FLJ10101 // FLJ13045 // Parf // RP11-216L13.9 // bA218L13.9 // pp8875 // MGC15438//PARF// RP11-216L13.7//RP11-216L13.9 // bA216L13.7 | 3194635 | chr9 | + | 136810077 | 138855460 | 13.66 |
| adhesion regulating molecule 1 | ADRM1 | NM_007002 | GP110 // MGC29536 // Rpn13 | 3892607 | chr20 | + | 60305485 | 60317305 | 13.51 |
| keratin 18 | KRT18 | NM_000224 | CYK18 // K18 | 3415576 | chr12 | + | 51596769 | 51632949 | 13.41 |
| heat shock 70kDa protein 98 (mortalin-2) | HSPA9 | NM_004134 | CSA // GRP75 // HSPA9B // MGC4500 // MOT // MOT2 // MTHSP75 // PBP74 // mot-2 | 2877508 | chr5 | - | 137917364 | 137939144 | 13.33 |
| 3-hydroxy-3-mothylglutaryl-Coenzyme A synthase 1 (soluble) | HMGCS1 | NM_002130 | HMGCS // MGC90332 | 2855501 | chr5 | - | 43324231 | 43349337 | 13.21 |
| neuroepithelial cell transforming gene 1 | NET1 | NM_005603 | ARHGEF8 // NET1A | 3233182 | chr10 | + | 5444535 | 5491001 | 13.02 |
| SMAD, mothers against DPP homolog 3 (Drosophila) | SMAD3 | NM_005902 | DKFZP586N0721 // DKFZ686J10186 // HSPC193 // HsT17438 // JV15-2 // MADH3 // MGC60396 // Smad 3 | 3598959 | chr15 | + | 65145043 | 65296818 | 12.88 |
| cyclin L2 | CCNL2 | NM_030937 | ANIA-6B // DKFZp761A1210 // DKFZp7620195 // HCLA-ISO // HLA-ISO // PCEE // S8138 | 4041923 | chr1_random | + | 359569 | 372958 | 12.84 |
| ST3 bata-galactoside alpha-2,3-sialyltransferase 1 | ST3GAL1 | NM_003033 | Gal-NAc6S // MGC8183 // SIAT4A // SIATFL // ST3GalA // ST3GalA.1 // ST3GalIA.1 // ST30 | 3154398 | chr8 | - | 134535811 | 134653449 | 12.77 |
| WD repeat domain 74 | WDR74 | NM_018093 | FLJ10439 // FLJ21730 | 3376235 | chr11 | - | 62356627 | 62365857 | 12.70 |
| cyclin D1 | CCND1 | NM_053056 | BCL1 // D11S287E // PRAD1 // U21B31 | 3338192 | chr11 | + | 69161881 | 69170408 | 12.66 |
| nucleoporin like 1 | NUPL1 | NM_014089 // NM_001008564 // NM 001008565 | KIAA0410 // PRO2463 | 3482219 | chr13 | + | 24773258 | 24622202 | 12.63 |
| WDR45-like | WDR45L | NM_019613 | WIPI-3 // WIPI3 | 3775157 | chr17 | - | 78165748 | 78199803 | 12.59 |
| ubiquitin specific peptidase 47 | USP47 | NM_017944 | DKFZp6876C3257 // FLJ20727 // TRFP | 3320604 | chr11 | + | 11819556 | 11937446 | 1232 |
| eukaryotic translation initiation factor 5 | E1F5 | NM_001969 | EIF-5A | 3553607 | chr14 | + | 102743652 | 102881108 | 12.30 |
| methionine adenosyltransferase II. alpha | MAT2A | NM_005911 | MATA2 // MATII // SAMS2 | 2491615 | chr2 | + | 85597809 | 85625908 | 12.29 |
| WEE1 homolog (S. pombe) | WEE1 | NM_003390 | DKFZp686118166 // FLJ16446 // WEE1hu | 3319937 | chr11 | + | 9550916 | 9579143 | 12.26 |
| GTP binding protein 4 | GTPBP4 | NM_012341 | CRFG // FLJ10686 // FLJ10690 // FLJ39774 // NGB | 3231774 | chr10 | + | 942450 | 1055862 | 12.24 |
| zinc finger protein 384 | ZNF384 | NM_133476 | CAGH1 // CAGH1A // CIZ // ERDA2 // NMP4 // NP // TNRC1 | 3442205 | chr12 | - | 6645924 | 6668961 | 12.13 |
| ATPase, Na+/K⁺ transporting, beta 1 polypeptide | ATP1B1 | NM_001677 // NM_001001787 | ATP1B // MQC1798 | 2366422 | chr1 | + | 167342204 | 167368946 | 12.11 |
| ADP-ribosylation factor interacting protein 2 (arfaptin 2) | ARFIP2 | NM_012402 | POR1 | 3360941 | chr11 | - | 6453496 | 6439171 | 12.04 |
| zinc finger protein 410 | ZNF410 | NM_021188 | APA-1 // APA1 | 3543884 | chr14 | + | 73423093 | 73468718 | 12.01 |
| DnaJ (Hsp40) homolog, subfamily B. member 1 | DNAJB1 | NM_006145 | HSPF1 // Hdj1 // Hsp40 | 3852783 | chr19 | - | 14485622 | 14501114 | 11.95 |
| guanine nucleotide binding protein (G protein), beta polypeptide 2-like 1 | GNB2L1 | NM_006098 | Gnb2-rs1 // H12.3 // HLC-7 // PIG21 // RACK1 | 2891052 | chr5 | - | 180596592 | 180612024 | 11.87 |
| Rho GTPase activating protein 12 | ARHGAP12 | NM_018287 | DKFZp779N2050 // FLJ10971 // FLJ20737 // FLJ21785 // FLJ45709 | 3283920 | chr10 | - | 32134245 | 32261226 | 11.60 |
| tight junction protein 1 (zona occludens 1) | TJP1 | NM_003257 // NM_175610 | DKFZp686M05161// MCC133289 // ZO-1 | 3615579 | chr15 | - | 27703228 | 28048334 | 11.55 |
| dyskeratosis congenita 1, dyskerin | DKC1 | NM_001363 | DKC // NAP57 // NOLA4 // XAP101 // dyskerin | 3996667 | chrX | ++ | 153644243 | 153659150 | 11.53 |
| BAT2 domain containing 1 | BAT2D1 | NM_015172 | BAT2-iso // XTP2 | 2367154 | chr1 | + | 169716078 | 169829202 | 11.26 |
| TERF1 (TRF1)-interacting nuclear factor 2 | TINF2 | NM_012461 | TIN2 | 3558012 | chr14 | - | 23776465 | 23781950 | 11.05 |
| YTH domain containing 1 | YTHDC1 | NM_001031732 | KIAA1986 // YT521 // YT521-B | 2772017 | chr4 | - | 68858700 | 68934802 | 10.66 |
| adenylate kinase 2 | AK2 | NM_001625 // NM_013411 | ADK2 | 2405364 | chr1 | - | 33245959 | 33275155 | 10.05 |

### Example 4: Analysis in PBMC

Since it is not easy to obtain a cancer tissue clinically, the measurement of the marker in hemocytes readily obtainable in peripheral blood would be more useful. It was then examined whether the marker gene obtained in cancer cells could change in peripheral blood mononuclear cells in a similar manner as observed in the cancer cells. Specifically peripheral blood was taken from three normal individuals (volunteers) and peripheral blood mononuclear cells were purified and treated with (8E,12E,14E)-7-((4-cycloheptylpiperazin-1-yl)carbonyl)oxy-3,6, 16,21-tetrahydroxy-6,10,12,16,20-pentamethyl-18,19-epoxytric osa-8,12,14-trien-11-olide for three hours, followed by measurement of change in expression of the precursor gene (pre-mRNA) by qPCR. In cases where an increase in the expression of pre-mRNA is used as a marker, the genes in Table 2 below, as a representative example, were found to be usable.

### (1) Isolation of peripheral blood mononuclear cells (PBMC)

Ficoll-Paque PLUS solution (Amersham, 17-1440-02) was slowly added to the blood taken (with heparin added) from healthy individuals to form a layer underneath the blood (15 mL of Ficoll-Paque PLUS solution was added to 25 mL of blood). After the mixture was centrifuged at 1500 rpm for 30 min, the upper part containing platelets was removed and then a layer containing mononuclear cells was transferred to another tube. The cells were suspended in PBS and centrifuged at 1500 rpm for 5 min, followed by removal of the supernatant. After these steps were repeated twice, the PBMC was suspended in RPMI1640 (containing 10% FBS, penicillin, and streptomycin) and the number of the cells was then counted.

### (2) Preparation of total RNA

PBMC was suspended in RPMI1640 medium (containing10% FBS, penicillin, and streptomycin) to 5x10⁶ cells/ml and 1 ml of the suspension was plated per well of a 24-well plate. Immediately, 111 µl of 10 times-concentrated (8E,12E,14E)-7-((4-cycloheptylpiperazin-1-yl)carbonyl)oxy-3,6, 16,21-tetrahydroxy-6,10,12,16,20-pentamethyl-18,19-epoxytric osa-8,12,14-trien-11-olide was added to the well (six wells per each concentration tested). The cells were then cultured in an incubator with 5% carbon dioxide gas at 37°C. Three hours later, the supernatant was collected and centrifuged at 1500 rpm for five minutes. TRI reagent (SIGMA) (1 ml) was added to each well of the plate from which the supernatant was removed to harvest the cells, which was added to the centrifuged pellet to dissolve. RNA was purified in accordance with the protocol of TRI reagent. RNA was further purified using RNeasy (QIAGEN) and, in accordance with the protocol, DNaseI was added to the samples during the procedure. Absorbance at 260 nm was measured to quantify an amount of RNA.

### (3) Measurement of the expression level of a precursor gene (pre-mRNA)

RNA was prepared to 30 ng/µl and cDNA was synthesized using TaqMan Reverse Transcription Reagents (Applied Biosystems). For the expression level of pre-mRNA of ID1, amplification was carried out by using TaqMan Universal PCR Master Mix (Applied Biosystems) with TaqMan Gene Expression Assays (Hs00704053_s1 Applied Biosystems) as a probe. For the expression level of pre-mRNA of DNAJB1, BZW1, NUP54, RIOK3, CDKN1B, STK17B, ADRM1, EIF4A1, FOXK2, GNB2L1, HSPA9B, HSPH1, KLHL18 and VIL2, reagents were prepared in accordance with each protocol by using POWER SYBR GREEN PCR MASTER MIX (Applied Biosystems) and primers with the sequence below (Invitrogen), and the expression level was measured with ABI7900 (Applied Biosystems). 18S rRNA was measured by both the TaqMan and SYBR methods. The expression level of 18S r RNA was measured with Hs99999901_s1 (Applied Biosystems) as primers for TaqMan and with 18S primers included in TaqMan Ribosomal RNA control reagents (4308329: Applied Biosystems) as primers for SYBR. Measured values were corrected using the expression level of 18S rRNA as an internal control. The expression level of each gene was calculated with the expression level in the cells treated without (8E,12E,14E)-7-((4-cycloheptylpiperazin-1-yl)carbonyl)oxy-3,6, 16,21-tetrahydroxy-6,10,12,16,20-pentamethyl-18,19-epoxytric osa-8,12,14-trien-11-olide as 1. Genes of which expression level in PBMC reached about 250% or more were shown in Table 2.

### DNAJB1:

Sense: 5'-GGCCTGATGGGTCTTATCTATGG-3' (SEQ ID NO: 18)
Antisense: 5'-TTAGATGGAAGCTGGCTCAAGAG-3' (SEQ ID NO: 19)

### BZW1:

Sense: 5'-GAACTTTGCCTCATTCTTTTGCA-3' (SEQ ID NO: 20)
Antisense: 5'-CTGAGCTCCAGTCTCTTGTATTTCTG-3' (SEQ ID NO: 21)

### NUP54:

Sense: 5'-CAAGGTAACCACTTCTAAGACCATAATTC-3' (SEQ ID NO: 22)
Antisense: 5'-CCTGCTTGAAGATTACATAACTTTTTGT-3' (SEQ ID NO: 23)

### RIOK3:

Sense: 5'-TCAATGGAGATAGCAAAGGTATTATAACC-3' (SEQ ID NO: 24)
Antisense: 5'-AGATTTACTTAGGAGCACATTATGAGTGTT-3' (SEQ ID NO: 25)

### CDKN1B (p27):

Sense: 5'-ATGTTTATCAACGGTCCGCCT-3' (SEQ ID NO: 26)
Antisense: 5'-CATCCCCAGTGGCTTTTAAGG-3' (SEQ ID NO: 27)

### STK17B:

Sense: 5'-TGCGCGGAGACAGCATAG-3' (SEQ ID NO: 28)
Antisense: 5'-TGGGTTCAAAATGCCAGGTT-3' (SEQ ID NO: 29)

### ADRM1:

Sense: 5'-GGCCCTTCAGAAATGCTTGTC-3' (SEQ ID NO: 30)
Antisense: 5'-CCCATTACACAATTCATGTGCTTAG-3' (SEQ ID NO: 31)

### EIF4A1:

Sense: 5'-CAGATCATCTAGAAGCAGCTGGTTT-3' (SEQ ID NO: 32)
Antisense: 5'-ACAGAATCTGGTGCCTACTAACAAAA-3' (SEQ ID NO: 33)

### FOXK2:

Sense: 5'-GAGCAGAAGGAAGCGTGGTT-3' (SEQ ID NO: 34)
Antisense: 5'-GACACATGAATTTCCACAACAGTAAA-3' (SEQ ID NO: 35)

### GNB2L1:

Sense: 5'-ACGTAATGACATTTTGGTCTGAGTAACT-3' (SEQ ID NO: 36)
Antisense: 5'-AAATGCTGCTAAACATCCTGGAA-3' (SEQ ID NO: 37)

### HSPA9B:

Sense: 5'-TGAATCCTGAATACTATGCCTCCTT-3' (SEQ ID NO: 38)
Antisense: 5'-TCCCTTCTTCTCAAGACTACTCAGTATG-3' (SEQ ID NO: 39)

### HSPH1:

Sense: 5'-ACCTTCTTCTCACAAGACTTTTTAAGC-3' (SEQ ID NO: 40)
Antisense: 5'-CCTTCTCAGCCACCATGGAA-3' (SEQ ID NO: 41)

### KLHL18:

Sense: 5'-CCAGGTTTCCCCTGCAGTT-3' (SEQ ID NO: 42)
Antisense: 5'-CCCCCTGACTTAGTCCTCGTT-3' (SEQ ID NO: 43)

### VIL2:

Sense: 5'-CCCACTGCCTTGATCTAGTTGAT-3' (SEQ ID NO: 44)
Antisense: 5'-GTCAAGCAATCTATGGCCTACCA-3' (SEQ ID NO: 45)
In Table 2, for the gene evaluated, abbreviated name (Gene Symbol), accession number (Accession), alias name (Synonym), gene name (Gene Name), chromosome number (Chromosome), type of strand (Strand), start region (Start), stop region (Stop), and the expression level at 0 hours, three hours, 10 hours and 30 hours after the E7107 treatment were respectively shown.

**[Table 2]**

| Gene Symbol | Accession | Synonym | Gene Name | E7107 (nM) | | | |
|---|---|---|---|---|---|---|---|
| | | | | 0 | 3 | 10 | 30 |
| DNAJB1 | NM_006145 | HSPF1 // Hdj1 // Hsp40 | DnaJ (Hsp40) homolog, subfamily B, member 1 | 1 | 2.25 | 4.46 | 7.42 |
| BZW1 | XM_001126385 // NM_014670 | BZAP45 // KIAA0005 // Nbla10236 | basic leucine zipper and W2 domains 1 | 1 | 1.72 | 2.34 | 3.26 |
| NUP54 | NM_017426 | MGC13407 | nucleoporin 54kDa | 1 | 2.31 | 2.46 | 1.93 |
| RIOK3 | NM_003831 // NM_145906 | DKFZp779L1370 // SUDD | RIO kinase 3 (yeast) | 1 | 2.59 | 3.57 | 2.89 |
| CDKN1B | NM_004064 | CDKN4 // KIP1 // P27KIP1 | cyclin-dependent kinase inhibitor 1B (p27, Kip1) | 1 | 1.27 | 2.35 | 4.9 |
| STK17B | NM_004226 | DRAK2 | serine/threonine kinase 17b | 1 | 1.94 | 3.44 | 3.99 |
| ID1 | NM_002165 // NM_181353 | ID | inhibitor of DNA binding 1, dominant negative helix-loop-helix protein | 1 | 3.31 | 6 | 10.57 |
| ADRM1 | NM_007002 // NM_175573 | GP110 // MGC29536 // Rpn13 | adhesion regulating molecule 1 | 1 | 1.8 | 2.68 | 2.59 |
| EIF4A1 | NM_001416 | DDX2A // EIF-4A // EIF4A | eukaryotic translation initiation factor 4A, isoform 1 | 1 | 2.17 | 3.64 | 4.36 |
| FOXK2 | XM_001134363 // XM_001134364 // NM_004514 | ILF // ILF-1 // ILF1 | forkhead box K2 | 1 | 2.1 | 2.68 | 2.31 |
| GNB2L1 | NM_006098 | Gnb2-rs1 // H12.3 // HLC-7 // PIG21 // RACK1 | guanine nucleotide binding protein (G protein), beta polypeptide 2-like 1 | 1 | 2.47 | 2.74 | 2.23 |
| HSPA9 | NM_004134 | CSA // GRP75 // HSPA9B // MGC4500 // MOT // MOT2 // MTHSP75 // PBP74 // most-2 | heat shock 70kDa protein 9 (mortalin) | 1 | 1.87 | 3.45 | 2.33 |
| HSPH1 | NM_006644 | DKFZp686M05240 // HSP105 // HSP105A // HSP105B // KIAA0201 // NY-CO-25 | heat shock 105kDa/110kDa protein 1 | 1 | 1.26 | 1.88 | 2.68 |
| KLHL18 | NM_025010 | FLJ13703 // KIAA0795 | kelch-like 18 (Drosophila) | 1 | 2.36 | 2.97 | 2.6 |
| VIL2 | NM_003379 | CVIL // CVL // DKFZp782H157 // FLJ26216 // MGC1584 | villin 2 (ezrin) | 1 | 1.27 | 2.87 | 4.89 |

### Example 5: Analysis in PBC

Since fractionation of hemocytes is required for emptying PBMC, use of PBMC in a clinical test is complicated. If change of pre-mRNA can be confirmed in whole blood (PBC), such a test would be clinically more useful. However, since it is difficult to culture PBC, it is not easy to monitor the change of pre-mRNA *in vitro.* If expression of mRNA in PBC, like in PBMC, is confirmed, the change of pre-mRNA can be monitored. Accordingly, in regard to the genes of Table 2 confirmed for PBMC (DNAJB1, BZW1, NUP54, RIOK3, CDKN1B, STK17B, and ID1), it is examined whether mRNA can be detected for RNA obtained from PBC by the same RNA purification method as in the clinical setting (Tempus PAX gene).

### (1) Preparation of RNA with Tempus Blood RNA Tube (Applied Biosystems)

Human peripheral blood was collected in the tube (3ml/tube) and combined with Stabilizing Reagent. In accordance with the protocol of the Tube, RNA was purified by the RNA Blood-DNA Method in ABI 6100 PrepStation (Applied Biosystems). Absorbance at 260nm was measured to quantify an amount of RNA.

### (2) Preparation of RNA with PAXgene Blood RNA Tube (QIAGEN)

Human peripheral blood was collected in the tube (2.5ml/tube), combined with Stabilizing Reagent, and left to stand overnight at room temperature. In accordance with the protocol of the Tube, RNA was purified with PAXgene Blood RNA Kit (QIAGEN). Absorbance at 260nm was measured to quantify an amount of RNA.

### (3) Quantification of the expression level of mRNA

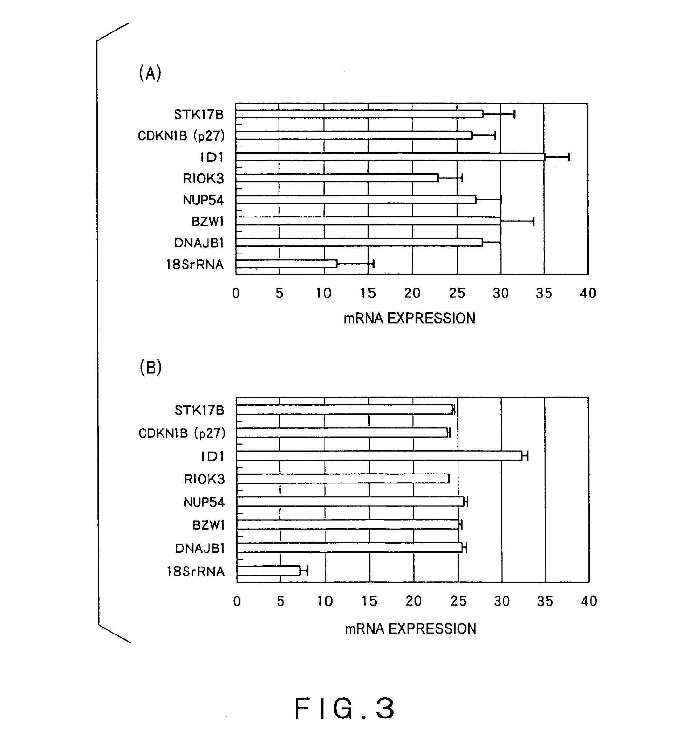

RNA was prepared to 30 ng/µL and cDNA was synthesized by using High Capacity cDNA Reverse Transcription Kits (Applied Biosystems). A probe corresponding respectively to DNAJB1, BZW1, NUP54, RIOK3, CDKN1B, STK17B, ID1 and 18S rRNA was purchased from TaqMan Gene Expression Assays (Applied Biosystems). Reagents were prepared in accordance with the protocol of TaqMan Universal PCR Master Mix (Applied Biosystems) and the expression level of mRNA was measured with ABI7900 (Applied Biosystems). As shown in Figure 3A (Tempus Blood RNA Tube) and Figure 3B (PAXgene Blood RNA Tube), it was demonstrated that gene expression could sufficiently be confirmed in RNA obtained by both methods.

### Example 6: Analysis by Western blotting

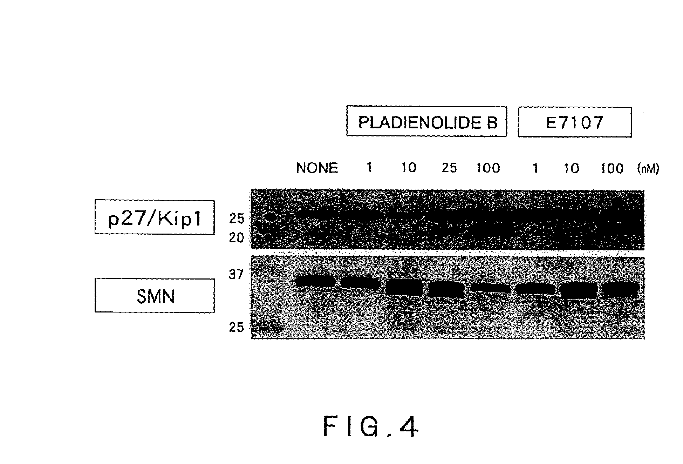

Since pre-mRNA contains a part of the introns, a protein translated from such pre-mRNA is one which does not normally exist. Consequently the protein may serves as a protein marker to monitor the action of (8E,12E,14E)-7-((4-cycloheptylpiperazin-1-yl)carbonyl)oxy-3,6, 16,21-tetrahydroxy-6,10,12,16, 20-pentamethyl-18,19-epoxytric osa-8,12,14-trien-11-olide. Whether the pre-mRNA-dependent abnormal protein practically emerged upon the treatment of (8E,12E,14E)-7-((4-cycloheptylpiperazin-1-yl)carbonyl)oxy-3,6, 16,21-tetrahydroxy-6,10,12,16,20-pentamethyl-18,19-epoxytric osa-8,12,14-trien-11-olide was examined by using cancer cells (HaLa). As a result, insertion of a intron sequence was found to introduce a termination codon, which led to generation of small-molecule p27 and SMN different from normal CDKN1B (p27) (Figure 4). Based on these findings, the protein generated from pre-mRNA can serve as the marker as well.

About 3x10⁵cells of HeLa cells were first seeded in a 24-well culture plate (bottom area: 2 cm²), and 18 hours later, the compound was added thereto at the indicated concentration, followed by additional 24-hour culturing. The cells were washed once with PBS(-), and then were lysed by treating with 0.2mlof M-PER reagent (PIERCE) for 30 minutes. The collected lysate was filtered with a 0.45µm filter to remove insoluble matters and then combined with an equal amount of 2xSDS-PAGE sample solution. The mixture was heated to be denatured at 95 °C for five minutes to yield a sample to be analyzed.

The analysis sample (15µl each) was separated with 5-20% SDS-PAGE and transferred onto a PVDF membrane (Hybond-P: GE-Amersham). The membrane was treated to be blocked with Blockace (Dainippon Pharma Co., Ltd.) by one-hour incubation. The membrane was then incubated with a primary antibody for two hours, washed three times for 10 minutes each, incubated with a secondary antibody for one hour, washed (10 minutes each) three times followed by signal detection.

Detection of p27/Kip1 was carried out as follows. Specifically, a mouse anti-p27/Kip1 monoclonal antibody (BD Bioscience, #610242) was used as the primary antibody at a 1:1000 dilution rate. An HRP-labeled mouse anti-IgG antibody (GE-Amersham) was used as the secondary antibody at a 1:2500 dilution rate. An ECL-Plus reagent (GE-Amersham) was used for the signal detection.

Detection of SMN was carried out as follows. Specifically, a mouse anti-SMN monoclonal- antibody (BD Bioscience, #610646) was used as the primary antibody at a 1:1000 dilution rate. An AP-labeled mouse anti-IgG antibody (CHEMICON) was used as the secondary antibody at a 1:2500 dilution rate. NBT/BCIP reagent (PIERCE) was used for signal detection.

As shown in Figure 4, only a 27 kDa band was detected for p27/Kip1 when the cells were treated without the compounds. On the other hand, appearance of a band with about 22 kDa was confirmed in the cells treated with about 100nM of Pladienolide B or (8E,12E,14E)-7-((4-cycloheptylpiperazin-1-yl)carbonyl)oxy-3,6, 16,21-tetrahydroxy-6,10,12,16,20-pentamethyl-18,19-epoxytric osa-8,12,14-trien-11-olide for 24 hours. In addition, only a 35 kDa band was detected for SMN in the cells treated without the compounds. When the cells were treated with 10nM and 25nM of Pladienolide B or 10nM and 100nM of (8E,12E,14E)-7-((4-cycloheptylpiperazin-1-yl)carbonyl)oxy-3,6, 16,21-tetrahydroxy-6,10,12,16, 20- pentamethyl-18,19-epoxytric osa-8,12,14-trien-11-olide for 24 hours, multiple bands with a molecular weight of less than 35 kDa were confirmed.

### Example 7: Analysis in a nude mouse

### (1) Administration of the pladienolide derivative to nude mice subcutaneously transplanted with WiDr human colon carcinoma cells.

Nude mice (BALB/cAJcl-nu/nu, 6 weeks old, female) were purchased from CLEA Japan, Inc. After an acclimated period of a week, the mice were subcutaneously transplanted WiDr cells suspended in Hanks' Balanced Salt Solution (GIBCO) (5x10⁶ cells per a mouse). Two weeks after the transplantation, when tumor volume was confirmed to grow to more than 200 mm³, the mice were administrated (8E,12E,14E)-7-((4-cycloheptylpiperazin-1-yl)carbonyl)oxy-3,6, 16,21-tetrahydroxy-6,10,12,16,20-pentamethyl-18,19-epoxytric osa-8,12,14-trien-11-olide (30 mg/kg) in a single dose via tail vein injection.

### (2) Blood collection and isolation of the tumor

At the time point of 30 minutes, 1 hour, 2 hours, 4 hours, and 8 hours, after the administration of (8E,12E,14E)-7-((4-cycloheptylpiperazin-1-yl)carbonyl)oxy-3,6, 16,21-tetrahydroxy-6,10,12,16,20-pentamethyl-18,19-epoxytric osa-8,12,14-trien-11-olide, two mice per each time point were put down by euthanasia with CO₂. Whole blood (with heparin added) was taken from abdominal aorta of each mouse, TRIzol LS Reagent (Invitrogen) was added thereto, and the mixture was stored at -20°C. The tumor was removed, cut in shape of a about 0.5 cm x 0.5 cm square, and stored -20°C in RNAlater (Ambion).

### (3) Preparation of RNA

RNA preparation from blood was carried out in accordance with the protocol of TRIzol LS Reagent (Invitrogen). The obtained RNA was further purified with RNeasy (QIAGEN). During the process, a treatment with DNase I was performed in accordance with the protocol. The tumor treated with RNA later (Ambion) was placed in TRI reagent (SIGMA) and grinded by a homogenizer, followed by the operations following the protocol of TRI reagent. The obtained RNA was then purified using RNeasy (QIAGEN). During the process, a treatment with DNase I was performed in accordance with the protocol. Absorbance at 260nm was measured to quantify an amount of each RNA.

### (4) Measurement of the expression level of the precursor gene (pre-mRNA) in blood

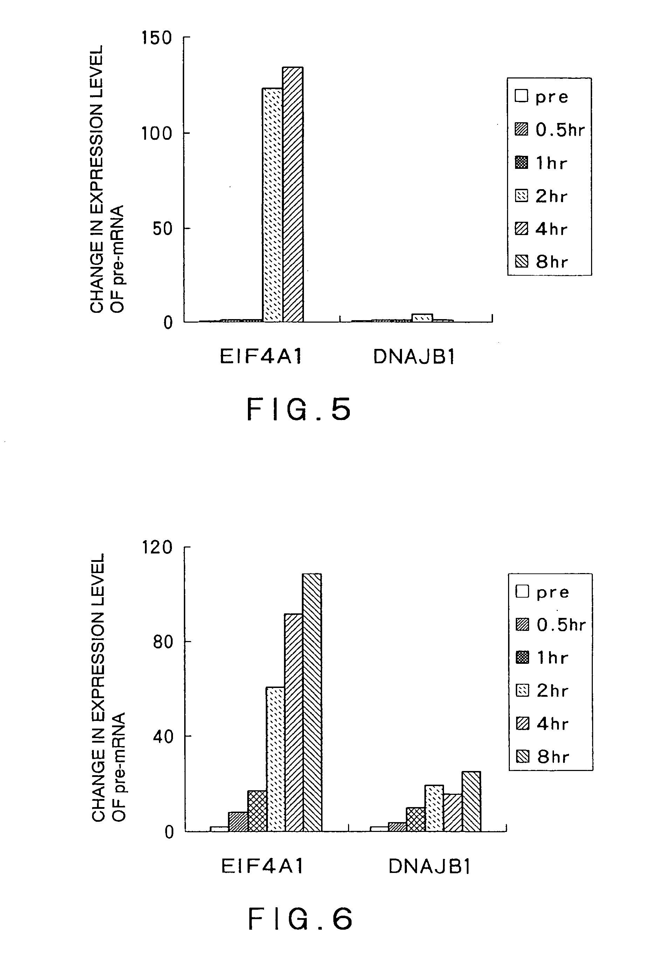

RNA was prepared to 100ng/µl and cDNA was synthesized by using TaqMan Reverse Transcription Reagents (Applied Biosystems). For the expression level of pre-mRNA of mouse DNAJB1 and mouse EIF4A, reagents were prepared in accordance with each protocol by using POWER SYBR GREEN PCR MASTER MIX (Applied Biosystems) and primers with the sequence below (Invitrogen), and the expression level was measured with ABI7900 (Applied Biosystems). The expression level of 18S rRNA was measured by using 18S primers included in TaqMan Ribosomal RNA control reagents (4308329: Applied Biosystems). Measured values were corrected using the expression level of 18S rRNA as an internal control. The expression level of each gene was calculated with the expression level in the group of mice treated without (8E,12E,14E)-7-((4-cycloheptylpiperazin-1-yl)carbonyl)oxy-3,6, 16,21-tetrahydroxy-6,10,12,16,20-pentamethyl-18,19-epoxytric osa-8,12,14-trien-11-olide as 1. Results were shown in Figure 5.

### [mouse DNAJB1]

Sense: 5'-TGCTGTGAGAATAATGGGTTGTG-3' (SEQ ID NO: 46)
Antisense: 5'-GGCTGGCTTAGGAGCTTCACT-3' (SEQ ID NO: 47)

### [mouse EIF4A1]

Sense: 5'-ACCTGCGGTTCCCACTTTATT-3' (SEQ ID NO: 48)
Antisense: 5'-ACCACTCCAAATGTCTAAGGTCACT-3' (SEQ ID NO:49)

### (5) Measurement of the expression level of the precursor gene (pre-mRNA) in tumor

RNA was prepared to 100ng/µl and cDNA was synthesized by using TaqMan Reverse Transcription Reagents (Applied Biosystems). For the expression level of pre-mRNA of human DNAJB1 and human EIF4A, reagents were prepared in accordance with each protocol by using POWER SYBR GREEN PCR MASTER MIX (Applied Biosystems) and primers with the sequence below (Invitrogen), and the expression level was measured with ABI7900 (Applied Biosystems). The expression level of 18S rRNA was measured by using 18S primers included in TaqMan Ribosomal RNA control reagents (4308329: Applied Biosystems). Measured values were corrected using the expression level of 18S rRNA as an internal control. The expression level of each gene was calculated with the expression level in the group of mice treated without (8E,12E,14E)-7-((4-cycloheptylpiperazin-1-yl)carbonyl)oxy-3,6, 16,21-tetrahydroxy-6,10,12,16,20-pentamethyl-18,19-epoxytric osa-8,12,14-trien-11-olide as 1. Results were shown in Figure 6.

### [Human DNAJB1]

Sense: 5'-GGCCTGATGGGTCTTATCTATGG-3' (SEQ ID NO: 50)
Antisense: 5'-TTAGATGGAAGCTGGCTCAAGAG-3' (SEQ ID NO: 51)

### [Human EIF4A1]

Sense: 5'-GAACTTTGCCTCATTCTTTTGCA-3' (SEQ ID NO: 52)
Antisense: 5'-CTGAGCTCCAGTCTCTTGTATTTCTG-3' (SEQ ID NO: .53)

### Example 8: Analysis with microarray (2)

Analysis conditions were reviewed in the data used in Example 3. Genes containing increased introns upon treatment with (8E,12E,14E)-7-((4-cycloheptylpiperazin-1-yl)carbonyl)oxy-3,6, 16,21-tetrahydroxy-6,10,12,16,20-pentamethyl-18,19-epoxytric osa-8,12,14-trien-11-olide were identified.

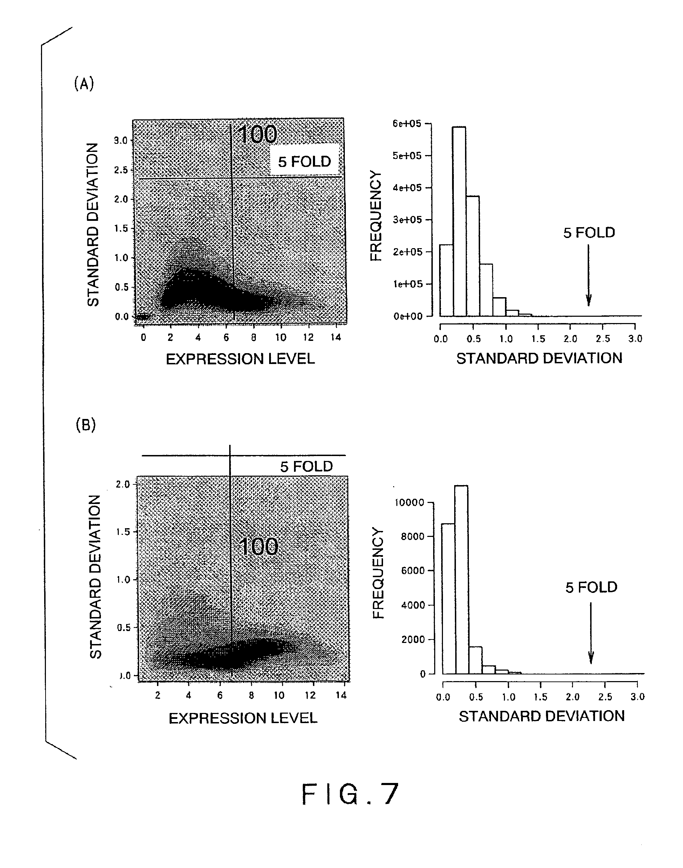

In order to consider the analysis conditions, variations in the data from the samples with identical conditions were studied. Figure 7 shows a scatter diagram of the expression level of probe sets and genes of the control cells, and standard deviation thereof. It turned out that, by analyzing under conditions where the expression level was more than 100 and change in the expression level was more than five times, a probe set with increased expression can be extracted.

Probe sets showing that the normalized change in the level expression of the probe set in the group with the treatment was more than 5 times; the expression level of the probe set and the gene in the group with the treatment were more than 100; and probe set annotation was "extended, full, free", were picked out. Welch's t-test was conducted with the probe sets extracted for the treated group and the control group to determine a p and q value. A gene containing the probe sets with the q value of less than 5% was determined as a candidate gene with increased expression level of the intron regions (Table 3).

In Table 3, for each gene evaluated, gene name (Gene Name), abbreviated name (Gene Symbol), accession number (Accession), alias name (Synonym), transcription cluster number in Human Exon 1.0 ST Array (Human Exon 1.0 ST Array Transcript Cluster ID), chromosome number (Chromosome), type of strand (Strand), start region (Start), stop region (Stop), and change in expression level (Fold Change) were respectively shown.

**[Table 3]**

| Gene Name | Gene Symbol | Accession | Synonym | Human Exon ST 1.0 Array | Human Genome hg18 | | | | Fold Change |
|---|---|---|---|---|---|---|---|---|---|
| | | | | Transcript Cluster ID | Chromo some | Strand | Start | Stop | |
| TSR1 , 20S rRNA accumulation, | TSR1 | NM 018128 | FLJ10534 // | 3740998 | chr17 | - | 2172438 | 2187551 | 44.77 |
| translocase of inner mitochondrial membrane 23 homolog (yeast) | TIMM23 | XM_001133798 // NM_006327 | MGC22767 // PR01197 // TIM23 // TIMM23B | 3289031 | chr10 | - | 51180787 | 51293382 | 28.32 |
| pellino homolog 1 (Drosophila) | PELI1 | NM_020651 | DKFZp686C18116 // MGC50990 | 2556302 | chr2 | - | 64173290 | 64225291 | 27.36 |
| chromosome 15 open reading frame 42 | C15orf42 | NM_152258 | FLJ41618 // MGC45866 | 3607698 | chr15 | + | 87857353 | 87975281 | 26.27 |
| RNA binding motif protein 26 | RBM26 | NM_02211 | C13orf10 // FLJ20957 // MGC133295 // MGC133296 // PRO1777 // RP11-255E21.1 // SE70-2 | 3519119 | chr13 | - | 78783968 | 78878613 | 21.52 |
| integrin, beta 1 (fibronectin receptor, beta polypeptide, antigen CD29 includes MDF2, MSK12) | ITGB1 | NM_002211 // NM_033666 // NM_033667 // NM_033668 // NM-033669 // NM 133376 | CD29 // FNRB // GPGA // MDF2 // MSK12 // VLAB | 3284188 | chr10 | - | 33211051 | 33321367 | 21.20 |
| ADP-ribosylation factor guanine nucleotide-exchange factor 1(brefeldin A-inhibited) | ARFGEF1 | NM_006421 | ARFGEP1 // BIG1 // D730028018Rik // DKFZP434L057 // P200 | 3139035 | chr8 | - | 68250009 | 68418446 | 21.19 |
| clathrin interactor 1 | CLINT1 | NM_014666 | COUNT // ENTH // EPN4 // EPNR // EPSINR // KIAA01 71 | 2883609 | chr5 | - | 157145339 | 157284818 | 20.51 |
| CTD (carboxy-terminal domain, RNA polymerase II. polypeptide A) small phosphatase like 2 | CTDSPL2 | NM_016396 | FLJ10523 // HSPC058 // HSPC129 | 3591909 | chr15 | + | 42506834 | 42607712 | 20.40 |
| cell division cycle associated 4 | CDCA4 | NM_017955 // NM_145701 | FLJ20764 // HEPP // MGC19517 | 3581386 | chr14 | - | 104546975 | 104585458 | 20.39 |
| WD repeat domain 73 | WDR73 | NM_032856 | FLJ14888 // HSPC264 | 3636956 | chr15 | - | 82987018 | 82998719 | 20.03 |
| zinc finger protein 317 // zinc finger protein 658 | ZNF317 // ZNF658 | NM_020933 // XM_001125688 // NM_033160 | KIAA1588 // DKFZp572C163 // FLJ32813 // MGC35232 | 3819880 | chr19 | + | 9112073 | 9135082 | 19.64 |
| integrin, alpha 6 | ITGA6 | NM_000210 // NM_001 079818 | CD49f // ITGA6B // VLA-6 | 2515627 | chr2 | + | 173000563 | 173079416 | 19.59 |
| ubiquitin protein ligase E3A (human papilloma virus E6-associated protein, Angelman syndrome) | UBE3A | NM_000462 // NM_130838 // NM_30839 | ANCR // AS // E6- AP // EPVE6AP // FLJ26981 // HPVE6A | 3614087 | chr15 | - | 23006655 | 23355108 | 19.35 |
| museleblind-like 2 (Drosophila) | MBNL2 | NM_144778 // NM_207304 | DKFZp781H1296 // MBLL // MBLL39 // MGC120625 // MGC120626 // MGC120628 // PR02032 | 3497586 | chr13 | + | 96672563 | 96844371 | 19.31 |
| chromosome 6 open reading frame 166 | C6orf166 | NM_018064 | FLJ10342 // dJ486L4.2 | 2963784 | chr6 | - | 88441297 | 88476721 | 19.23 |
| homocysteine-inducible, endoplasmic reticulum stress-inducible, ubiquitin-like domain member 1 | HERPUD1 | NM_001010989 // NM_001010990 // NM_014685 | HERP // KIAA0025 // Mif1 // SUP | 3662387 | chr16 | + | 55523269 | 55552122 | 19.03 |
| RAP1 interacting factor homolog (yeast) | RIF1 | NM_018151 | DKFZp781N1478 // FLJ12870 | 2510485 | chr2 | + | 151974674 | 152072768 | 18.85 |
| development and differentiation enhancing factor 2 | DDEF2 | NM_003887 | AMAP2 // FLJ42910 // KIAA0400 // PAG3 // PAP // Pap-alpha // SHAG1 | 2468811 | chr2 | + | 9264345 | 9463243 | 18.43 |
| cell division cycle 20 homolog (S. cerevisiae) | CDC20 | NM_001255 | CDC20A // MGC102824 // bA276H19.3 // p55CDC | 2333136 | chr1 | + | 43597180 | 43601431 | 18.22 |
| BMI1 polycomb ring finger oncogene | BMI1 | NM_005180 | MGC12685 // PCGF4 // RNF51 | 3238491 | chr10 | + | 22650103 | 22660820 | 18.11 |
| golgi-specific brefeldin A resistance factor 1 | GBF1 | NM_004193 | FLJ21263 // FLJ21500 // KIAA0248 // MGC134877 // MGC134878 | 3261544 | chr10 | + | 103993260 | 104132642 | 17.47 |
| strawberry notch homolog (Drosophila) | SBNO1 | NM_018183 | FLJ10701 // FLJ10833 // FLJ16176 // MOP3 // Sno | 3476130 | chr12 | - | 122342423 | 122435284 | 17.44 |
| protein phosphatase 4, regulatory subunit 2 | PPP4R2 | NM_174907 | MGC131930 | 2629693 | chr3 | + | 73019946 | 73201035 | 17.19 |
| brix domain containing 2 | BXDC2 | NM_018321 | BRIX FLJI 1100 | 2806231 | chr5 | + | 34951248 | 34962845 | 17.08 |
| far upstream element (FUSE) binding protein 1 | FUBP1 | NM_003902 | FBP // FUBP | 2419235 | chr1 | - | 78182330 | 78217881 | 16.59 |
| RAE1 RNA export 1 homolog (S. pombe) | RAE1 | NM_001015885 // NM_003610 | FLJ30608 // MGC117333 // MGC126076 // MGC126077 // MIG14 // MRNP41 // Mnrp41 // dJ481 F12.3 // dJ800J21.1 | 3890555 | chr20 | + | 55359493 | 55386992 | 16.46 |
| CCHC-type zinc finger, nucleic acid binding protein | CNBP | NM_003418 | CNBP1 // DM2 // PROMM // RNF163 // ZCCHC22 // ZNF9 | 2694644 | chr3 | - | 130369369 | 130385467 | 16.43 |
| acyl-CoA synthetase long-chain family member 3 | ACSL3 | NM_004457 // NM 203372 | ACS3 // FACL3 // PRO2194 | 2529546 | chr2 | + | 223433916 | 223539136 | 16.27 |
| peptidase (mitochondrial processing) alpha | PMPCA | NM_015160 | Alpha-MPP // INPP5E // KIAA0123 // MGC104197 | 3194338 | chr9 | + | 138424942 | 138438030 | 16.01 |
| CGI-09 protein | CGI-09 | NM 015939 | MGC5029 | 3896524 | chr20 | - | 5865881 | 5879370 | 16.01 |
| baculoviral IAP repeat-containing 6 (apollon) | BIRC6 | NM_016252 | APOLLON // BRUCE // FLJ13726 // FLJ13786 // KIAA1289 | 2476219 | chr2 | + | 32435095 | 32697447 | 15.96 |
| BCL2/adenovirus E1B 19kDa interacting protein 2 | BNIP2 | NM_004330 | BNIP-2 // NIP2 | 3627076 | chr15 | - | 57742025 | 57768916 | 15.96 |
| DEAD (Asp-Glu-Ala-Asp) box polypeptide 23 | DDX23 | NM_004818 | MGC8416 // PRPF28 // U5-100K // prp28 | 3453319 | chr12 | - | 47509816 | 47532322 | 15.78 |
| DEAD (Asp-Glu-Ala-Asp) box polypeptide 17 | DDX17 | NM_006386 // NM_030881 | DKFZp761H2016 // P72 // RH70 | 3960629 | chr22 | - | 37202800 | 37232288 | 15.42 |
| SAPS domain family, member 3 | SAPS3 | NM_018312 | C11orf23 // DKFZp781E17107 // DKFZp781E2374 // DKFZp78102362 // FLJl1058 // FLJ43065 // MGC125711 // MGC125712 // PP6R3 // SAP190 // SAPL // SAPLa | 3337618 | chr11 | + | 67984785 | 68173381 | 15.25 |
| PAK1 interacting protein 1 // chromosome 20 open reading frame 7 | PAKlIP1 // C20orf7 | NM_017906 // NM_001039375 // NM_024120 | FLJ20624 // MAK11 // PIP1 // RP11- 421M1.5 // WDR84 // bA421M1.5 // hPIP1 // FLJ22324 // MGC90272 // bA526K24.2 // dJ842G6.1 | 2894663 | chr6 | + | 10799016 | 10817955 | 15.04 |
| sprouty-related, EVH1 domain containing 2 | SPRED2 | NM_181784 | FLJ21897 // FLJ31917 // MGC163164 // Spred-2 | 2556752 | chr2 | - | 65391279 | 65513247 | 15.03 |
| kelch-like 18 (Drasophila) | KLHL18 | NM_025010 | FLJ13703 // KIAA0795 | 2621275 | chr3 | + | 47298888 | 47367590 | 15.00 |
| suppressor of var1, 3-like 1 (S. | SUPV3L1 | NM_003171 | SUV3 | 3250204 | chr10 | + | 70609946 | 70638996 | 14.99 |
| cerevisiae) | K1AA0174 | NM_014761 | MGC117220 | 3667766 | chr16 | + | 70481983 | 70522196 | 14.78 |
| KLAA0174 flap structure-specific endonuclease | FEN1 | NM_004111 | FEN-1 // MF1 // RAD2 | 3333226 | chr11 | + | 61312929 | 61350560 | 14.75 |
| 1 atrophin 1 | ATN1 | NM_001007026 // NM 001940 | B37 // D12S755E // DRPLA // NOD | 3403045 | chr12 | + | 6903897 | 6921744 | 14.70 |
| poliovirus receptor | PVR | NM_006505 | CD155 // HVED // NECL5 // Necl-5 // PVS // TAGE4 | 3835645 | chr19 | + | 49839030 | 49860075 | 14.67 |
| KIAA0317 | K1AA0317 | NM_001039479 | | 3572041 | chr14 | - | 74189599 | 74249655 | 14.49 |
| DEAD (Asp-Glu-Ala-Asp) box polypeptide 3, X-linked | DDX3X | XM_001129278 // NM_001356 | DBX // DDX14 // DDX3 // HLP2 | 3974838 | chrX | + | 41055591 | 41108668 | 14.39 |
| brix domain containing 1 | BXDC1 | NM_032194 | FLJ21087 // RPF2 // bA397G5.4 | 2921374 | chr6 | + | 111409800 | 111458743 | 14.38 |
| actinin, alpha 1 | ACTN1 | NM_001102 | FLJ40884 | 3569814 | chr14 | - | 68409817 | 68515815 | 14.26 |
| Rho guanine nucleotide exchange factor (GEF) 12 | ARHGEF12 | NM_015313 | DKFZp68602372 // KIAA0382 // LARG // PRO2792 | 3352503 | chr11 | + | 119711882 | 119896306 | 14.21 |
| KlAA0859 | K1AA0869 | NM_001007239 11 NM_014955 // NM_015935 | 5630401 D24Rik // CGI-01 // FLJ10310 | 2367287 | chr1 | + | 170017287 | 170053841 | 14.17 |
| THO complex 5 | THOC5 | NM_001002877 // NM_001002878 // NM_001002879 // NM_003678 | C22orf19 // Fmip // PK1.3 | 3956854 | chr22 | - | 28224935 | 28280423 | 14.15 |
| polo-like kinase 2 (Drosophila) | PLK2 | NM_006622 | SNK | 2858023 | chr5 | - | 57785572 | 57792624 | 14.01 |
| forkhead box K2 | FOXK2 | XM_001134363 // XM_001134364 // NM 004514 | ILF //ILF-1 // ILF1 | 3738969 | chr17 | + | 78070883 | 78195807 | 14.01 |
| calcium binding protein 39 | CAB39 | NM_016289 | CGI-66 // FLJ22682 // MO25 | 2531522 | chr2 | + | 231285781 | 231394027 | 13.97 |
| ubiquitin specific peptidase 38 | USP38 | NM_032557 | FLJ35970 // HP43.8KD // KIAA1691 | 2745499 | chr4 | + | 144325529 | 144364549 | 13.94 |
| SMAD specific E3 ubiquitin protein ligase 2 | SMURF2 | NM_022739 | DKFZp686F0270 // MGC138150 | 3766960 | chr17 | - | 59968891 | 60088896 | 13.92 |
| RIO kinase 3 (yeast) | RIOK3 | NM_003831 // NM_145906 | DKFZp779L1370 // SUDD | 3781654 | chr18 | + | 19161035 | 19320545 | 13.87 |
| protein phosphatase 1, catalytic subunit, gamma isoform | PPP1CC | NM_002710 | PPP1G | 3471374 | chr12 | - | 109642007 | 109665131 | 13.87 |
| erbb2 interacting protein // caspase 6, apoptosis-related cysteine peptidase | ERBB21P // CASP6 | NM_001006600 // NM_018695 // NM_001226 // NM_032992 | ERBIN // LAP2 // MCH2 | 2812435 | chr5 | + | 65258079 | 65451372 | 13.87 |
| MKI67 (FHA domain) interacting nucleolar phosphoprotein | MKI671P | NM_032390 | NiFK // Nopp34 | 2573786 | chr2 | - | 122132489 | 122231052 | 13.76 |
| tyrosine 3-monooxygenase/tryptophan 5-monooxygenase activation protein, | YWHAB | NM_003404 // NM_139323 | GW128 // HSt // KCIP-1 | 3886639 | chr20 | + | 42927275 | 42970748 | 13.72 |
| Ctr9, Paf1 /RNA polymerase II complex component, homolog (S. cerevisiae) // tRNA 5-methylaminomethyl-2-thiouridylate methyltransferase | CTR9 // TRMU | NM_014633 // NM_018006 | KIAA0155 // SH2BP1 // TSBP // p150 // p150TSP // MGC99627 // MTO2 // MTU1 // TRMT // TRMT1 // TRNT1 | 3320301 | chr11 | + | 10684027 | 10774985 | 13.68 |
| karyopherin (importin) beta 1 | KPNB1 | NM_002265 | IMB1 // IPOB // lmpnb // MGC2155 // MGC2156 // MGC2157 // NTF97 | 3724782 | chr17 | + | 43082272 | 43117868 | 13.68 |
| A kinase (PRKA) anchor protein 13 | AKAP13 | NM_006738 // NM_007200 // NM_144767 | AKAP-Lbc // BRX // FLJ11952 // FLJ43341 // HA-3 // Ht31 // LBC // PROTO-LB // PROTO-LBC // c-Ibc | 3606304 | chr15 | + | 83681343 | 84102785 | 13.65 |
| nucleolar protein 9 | NOL9 | NM_024654 | FLJ23323 // MGC131821 // MGC138483 | 2394784 | chr1 | - | 6504004 | 6537329 | 13.58 |
| ADP-ribosylation factor-like 6 interacting protein 2 | ARL61P2 | NM_022374 | ARL3IP2 // ATL2 // FLJ23293 // atlastin2 | 2548776 | chr2 | - | 38374621 | 38516141 | 13.57 |
| pumilio homolog 2 (Drosophila) | PUM2 | NM_015317 | FLJ36528 // KLAA0235 // MGC138251 // MGC138253 // PUMH2 // PUML2 | 2542816 | chr2 | - | 20291440 | 20450197 | 13.55 |
| calmodulin 2 (phosphorylase kinase, delta) | CALM2 | NM_001743 | CAMII // PHKD // PHKD2 | 2551924 | chr2 | - | 47232159 | 47286178 | 13.54 |
| nucleolar protein family A, member 1 (H/ACA small nucleolar RNPs) | NOLA1 | NM_018983 // NM_032993 | GAR1 | 2739242 | chr4 | + | 110956115 | 110965340 | 13.42 |
| chromosome 6 open reading frame 94 // LAV1 homolog (S. cerevisiae) | C6orf94 // LTV 1 | XM_928657 // XM_941265 // NM_032860 | dJ468K18.5 // C6orf93 // FLJ14909 // dJ488K18.4 | 2929036 | chr6 | + | 144194149 | 144227146 | 13.35 |
| PPAR binding protein | PPARBP | NM_004774 | CRSP1 // CRSP200 // DRIP205 // DRIP230 // MEND // MGC71488 // PBP // PPARGBP // RB18A // TRAP220 // TRIP2 | 3755714 | chr17 | - | 34801544 | 34861069 | 13.29 |
| 3-hydroxy-3-methylglutaryl-Coenzyme A reductase | HMGCR | NM_000859 | - | 2815965 | chr5 | + | 74668800 | 74700136 | 13.12 |
| 15 kDa selenoprotein | 15-Sep | NM_004261 // NM_203341 | - | 2421271 | chr1 | - | 87100720 | 87153273 | 13.02 |
| phosphatidylinositol binding clathrin assembly protein | PICALM | NM_001008660 // NM_007166 | CALM // CLTH // LAP | 3385175 | chr11 | - | 85345353 | 85458481 | 13.00 |
| proteasome (prosome, macropain) 26S subunit, non-ATPase, 3 | PSMD3 | NM_002809 | P58 // RPN3 // S3 | 3720636 | chr17 | + | 35390438 | 35407732 | 12.85 |
| COP9 constitutive photomorphogenic homolog subunit 2 (Arabidopsis) | COPS2 | NM_004236 | ALIEN // CSN2 // SGN2 // TRIP15 | 3623424 | chr15 | - | 47203877 | 47235198 | 12.73 |
| B-cell CLL/lymphoma 10 | BCL10 | NM_003921 | CARMEN // CIPER // CLAP // c-E10 // mE10 | 2420808 | chr1 | - | 85504067 | 85516171 | 12.67 |
| villin 2 (ezrin) | VIL2 | NM_003379 | CVIL // CVL // DKFZp762H157 // FLJ26216 // MGC1584 | 2981912 | chr6 | - | 159106770 | 159160432 | 12.62 |
| solute carrier family 25, member 32 | SLC25A32 | NM 030780 | FLJ23872 // MFTC | 3147726 | chr8 | - | 104247068 | 104496640 | 12.60 |
| DEAD (Asp-Glu-Als-Asp) box polypeptide 21 | DDX21 | NM_004728 | DKFZp686F21172 // GUA // GURDB // RH-II/GU // RH- II/GuA | 3250055 | chr10 | + | 70380584 | 70414821 | 12.56 |
| WEE1 homolog (S. pombe) | WEE1 | NM_003390 | DKFZp686I18166 // FLJ16446 // WEE1hu | 3319937 | chr11 | + | 9550916 | 9579143 | 12.54 |
| N-acetyltransferase 10 | NAT10 | NM_024662 | ALP // DKFZp434C116 // FLJ10774 // FLJ12179 // FLJ23850 // KIAA1709 // hALP | 3326252 | chr11 | + | 34083688 | 34125769 | 12.51 |
| transcription elongation factor B (SIII), polypeptide 3 (110kDa, elongin | TCEB3 | NM_003198 | SIII // TCEB3A | 2325251 | chr1 | + | 23942459 | 23961135 | 12.38 |
| microtubule-associated protein, RP/EB family, member 1 | MAPRE1 | NM_012325 | EB1 // MGC117374 // MGC129946 | 3882069 | chr20 | + | 30871304 | 30901864 | 12.37 |
| Cdk5 and Abl enzyme substrate 1 | CABLES1 | NM_38375 | FLJ35924 // HsT2563 | 3781531 | chr18 | + | 18940699 | 19101596 | 12.37 |
| ubiquitin specific peptidase 47 | USP47 | NM_017944 | DKFZp686C13257 // FLJ20727 // TRFP | 3320604 | chr11 | + | 11819556 | 11937446 | 12.32 |
| DEAD (Asp-Glu-Ala-Asp) box polypeptide 31 | DDX31 | NM_022779 // NM_138620 | FLJ13633 // FLJ14578 // FLJ23349 // helicain A // helicain B // helicain C | 3228191 | chr9 | - | 134456969 | 134535609 | 12.21 |
| chromosome 8 open reading frame 76 // zinc fingers and homeoboxes 1 | C8orf78 // ZHX1 | NM_032847 // NM_001017926 // NM_007222 | FLJ14825 // MGC9784 // - | 3151473 | chr8 | - | 124291083 | 124356860 | 12.17 |
| chromosome 10 open reading frame 18 | C10orf18 | XM_374765 // XM_937970 | DKFZp781E1986 // FLJ20360 // bA318E3.2 | 3233322 | chr10 | + | 5766851 | 5846629 | 12.16 |
| guanine nucleotide binding protein-like 2 (nucleolar) | GNL2 | NM.013285 | FLJ40906 // HUMAUANTIG // NGP1 // Ngp-1 // dJ423B22.6 | 2407191 | chr1 | - | 37805043 | 37834120 | 12.14 |
| eukaryotic translation initiation factor 4 gamma, 1 | EIF4G1 | NM_004953 // NM-182917 // NM_198241 // NM_198242 // NM_198244 | DKFZp686A1451 // EIF4F // EIF4G // p220 | 2655688 | chr3 | + | 185514970 | 185535829 | 12.13 |
| peter pan homolog (Drosophila) // PPAN-P2RY11 | PPAN // PPAN-P2RY11 | NM_020230 // NM_001040664 | BXDC3 // MGC14226 // MGC45852 // SSF // SSF1 // SSF2 // | 3820342 | chr19 | + | 10077989 | 10082841 | 12.07 |
| Rho GTPase activating protein 11A | ARHGAP11A | NM_014783 // NM_199357 | GAP (1-12) // KIAA0013 // MGC70740 | 3587457 | chr15 | + | 30693622 | 30719422 | 12.06 |
| high-mobility group nucleosome binding domain 1 | HMGN1 | NM_004965 | FLJ27265 // FLJ31471 // HMG14 II MGC104230 MGC117425 | 3932270 | chr21 | - | 39636124 | 39643423 | 11.92 |
| mortality factor 4 // mortality factor 4 like 1 | MORF4 // MORF4L1 | NM_006792 // NM_006791 // NM_206839 | CSR // CSRB // SEN // SEN1 // Eaf3 // FWP006 // HsT17725 // MGC10631 // MORFRG15 // MRG15 // S863-6 | 3603532 | chr15 | + | 76889894 | 76979806 | 11.90 |
| cytotoxic and regulatory T cell molecule | KIAA0690 // CRTAM | NM_019604 | - | 3302240 | chr10 | - | 99098933 | 99175486 | 11.86 |
| TERF1 (TRF1)-interacting nuclear factor 2 | TINF2 | NM_012461 | TIN2 | 3558012 | chr14 | - | 23776465 | 23781950 | 11.82 |
| DCN1, defective in cullin neddylation 1, domain containing 5 (S. cerevisiae) | DCUN1D5 | NM_032299 | FLJ32431 // MGC2714 | 3388914 | chr11 | - | 102342328 | 102485844 | 11.81 |
| isopentenyl-diphosphate delta isomerase 1 | ID11 | NM_004508 | IPP1 // IPPI1 | 3273601 | chr10 | - | 1075869 | 1092634 | 11.81 |
| exosome component 9 | EXOSC9 | NM_001034194 // NM_005033 | PM/Scl-75 // PMSCL1 // RRP45 // Rrp45p // p5 // p6 | 2741768 | chr4 | + | 122941935 | 122957724 | 11.79 |
| diablo homolog (Drosophila) | D1ABLO | NM_019887 // NM_38929 // NM_138930 | DIABLO-S // FLJ10537 // FLJ25049 // SMAC // SMAC3 | 3475511 | chr12 | - | 121257461 | 121277963 | 11.73 |
| eukaryotic translation elongation factor 2 | EEF2 | NM_001961 | EEF-2 // EF2 | 3846538 | chr19 | - | 3926677 | 3936451 | 11.73 |
| calcium activated nucleotidase 1 | CANT1 | NM 138793 | SCAN-1 // SHAPY | 3772775 | chr17 | - | 74499403 | 74517744 | 11.68 |
| moesin | MSN | NM 002444 | | 3979659 | chrX | + | 64804248 | 64878488 | 11.57 |
| aquarius homolog (mouse) | AQR | NM_014691 | KIAA0560 | 3617757 | chr15 | - | 32935042 | 33160557 | 11.56 |
| EPH receptor A2 | EPHA2 | No 004431 | ECK | 2397948 | chr1 | - | 16323428 | 16355151 | 11.54 |
| ATPase, Na+/K+ transporting, beta 1 polypeptide | ATP1B1 | NM_001001787 // NM_001677 | ATP1B // MGC1798 | 2366422 | chr1 | + | 167342204 | 167368946 | 11.44 |
| ring finger protein 40 | RNF40 | NM_014771 | BRE1B // DKFZp686K191 // KIAA0661 // MGC13051 // RBP95 // STARING | 3656555 | ehr16 | + | 30681120 | 30695182 | 11.42 |
| KIAA0406 | KIAA0406 | NM_014657 | | 3905073 | chr20 | - | 36044840 | 36095268 | 11.38 |
| TIMP metallopeptidase inhibitor 1 | TIMP1 | NM_003254 | CLGI // EPA // EPO // FLJ90373 // HCI // TIMP | 3976341 | chrX | + | 47326654 | 47332978 | 11.27 |
| DCP1 decapping enzyme homolog A (S. cerevisiae) | DCP1A | NM_018403 | HSA275986 // Nbla00360 // SMAD4IP1 // SMIF | 2676726 | chr3 | - | 53296412 | 53356692 | 11.27 |
| heat shock 70kDa protein 5 (glucose-regulated protein, 78kDa) | HSPA5 | NM_005347 | SIP // FLJ26106 // GRP78 // MIF2 | 3225398 | chr9 | - | 127036953 | 127064590 | 11.27 |
| chromosome 4 open reading frame 23 | C4orf23 | NM_152544 | FLJ12891 // FLJ35725 | 2717757 | chr4 | + | 8474582 | 8545892 | 11.25 |
| RAB5A, member RAS oncogene | RAB5A | NM_004162 | RAB5 | 2613386 | chr3 | + | 19963571 | 20002852 | 11.19 |
| importin 7 | IPO7 | NM_006391 | FLJ14581 // MGC138673 // RANBP7 | 3319840 | chr11 | + | 9362702 | 9426296 | 11.19 |
| nucleoporin 214kDa | NUP214 | NM_005085 | CAIN // CAN // D9S46E // MGC104525 // N214 | 3191900 | chr9 | + | 132990780 | 133102339 | 11.18 |
| PRP8 pre-mRNA processing factor 8 cerevisiae) | PRPF8 | NM_006445 | HPRP8 // PRP8 // PRPC8 // RP13 | 3740479 | chr17 | - | 1500681 | 1534906 | 11.12 |
| homolog (S. Snf2-related CBP activator protein | SRCAP | NM_006662 | KIAA0309 | 3656418 | chr16 | + | 30616551 | 30663998 | 11.09 |
| keratin 18 | KRT18 | NM_000224 // NM 199187 | CYK18 // K18 | 3415576 | chr12 | + | 51596769 | 51632949 | 11.07 |
| adiponectin receptor 2 | ADIPOR2 | NM_024551 | ACDCR2 // FLJ21432 // MGC4640 // PAQR2 | 3400625 | chr12 | + | 1670418 | 1768071 | 11.05 |
| splicing factor, arginine/serine-rich 3 | SFRS3 | NM_003017 | SRp20 | 2905118 | chr6 | + | 36669658 | 36741293 | 11.05 |
| cyclin B1 | CCNB1 | NM_031966 | CCNB | 2813414 | chr5 | + | 68498462 | 68509822 | 11.03 |
| GrpE-like 2, mitochondrial (E. coli) | GRPEL2 | NM_152407 | DKFZp451 C205 // FLJ23713 // FLJ33918 // Mt- GrpE#2 | 2835006 | chr5 | + | 148705190 | 148714338 | 11.03 |
| Yes-associated protein 1, 65kDa | YAP1 | NM_006106 | YAP // YAP2 // YAP65 | 3346453 | chr11 | + | 101486379 | 101609466 | 11.01 |
| nuclear factor (erythroid-derived 2)-like 3 | NFE2L3 | NM_004289 | NRF3 | 2993590 | chr7 | + | 26158385 | 26194013 | 11.01 |
| nucleoporin like 1 | NUPL1 | NM_001008564 // NM_001008565 // NM_014089 | KIAA0410 // PRO2463 | 3482219 | chr13 | + | 24773258 | 24822202 | 10.97 |
| RAB14, member RAS oncogene | RAB14 | NM_016322 | FBP // RAB-14 | 3223872 | chr9 | - | 122980236 | 123025093 | 10.96 |
| sperm specific antigen 2 | SSFA2 | NM_006751 | CS-1 // OS1 // DKFZp31301039 // DKFZp779G0129 // FLJ45996 // KIAA1927 // KRAP // SPAG13 | 2518428 | chr2 | + | 182464815 | 182516732 | 10.95 |
| DnaJ (Hsp40) homolog, subfamily B, member 1 | DNAJB1 | NM_006145 | HSPF1 // Hdj1 // Hsp40 | 3852783 | chr19 | - | 14485622 | 14501114 | 10.94 |
| cyclin D1 | CCND1 | NM_053056 | BCL1 // D11S287E // PRAD1 // U21B31 | 3338192 | chr11 | + | 69161881 | 69178408 | 10.91 |
| chromosome 20 open reading frame 77 | C20orf77 | NM_021215 | CREPT // DKFZp434P0735 // FLJ44520 // dJ1057B20.2 | 3884450 | chr20 | + | 36095232 | 36189576 | 10.91 |
| SWI/SNF related, matrix associated, actin dependent regulator of chromatin, subfamily d, member 1 | SMARCD1 | NM_003076 // NM_139071 | BAF60A // CRACD1 // Rsc6p | 3414390 | chr12 | + | 48765279 | 48780742 | 10.90 |
| kinesin family member 11 | KIF11 | NM_004523 | EG5 // HKSP // KNSL1 // TRIP5 | 3258168 | chr10 | + | 94292951 | 94405130 | 10.90 |
| dickkopf homolog 1 (Xenopus laevis) | DKK1 | NM_012242 | DKK-1 // SK | 3247172 | chr10 | + | 53744067 | 53807545 | 10.89 |
| polo-like kinase 4 (Drosophila) | PLK4 | NM_014264 | SAK // STK18 | 2742985 | chr4 | + | 129021450 | 129039809 | 10.88 |
| OTU domain containing 4 | OTUD4 | NM_017493 // NM_199324 | DKFZp434I0721 // HIN1 // HSHIN1 // KIAA1046 | 2788195 | chr4 | - | 146274170 | 146471942 | 10.88 |
| ring finger and SPRY domain containing 1 | RSPRY1 | NM_133368 | KIAA1972 | 3662612 | chr16 | + | 55777703 | 55831882 | 10.87 |
| leucine rich repeat containing 59 | LRRC59 | M_018509 | FLJ21675 // | 3762355 | chr17 | - | 45813504 | 45836593 | 10.85 |
| exosome component 2 | EXOSC2 | NM_014285 | RRP4 // Rrp4p // hRrp4p // p7 | 3191695 | chr9 | + | 132559000 | 132572797 | 10.83 |
| polo-like kinase 1 (Drosophila) | PLK1 | NM_005030 | PLK // STPK13 | 3653072 | chr16 | + | 23597664 | 23609180 | 10.82 |
| chromosome 20 open reading frame 24 | C20orf24 | NM_018840 // NM 199483 | PNAS-11 // RIP5 | 3883971 | chr20 | + | 34667571 | 34674368 | 10.81 |
| KIAA1984 // chromosome 9 open reading frame 86 | KIAA1984 // C9orf86 | NM_001039374 // NM_024718 | MGC15438 // PARF // RP11-216L13.7 // RP11-216L13.9 // bA216L13.7 // FLJ10101 // FLJ13045 // Parf // bA216L13.9 // pp8875 | 3194635 | chr9 | + | 138810077 | 138855460 | 10.81 |
| nucleolar protein 1, 120kDa | NOL1 | NM_001033714 // NM_006170 | MGC117384 // MGC149287 // MGC149288 // NOP120 // NSUN1 // p120 | 3442024 | chr12 | - | 6536290 | 6547817 | 10.79 |
| chromosome 3 open reading frame 17 | C3orf17 | NM_001025072 // NM_001025073 // NM_015412 | DKFZP434F2021 | 2688882 | chr3 | - | 113926517 | 114253449 | 10.73 |
| DOT1-like, histone H3 methyltransferase (S. cerevisiae) | DOT1L | NM_032482 | DOT1 // KIAA1814 | 3816264 | chr19 | + | 2114945 | 2183576 | 10.70 |
| dullard homology (Xenopus laevis) | DULLARD | NM_015343 | HSA011916 | 3743501 | chr17 | - | 7087644 | 7096514 | 10.70 |
| v-raf-1 murine leukemia viral oncogene homolog 1 // microtubule-associated protein 2 | RAF1 // MAP2 | NM_002880 // NM_001039538 // NM_002374 // NM_031845 // NM_031847 | CRAF // Raf-1 // c- Raf // DKFZp68612148 // MAP2A // MAP2B // MAP2C | 2663244 | chr3 | - | 12600117 | 12680654 | 10.61 |
| solute carrier family 39 (zinc transporter), member 7 // ring finger protein 1 | SLC39A7 // RING1 | NM_001077516 // NM_006979 // NM_002931 | D6S115E // D6S2244E // H2-KE4 // HKE4 // KE4 // RING5 // ZIP7 // RNF1 | 2903470 | chr6 | + | 33276220 | 33280187 | 10.55 |
| testis derived transcript (3 UM domains) | TES | NM_015641 // NM_152829 | DKFZP586B2022 // MGC1146 // TESS // TESS-2 // TESTIN | 3020192 | chr7 | + | 115637811 | 115743800 | 10.53 |
| chromosome 12 open reading frame 11 | C12orf11 | NM_0181 64 | FLJ10630 // FLJ10637 | 3448428 | chr12 | - | 26949277 | 26982501 | 10.50 |
| golgi associated PDZ and coiled-coil motif containing // v-ros UR2 sarcoma virus oncogene homolog 1 (avian) | GOPC // ROS1 | NM_001017408 // NM_020399 // NM_002944 | CAL // FIG // GOPC1 // PIST // dJ94G16.2 // MCF3 // ROS | 2971378 | chr6 | - | 117988142 | 118030384 | 10.49 |
| protein regulator of cytokinesis 1 // interFeron-related developmental regulator 1 | PRC1 // IFRD1 | NM_003981 // NM_199413 // NM_199414 // NM_001007245 // NM_001550 | ASE1 // MGC1671 // MGC3669 // PC4 // TIS7 | 3639031 | chr15 | - | 89310285 | 89338950 | 10.49 |
| RNA methyltransferase like 1 | RNMTL1 | NM_018146 | FLJ10581 // HC90 | 3705539 | chr17 | + | 632263 | 642481 | 10.45 |
| forkhead box 01A (rhabdomyosarcoma) | FOXO1A | NM_002015 | FKH1 // FKHR // FOXO1 | 3510858 | chr13 | - | 40027817 | 40162098 | 10.41 |
| mitochondrial ribosomal protein S7 | MRPS7 | NM_015971 | MRP-S // MRP-S7 // RP-S7 // RPMS7 | 3734760 | chr17 | + | 70769374 | 70774626 | 10.41 |
| suppressor of Ty 6 homolog (S. cerevisiae) | SUPT6H | NM_170 | K1AA0162 // MGC87943 // SPT6 // SPT6H // emb-5 | 3715703 | chr17 | + | 24013260 | 24053809 | 10.38 |
| splicing factor, arginine/serine-rich 1 (splicing factor 2, alternate splicing factor) | SFRS1 | NM_001078166 // NM_006924 | ASF // MGC5228 // SF2 // SF2p33 // SRp30a | 3764103 | chr17 | - | 53421412 | 53439635 | 10.37 |
| heterogeneous nuclear ribonucleoprotein M | HNRPM | NM_005968 // NM_31203 | DKFZp547H118 // HNRNPM // HNRNPM4 // HNRPM4 // HTGR1 | 3819543 | chr19 | + | 8415651 | 8459993 | 10.37 |
| neuroepithelial cell transforming gene 1 | NET1 | NM_01047160 // NM_005863 | ARHGEF8 // NET1A | 3233182 | chr10 | + | 5444535 | 5491001 | 10.35 |
| ribonucleotide reductase M2 polypeptide | RRM2 | NM_001034 | R2 // RR2M | 2469252 | chr2 | + | 10179982 | 10279478 | 10.35 |
| chromosome 16 open reading frame | C16orf80 | NM_013242 | EVORF // GTL3 | 3693511 | chr16 | - | 56683698 | 56739201 | 10.32 |
| 80 exosome component 4 | EXOSC4 | NM_019037 | FLJ20591 // RRP41 // RRP41A // Rrp4lp // SK16 // Ski6p // hRrp41p // p12A | 3120008 | chr8 | + | 145205527 | 145207529 | 10.30 |
| UTP6, small subunit (SSU) processome component, homolog (yeast) | UTP6 | NM_018428 | C17orf40 // HCA66 | 3752437 | chr17 | - | 27211153 | 27252839 | 10.30 |
| polymerase (RNA) I polypeptide C, 30kDa | POLR1C | NM_004875 // NM_203290 | RPA39 // RPA40 // RPA5 // RPAC1 | 2908052 | chr6 | + | 43592769 | 43605071 | 10.29 |
| centromere protein L | CENPL | NM_033319 | Clorf155 // CENP-L // FLJ31044 // RP3- 383J4.1 // dJ383J4.3 | 2444451 | chr1 | - | 172035313 | 172104622 | 10.28 |
| coronin actin binding protein, 1C | CORO1C | NM_014325 | HCRNN4 // coronin-3 | 3470549 | chr12 | - | 107563018 | 107697353 | 10.27 |
| ATP-binding cassette, sub-family F (GCN20), member 3 | ABCF3 | NM_018358 | EST201864 // FLJ11198 | 2655511 | chr3 | + | 185386580 | 185395316 | 10.26 |
| telomeric repeat binding factor 2, interacting protein | TERF2IP | NM_018975 | DRIP5 // RAP1 | 3669092 | chr16 | + | 74238853 | 74353272 | 10.25 |
| ADP-ribosylation factor interacting protein 2 (arfaptin 2) // solute carrier organic anion transporter family, member 6A1 | ARFIP2 // SLC06A1 | NM_012402 // NM_173488 | POR1 // GST // MGC26949 // OATP6A1 // OATPY | 3360941 | chr11 | - | 6453496 | 6459171 | 10.24 |
| RNA binding motif protein 25 | RBM25 | NM_021239 | MGC105088 // MGC117168 // RNPC7 // S164 | 3543411 | chr14 | + | 72594776 | 72658577 | 10.23 |
| zinc finger protein 410 | ZNF410 | NM_021188 | APA-1 // APA1 | 3543884 | chr14 | + | 73423093 | 73468718 | 10.23 |
| 3-hydroxy-3-methylglutaryl-Coenzyme A synthase 1 (soluble) | HMGCS1 | NM_002130 | HMGCS // MGC90332 | 2855501 | chr5 | - | 43324231 | 43349337 | 10.19 |
| transcription elongation regulator 1 | TCERG1 | NM_001040006 // NM_006706 | CA150 // MGC133200 // | 2834093 | chr5 | + | 145754916 | 145871713 | 10.18 |
| WD repeat domain 74 | WDR74 | XM_001125771 // NM_018093 | FLJ10439 // FLJ21730 | 3376235 | chr11 | - | 62356627 | 62365857 | 10.13 |
| sperm associated antigen 5 | SPAG5 | NM_006461 | DEEPEST // MAP126 // hMAP126 | 3750785 | chr17 | - | 23928719 | 23950424 | 10.12 |
| G protein-coupled receptor 126 | GPR126 | NM_001032394 // NM_001032395 // NM_020455 // NM_198569 | DREG // PS1TP2 // VIGR | 2928461 | chr6 | + | 142622079 | 142853981 | 10.05 |
| DnaJ-like protein // guanine nucleotide binding protein (G protein), gamma 10 | bA16L21.2.1 // GNG10 | NM_001015882 // NM_001017998 | - | 3184940 | chr9 | + | 113412172 | 113474576 | 10.05 |
| general transcription factor IIIC, polypeptide 4, 90kDa | GTF3C4 | NM_012204 | FLJ21002 // MGC138450 // TFIII90 // TFIIIC90 // TFIIICdelta // TFiiiC2-90 | 3192525 | chr9 | + | 134535243 | 134581784 | 10.00 |
| eukaryotic translation initiation factor 5 | EIF5 | NM_001969 // NM_183004 | EIF-5A | 3553607 | chr14 | + | 102743652 | 102881108 | 10.00 |
| programmed cell death 7 | PDCD7 | NM_005707 | ES18 // HES18 // MGC22015 | 3629475 | chr15 | - | 63196772 | 63213308 | 9.97 |
| dual specificity phosphatase 14 | DUSP14 | NM_007026 | MKP-L // MKP6 | 3719515 | chr17 | + | 32922795 | 32947707 | 9.97 |
| RAD18 homolog (S. cerevisiae) | RAD18 | NM_020165 | RNF73 | 2662020 | chr3 | - | 8792108 | 8980146 | 9.94 |
| myeloid cell leukemia sequence 1 (BCL2-related) | MCL1 | NM_021960 // NM_182763 | EAT // MCL1L // MCL1S // MGC104264 // | 2434438 | chr1 | - | 148799312 | 148818878 | 9.93 |
| oxysterol binding protein-like 2 | OSBPL2 | NM_014835 // NM_144498 | FLJ20223 // KIAA0772 // MGC4307 // MGC8342 // ORP-2 // ORP2 | 3892565 | chr20 | + | 60245389 | 60304834 | 9.91 |
| inhibitor of DNA binding 2B, dominant negative helix-loop-helix protein // inhibitor of DNA binding 2, dominant negative helix-loop-helix protein | ID2B // ID2 | NM_001039082 // NM_002166 | - // GIG8 // ID2A // ID2H // MGC26389 | 2468622 | chr2 | + | 8513132 | 8750492 | 9.91 |
| cytoskeleton associated protein 5 | CKAP5 | NM_001008938 // NM_014758 | FLJ35359 // KIAA0097 // TOG // TOGp // ch-TOG | 3371719 | chr11 | - | 46721250 | 46824660 | 9.90 |
| thymosin, beta 10 | TMSB10 | NM_021103 | MIG12 | 2491271 | chr2 | + | 84959319 | 84988131 | 9.85 |
| DEAD (Asp-Glu-Ala-Asp) box polypeptide 27 | DDX27 | NM_017895 | DKFZp667N057 // FLJ12917 // FLJ20596 // FLJ22238 // HSPC259 // MGC1018 // MGC163147 // PP3241 // RHLP // | 3888217 | chr20 | + | 47237780 | 47294013 | 9.83 |
| TAR DNA binding protein | TARDBP | NM_007375 | TDP-43 | 2320048 | chr1 | + | 10995025 | 11013170 | 9.82 |
| pyruvate dehydrogenase complex, component X // mitogen-activated protein kinase 10 | PDHX // MAPK10 | NM_003477 // NM_002753 // NM_138980 // NM_138981 // NM_138982 | DLDBP // E3BP // OPDX // PDX1 // proX // FLJ12099 // FLJ33785 // JNK3 // JNK3A // MGC50974 // PRKM10 // p493F12 // | 3326540 | chr11 | + | 34870003 | 34998695 | 9.81 |
| dihydrouridine synthase 3-like (S. cerevisiae) | DUS3L | NM_020175 | DUS3 // FLJ13896 | 3847420 | chr19 | - | 5735867 | 5759311 | 9.80 |
| splicing factor proline/glutamlne-rich (polypyrimidine tract binding protein associated) | SFPQ | NM_005066 | POMP100 // PSF | 2406064 | chr1 | - | 35351696 | 35506894 | 9.80 |
| phosphoinositlde-3-kinase, regulatory subunit 4, p150 | PIK3R4 | NM_014602 | MGC102700 // VPS15 // p150 | 2695200 | chr3 | - | 131801268 | 132026111 | 9.79 |
| RNA binding motif, single stranded interacting protein 2 | RBMS2 | NM_002898 | SCR3 | 3417583 | chr12 | + | 55202025 | 55277435 | 9.78 |
| eukaryotic translation initiation factor 2C, 2 | EIF2C2 | NM_012154 | AGO2 // MGC3183 //Q10 | 3156193 | chr8 | - | 141599440 | 141726037 | 9.74 |
| inner centromere protein antigens 135/155kDa | INCENP | NM_001040694 // NM_020238 | FLJ31633 // MGC111393 | 3333358 | chr11 | + | 61648046 | 61677757 | 9.73 |
| cofilin 1 (non-muscle) | CFL1 | NM_005507 | CFL | 3377886 | chr11 | - | 65347069 | 65386796 | 9.72 |
| heat shock 105kDa/110kDa protein 1 | HSPH1 | NM_006644 | DKFZp686M05240 // HSP105 // HSP105A // HSP105B // KIAA0201 // NY-CO- 25 | 3508330 | chr13 | - | 30558258 | 30651692 | 9.68 |
| nuclear import 7 homolog (S. cerevisiae) | NIP7 | NM_016101 | CGI-37 // FLJ10296 // HSPC031 // KD93 | 3666686 | chr16 | + | 67930869 | 67935044 | 9.64 |
| epiregulin | EREG | NM_001432 | ER | 2731513 | chr4 | + | 75440309 | 75473341 | 9.62 |
| mannosidase, alpha, class 1A, member 2 // urothelial cancer | MAN1A2 // UCA1 | NM_006699 // - | MAN1B //- | 2353881 | chr1 | + | 117689854 | 117931990 | 9.61 |
| tumor necrosis factor receptor superfamily, member 1A | TNFRSF1 A | NM_001065 | CD120a // FPF // MGC19588 // TBP1 // TNF-R // TNF-R-I // TNF-R55 // TNFAR // TNFRI // TNFR55 // TNFR60 // p55 // p55-R // | 3441849 | chr12 | - | 6308185 | 6321522 | 9.60 |
| cyclin L1 | CCNL1 | NM_020307 | BM-001 // PR01073 // ania-6a | 2702307 | chr3 | - | 158305077 | 158361233 | 9.60 |
| RNA binding motif protein 4 | RBM4 | NM_002896 | DKFZp547K0918 // LARK // MGC75138 // RBM4A // ZCCHC21 // ZCRB3A | 3336422 | chr11 | + | 66161951 | 66190547 | 9.60 |
| KIAA1429 | KIAA1429 | NM_015496 // NM_183009 | DKFZP4341.116 // DKFZp781B2117 // MGC138493 // MGC141940 // MSTP054 | 3145020 | chr8 | - | 95569109 | 95634851 | 9.57 |
| nucleoporin 153kDa | NUP153 | NM 005124 | HNUP153 // N153 | 2943808 | chr6 | - | 17723255 | 17855638 | 9.56 |
| zinc finger protein 91 homolog (mouse) // ciliary neurotrophic factor | ZFP91 // CNTF | NM_053023 // NM_170768 // NM_000614 | FK5G11 // PZF // ZNF757 // HCNTF | 3331730 | chr11 | + | 58101868 | 58149782 | 9.54 |
| tetraspanin 5 | TSPAN5 | NM_005723 | NET-4 // TM4SF9 // TSPAN-5 | 2778856 | chr4 | - | 99589201 | 99798839 | 9.53 |
| resistance to inhibitors of cholinesterase 8 homolog A (C. etegans) | RIC8A | NM_021932 | MGC104517 // MGC131931 // MQC148073 // MGC148074 // RIOS // synembryn | 3315512 | char11 | + | 197139 | 206202 | 9.52 |
| splicing factor 3b, subunit 1, 155kDa | SF3B1 | NM_001005526 // NM_012433 | PRP10 // PRPF10 // SAP155 // SF3b155 | 2593670 | chr2 | - | 197962756 | 198039327 | 9.52 |
| DEAD (Asp-Glu-Ala-Asp) box polypeptide 47 // apolipoprotein L domain containing 1 | DDX47 // APOLD1 | NM_016355 // NM_201224 // NM_030817 | DKFZp5640176 // E4-DBP // FLJ30012 // HQ0256 // MSTP162 // DKFZP434F0318 // | 3405531 | chrl2 | + | 12856462 | 12874176 | 9.51 |
| tensin 4 | TNS4 | NM_032865 | CTEN // FLJ14950 // PP14434 | 3756262 | chr17 | - | 35881491 | 35911365 | 9.51 |
| heterogeneous nuclear ribonucleoprotein D (AU-rich element RNA binding protein 1, 37kDa) | HNRPD | NM_001003810 // NM_002138 // NM_031369 // NM 031370 | AUF1 // AUF1A // P37 // hnRNPD0 | 2775463 | chr4 | - | 83492676 | 83515077 | 9.50 |
| v-yes-1 Yamaguchi sarcoma viral oncogene homolog 1 | YES1 | NM_005433 | HsT441 // P61-YES // Yes // c-yes | 3795942 | chr18 | - | 711592 | 803236 | 9.48 |
| nuclear factor (erythroid-derived 2)-like 2 // hypothetical protein DKFZp451M2119 | NFE2L2 // DKFZp451M 2119 | NM_006164// NM_182585 | NRF2 // - | 2588827 | chr2 | - | 177794809 | 177837753 | 9.48 |
| eukaryotic translation initiation factor 4A, isoform 1 // SUMO1/sentrin/SMT3 specific peptidase 3 | EIF4A1 // SENP3 | NM_001416 // NM_015670 | DDX2A // EIF-4A // EIF4A // DKFZP586K0919 // DKFZp762A152 // SMT3IP1 // SSP3 | 3708826 | chr17 | + | 7416768 | 7423040 | 9.46 |
| nucleolar protein 11 | NOL11 | NM_015462 | DKFZP586L0724 | 3732373 | chr17 | + | 63144526 | 63205942 | 9.45 |
| adenylate kinase 2 | AK2 | NM_001625 // NM_013411 | ADK2 | 2405364 | chr1 | - | 33245959 | 33275155 | 9.45 |
| splicing factor, arginine/serine-rich 5 | SFRS5 | NM_001039465 // NM_006925 | HRS // SRP40 | 3542207 | chr14 | + | 69263363 | 69308465 | 9.42 |
| ancient ubiquitous protein 1 // DEAQ box polypeptide 1 (RNA-dependent ATPase) | AUP1 // DQX1 | NM_181575 // NM_133637 | - // FLJ23757 | 2560254 | chr2 | - | 74607294 | 74610455 | 9.38 |
| AF4/FMR2 family, member 4 | AFF4 | NM_014423 | AF5Q31 // MCEF // MGC75036 | 2875555 | chr5 | - | 132238768 | 132327205 | 9.38 |
| guanine nucleotide binding protein (G protein), beta polypeptide 2-like 1 | GNB2L1 | NM_006098 | Gnb2-rs1 // H12.3 // HLC-7 // PIG21 // RACK1 | 2891052 | chr5 | - | 180596592 | 180612024 | 9.38 |
| survival motor neuron domain containing 1 | SMNDC1 | NM_005871 | SMNR // SPF30 | 3306516 | chr10 | - | 112042540 | 112106248 | 9.38 |
| BUB1 budding uninhibited by benzimidazoles 1 homolog (yeast) | BUB1 | NM_004336 | BUB1A // BUB1L // hBUB1 | 2570616 | chr2 | - | 111088456 | 111174950 | 9.36 |
| SET domain containing 5 | SETD5 | XM_926615 // XM_931376 // XM_931417 // XM_931444 // XM_931447 11 XM_931455 // XM_931478 // XM_939712 // XM_944260 // XM_944276 // XM_944280 // XM_944295 // XM_944298 // XM_944303 // NM_001080517 | DKFZp686J18276 // FLJ10707 // K1AA1 1757 | 2609608 | chr3 | + | 9414309 | 9495916 | 9.36 |
| heat shock protein 90kDa beta (Grp94), member 1 // thymine-DNA glycosylase // heat shock protein 90kDa beta (Grp94), member 2 (pseudogene) | HSP90B1 // TDG // HSP90B2P | NM_003299 // NM_003211 // - | ECGP // GP96 // GRP94 // TRA1 // - // GRP94P1 // GRP94b // HSP // HSPCP2 // HsGrp94b // TRA1P1 // TRAP1 | 3429312 | chr12 | + | 102848291 | 102871551 | 9.34 |
| actin, gamma 1 | ACTG1 | NM_001614 | ACT // ACTG // DFNA20 // DFNA26 | 3773932 | chr17 | - | 77091456 | 77105797 | 9.32 |
| dual specificity phosphatase 6 | DUSP6 | NM_001946 // NM_022652 | MKP3 // PYST1 | 3464860 | chr12 | - | 88223376 | 88273467 | 9.31 |
| LATS, large tumor suppressor, homolog 2 (Drosophila) | LATS2 | NM_014572 | FLJ13161 // KPM | 3504526 | chr13 | - | 20445178 | 20533718 | 9.28 |
| SERPINE1 mRNA binding protein 1 | SERBP1 | NM_001018067 // NM_001018068 // NM_001018069 // NM_015640 | CGI-55 // CHD3IP // DKFZp564M2423 // FLJ90489 // HABP4L // PAI-RBP1 // PAIRBP1 | 2417174 | chr1 | - | 67646101 | 67669020 | 9.26 |
| DnaJ (Hsp40) homolog, subfamily A, member 3 | DNAJA3 | NM_005147 | FLJ45758 // TID1 // hTid-1 | 3646164 | chr16 | + | 4415465 | 4446773 | 9.23 |
| nuclear cap binding protein subunit 2, 20kDa | NCBP2 | NM_001042540 // NM_007362 | CBC2 // CBP20 // NIP1 // PIG55 | 2713074 | chr3 | - | 198146677 | 198154763 | 9.23 |
| TRK-fused gene | TFG | NM_001007565 // NM 006070 | FLJ36137 // TF6 // TRKT3 | 2633773 | chr3 | + | 101910681 | 101950800 | 9.22 |
| LIM domain 7 | LMO7 | NM_005358 | FBX20 // FBX020 // KIAA0858 // LOMP | 3494137 | chr13 | + | 75092571 | 75566974 | 9.22 |
| KIAA0090 | KIAA0090 | NM_015047 | RP1-43E13.1 | 2399620 | chr1 | - | 19413665 | 19450633 | 9.22 |
| RNA binding motif protein 23 // chromosome 9 open reading frame 102 | RBM23 // C9orf102 | NM_001077351 // NM_001077352 // NM_018107 // NM_001034155 // NM_020207 | CAPERbeta // FLJ10482 // MGC4458 // PP239 // RNPC4 // FLJ37706 // SR278 | 3556888 | chr14 | - | 22439714 | 22458231 | 9.18 |
| suppressor of Ty 7 (S. cerevisiae)-like | SUPT7L | NM_014860 | ART1 // KLAA0764 // MGC90306 // SPT7L // STAF65 // STAF65(gamma) | 2546008 | chr2 | - | 27718957 | 27740160 | 9.15 |
| transforming, acidic coiled-coil containing protein 3 | TACC3 | NM_006342 | ERIC1 // MGC117382 // MGC133242 | 2714955 | chr4 | + | 1692616 | 1716693 | 9.12 |
| signal transducing adaptor molecule (SH3 domain and ITAM motif) 1 | STAM | NM_003473 | DKFZp686J2352 // STAM1 | 3237088 | chr10 | + | 17726140 | 17798809 | 9.11 |
| neurotensin receptor 1 (high affinity) | NTSR1 | NM_002531 | NTR | 3892873 | chr20 | + | 60810575 | 60864568 | 9.10 |
| sorting nexin associated golgi protein 1 | SNAG1 | NM_052870 | MGC150827 // MGC150829 // SH3PX2 // SH3PXD3B // SNX18 | 2809628 | chr5 | + | 53849348 | 53878172 | 9.07 |
| DEAD (Asp-Glu-Ala-Asp) box polypeptide 42 | DDX42 | NM_007372 // NM_203499 | FLJ43179 // RHELP // RNAHP // | 3730899 | chr17 | + | 59204975 | 59250510 | 9.05 |
| adhesion regulating molecule 1 | ADRM1 | NM_007002 // NM_175573 | GP110 // MGC29536 // Rpn13 | 3892607 | chr20 | + | 60305485 | 60317305 | 9.05 |
| dyskeratosis congenita 1, dyskerin | DKC1 | NM_001363 | DKC // NAP57 // NOLA4 // XAP101 // dyskerin | 3996667 | chrX | + | 153644243 | 153659150 | 9.04 |
| GNAS complex locus | GNAS | NM_000516 // NM_001077468 // NM_001077489 // NM_001077490 // NM_016592 // NM_080425 // NM_080426 // NR_003259 | AHO // C20orf45 // GNAS 1 // GPSA // GSA // GSP // MGC33735 // PHP1A // PHP1B // POH // dJ309F20.1.1 // dJ806M20.3.3 | 3891163 | chr20 | + | 56848165 | 56919604 | 9.04 |
| protein inhibitor of activated STAT, 1 | PIAS1 | M_016166 | DDXBP1 // GBP // GU/RH-II // MGC141878 // MGC141879 // ZMIZ3 | 3599280 | chr15 | + | 65898917 | 66277975 | 9.03 |
| WD repeat domain 36 | WDR36 | NM_139281 | DKFZp68611650 // GLC1G // TA-WDRP // TAWDRP // | 2823820 | chr5 | + | 110455769 | 110517812 | 9.03 |
| tripartite motif-containing 44 | TRIM44 | NM_017583 | DIPB // HSA249128 // MC7 // MGC3490 | 3326842 | chr11 | + | 35600246 | 35826001 | 9.03 |
| transforming growth factor beta regulator 4 | TBRG4 | NM_004749 // NM_030900 // NM_199122 | CPR2 // FASTKD4 // KIAA0948 | 3049025 | chr7 | - | 45103738 | 45128368 | 9.01 |
| guanine nucleotide binding protein-like 3 (nucleolar) | GNL3 | NM_014366 // NM_206825 // NM 206826 | C77032 // E21G3 // MGC800 // NS | 2624074 | chr3 | + | 52694976 | 52705212 | 9.00 |
| BCL2-associated transcription factor 1 | BCLAF1 | NM_001077440 // NM_001077441 // NM_014739 | BTF // KIAA0164 // bK211L9.1 | 2975680 | chr6 | - | 136619703 | 136652847 | 8.99 |
| cyclin L2 // aurora kinase A interacting protein 1 | CCNL2 // AURKAIP1 | NM_001039577 // NM_030937 // NM_017900 | ANIA-6B // DKFZp761A1210 // DKFZp7620195 // HCLA-ISO // HLA- ISO // PCEE // SB138 // AIP // AKIP // FLJ20608 | 4041923 | chr1_ran dom | + | 359569 | 372958 | 8.99 |
| cirrhosis, autosomal recessive 1A (cirhin) | CIRH1A | NM_032830 | CIRHIN // FLJ14728 // KIAA1988 // NAIC // TEX292 | 3666566 | chr16 | + | 67722728 | 67761072 | 8.98 |
| SMAD family member 3 | SMAD3 | NM_005902 | DKFZP586N0721 // DKFZp686J10186 // HSPC193// HsT17436 // JV15-2 // MADH3 // MGC60396 // Smad 3 | 3598959 | chr15 | + | 65145043 | 65296818 | 8.97 |
| proteasome (prosome, macropain) subunit, beta type, 7 | PSMB7 | NM_002799 | Z | 3225003 | chr9 | - | 126155580 | 126223284 | 8.97 |
| cyclin L2 // aurora kinase A interacting protein 1 | CCNL2 // AURKAIP1 | NM_001039577 // NM_030937 // NM_017900 | ANIA-6B // DKFZp761A1210 // DKFZp7620195 // HCLA-ISO // HLA- ISO // PCEE // SB138 // AIP // AKIP // FLJ20608 | 2391532 | chr1 | - | 1314609 | 1324575 | 8.96 |
| mediator of RNA polymerase II transcription, subunit 28 homolog (S. cerevisiae) | MED28 | NM_025205 | 1500003D12Rik // DKFZP434N185 // EG1 // magicin | 2720181 | chr4 | + | 17225344 | 17244808 | 8.95 |
| Rho GTPase activating protein 12 | ARHGAP12 | N_018287 | DKFZp779N2050 // FLJ10971 // FLJ20737 // FLJ21785 // FLJ45709 | 3283920 | chr10 | - | 32134245 | 32261226 | 8.93 |
| proteasome (prosome, macropain) 26S subunit, non-ATPase, 7 (Mov34 homolog) | PSMD7 | NM_002811 | MOV34 // P40 // S12 | 3668617 | chr16 | + | 72888182 | 72901528 | 8.92 |
| hypothetical protein MGC10433 | MGC10433 | NM_024321 | - | 3830612 | chr19 | + | 40811767 | 40820425 | 8.91 |
| ubiquitin associated protein 2-like | UBAP2L | NM_014847 | FLJ42300 // KIAA0144 // NICE-4 | 2360083 | chr1 | + | 152459139 | 152510701 | 8.91 |
| solute carrier family 20 (phosphate transporter), member 1 | SLC20A1 | NM_005415 | DKFZp686J2397 // FLJ41426 // GLVR1 // Glvr-1 // PIT1 // PiT-1 | 2500919 | chr2 | + | 113117773 | 113137861 | 8.90 |
| protein phosphatase 1, regulatory (inhibitor) subunit 10 | PPP1R10 | NM_002714 | CAT53 // FB19 // PNUTS | 2948425 | chr6 | - | 30676177 | 30694348 | 8.87 |
| SAP30 binding protein // cardiotrophin-like cytokine factor 1 | SAP30BP // CLCF1 | NM_013260 // NM_013246 | DKFZp586L2022 // HCNGP // HTRG // HTRP // BSF3 // CISS2 // CLC // NNT1 // NR6 | 3735107 | chr17 | + | 71174801 | 71215729 | 8.86 |
| ATP-binding cassette, sub-family E (OABP), member 1 | ABCE1 | NM_001040876 // NM_002940 | ABC38 // OABP // RU // RNASEL1 // RNASELI // RNS41 | 2746024 | chr4 | + | 146238626 | 146270126 | 8.86 |
| BTAF1 RNA polymerase II, B-TFIID transcription factor-associated, 170kDa (Mot1 homolog, S. cerevisiae) | BTAF1 | NM_003972 | KLAA0940 // MGC138406 // MOT1 // TAF(II)170 // TAF172 // TAFII170 | 3257938 | chr10 | + | 93673509 | 93821994 | 8.84 |
| syntaxin 5 | STX5 | XM_001128716 // NM_003164 | SED5 // STX5A | 3376193 | chr11 | - | 62330946 | 62356126 | 8.84 |
| cyclin A2 | CCNA2 | NM_001237 | CCN1 // CCNA | 2784113 | chr4 | - | 122956335 | 122964516 | 8.81 |
| integrator complex subunit 7 | INTS7 | NM_015434 | C1 orf73 // DKFZP434B168 // INT7 | 2454532 | chr1 | - | 210180312 | 210275613 | 8.81 |
| regulator of chromosome condensation 1 // small nucleolar RNA host gene (non-protein coding) 3 | RCC1 // SNHG3 | NM_001048194 // NM_001048195 // NM_001269 // NR_002909 | CHC1 // RCC1-I // RNU17C // RNU17D // U17HG // U17HG- A | 2327482 | chr1 | + | 28705062 | 28802493 | 8.81 |
| LEM domain containing 3 | LEMD3 | M_014319 | MAN1 | 3420079 | chr12 | + | 63806051 | 63928317 | 8.80 |
| upstream binding protein 1 (LBP-1 a) | UBP1 | NM_014517 | DKFZp686L1745 // LBP-1B // LBP-1a // LBP1A // LBP1B | 2668351 | chr3 | - | 33404835 | 33456874 | 8.80 |
| zinc finger protein 384 | ZNF384 | NM_001039916 // NM_001039917 // NM_001039918 // NM_001039919 // NM_001039920 // NM_133476 | CAGH1 // CAGH1A // CIZ // ERDA2 // NMP4 // NP // TNRC1 | 3442205 | chr12 | - | 6645924 | 6668961 | 8.79 |
| caveolin 2 | CAV2 | NM_001233 // NM_198212 | CAV // MGC12294 | 3020273 | chr7 | + | 115926553 | 115935830 | 8.78 |
| actin, beta | ACTB | NM_001101 | PS1TP5BP1 | 3036924 | chr7 | - | 5533433 | 5537011 | 8.77 |
| acyl-Coenzyme A binding domain containing 3 | ACBD3 | NM_022735 | GCP60 // GOCAP1 // GOLPH1 // PAP7 | 2458701 | chr1 | - | 224398953 | 224463924 | 8.76 |
| eukaryotic translation initiation factor 3, subunit 10 theta, 150/170kDa | EIF3S10 | NM_003750 | EIF3 // EIF3A // KIAA0139 // P167 // eIF3-p170 // eIF3- theta // p180 // p185 | 3309215 | chr10 | - | 120621551 | 120830522 | 8.75 |
| CDC42 small effector 2 | CDC42SE2 | NM_001038702 // NM_020240 | FLJ21967 // SPEC2 | 2828146 | chr5 | + | 130616639 | 130781182 | 8.75 |
| zinc finger, AN1-type domain 2B | ZFAND2B | NM 138802 | - | 2528308 | chr2 | + | 219779769 | 219782603 | 8.72 |
| ribosomal protein S20 | RPS20 | NM_00023 | FLJ27451 // MGC102930 | 3136129 | chr8 | - | 57139946 | 57185891 | 8.72 |
| exportin 1 (CRM1 homolog, yeast) // thyroid hormone receptor, beta (erythroblastic leukemia viral (v-erb- a) oncogene homolog 2, avian) | XPO1 // THRB | NM_003400 // NM_000461 | CRM1 // DKFZp686B1823 // ERBA-BETA // ERBA2 // GRTH // MGC126109 // MGC126110 // NR1A2 // THR1 // THRB1 // THRB2 | 2555490 | chr2 | - | 61558490 | 61619343 | 8.71 |
| D4, zinc and double PHD fingers family 2 | DPF2 | NM_006268 | MGC10180 // REQ // UBID4 // ubi-d4 | 3335089 | chur11 | + | 64857703 | 64877276 | 8.71 |
| basic leucine zipper and W2 domains 1 | BZW1 | XM_001126385 // NM_014670 | BZAP45 // KIAA0005 // Nbla10236 | 2522439 | chr2 | + | 201384456 | 201418386 | 8.71 |
| pogo transposable element with ZNF domain | POGZ | NM_015100 // NM_145796 // NM_207171 | KIAA0461 // MGC71543 // SUHW5 // ZNF635 | 2435044 | chr1 | - | 149641830 | 149753035 | 8.70 |
| basic helix-loop-helix domain containing, class B, 3 | BHLHB3 | NM_030762 | DEC2 // SHARP-1 // SHARP1 | 3448088 | chr12 | - | 26141435 | 26179615 | 8.69 |
| family with sequence similarity 83, member D | FAM83D | NM_030919 | C20orf129 // FLJ38341 // dJ616B8.3 | 3884892 | chr20 | + | 36968369 | 37015223 | 8.69 |
| thioredoxin domain containing 14 // catenin (cadherin-associated protein), delta 1 | TXNDC14 // CTNND1 | NM_015959 // XM_001126721 // XM_001126756 // XM_001126767 // XM_001126781 // XM_001126793 // XM_001126823 // NM 001331 | CGl-31 // DKFZp781O202 // MGC111151 // PIG26 // TMX2 // CAS // CTNND // KIAA0384 // P120CAS // P120CTN // p120 | 3331487 | chr11 | + | 57235839 | 57391291 | 8.68 |
| thymopoietin | TMPO | NM_001032283 // NM_001032284 // NM 003276 | CMD1T // LAP2 // MGC61508 // PRO0868 // TP | 3427767 | chr12 | + | 97395927 | 97502543 | 8.68 |
| jumonji, AT rich interactive domain 1A | JARID1A | NM_001042603 // NM_005056 | RBBP2 // RBP2 | 3439603 | chr12 | - | 259504 | 368944 | 8.67 |
| sorting nexin 19 | SNX19 | NM_014758 | CHET8 // DKFZp667I205 // KIAA0254 | 3398482 | chr11 | - | 130250320 | 130291615 | 8.67 |
| Der1-like domain family, member 1 | DERL1 | NM_024295 | DER-1 // DER1 // FLJ13784 // FLJ42092 // MGC3067 // | 3151401 | chr8 | - | 124094605 | 124123781 | 8.67 |
| eukaryotic translation initiation factor 3, subunit 9 eta, 116kDa | EIF3S9 | NM_001037283 // NM_003751 | EIF3-ETA // EIF3- P110 // EIF3-P116 // MGC104664 // MGC131875 // PRT1 // eIF3b | 2987441 | chr7 | + | 2315929 | 2386896 | 8.66 |
| WDR45-like | WDR45L | NM_019613 | WIPI-3 // WIPI3 | 3775157 | chr17 | - | 78165748 | 78199803 | 8.65 |
| conserved helix-loop-helix ubiquitous kinase | CHUK | NM_001278 | IKBKA // IKK-alpha // IKK1 // IKKA // NFKBIKA // TCF1 6 | 3303300 | chr10 | - | 101938115 | 101979345 | 8.62 |
| WD repeat domain 77 // oviductal glycoprotein 1, 120kDa (mucin 9, oviductin) | WDR77 // OVGP1 | NM_024102 // NM_002557 | HKMT1069 // MEP50 // MGC2722 // Nubia10071 // RP11- 552M11.3 // CHIT5 // EGP // MUC9 // | 2427930 | chr1 | - | 111782351 | 111793512 | 8.60 |
| Paf1, RNA polymerase II associated factor, homolog (S. cerevisiae) | PAF1 | NM_019088 | F23149₋1 // FLJ11123 // PD2 | 3861978 | chr19 | - | 44568113 | 44592162 | 8.60 |
| kinesin family member 23 | KIF23 | NM_004856 // NM_138555 | CHO1 // KNSL5 // MKLP-1 // MKLP1 | 3599811 | chr15 | + | 67493677 | 67527808 | 8.60 |
| BMS1-like, ribosome assembly protein (yeast) // tetratricopeptide repeat domain 7B | BMS1L // TTC7B | NM_014753 // NM_001010854 | KIAA0187 // TTC7L1 // c14_5685 | 3243708 | chr10 | + | 42597978 | 42682443 | 8.58 |
| insulin induced gene 2 | INSIG2 | NM_016133 | MGC26273 | 2502424 | chr2 | + | 118558758 | 118593436 | 8.57 |
| ubiquitin specific peptidase 9, X-linked // ubiquitin specific peptidase 9, Y-linked (fat facets-like, | USP9X // USP9Y | NM_001039590 // NM_001039591 // NM_004654 | DFFRX // FAF // AZF // AZFA // DFFRY // SP3 | 3974708 | chrX | + | 40829542 | 40980776 | 8.57 |
| squamous cell carcinoma antigen recognized by T cells 3 | SART3 | NM_014706 | KIAA0156 // MGC138188 // RP11- 13G14 // TIP110 // p110(nrb) | 3470253 | chr12 | - | 107440140 | 107479296 | 8.57 |
| LIM domain only 4 | LMO4 | NM_006769 | - | 2345286 | chr1 | + | 87458333 | 87588817 | 8.56 |
| peptidylprolyl isomerase G (cyclophilin G) | PPIG | NM_004792 | CARS-Cyp // CYP // MGC133241 // | 2514441 | chr2 | + | 170149096 | 170206156 | 8.55 |
| guanine nucleotide binding protein (G protein), q polypeptide | GNAQ | NM_002072 | G-ALPHA-q // GAQ | 3210808 | chr9 | - | 79475043 | 79836455 | 8.53 |
| TSC22 domain family, member 1 | TSC22D1 | NM_006022 // NM_183422 | DKFZp686019206 // MGC17597 // RP11- 269C23.2 // TGFB114 // TSC22 | 3512294 | chr13 | - | 43905532 | 44202063 | 8.53 |
| serine/arginine repetitive matrix 2 | SRRM2 | NM_016333 | 300-KD // DKFZp686015166 // FLJ21926 // FLJ22250 // KIAA0324 // MGC40295 // SRL300 // SRm300 | 3645253 | chr16 | + | 2742650 | 2761908 | 8.52 |
| methionine adenosyltransferase II, alpha | MAT2A | NM_005911 | MATA2 // MATII // SAMS2 | 2491615 | chr2 | + | 85597809 | 85625908 | 8.51 |
| ubinuclein 1 | UBN1 | NM_001079514 // NM_016936 | VT // VT4 | 3646434 | chr16 | + | 4836677 | 4872358 | 8.51 |
| solute carrier family 38, member 2 | SLC38A2 | NM_018976 | ATA2 // KIAA1382 // PRO1068 // SAT2 // SNAT2 | 3452323 | chr12 | - | 45038242 | 45132603 | 8.50 |
| GrpE-like 1, mitochondrial (E. coli) | GRPEL1 | NM 025196 | FLJ25609 // HMGE | 2759404 | chr4 | - | 7107908 | 7187815 | 8.48 |
| BAT2 domain containing 1 | BAT2D1 | NM_015172 | BAT2-iso // XTP2 | 2367154 | chr1 | + | 169716078 | 169829262 | 8.48 |
| eukaryotic translation initiation factor 3, subunit 7 zeta, 66/67kDa | EIF3S7 | NM_003753 | MGC126526 // MGC17258 // eIF3- p66 // eIF3-zeta // eIF3d | 3959631 | chr22 | - | 35236857 | 35255429 | 8.48 |
| polymerase (DNA directed) sigma | POLS | NM_006999 | LAK-1 // POLK // TRF4 // TRF4-1 | 2800503 | chr5 | + | 6736994 | 6893446 | 8.47 |
| interleukin enhancer binding factor 2, 45kDa | ILF2 | NM_004515 | MGC8391 // NF45 // PRO3063 | 2436132 | chr1 | - | 151900372 | 151910103 | 8.46 |
| chromosome X open reading frame | CXorf39 | NM_207318 | | 3985866 | chrX | + | 103297819 | 103323642 | 8.45 |
| lysosomal-associated membrane protein 1 | LAMP1 | NM_005561 | CD107a // LAMPA // LGP120 | 3502570 | chr13 | + | 112999460 | 113025981 | 8.45 |
| YTH domain containing 1 | YTHDC1 | NM_001031732 // NMJ_33370 | KIAA1966 // YT521 // YT521-B | 2772017 | chr4 | - | 68858700 | 68934802 | 8.45 |
| RAD21 homolog (S. pombe) | RAD21 | NM_006265 | FLJ25655 // FLJ40596 // HR21 // HRAD21 // KIA0078 // MCD1 // NXP1 // SCC1 // hHR21 | 3149843 | chr8 | - | 117909861 | 117956284 | 8.45 |
| TATA box binding protein | TBP | NM_003194 | GTF2D // GTF2D1 // MGC117320 // MGC126054 // MGC126055 // SCA17 // TFIID | 2937984 | chr6 | + | 170705200 | 170723945 | 8.44 |
| Rho GTPase activating protein 29 | ARHGAP29 | NM_004815 | PARG1 // RP11- 255E17.1 | 2423829 | chr1 | - | 94409906 | 94549874 | 8.43 |
| PRP4 pre-mRNA processing factor 4 homolog (yeast) | PRPF4 | NM_004697 | HPRP4 // HPRP4P // PRP4 // Prp4p | 3185558 | chr9 | + | 115077230 | 115095267 | 8.42 |
| myosin phosphatase-Rho interacting protein | M-RIP // C17orf84 | NM_015134 // NM_201274 | KIAA0864 // RHOIP3 // p116Rip | 3712363 | chr17 | + | 16886542 | 17062286 | 8.42 |
| E2F transcription factor 4, p107/p130-binding | E2F4 | NM_001950 | E2F-4 | 3665288 | chr16 | + | 65783266 | 65790299 | 8.42 |
| glutamine-rich 1 | QRICH1 | NM_017730 // NM 198880 | FLJ20259 // MGC131838 | 2673902 | chr3 | - | 49042148 | 49106498 | 8.42 |
| annexin A1 | ANXA1 | NM_000700 | ANX1 // LPC1 | 3174816 | chr9 | + | 74914324 | 74983394 | 8.41 |
| serine/threonine kinase 4 | STK4 | NM_006282 | DKFZp686A2068 // KRS2 // MST1 // YSK3 | 3886704 | chr20 | + | 43028534 | 43142041 | 8.38 |
| chromosome X open reading frame 15 | CXorf15 | NM_018360 | FIAT // FLJ11209 // LSR5 // MGC126621 // MGC126625 | 3970166 | chrX | + | 16714433 | 16772561 | 8.36 |
| transportin 2 (importin 3, karyopherin beta 2b) | TNPO2 | NM_013433 | FLJ12155 // IPO3 // KPNB2B // TRN2 | 3851651 | chr19 | - | 12670912 | 12695776 | 8.35 |
| pleckstrin homology-like domain, family B, member 1 | PHLDB1 | NM_015157 | DKFZp686H039 // DKFZp686024210 // FLJ00141 // FLJ90266 // KIAA0638 // LL5A // MGC111531 | 3351564 | chr11 | + | 117982385 | 118033939 | 8.34 |
| tumor necrosis factor receptor superfamily, member 12A | TNFRSF12A | NM_016639 | CD266 // FN14 // TWEAKR | 3645555 | chr16 | + | 3010334 | 3012380 | 8.34 |
| SLC7A5 pseudogene | LAT1-3TM // IMAA | NR_002593 // NR_002594 | DC49 // MLAS // hLAT1-3TM // MMAA | 3684100 | chr16 | - | 21320984 | 22449842 | 8.33 |
| CD3e molecule, epsilon associated protein | CD3EAP | NM_012099 | ASE-1 // CAST // MGC118851 // PAF49 | 3836243 | chr19 | + | 50597660 | 50605831 | 8.33 |
| kinesin family member 2C | KIF2C | NM_006845 | KNSL6 // MCAK | 2334098 | chr1 | + | 44978097 | 45005994 | 8.33 |
| CD55 molecule, decay accelerating factor for complement (Cromer blood group) | CD55 | NM_000574 | CR // DAF // TC | 2377229 | chr1 | + | 205561488 | 205607671 | 8.32 |
| aldolase A, fructose-bisphosphate | ALDOA | NM_000034 // NM_184041 // NM_184043 | ALDA // MGC10942 // MGC17716 // MGC17767 | 3655920 | chr16 | + | 29968869 | 29991009 | 8.31 |
| denticleless homolog (Drosophila) | DTL | NM_016448 | CDT2 // DCAF2 // L2DTL // RAMP | 2378937 | chr1 | + | 210275565 | 210399046 | 8.30 |
| adhesion molecule with Ig-like domain 2 | AMIGO2 | NM_181847 | ALI1 // DEGA | 3452478 | chr12 | - | 45726359 | 45874177 | 8.30 |
| ST3 beta-galactoside alpha-2,3-sialyltransferase 1 | ST3GAL1 | NM_003033 // NM_173344 | Gal-NAc6S // MGC9183 // SIAT4A // SIATFL // ST3GalA // ST3GalA.1 // | 3154398 | chr8 | - | 134535811 | 134653449 | 8.29 |
| tripartite motif-containing 8 | TRIM8 | NM_030912 | GERP // RNF27 | 3261820 | chr10 | + | 104384137 | 104419190 | 8.29 |
| fibroblast growth factor (acidic) intracellular binding protein | FIBP | NM_004214 // NM 198897 | FGF1BP // FIBP-1 | 3377964 | chr11 | - | 65407787 | 65412586 | 8.29 |
| PAP associated domain containing 1 // collagen, type XI, alpha 1 | PAPD1 // COL11A1 | NM_018109 // NM_001854 // NM_080629 // NM 080630 | FLJ10486 // RP11- 305E6.3 // mtPAP // CO11A1 // COLL6 // STL2 | 3283378 | chr10 | - | 30511316 | 30764402 | 8.26 |
| exosome component 10 | EXOSC10 | NM_001001998 // NM_002685 | PM-Scl // PM/Scl- 100 // PMSCL // PMSCL2 // RRP6 // Rrp6p // p2 // p3 // p4 | 2396480 | chr1 | - | 11049173 | 11082811 | 8.26 |
| B-cell CLL/lymphoma 9-like | BCL9L | NM 182557 | BCL9-2 // DLNB11 | 3393993 | chr11 | - | 118272076 | 118304947 | 8.23 |
| 1-acylglycerol-3-phosphate O-acyltransferase 5 (lysophosphatidic acid acyltransferase, epsilon) | AGPAT5 | NM_018361 | 1-AGPAT // LPAAT-e // LPAAT- epsilon | 3083936 | chr8 | + | 6553307 | 6633056 | 8.22 |
| chaperonin containing TCP1, subunit 6A (zeta 1) | CCT6A | NM_001009186 // NM_001762 | CCT-zeta // CCT- zeta-1 // CCT6 // Cctz // HTR3 // MGC126214 // MGC126215 // MoDP-2 // TCP-1- zeta // TCP20 // TCPZ // TTCP20 | 3003193 | chr7 | + | 56086894 | 56099148 | 8.22 |
| jumonji, AT rich interactive domain 1B | JARID1B | NM_006618 | FLJ10538 // FLJ12459 // FLJ12491 // FLJ16281 // FLJ23670 // PLU-1 // PUT1 // RBBP2H1A | 2451309 | chr1 | - | 200963111 | 201048571 | 8.22 |
| solute carrier family 25, member 37 | SLC25A37 | XM_001128238 // XM_001128248 // XM_001128255 // XM_001128269 // NM_016612 | HT015 // MFRN // MSC // MSCP // PRO1278 // PRO1584 // PRO2217 | 3090006 | chr8 | + | 23439117 | 23485313 | 8.21 |
| nucleoporin 50kDa | NUP50 | NM_007172 // M_153645 | MGC39961 // NPAP60 // NPAP60L | 3948461 | chr22 | + | 43938390 | 43960160 | 8.21 |
| mediator of RNA polymerase II transcription, subunit 8 homolog (S. cerevisiae) | MED8 | NM_001001651 // NM_001001653 // NM_001001654 // NM_052877 // NM_201542 | ARC32 // MGC17544 // MGC19641 | 2409344 | chr1 | - | 43622176 | 43628144 | 8.20 |
| eukaryotic translation initiation factor 4A, isoform 2 | EIF4A2 | NM_001967 | BM-010 // DDX2B // EIF4A // EIF4F | 2656738 | chr3 | + | 187961636 | 187990742 | 8.20 |
| GA binding protein transcription factor, beta subunit 2 | GABPB2 | NM_002041 // NM_005254 // NM_016654 // NM_016655 // NM_181427 | BABPB2 // E4TF1 // E4TF1-47 // E4TF1- 53 // E4TF1B // GABPB // GABPB1 // NRF2B1 // | 3623683 | chr15 | - | 48356681 | 48434794 | 8.18 |
| RNA binding motif protein 22 | RBM22 | NM_018047 | FLJ10290 // ZC3H16 | 2881521 | chr5 | - | 150050460 | 150060885 | 8.16 |
| meningioma expressed antigen 5 (hyaluronidase) | MGEA5 | NM_012215 | FLJ11229 // FLJ23355 // KIAA0679 // MEA5 // NCOAT | 3304012 | chr10 | - | 103534201 | 103568420 | 8.15 |
| low density lipoprotein receptor hypercholesterolemia) | LDLR | NM_000527 | FH // FHC | 3821015 | chr19 | + | 11061114 | 11115050 | 8.15 |
| (familial cell division cycle 40 homolog (S. cerevisiae) | CDC40 | NM_015891 | EHB3 // FLJ10564 // MGC102802 // PRP17 // PRPF17 | 2921086 | chr6 | + | 110606544 | 110682171 | 8.13 |
| hypothetical protein HSPC111 | HSPC111 | NM_016391 | HSPC185 | 2888284 | chr5 | - | 175743557 | 175749348 | 8.13 |
| solute carrier family 2 (facilitated glucose transporter), member 1 | SLC2A1 | NM_006516 | GLUT // GLUT1 // MGC141895 // MGC141896 | 2409104 | chr1 | - | 43124794 | 43338329 | 8.12 |
| caspase 2, apoptosis-related cysteine peptidase (neural precursor cell expressed, developmentally down-regulated 2) | CASP2 | NM_032982 // NM_032983 | CASP-2 // ICH-1L // ICH-1L/1S // ICH1 // NEDD2 | 3029030 | chr7 | + | 142695524 | 142714904 | 8.11 |
| HECT domain containing 1 | HECTD1 | NM_015382 | FLJ38315 // KIAA1131 | 3559570 | chr14 | - | 30629936 | 31002364 | 8.10 |
| ariadne homolog, ubiquitin-conjugating enzyme E2 binding protein, 1 (Drosophila) | ARIH1 | NM_005744 | ARI // HARI // HHARI // UBCH7BP | 3600744 | chr15 | + | 70553731 | 70666726 | 8.09 |
| RNA binding motif protein, X-linked 2 | RBMX2 | NM_016024 | CGI-79 | 3990795 | chrX | + | 129356824 | 129381607 | 8.09 |
| family with sequence similarity 29, member A | FAM29A | NM_017645 | MGC102696 // MGC138798 // MGC138799 // RP11- 296P7.3 | 3200611 | chr9 | - | 19042752 | 19093153 | 8.09 |
| NGFI-A binding protein 1 (EGR1 binding protein 1) | NAB1 | NM_005966 | - | 2520225 | chr2 | + | 191205069 | 191437200 | 8.08 |
| zinc finger protein 706 | ZNF706 | NM_001042510 // NM_001042511 // NM_016096 | HSPC038 // PNAS- 106 // PNAS-113 | 3147020 | chr8 | - | 102226101 | 102376609 | 8.08 |
| chaperonin containing TCP1, subunit 4 (delta) | CCT4 | NM_006430 | Cctd // MGC126164 // MGC126165 // SRB | 2555630 | chr2 | - | 61939693 | 61970469 | 8.07 |
| zinc finger, CCHC domain containing | ZCCHC8 | NM_017612 | DKFZp434E2220 | 3475679 | chr12 | - | 121522105 | 121551469 | 8.07 |
| methyltransferase like 2B // methyltransferase like 2A | METTL2B // METTL2A | NM_018396 // NM_001005372 // NM_181725 | FLJ11350 // FLJ12760 // METL // METTL2 // METTL2A // PSENIP1 | 3730211 | chr17 | + | 57854988 | 57881176 | 8.06 |
| fibrosin 1 | FBS1 | NM_022452 | FBS // FLJ11618 | 3656362 | chr16 | + | 30577810 | 30590035 | 8.06 |
| splicing factor, arginine/serine-rich 8 (suppressor-of-white-apricot homolog, Drosophila) | SFRS8 | NM_004592 | SWAP | 3438417 | chr12 | + | 130761587 | 130852472 | 8.05 |
| CCR4-NOT transcription complex, subunit 7 | CNOT7 | NM_013354 // NM 054026 | CAF1 // hCAF-1 | 3125775 | chr8 | - | 17128912 | 17148758 | 8.05 |
| BTB (POZ) domain containing 7 | BTBD7 | NM_001002860 // NM_018167 | DKFZp686N0544 // FUP1 // MGC48310 | 3577277 | chr14 | - | 92773656 | 92966460 | 8.05 |
| chromosome 17 open reading frame 79 | C17orf79 | NM_018405 | FLJ21119 // HSA272196 // TTP1 | 3752424 | chr17 | - | 27203014 | 27210424 | 8.04 |
| transcription factor 12 (HTF4, helix-loop-helix transcription factors 4) | TCF12 | NM_003205 // NM_207036 // NM_207037 // NM_207038 // NM_207040 | HEB // HTF4 // HsT17266 | 3595096 | chr15 | + | 54963208 | 55402126 | 8.03 |
| ATP binding domain 1 family, member B | ATPBD18 | NM_018066 | FLJ10349 | 2402838 | chr1 | - | 27073870 | 27089311 | 8.03 |
| PRP3 pre-mRNA processing factor 3 homolog (S. cerevisiae) // KIAA0460 | PRPF3 // KIAA0460 | NM_004698 // NM_015203 | HPRP3 // HPRP3P // PRP3 // Prp3p // RP18 // FLJ32145 // HSPC099 | 2358171 | chr1 | + | 148560583 | 148592321 | 8.03 |
| G1 to S phase transition 1 | GSPT1 | NM_002094 | 551G9.2 // ETF3A // GST1 // eRF3a | 3680610 | chr16 | - | 11865619 | 11917408 | 8.00 |
| zinc finger protein 408 | ZNF408 | NM_024741 | FLJ12827 | 3329461 | chr11 | + | 46678944 | 46684037 | 7.99 |
| chromosome 20 open reading frame 4 | C20orf4 | NM_015511 | CGI-23 // DKFZp564N1363 // bA234K24.2 | 3883787 | chr20 | + | 34287657 | 34322234 | 7.99 |
| DnaJ (Hsp40) homolog, subfamily A, member 2 | DNAJA2 | NM_005880 | CPR3 // DJA2 // DNAJ // DNJ3 // HIRIP4 // PRO3015 // RDJ2 | 3690084 | chr16 | - | 45546783 | 45565155 | 7.98 |
| splicing factor, arginine/serine-rich 10 (transformer 2 homolog, Drosophila) | SFRS10 | NM_004593 | DKFZp686F18120 // Htra2-beta // SRFS10 // TRA2- BETA // TRA2B | 2709062 | chr3 | - | 187048509 | 187160523 | 7.98 |
| trinucleotide repeat containing 6A | TNRC6A | NM_020847 // NM_014494 | CAGH26 // DKFZp666E117 // FLJ22043 // GW1 // GW182 // KIAA1460 // MGC75384 // TNRC6 | 3653398 | chr16 | + | 24583665 | 24748478 | 7.98 |
| testis expressed sequence 10 | TEX10 | NM_017746 | FLJ20287 // bA208F1.2 | 3217807 | chr9 | - | 102104197 | 102155471 | 7.98 |
| nucleoporin 107kDa | NUP107 | NM_020401 | NUP84 | 3421177 | chr12 | + | 67366998 | 67423025 | 7.97 |
| hypoxia-inducible factor 1, alpha subunit inhibitor | HIF1AN | NM_017902 | DKFZp762F1811 // FIH1 // FLJ20615 // FLJ22027 | 3260666 | chr10 | + | 102279093 | 102303662 | 7.97 |
| myosin IE | MYO1E | NM_004998 | HuncM-IC // MGC104638 // MYO1C | 3626826 | chr15 | - | 57215720 | 57452363 | 7.97 |
| v-raf murine sarcoma 3611 viral oncogene homolog | ARAF | NM_001654 | A-RAF // ARAF1 // PKS2 // RAFT1 | 3976299 | chrX | + | 47300826 | 47316249 | 7.96 |
| nucleolar protein family 6 (RNA-associated) | NOL6 | NM_130793 // NM_022917 // NM_139235 | FLJ21959 // MGC14896 // MGC14921 // MGC20838 // NRAP // UTP22 // bA311H10.1 | 3203582 | chr9 | - | 33442676 | 33464087 | 7.96 |
| chromosome 21 open reading frame 66 | C21 orf66 | NM_145328 // NM_013329 // NM_016631 // NM_058191 | BM-020 // FLJ90561 // GCFC | 3929395 | chr21 | - | 33028084 | 33066030 | 7.96 |
| casein kinase 1, alpha 1 | CSNK1A1 | NM_001025105 // NM_001892 | CK1 // HLCDGP1 // PRO2975 | 2880932 | chr5 | - | 148800168 | 148922811 | 7.96 |
| hypothetical protein DKFZp762E1312 | DKFZp762E1 312 | NM_018410 | hFLEG1 | 2604254 | chr2 | - | 234406876 | 234427931 | 7.95 |
| PRP6 pre-mRNA processing factor 6 homolog (S. cerevisiae) | PRPF6 | NM_012469 | ANT-1 // C20orf14 // TOM // U5-102K // hPrp6 | 3893849 | chr20 | + | 62082901 | 62134891 | 7.95 |
| tumor suppressing subtransferable candidate 4 | TSSC4 | NM_005706 | - | 3317338 | chr11 | + | 2378304 | 2381662 | 7.93 |
| cell division cycle 25 homolog B (S. pombe) | CDC25B | NM_004358 // NM_021872 // NM_021873 | - | 3874438 | chr20 | + | 3715655 | 3734756 | 7.93 |
| megakaryoblastic leukemia (translocation) 1 | MKL1 TOP1 | NM_020831 | BSAC // MAL // MRTF-A | 3961496 | chr22 | - | 39136248 | 39362660 | 7.93 |
| topoisomerase (DNA) I | TOP1 | NM 003286 | TOPI | 3885464 | chr20 | + | 39090891 | 39186535 | 7.93 |
| WW domain containing adaptor with coiled-coil | WAC | NM_016628 // NM_100264 // NM_100486 | BM-016 // MGC10753 // PRO1741 // Wwp4 // bA48B24 // | 3240340 | chr10 | + | 28828043 | 29005095 | 7.92 |
| ubiquitin specific peptidase 36 | USP36 | NM_025090 | DUB1 | 3772581 | chr17 | - | 74295075 | 74348574 | 7.92 |
| t-complex 1 | TCP1 | NM_001008897 // NM_030752 | CCT-alpha // CCT1 // CCTa // D6S230E // TCP-1-alpha | 2982381 | chr6 | - | 160119521 | 160130731 | 7.92 |
| protein phosphatase 6, catalytic subunit | PPP6C | NM_002721 | - | 3225348 | chr9 | - | 126948675 | 126991992 | 7.91 |
| CDC-like kinase 3 | CLK3 | NM_001292 // NM_003992 | FLJ22858 | 3601741 | chr15 | + | 72677906 | 72719105 | 7.91 |
| anillin, actin binding protein | ANLN | NM_018685 | ANILLIN // DKFZp779A055 // Scraps // scra | 2997376 | chr7 | + | 36395957 | 36462017 | 7.90 |
| adenosylmethionine decarboxylase 1 | AMD1 | NM_001033059 // NM_001634 | ADOMETDC // AMD // DKFZp313L1234 // FLJ26964 | 2921296 | chr6 | + | 111287588 | 111324441 | 7.90 |
| famesyl-diphosphate farnesyltransferase 1 | FDFT1 | NM_004462 | DGPT // ERG9 // SQS // SS | 3086206 | chr8 | + | 11690497 | 11740987 | 7.90 |
| PRP40 pre-mRNA processing factor 40 homolog A (S. cerevisiae) | PRPF40A | XM_371575 // XM_938514 | FBP-11 // FBP11 // FLAF1 // FLJ20585 // FNBP3 // HIP10 // HYPA // NY-REN- 6 | 2581548 | chr2 | - | 153212414 | 153283546 | 7.89 |
| death effector domain containing | DEDD | NM_001039711 // NM_001039712 // NM_032998 | CASP8IP1 // DEDD1 // DEFT // FLDED1 // KE05 | 2440625 | chr1 | - | 159357395 | 159369167 | 7.89 |
| integrin, beta 6 | ITGB6 | NM 000888 | - | 2583465 | chr2 | - | 160664438 | 160818586 | 7.88 |
| zinc finger protein 473 | ZNF473 | NM_001006656 // NM_015428 | HZFP100 // ZN473 | 3839142 | chr19 | + | 55220811 | 55248476 | 7.87 |
| HLA-B associated transcript 1 // ATPase, H+ transporting, lysosomal 13kDa, V1 subunit G2 | BAT1 // ATP6V1G2 | NM_004640 // NM_080598 // NM_130463 // NM_138282 | D6S81E // DDX39B // UAP56 // ATP6G // ATP6G2 // NG38 // VMA10 | 2949038 | chr6 | - | 31605990 | 31622606 | 7.87 |
| casein kinase 2, alpha 1 polypeptide // casein kinase 2, alpha 1 polypeptide pseudogene | CSNK2A1 // CSNK2A1P | NM_001895 // NM_77559 // NM_177560 // NR_002207 | CK2A1 // CKII // CKII alpha // - | 3894228 | chr20 | - | 402069 | 472534 | 7.87 |
| peroxisome proliferator-activated receptor gamma, coactivator-related 1 | PPRC1 | NM_015062 | KIAA0595 // MGC74642 // PRC // RP11-302K17.6 | 3261447 | chr10 | + | 103882745 | 103900072 | 7.86 |
| YME1-like 1 (S. cerevisiae) | YME1L1 | NM_014263 // NM_139312 // NM_139313 | FTSH // MEG4 // PAMP // YME1L | 3282213 | chr10 | - | 27439066 | 27484191 | 7.84 |
| thyroid hormone receptor associated protein 1 | THRAP1 | NM_005121 | ARC250 // DRIP250 // HSPC221 // KIAA0593 // MED13 // TRAP240 | 3765689 | chr17 | - | 57374750 | 57498031 | 7.84 |
| YTH domain family, member 1 | YTHDF1 | NM_017798 | C20orf21 // | 3913712 | chr20 | - | 61297230 | 61330873 | 7.83 |
| B-cell CLL/lymphoma 7B | BCL7B | NM_001707 // NM_138707 | - | 3056108 | chr7 | - | 72588624 | 72610244 | 7.82 |
| HIR histone cell cycle regulation defective homology A (S. cerevisiae) | HIRA | NM_003325 | DGCR1 // TUP1 // TUPLE1 | 3952637 | chr22 | - | 17696576 | 17799452 | 7.82 |
| Josephin domain containing 3 // small nucleolar RNA, H/ACA box 25 // small nucleolar RNA, H/ACA box 32 // small nucleolar RNA, H/ACA | JOSD3 // SNORA25 // SNORA32 // SNORA18 | NM_024116 // NR_003028 // NR_003032 // NR_002959 | MGC5306 // ACA25 // ACA32 // ACA18 | 3386814 | chr11 | - | 93102831 | 93114308 | 7.81 |
| GTP binding protein 4 | GTPBP4 | NM_012341 | CRFG // FLJ10686 // FLJ10690 // FLJ39774 // NGB | 3231774 | chr10 | + | 942450 | 1055862 | 7.79 |
| eukaryotic translation elongation factor 1 beta 2 | EEF1B2 | NM_001037663 // NM_001959 // NM_021121 | EEF1B // EEF1B1 // EF1B | 2524577 | chr2 | + | 206732303 | 206735884 | 7.78 |
| SMAD family member 5 | SMAD5 | NM_001001419 // NM_001001420 // NM_005903 | DKFZp781C1895 // DKFZp78101323 // Dwfc // JV5-1 // MADH5 | 2830010 | chr5 | + | 135464462 | 135552314 | 7.77 |
| epidermal growth factor receptor (erythroblastic leukemia viral (v-erb- b) oncogene homolog, avian) | EGFR | NM_005228 // NM_201282 // NM_201283 // NM_201284 | ERBB // ERBB1 // mENA | 3002640 | chr7 | + | 55054070 | 55291773 | 7.77 |
| mitogen-activated protein kinase kinase kinase 11 | MAP3K11 | NM_002419 | MGC17114 // MLK-3 // MLK3 // PTK1 // SPRK | 3377752 | chr11 | - | 65121806 | 65139693 | 7.77 |
| apoptosis inhibitor 5 | API5 | NM_006595 | AAC-11 // AAC11 // API5L1 | 3327906 | chr11 | + | 43290109 | 43322649 | 7.77 |
| coiled-coil-helix-coiled-coil-helix domain containing 3 | CHCHD3 | NM_017812 | FLJ20420 | 3073597 | chr7 | - | 132062426 | 132500566 | 7.75 |
| TBC1 domain family, member 10B | TBC1D10B | NM_015527 | DKFZP434P1750 // FP2461 | 3687715 | chr16 | - | 30275789 | 30289100 | 7.74 |
| nucleolar protein NOP5/NOP58 | NOP5/NOP5 8 | NM_015934 | HSPC120 | 2523144 | chr2 | + | 202811346 | 202926967 | 7.73 |
| ribosomal protein S6 kinase, 70kDa, polypeptide 1 | RPS6KB1 | NM_003161 | PS6K // S6K // S6K1 // STK14A // p70(S6K)-alpha // p70-S6K // p70-alpha | 3729294 | chr17 | + | 55325239 | 55429105 | 7.73 |
| GDP dissociation inhibitor 1 | GDI1 | NM_001493 | FLJ41411 // GDIL // MRX41 // MRX48 // OPHN2 // RABGD1A // RABGDIA // XAP- | 3996404 | chrX | + | 153318480 | 153324978 | 7.73 |
| PHD finger protein 12 | PHF12 | NM_001033561 // NM 020889 | KIAA1523 // MGC131914 // PF1 | 3751184 | chr17 | - | 24256404 | 24302905 | 7.73 |
| Smad nuclear interacting protein 1 | SNIP1 | NM_024700 | FLJ12553 // RP3- 423B22.3 // dJ423B22.2 | 2407163 | chr1 | - | 37774050 | 37792490 | 7.72 |
| stress-induced-phosphoprotein 1 (Hsp70/Hsp90-organizing protein) | STIP1 | NM_006819 | HOP // IEF-SSP- 3521 // P60 // STI1 L | 3334224 | chr11 | + | 63709341 | 63728586 | 7.71 |
| speckle-type POZ protein | SPOP | NM_001007226 // NM_001007227 // NM_001007228 // NM_001007229 // NM_001007230 // NM_003563 | TEF2 | 3761905 | chr17 | - | 45031245 | 45110541 | 7.71 |
| eukaryotic translation elongation factor 1 alpha 1 | EEF1A1 | NM_001402 | CCS-3 // CCS3 // EEF-1 // EEF1A // EF-Tu // EF1A // FLJ25721 // GRAF- 1EF // HNGC:16303 // LENG7 // MGC102687 // MGC131894 // MGC16224 // PTI1 // eEF1A-1 | 2960903 | chr6 | - | 74281167 | 74335800 | 7.70 |
| mitogen-activated protein kinase kinase 3 | MAP2K3 | XM_001130488 // NM_145110 // NM_002756 // NM_145109 | MAPKK3 // MEK3 // MKK3 // PRKMK3 | 3714729 | chr17 | + | 21128581 | 21159113 | 7.70 |
| DEAD (Asp-Glu-Ala-Asp) box polypeptide 18 // wingless-type MMTV integration site family, | DDX18 // WNT8B | NM_006773 // NM_003393 | FLJ33908 // MrDb // | 2502300 | chr2 | + | 118243834 | 118323562 | 7.70 |
| ATPase family, AAA domain containing 2 | ATAD2 | NM_014109 | DKFZp667N1320 // MGC131938 // MGC142216 // MGC29843 // MGC5254 // | 3151534 | chr8 | - | 124393174 | 124477871 | 7.69 |
| chromosome 1 open reading frame 160 | C1orf160 | NM_032125 | DKFZP564D0478 // MGC111002 // RP11- 4K3 A.4 | 2326954 | chr1 | + | 27521221 | 27535639 | 7.69 |
| RNA binding motif protein 5 | RBM5 | NM_005778 | FLJ39876 // G15 // H37 // LUCA15 // RMB5 | 2622469 | chr3 | + | 50101372 | 50134000 | 7.69 |
| thioredoxin domain containing 9 // lysozyme-like | TXNDC9 // LYG2 | NM_005783 // NM_175735 | APACD // LYGH // MGC119046 // MGC119047 // MGC119049 | 2566740 | chr2 | - | 99301923 | 99319337 | 7.69 |
| zinc finger protein 148 | ZNF 48 | NM_021964 | BERF-1 // BFCOL1 // HT-BETA // ZBP- 89 // ZFP148 // pHZ-52 | 2693081 | chr3 | - | 126427204 | 126577074 | 7.69 |
| insulin receptor substrate 2 | IRS2 | NM_003749 | - | 3525234 | chr13 | - | 109196024 | 109238228 | 7.68 |
| interleukin enhancer binding factor 3, 90kDa | ILF3 | NM_004516 // NM_012218 // NM_153464 | DRBF // DRBP76 // MMP4 // MPHOSPH4 // MPP4 // NF-AT- 90 // NF90 // NFAR // NFAR-1 // TCP80 | 3820663 | chr19 | + | 10625547 | 10664074 | 7.68 |
| ubiquitin-conjugating enzyme E2, J1 (UBC6 homolog, yeast) | UBE2J1 | NM_016021 | CGI-76 // HSPC153 // HSPC205 // HSU93243 // MGC12555 // NCUBE1 // Ubc6p | 2964200 | chr6 | - | 90093065 | 90119338 | 7.66 |
| START domain containing 7 | STARD7 | NM_020151 // NM_139267 | GTT1 | 2565143 | chr2 | - | 96214334 | 96238296 | 7.65 |
| DPH1 homolog (S. cerevisiae) // candidate tumor suppressor in ovarian cancer 2 | DPH1 // OVCA2 | NM_001383 // NM_060822 | DPH2L // DPH2L1 // FLJ33211 // OVCA1 //- | 3706071 | chr17 | + | 1874907 | 1895099 | 7.64 |
| non-POU domain containing, octamer-binding | NONO | I NM_007363 | NMT55 // NRB54 // P54 // P54NRB | 3980887 | chrX | + | 70420006 | 70437726 | 7.64 |
| scaffold attachment factor 82 | SAFB2 | I NM_014649 | KIAA0138 | 3847252 | chr19 | - | 5533105 | 5575015 | 7.64 |
| WD repeat domain 3 | WDR3 | I NM_006784 | FLJ12796 | 2354082 | chr1 | + | 118273827 | 118304572 | 7.63 |
| metal response element binding transcription factor 2 | MTF2 | I NM_007368 | M96 // PCL2 // RP5-976013.1 // dJ976O13.2 | 2346934 | chr1 | + | 93147511 | 93377202 | 7.63 |
| general transcription factor IIIC, polypeptide 2, beta 110kDa | GTF3C2 | NM_001035521 // NM_001521 | KIAA0011 // TFIIIC- BETA // TFIIIC110 | 2545681 | chr2 | - | 27402231 | 27433372 | 7.63 |
| general transcription factor IIH, polypeptide 1, 62kDa | GTF2H1 | NM_005316 | BTF2 // TFIIH | 3322717 | chr11 | + | 18300412 | 18345156 | 7.62 |
| mitochondrial ribosomal protein L17 | MRPL17 | NM_022061 | MRP-L26 // RPL17L // RPML26 | 3361116 | chr11 | - | 6657673 | 6690484 | 7.62 |
| Yip1 domain family, member 3 // chromosome 6 open reading frame 154 | YIPF3 // C6orf154 | NM_015388 // NM_001012974 | C6orf109 // DKFZP566C243 // FinGER3 // KLIP1 // dJ337H4.3 // FLJ44836 // MGC131686 // dJ337H4.2 | 2954646 | chr6 | - | 43587500 | 43592701 | 7.61 |
| eukaryotic translation initiation factor 4 gamma, 2 | EIF4G2 | NM_001042559 // NM_001418 | AAG1 // DAP5 // FLJ41344 // NAT1 // p97 | 3362719 | chr11 | - | 10487574 | 10788746 | 7.60 |
| MYST histone acetyltransferase 2 | MYST2 | NM_007067 | HBO1 // HBOA | 3725779 | chr17 | + | 45196447 | 45261455 | 7.60 |
| SIN3 homolog A, transcription regulator (yeast) | SIN3A | NM_015477 | DKFZP434K2235 // FLJ90319 // KIAA0700 | 3633403 | chr15 | - | 73448786 | 73535167 | 7.60 |
| centaurin, delta 1 | CENTD1 | NM_015230 // NM_139182 | ARAP2 // FLJ13675 // FLJ4-0916 // KIAA0580 // PARX | 2765590 | chr4 | - | 35626248 | 35922356 | 7.60 |
| KIT ligand | KITLG | NM_000899 // NM_003994 | DKFZp686F2250 // KL-1 // Kitl // MGF // SCF // SF | 3464747 | chr12 | - | 87349442 | 87498371 | 7.58 |
| F-box and leucine-rich repeat protein 11 | FBXL11 | NM_012308 | CXXC8 // DKFZP434M1735 // FBL11 // FBL7 // FLJ00115 // FLJ46431 // JHDM1A // KIAA1004 // LILINA | 3336696 | chr11 | + | 66643299 | 66782124 | 7.57 |
| POM (POM121 homolog, rat) and ZP3 fusion | POMZP3 | NM_012230 // NM_152992 | MGC8359 // POM- ZP3 // POM121 | 3057755 | chr7 | - | 76037679 | 76196100 | 7.56 |
| VAMP (vesicle-associated membrane protein)-associated protein A, 33kDa | VAPA | NM_003574 // NM_194434 | MGC3745 // VAP-33 // VAP-A // VAP33 // hVAP-33 | 3778601 | chr18 | + | 9903984 | 9950012 | 7.56 |
| integrin beta 4 binding protein | ITGB4BP | NM_002212 // NM_181466 // NM_181467 // NM_181468// NM_181469 | CAB // EIF3A // EIF6 // b(2)gcn // p27BBP | 3903836 | chr20 | - | 33276003 | 33336225 | 7.55 |
| chromosome 10 open reading frame 119 | C10orf119 | NM_024834 | FLJ13081 // FLJ36756 | 3309755 | chr10 | - | 121568302 | 121642742 | 7.55 |
| chromosome 11 open reading frame 57 | C11orf57 | NM_018195 | FLJ10726 | 3348891 | chr11 | + | 111450087 | 111461067 | 7.54 |
| heat shock protein 90kDa alpha (cytosolic), class A member 1 // heat shock protein 90kDa alpha (cytosolic), class A member 2 // heat shock protein 90kDa alpha (cytosolic), class A member 6 (pseudogene) | HSP90AA1 // HSP90AA2 // HSP90AA6P | NM_001017963 // NM_005348 // NM_001040141 // - | FLJ31884 // HSP86 // HSP90A // HSP90N // HSPC1 // HSPCA // HSPCAL1 // HSPCAL4 // HSPN // Hsp89 // Hsp90 // LAP2 // HSP90ALPHA // HSPCAL3 // | 3580179 | chr14 | - | 101616842 | 101676327 | 7.53 |
| peroxisomal biogenesis factor 5 | PEX5 | NM_000319 | PTS1R // PXR1 | 3403299 | chr12 | + | 7232607 | 7262437 | 7.53 |
| chromosome 4 open reading frame 30 | C4orf30 | NM_017741 | FLJ20280 // MGC126765 // MGC126767 | 2762468 | chr4 | - | 17404173 | 17421482 | 7.53 |
| transmembrane protein 2 | TMEM2 | NM 013390 | - | 3209384 | chr9 | - | 73452177 | 73573229 | 7.52 |
| forkhead box M1 | FOXM1 | NM_021953 // NM_202002 // NM_202003 | FKHL16 // FOXM1B // HFH-11 // HFH11 // HNF-3 // INS-1 // MPHOSPH2 // MPP-2 // MPP2 // PIG29 // TRIDENT | 3440598 | chr12 | - | 2836207 | 2856564 | 7.52 |
| ceramide kinase | CERK | NM_022766 // NM_182661 | DKFZp434E0211 // FLJ21430 // FLJ23239 // KIAA1646 // LK4 // MGC131878 // dA59H18.2 // dA59H18.3 // hCERK | 3964154 | chr22 | - | 45454343 | 45537349 | 7.52 |
| small nuclear ribonucleoprotein 70kDa polypeptide (RNP antigen) | SNRP70 | NM_001009820 // NM_003089 | RNPU1Z // RPU1 // U170K // U1AP // U1RNP | 3838185 | chr19 | + | 54280358 | 54304714 | 7.52 |
| anthrax toxin receptor 2 | ANTXR2 | NM_058172 | CMG-2 // CMG2 // FLJ31074 // ISH // JHF // MGC111533 // MGC45856 | 2774971 | chr4 | - | 80762874 | 81219758 | 7.51 |
| antizyme inhibitor 1 | AZIN1 | NM_015878 // NM_148174 | MGC3832 // MGC691 // OAZI // OAZIN // ODC1L | 3147591 | chr8 | - | 103907725 | 103953967 | 7.51 |
| chromosome 12 open reading frame 44 | C12orf44 | NM_021934 | FLJ11773 | 3415273 | chr12 | + | 50749317 | 50757534 | 7.51 |
| cofactor required for Sp1 transcriptional activation, subunit 6, | CRSP6 | NM_004268 | CRSP77 // DRIP80 // FLJ10812 // MED17 // TRAP80 | 3344897 | chr11 | + | 93157019 | 93186225 | 7.49 |
| 77kDa KIAA1967 | KIAA1967 | NM_021174 // NM_199205 | DBC-1 // DBC1 | 3089597 | chr8 | + | 22518038 | 22534618 | 7.49 |
| serologically defined colon cancer antigen 3 // small nuclear RNA activating complex, polypeptide 4, 190kDa | SDCCAG3 // SNAPC4 | NM_001039707 // NM_001039708 // NM_006643 // NM_003086 | NY-CO-3 // FLJ13451 // PTFalpha // SNAP190 | 3229943 | chr9 | - | 138416210 | 138425418 | 7.49 |
| potassium channel tetramerisation domain containing 10 | KCTD10 | NM_031954 | FLJ41739 // MSTP028 // ULRO61 | 3470793 | chr12 | - | 108370845 | 108399528 | 7.48 |
| NADH dehydrogenase (ubiquinone) 1 alpha subcomplex, 5, 13kDa | NDUFA5 | NM_005000 | B13 // CI-13KD-B // DKFZp781K1356 // FLJ12147 // NUFM // UQOR13 | 3070658 | chr7 | - | 122968075 | 122985216 | 7.48 |
| interferon stimulated exonuclease 20kDa-like 1 | 1SG20L1 | NM_022767 | AEN // FLJ12484 // FLJ12562 // pp12744 | 3607232 | chr15 | + | 86948254 | 86976501 | 7.47 |
| gene paxillin | PXN | NM_002859 | FLJ16691 | 3474372 | chr12 | - | 119132644 | 119187926 | 7.47 |
| synaptotagmin binding, cytoplasmic RNA interacting protein | SYNCRIP | NM_006372 | GRY-RBP // HNRPQ1 // NSAP1 // RP1-3J17.2 // dJ3J17.2 // pp68 | 2963407 | chr6 | - | 86374062 | 86410282 | 7.47 |
| proteasome (prosome, macropain) 26S subunit, non-ATPase, 6 | PSMD6 | NM_014814 | KIAA0107 // S10 // SGA-113M // p44S10 | 2679864 | chr3 | - | 63971281 | 63984700 | 7.47 |
| chromosome 7 open reading frame | C7orf30 | NM 138446 | - | 2992863 | chr7 | + | 23305462 | 23317894 | 7.46 |
| heterogeneous nuclear ribonucleoprotein K | HNRPK | NM_002140 // NM_031262 // NM 031263 | CSBP // FLJ41122 // HNRNPK // TUNP | 3212294 | chr9 | - | 85772377 | 85785355 | 7.45 |
| heterogeneous nuclear ribonucleoprotein L | HNRPL | NM_001005335 // NM_001533 | FLJ35509 // P/OKcl.l4 // hnRNP- L | 3861617 | chr19 | - | 44018883 | 44034809 | 7.45 |
| SEC14-like 1 (S. cerevisiae) | SEC14L1 | NM_001039573 // NM_003003 | DKFZp686C06176 // PRELID4A // SEC14L | 3735752 | chr17 | + | 72596335 | 72767744 | 7.45 |
| hippocampus abundant transcript-like 1 | HIATL1 | XM-001130181 // NM_032558 | FLJ14753 // MGC117350 | 3180263 | chr9 | + | 96176674 | 96325754 | 7.43 |
| eukaryotic translation initiation factor 5A | EIF5A | NM_001970 | EIF-5A // EIF5A1 // MGC104255 // MGC99547 // uORF // uORF A | 3708422 | chr17 | + | 7151037 | 7156478 | 7.42 |
| discoidin, CUB and LCCL domain containing 2 | DCBLD2 | NM_080927 | CLCP1 // ESDN | 2686023 | chr3 | - | 99997526 | 100124598 | 7.42 |
| voltage-dependent anion channel 2 | VDAC2 | NM 003375 | FLJ23841 | 3252577 | chr10 | + | 76639941 | 76661389 | 7.41 |
| polycomb group ring finger 3 | PCGF3 | NM_006315 | DKFZp686D20235 // DONG1 // FLJ36550 // FLJ43813 // MGC129615 // MGC40413 // RNF3 // RNF3A | 2714230 | chr4 | + | 689573 | 754428 | 7.41 |
| NADH dehydrogenase (ubiquinone) Fe-S protein 3, 30kDa (NADH-coenzyme Q reductase) | NDUFS3 | NM_004551 | - | 3329904 | chr11 | + | 47557156 | 47562663 | 7.41 |
| signal recognition particle 68kDa | SRP68 | NM_014230 | - | 3771297 | chr17 | - | 71546467 | 71580323 | 7.41 |
| thioredoxin reductase 1 | TXNRD1 | NM_003330 // NM_18272.9 // NM_182742 // NM 182743 | GRIM-1 // MGC9145 // TR // TR1 // TRXR1 // TXNR | 3429460 | chr12 | + | 103130628 | 103354079 | 7.39 |
| PDZ domain containing 8 | PDZD8 | NM_173791 | FLJ25412 // FLJ34427 // PDZK8 // bA129M16.2 | 3308619 | chr10 | - | 119029422 | 119124958 | 7.39 |
| IKAROS family zinc finger 5 (Pegasus) | IKZF5 | NM_022466 | DKFZp781B0249 // FLJ22973 // PEGASUS // | 3310757 | chr10 | - | 124740316 | 124774190 | 7.38 |
| TPX2, microtubule-associated, homolog (Xenopus laevis) | TPX2 | NM_012112 | C20orf1 // C20orf2 // DIL-2 // DIL2 // FLS353 // GD:C20orf1 // HCA519 // HCTP4 // | 3881443 | chr20 | + | 29774755 | 29853260 | 7.37 |
| RNA binding motif protein 39 | RBM39 | NM_184237 // NM_184241 // NM_184244 // NM_004902 // NM_184234 | CAPER // CAPERalpha // CC1.3 DKFZp781 C0423 // FLJ44170 // HCC1 // RNPC2 | 3904226 | chr20 | - | 33754569 | 33793768 | 7.35 |
| ubiquitin specific peptidase 53 | USP53 | NM 019050 | DKFZp781E1417 | 2741236 | chr4 | + | 120353195 | 120436746 | 7.35 |
| vesicle-associated membrane protein 3 (cellubrevin) | VAMP3 | NM_004781 | CEB | 2318637 | chr1 | + | 7753916 | 7764072 | 7.35 |
| DEAH (Asp-Glu-Ala-His) box polypeptide 36 | DHX36 | NM_020865 | DDX36 // KIAA1488 // MLEL1 | 2701595 | chr3 | - | 155475346 | 155524965 | 7.33 |
| fibronectin type III domain containing 3B | FNDC3B | NM_022763 | DKFZp686D14170 // DKFZp762K137 // FAD104 // FLJ23399 // MGC10002 // PRO4979 // YVTM2421 | 2652410 | chr3 | + | 173240112 | 173601176 | 7.33 |
| E74-like factor 1 (ets domain transcription factor) | ELF1 | NM_172373 | | 3511031 | chr13 | - | 40394613 | 40533811 | 7.32 |
| keratin 19 | KRT19 | NM_002276 | CK19 // K19 // K1CS // MGC15366 | 3757108 | chr17 | - | 36933431 | 36938160 | 7.32 |
| integrin, alpha 2 (CD49B, alpha 2 subunit of VLA-2 receptor) | ITGA2 | NM_002203 | BR // CD49B // GPIa // VLA-2 // VLAA2 | 2809245 | chr5 | + | 52320949 | 52432778 | 7.31 |
| ribosomal protein L30 | RPL30 | NM_000989 | - | 3145953 | chr8 | - | 99123079 | 99127371 | 7.31 |
| IMP4, U3 small nucleolar ribonucleoprotein, homolog (yeast) | IMP4 | NM_033416 | BXDC4 // MGC19606 | 2505501 | chr2 | + | 130816288 | 130828310 | 7.31 |
| origin recognition complex, subunit 2-like (yeast) | ORC2L // MGC39518 | NM_006190 | ORC2 | 2594569 | chr2 | - | 201482783 | 201536750 | 7.31 |
| Kruppel-like factor 10 | KLF10 | NM_001032282 // NM_005655 | EGRA // TIEG // TIEG1 | 3147508 | chr8 | - | 103682306 | 103767558 | 7.30 |
| tight junction protein 1 (zona occludens 1) | TJP1 | NM_003257 // NM_175610 | DKFZp686M05161 // MGC133289 // ZO-1 | 3615579 | chr15 | - | 27703228 | 28048334 | 7.30 |
| tRNA 5-methylaminomethyl-2-thiouridylate methyltransferase | TRMU | NM_018006 | MGC99627 // MTO2 // MTU1 // TRMT // TRMT1 // TRNT1 | 3949097 | chr22 | + | 45109982 | 45131896 | 7.29 |
| ribosomal protein L18 | RPL18 | NM_000979 | - | 3867223 | chr19 | - | 53810441 | 53814585 | 7.29 |
| Nance-Horan syndrome (congenital cataracts and dental anomalies) | NHS | XM_001134060 // NM_198270 | DKFZp781F2016 // DKFZp781L0254 // SCML1 | 3970338 | chrX | + | 17303441 | 17664033 | 7.29 |
| chromosome 16 open reading frame 72 | C16orf72 | NM_014117 | FLJ41272 // PRO0149 | 3647632 | chr16 | + | 9092682 | 9121055 | 7.28 |
| ubiquitin specific peptidase 10 | USP10 | NM_005153 | KIAA0190 // MGC2621 // UBPO | 3671873 | chr16 | + | 83291080 | 83371023 | 7.27 |
| A kinase (PRKA) anchor protein (gravin) 12 | AKAP12 | NM_005100 // NM_144497 | AKAP250 // DKFZp686M0430 // DKFZp68600331 | 2931569 | chr6 | + | 151602720 | 151721361 | 7.27 |
| ornithine decarboxylase 1 | ODC1 | NM_002539 | - | 2540157 | chr2 | - | 10485474 | 10606340 | 7.27 |
| matrin 3 | MATR3 | NM_018834 // NM_199189 | DKFZp686K0542 // DKFZp686K23100 // KL4A0723 // MGC9105 | 2831124 | chr5 | + | 138505686 | 138695245 | 7.26 |
| chromosome 5 open reading frame 22 | C5orf22 | NM_018356 | DKFZp667N066 // FLJ11193 // FLJ23805 // MGC33010 | 2805176 | chr5 | + | 31568161 | 31590902 | 7.26 |
| nuclear mitotic apparatus protein 1 | NUMA1 | NM_006185 | NUMA | 3380901 | chr11 | - | 71389909 | 71469367 | 7.25 |
| macrophage erythroblast attacher | MAEA | NM_001017405 // NM_005882 | EMP // HLC-10 // PIG5 | 2714672 | chr4 | + | 1273702 | 1323891 | 7.25 |
| nucleolar protein 8 | NOL8 | NM_017948 | C9orf34 // DKFZp686P12242 // Fly20736 // Nop132 // bA62C3.3 // bA62C3.4 | 3214749 | chr9 | - | 94099471 | 94127688 | 7.25 |
| proliferation-associated 2G4, 38kDa // dihydrolipoamide S-succinyltransferase (E2 component of 2-oxo-alutarate complex) | PA2G4 // DLST | NM_006191 // NM_001933 | EBP1 // HG4-1 // p38-2G4 // DLTS | 3417309 | chr12 | + | 54784566 | 54793956 | 7.23 |
| mitochondrial ribosomal protein L46 | MRPL46 | NM_022163 | C15orf4 // LIECG2 // MGC22762 // | 3638048 | chr15 | - | 86795015 | 86811600 | 7.23 |
| phosphatidylinositol glycan anchor biosynthesis, class G | PIGG | NM_017733 | FLJ20265 // FLJ39925 // GP17 // LAS21 // MGC131903 // PRO4405 // RLGS1930 | 2714025 | chr4 | + | 483010 | 523985 | 7.22 |
| SON DNA binding protein | SON | NM_032195 // NM_138927 | BASS1 // C21orf50 // DBP-5 // FLJ21099 // FLJ33914 // KIAA1019 // NREBP // SON3 | 3918696 | chr21 | + | 33836804 | 33872881 | 7.20 |
| kelch-like ECH-associated protein 1 // ADAM metallopeptidase with thrombospondin type 1 motif, 6 | KEAP1 // ADAMTS6 | NM_012289 // NM_203500 // NM_197941 | INrf2 // KIAA0132 // KLHL19 // MGC10630 // MGC1114 // MGC20887 // MGC4407 // MGC9454 // ADAM- | 3850363 | chr19 | - | 10457571 | 10475233 | 7.20 |
| bromodomain containing 2 | BRD2 | NM_005104 | D6S113E // DKFZp686NO336 // FLJ31942 // FSRG1 // KIAA9001 // NAT // RING3 // RNF3 | 2903343 | chr6 | + | 33044392 | 33057253 | 7.20 |
| squalene epoxidase | SQLE | NM_003129 | - | 3114832 | chr8 | + | 126063738 | 126105522 | 7.20 |
| lamin B receptor | LBR | NM_002296 // NM_194442 | DHCR148 // LMN2R // MGC9041 // PHA | 2458289 | chr1 | - | 223655840 | 223683230 | 7.19 |
| Ewing sarcoma breakpoint region 1 | EWSR1 | NM_005243 // NM_013986 | EWS | 3941907 | chr22 | + | 27994201 | 28026501 | 7.19 |
| chromosome 19 open reading frame 48 | C19orf48 | NM_199249 // NM_199250 | MGC13170 | 3868659 | chr19 | - | 55992774 | 56012596 | 7.17 |
| upstream transcription factor 1 // Rho GTPase activating protein 30 | USF1// ARHGAP30 | NM_007122 // NM_207005 // NM_001025598 // NM_181720 | FCHL // FCHL1 // HYPUP1 //MLTF // MLTFI // UEF // FLJ00267 // FLJ44128 // RP11- 544M22.6 | 2440523 | chr1 | - | 159275665 | 159282355 | 7.17 |
| WD repeat domain 46 | WDR46 | NM_005452 | BING4 // C6orf11 // FP221 | 2950561 | chr6 | - | 33354871 | 33365259 | 7.16 |
| topoisomerase (DNA) II alpha 170kDa | TOP2A | NM_001067 | TOP2 // TP2A | 3756193 | chr17 | - | 35717597 | 35847034 | 7.15 |
| ATP synthase, H+ transporting, mitochondrial F0 complex, subunit E | ATP5I | NM_007100 | ATP5K // MGC12532 | 2756497 | chr4 | - | 654679 | 658269 | 7.14 |
| splicing factor 3b, subunit 2, 145kDa | SF3B2 | NM_006842 | SAP145 // SF3B145 // SF3b1 // SF3b150 | 3335907 | chr11 | + | 65573499 | 65593343 | 7.14 |
| splicing factor, arginine/serine-rich 11 | SFRS11 | NM₋004768 | DKFZp686M13204 // dJ677H15.2 // p54 | 2341565 | chr1 | + | 70443686 | 70492014 | 7.12 |
| aminopeptidase puromycin sensitive | NPEPPS | XM_001128588 // NM_006310 | MP100 // PSA | 3724698 | chr17 | + | 42955347 | 43057168 | 7.11 |
| influenza virus NS1A binding protein | IVNS1ABP | NM_006469 // NM_016389 | DKFZp688K06216 // FLARA3 // FLJ10069 // FLJ10411 // FLJ10962 // FLJ35593 // FLJ36593 // HSPC068 // KIAA0850 // ND1 // NS-1 // NS1-BP // | 2448073 | chr1 | - | 183532162 | 183553081 | 7.10 |
| Smg-7 homolog, nonsense mediated mRNA decay factor (C. elegans) | SMG7 | NM_014837 // NM_173156 // NM_201568 // NM_201569 | C1orf16 // EST1C // FLJ23717 // KIAA0250 // SGA56M // SMG-7 | 2371255 | chr1 | + | 181708036 | 181834004 | 7.09 |
| helicase with zinc finger | HELZ | NM_014877 | DRHC // HUMORF5 // K1AA0054 // MGC163454 | 3768015 | chr17 | - | 62485987 | 62672547 | 7.08 |
| cytochrome P450, family 1, subfamily B, polypeptide 1 | CYP1B1 | NM_000104 | CP1B // GLC3A | 2548699 | chr2 | - | 38138730 | 38196009 | 7.08 |
| sprouty homolog 2 (Drosophila) | SPRY2 | NM 005842 | MGC23039 // | 3519309 | chr13 | - | 79683810 | 79813918 | 7.07 |
| golgi phosphoprotein 4 | GOLPH4 | NM_014498 | GIMPC // GPP130 // P138 | 2704267 | chr3 | - | 169208832 | 169296426 | 7.07 |
| Rab geranylgeranyltransferase, beta subunit // mutS homolog 4 (E. coli) | RABGGTB // MSH4 | NM_004582 // NM_002440 | GGTB // - | 2342624 | chr1 | + | 76024474 | 76033333 | 7.07 |
| angiomotin like 2 | AMOTL2 | NM_016201 | LCCP | 2696309 | chr3 | - | 135527277 | 135576097 | 7.06 |
| MAK10 homolog, amino-acid N- acetyltransferase subunit, (S. cerevisiae) | MAK10 | NM_024635 | FLJ21613 // FLJ22643 // bA379P1.1 | 3177563 | chr9 | + | 87733620 | 87828205 | 7.06 |
| NOL1/NOP2/Sun domain family, member 2 | NSUN2 | NM_017755 | FLJ20303 // SAKI // TRM4 | 2847292 | chr5 | - | 6510364 | 6686394 | 7.06 |
| leptin receptor overlapping transcript-like 1 | LEPROTL1 | NM_015344 | HSPC112 // Vps55 // my047 | 3092276 | chr8 | + | 30059542 | 30115386 | 7.06 |
| geminin, DNA replication inhibitor | GMNN | NM_015895 | Gem // RP3- | 2898597 | chr6 | + | 24857262 | 24894306 | 7.06 |
| FAT tumor suppressor homolog 1 (Drosophila) | FAT | NM_005245 | CDHF7 // FAT1 // ME5 // hFat1 | 2797393 | chr4 | - | 187745970 | 187885048 | 7.05 |
| forkhead box J3 | FOXJ3 | NM_014947 | - | 2408855 | chr1 | - | 42414807 | 42574125 | 7.05 |
| nucleoporin 54kDa | NUP54 | NM_017426 | MGC13407 | 2773997 | chr4 | - | 77254731 | 77288679 | 7.04 |
| kinesin family member 5B | KIF5B | NM_004521 | KINH // KNS // KNS1 // UKHC | 3283991 | chr10 | - | 32337798 | 32385342 | 7.04 |
| interferon gamma receptor 1 | IFNGR1 | NM_000416 | CD119 // FLJ45734 // IFNGR | 2976113 | chr6 | - | 137560317 | 137582279 | 7.04 |
| mortality factor 4 like 2 | MORF4L2 | NM_012286 | KIAA0026 // MORFL2 // MRGX | 4016572 | chrX | - | 102817114 | 102844278 | 7.03 |
| mastermind-like 1 (Drosophila) | MAML1 | XM_001126853 // NM_014757 | KIAA0200 // Mam-1 // Mam1 | 2844410 | chr5 | + | 179092457 | 179151795 | 7.03 |
| zinc finger and BTB domain containing 11 | ZBTB11 | NM_014415 | FLJ13426 // MGC133303 // ZNF- U69274 | 2686727 | chr3 | - | 102780272 | 102878819 | 7.02 |
| mitochondrial ribosomal protein S2 | MRPS2 | NM_016034 | CGI-91 // MRP-S2 | 3193900 | chr9 | + | 137531661 | 137536331 | 7.02 |
| adaptor-related protein complex 2. | AP2A2 | NM_012305 | ADTAB // CLAPA2 // HIP9 // HYPJ | 3316447 | chr11 | + | 912387 | 1002226 | 7.01 |
| alpha 2 subunit serine arginine-rich pre-mRNA SR-A1 | SR-A1 | NM_021228 | SRA1 | 3838757 | chr19 | + | 54837204 | 54853671 | 7.01 |
| splicing factor NMDA receptor regulated 1 | NARG1 | NM_057175 | Ga19 // NATH // TBDN100 | 2744597 | chr4 | + | 140442091 | 140560627 | 7.00 |
| triple functional domain (PTPRF | TRIO | NM_007118 | tgat | 2802398 | chr5 | + | 14196590 | 14585215 | 7.00 |
| interacting) myotubularin related protein 12 | MTMR12 | NM 001040446 | 3-PAP // PIP3AP | 2852274 | chr5 | - | 32262872 | 32348897 | 7.00 |
| cyclin G associated kinase | GAK | XM_001127411 // NM_005255 | FLJ40395 // MGC99654 | 2756673 | chr4 | - | 833110 | 916149 | 7.00 |
| GRB2-associated binding protein 1 | GAB1 | NM_002039 // NM_207123 | - | 2745547 | chr4 | + | 144476834 | 144615158 | 7.00 |
| senataxin | SETX | NM_015046 | ALS4 // AOA2 // DKFZp781B151 // FLJ12840 // FLJ43459 // KIAA0625 // SCAR1 // bA479K20.2 | 3228007 | chr9 | - | 134126584 | 134220359 | 7.00 |
| chromosome 12 open reading frame 41 | C12orf41 | NM_017822 | FLJ12670 // FLJ20436 | 3453177 | chr12 | - | 47333281 | 47362268 | 7.00 |
| WNK lysine deficient protein kinase 1 | WNK1 | NM_018979 | KDP // KIAA0344 // PHA2C // PRKWNK1 | 3400034 | chr12 | + | 278104 | 891189 | 6.98 |
| myotubularin related protein 1 | MTMR1 | NM_003828 | - | 3994846 | chrX | + | 149612503 | 149684226 | 6.97 |
| met proto-oncogene (hepatocyte growth factor receptor) | MET | NM_000245 | HGFR // RCCP2 | 3020343 | chr7 | + | 116099694 | 116230307 | 6.97 |
| ADP-ribosylation factor 4 | ARF4 | XM_001132763 // NM_001660 | - | 2678090 | chr3 | - | 57532172 | 57558635 | 6.96 |
| four and a half LIM domains 2 | FHL2 | NM_001039492 // NM_001450 // NM_201555 // NM 201557 | AAG11 // DRAL // SLIM3 | 2568687 | chr2 | - | 105335877 | 105501005 | 6.96 |
| polypyrimidine tract binding protein 1 | PTBP1 | NM_002819 // NM_031990 // NM_031991 // NM_175847 | HNRNPI // HNRPI // MGC10830 // MGC8461 // PTB // PTB-1 // PTB-T // PTB2 // PTB3 // PTB4 // pPTB | 3815165 | chr19 | + | 747768 | 763505 | 6.96 |
| glutamate-rich WD repeat containing 1 | GRWD1 | NM_031485 | GRWD // KIAA1942 // RRB1 // WDR28 | 3837796 | chr19 | + | 53640874 | 53652090 | 6.95 |
| signal sequence receptor, alpha (translocon-associated protein alpha) | SSR1 | NM_003144 | DKFZp781N23103 // TRAPA | 2940551 | chr6 | - | 7213542 | 7258526 | 6.94 |
| GC-rich promoter binding protein 1-like 1 | GPBP1L1 | NM_021639 | RP11-767N6.1 // SP192 | 2410330 | chr1 | - | 45865575 | 45926352 | 6.94 |
| chromosome 20 open reading frame 72 | C20orf72 | NM_052865 | FLJ14597 // bA504H3.4 | 3878220 | chr20 | + | 17896912 | 17919759 | 6.94 |
| ELAV (embryonic lethal, abnormal vision, Drosophila)-like 1 (Hu antigen R) // translocase of inner mitochondrial membrane 44 homolog (yeast) | ELAVL1 // TIMM44 | NM_001419 // NM_006351 | ELAV1 // HUR // Hua // MelG // DKFZp686H05241 // TIM44 | 3848689 | chr19 | - | 7929463 | 7999655 | 6.93 |
| heat shock protein 90kDa alpha (cytosolic), class B member 1 | HSP90AB1 | NM_007355 | D6S182 // FLJ26984 // HSP90-BETA // HSP90B // HSPC2 // HSPCB | 2908474 | chr6 | + | 44319983 | 44330383 | 6.93 |
| TSC22 domain family, member 2 | TSC22D2 | NM_014779 | KIAA0669 // TlLZ4a // TILZ4b // TILZ4c | 2647647 | chr3 | + | 151585441 | 151660841 | 6.91 |
| Pentatricopeptide repeat domain 3 // kinesin family member 5A | PTCD3 // KIF5A | NM_017952 // NM_004984 | DKFZp666K071 // FLJ20758 // D12S1889 // MY050 // NKHC // SPG10 | 2491935 | chr2 | + | 86186849 | 86222791 | 6.91 |
| kelch-like 20 (Drosophila) | KLHL20 | NM_014458 | KHLHX // KLEIP // KLHLX // RP3- 383J4.3 | 2367793 | chr1 | + | 171950703 | 172028914 | 6.90 |
| sorting nexin 5 | SNX5 | NM_014426 // NM_152227 | FLJ10931 | 3899346 | chr20 | - | 17855335 | 17897603 | 6.89 |
| KIAA0179 | KIAA0179 | NM 015056 | - | 3923218 | chr21 | + | 43903644 | 43940364 | 6.87 |
| protease, serine, 23 | PRSS23 | NM_007173 | MGC5107 // SIG13 // SPUVE // ZSIG13 | 3343452 | chr11 | + | 86189210 | 86341580 | 6.87 |
| transmembrane, prostate androgen induced RNA | TMEPAI | NM_020182 // NM_199169 // NM_199170 // NM_199171 | PMEPA1 // STAG1 | 3911217 | chr20 | - | 55656877 | 55781123 | 6.87 |
| zinc finger protein 330 | ZNF330 | NM_014487 | HSA6591 // NOA36 | 2745220 | chr4 | + | 142336669 | 142381405 | 6.87 |
| chromodomain helicase DNA binding protein 2 | CHD2 | NM_001042572 // NM_001271 | DKFZp781D1727 | 3609138 | chr15 | + | 91227096 | 91387351 | 6.86 |
| development and differentiation enhancing factor 1 | DDEF1 | NM_018482 | AMAP1 // ASAP1 // KIAA1249 // PAG2 // PAP // ZG14P | 3153428 | chr8 | - | 131133540 | 131525100 | 6.85 |
| mitogen-activated protein kinase kinase kinase 14 | MAP3K14 | NM_003954 | FTDCR1B // HS // HSNIK // NIK | 3759704 | chr17 | - | 40696283 | 40750550 | 6.85 |
| zinc finger protein 289, ID1 regulated | ZNF289 | NM_032389 | FLJ14576 // FLJ26000 // IRZ // Nbla 10535 // Zfp289 | 3371928 | chur11 | - | 47142451 | 47155114 | 6.84 |
| heterogeneous nuclear ribonucleoprotein R | HNRPR | NM_005826 | FLJ25714 // HNRNPR // hnRNP-R | 2401275 | chr1 | - | 23486545 | 23543926 | 6.84 |
| coiled-coil domain containing 47 | CCDC47 | NM_020198 | GK001 // MSTP041 | 3766334 | chr17 | - | 59176353 | 59204726 | 6.84 |
| CUG triplet repeat, RNA binding protein 1 | CUGBP1 | NM_001025596 // NM_006560 // NM_198700 | BRUNOL2 // CUG- BP // CUGBP // NAB50 // hNab50 | 3372253 | chr11 | - | 47444068 | 47543613 | 6.83 |
| cancer susceptibility candidate 3 | CASC3 | NM_007359 | BTZ // MLN51 | 3720739 | chr17 | + | 35518075 | 35586901 | 6.82 |
| SUMO1/sentrin/SMT3 specific peptidase 3 | SENP3 | NM_015670 | DKFZP586K0919 // DKFZp762A152 // SMT3IP1 // SSP3 | 3708798 | chr17 | + | 7405827 | 7416006 | 6.82 |
| MAX dimerization protein 1 | MXD1 | NM_002357 | MAD // MAD 1 // MGC104659 | 2487549 | chr2 | + | 69995330 | 70023575 | 6.82 |
| protein phosphatase 2 (formerly 2A), regulatory subunit B, alpha isoform | PPP2R2A | NM_002717 | B55A // FLJ26613 // MGC52248 // PR52A // PR55A | 3090922 | chr8 | + | 26156860 | 26286261 | 6.82 |
| fusion (involved in t(12;16) in malignant liposarcoma) | FUS | NM_001010850 // NM_004960 | CHOP // FUS-CHOP // FUS1 // TLS // TLS/CHOP | 3656904 | chr16 | + | 31093395 | 31111003 | 6.82 |
| programmed cell death 6 // aryl-hydrocarbon receptor repressor | PDCD6 // AHRR | NM_013232 // NM_020731 | ALG-2 // MGC111017 // MGC119050 // MGC9123 // PEF1B // AHH // AHHR // | 2798586 | chr5 | + | 324634 | 491368 | 6.81 |
| nucleolar protein 5A (56kDa with KKE/D repeat) | NOL5A | NM_006392 | NOP56 | 3873874 | chr20 | + | 2580791 | 2587030 | 6.81 |
| WAS protein family, member 2 | WASF2 | NM_006990 | SCAR2 // WAVE2 // dJ393P12.2 | 2403111 | chr1 | - | 27603331 | 27689256 | 6.81 |
| calmodulin regulated spectrin-associated protein 1 | CAMSAP1 | NM_015447 | DKFZP434F195 // DKFZp434G2311 // FLJ31228 // MGC163452 // PRO2405 // bA100C15.1 | 3229529 | chr9 | - | 137840157 | 137938826 | 6.81 |
| chromosome 18 open reading frame 25 | C18orf25 | NM_001008239 // NM_145055 | ARKL1 // MGC12909 // MGC87799 | 3787031 | chr18 | + | 42008011 | 42123527 | 6.78 |
| cullin 3 | CUL3 | NM_003590 | | 2601544 | chr2 | - | 225025520 | 225158348 | 6.78 |
| methylthioadenosine phosphorylase | MTAP | NM_002451 | MSAP // c86fus | 3164914 | chr9 | + | 21792645 | 22022975 | 6.77 |
| translocase of outer mitochondrial membrane 70 homolog A (S. cerevisiae) | TOMM70A | NM_014820 | FLJ90470 | 2686371 | chr3 | - | 101565001 | 101614635 | 6.76 |
| RNA pseudouridylate synthase domain containing 4 | RPUSD4 | NM_032795 | FLJ14494 | 3396883 | chr11 | - | 125577202 | 125586743 | 6.76 |
| sprouty-related, EVH1 domain containing 1 | SPRED1 | NM_152594 | FLJ33903 | 3589141 | chr15 | + | 36331548 | 36468943 | 6.76 |
| EH-domain containing 4 | EHD4 | NM 139265 | PAST4 | 3620276 | chr15 | - | 39977674 | 40052063 | 6.75 |
| proteasome (prosome, macropain) 26S subunit, non-ATPase, 12 | PSMD12 | XM_001134070 // XM_001134072 // XM_001134073 // NM 002816 | MGC75406 // p55 | 3768103 | chr17 | - | 62764497 | 62804392 | 6.75 |
| nucleoporin 160kDa | NUP160 | NM_015231 | MGC150678 // MGC150679 | 3371986 | chr11 | - | 47169280 | 47858048 | 6.74 |
| EPS8-like 1 | EPS8L1 | NM_017729 // NM_133180 // NM_139204 | DRC3 // EPS8R1 // FLJ20258 // MGC23164 // MGC4642 // PP10566 | 3841949 | chr19 | + | 60273799 | 60291530 | 6.74 |
| chromatin modifying protein 4B | CHMP4B | NM_176812 | C20orf178 // CHMP4A // SNF7-2 | 3882681 | chr20 | + | 31862780 | 31905829 | 6.74 |
| thioredoxin interacting protein | TXNIP | NM_006472 | // Shax1 // EST01027 // HHCPA78 // THIF // VDUP1 | 2356115 | chr1 | + | 144149846 | 144164251 | 6.72 |
| eukaryotic translation initiation factor 1A, X-linked // eukaryotic | EIF1AX // EIF1AP1 | XM_001134077 // NM_001412 // - | EIF1A // EIF4C // eIF-1A // eIF-4C // | 4002148 | chrX | - | 20052567 | 20069880 | 6.71 |
| translation initiation factor 1A mitochondrial ribosomal protein S30 | MRPS30 | NM_016640 | DKFZp566B2024 // MRP-S30 // PAP // PDCD9 | 2808612 | chr5 | + | 44844784 | 45054668 | 6.71 |
| Rho GTPase activating protein 21 | ARHGAP21 | NM_020824 | ARHGAP10 // DKFZp761 L0424 // FLJ33323 | 3281621 | chr10 | - | 24912552 | 25052583 | 6.71 |
| regulator of G-protein signalling 3 | RGS3 | NM_017790 // NM_021106 // NM_130795 // NM_134427 // NM_144488 // | C2PA // FLJ20370 // FLJ31516 // FLJ90496 // PDZ- RGS3 // RGP3 | 3185643 | chr9 | + | 115246842 | 115399812 | 6.71 |
| NFKB inhibitor interacting Ras-like 2 | NKIRAS2 | NM_144489 NM_001001349 // NM_017595 | DKFZP434N1526 // KBRAS2 // MGC74742 // | 3721579 | chr17 | + | 37422156 | 37431177 | 6.70 |
| polypyrimidine tract binding protein 2 | PTBP2 | NM_021190 | FLJ34897 // MIBP // PTB // PTBLP // brPTB // nPTB // nPTB5 // nPTB6 // nPTB7 // nPTB8 | 2348060 | chr1 | + | 96959805 | 97144643 | 6.70 |
| splicing factor 1 | SF1 | NM_004630 // NM_201995 // NM_201997 // NM_201998 | D11S636 // ZFM1 // ZNF162 | 3377044 | chr11 | - | 64287827 | 64302817 | 6.69 |
| Nipped-B homolog (Drosophila) // nuclear receptor binding protein 1 | NIPBL // NRBP1 | NM_015384 // NM_133433 // NM_013392 | CDLS // DKFZp434L1319 // FLJ11203 // FLJ12597 // FLJ13354 // FLJ13648 // FLJ44854 // IDN3 // IDN3-B // BCON3 // FLJ27109 // FLJ35541 // MADM // MUDPNP // NRBP | 2806799 | chr5 | + | 36894163 | 37115932 | 6.68 |
| ring finger and repeat WD domain 3 | RFWD3 | NM 018124 | FLJ10520 // RNF201 | 3699044 | chr16 | - | 73212799 | 73258283 | 6.68 |
| BCL2-associated athanogene | BAG1 | NM_004323 | - | 3203482 | chr9 | - | 33237838 | 33255392 | 6.68 |
| chromosome 6 open reading frame 111 // ubiquitin specific peptidase 45 | C6orf111 // USP45 | NM_032870 // XM_371838 // XM_931094 // XM_931115 // XM_931124 // XM_939661 // XM_944255 // XM_944271 // XM_944273 // NM_001080481 | DKFZp564B0769 // FLJ14752 // FLJ14853 // FLJ14992 // FLJ90147 // HSPC306 // MGC104269 // RP11- 98I9.2 // SRrp130 // bA98I9.2 // MGC14793 | 2966253 | chr6 | - | 99952648 | 99980235 | 6.68 |
| dynactin 5 (p25) | DCTN5 | NM _032486 | MGC3248 // p25 | 3653042 | chr16 | + | 23560236 | 23588668 | 6.67 |
| integrator complex subunit 10 | INTS10 | NM_018142 | C8orf35 // FLJ10569 //INT10 | 3088405 | chr8 | + | 19666766 | 19753848 | 6.67 |
| arrestin domain containing 3 | ARRDC3 | NM_020801 | KIAA1376 // TLIMP | 2866704 | chr5 | - | 90700311 | 90782084 | 6.67 |
| CD59 molecule, complement regulatory protein | CD59 | NM_000611 // NM_203329 // NM_203330 // NM_203331 | 16.3A5 // EJ16 // EJ30 // EL32 // G344 // MGC2354 // MIC11 // MIN1 // MIN2 // MIN3 // MSK21 // PROTECTIN // p18- | 3368707 | chr11 | - | 33676397 | 33714806 | 6.65 |
| opioid growth factor receptor | OGFR | NM_007346 | | 3892941 | chr20 | + | 60906461 | 60915796 | 6.65 |
| chromosome 20 open reading frame 11 | C20orf11 | NM_017896 | TWA1 | 3893072 | chr20 | + | 61039681 | 61050570 | 6.63 |
| ERBB receptor feedback inhibitor 1 | ERRFI1 | NM_018948 | GENE-33 // MIG-6 // MIG6 // RALT | 2395177 | chr1 | - | 7987071 | 8194824 | 6.62 |
| DEAD (Asp-Glu-Ala-Asp) box polypeptide 5 | DDX5 | NM_004396 | DKFZp686J01190 // G17P1 // HLR1 // HUMP68 // p68 | 3766893 | chr17 | - | 59924843 | 59935796 | 6.62 |
| chaperonin containing TCP1, subunit 2 (beta) | CCT2 | NM_006431 | 99D8.1 // CCT-beta // CCTB // MGC142074 // MGC142076 // PRO1633 // TCP-1- beta | 3421630 | chr12 | + | 68265508 | 68281612 | 6.62 |
| TROVE domain family, member 2 | TROVE2 | NM_001042369 // NM_001042370 // NM_004600 | SSA2 | 2372924 | chr1 | + | 191281274 | 191327514 | 6.62 |
| casein kinase 1, delta | CSNK1D | XM_001132734 // NM_001893 // NM_139062 | HCKID | 3774823 | chr17 | - | 77789955 | 77824878 | 6.62 |
| nucleoside phosphorylase | NP | NM_000270 | MGC117396 // MGC125915 // MGC125916 // PNP // PRO1837 // PUNP | 3527514 | chr14 | + | 20007398 | 20015985 | 6.61 |
| ubiquitin C | UBC | XM_001132949 // NM 021009 | HMG20 | 3476741 | chr12 | - | 123962213 | 123965631 | 6.61 |
| general transcription factor IIF, polypeptide 2, 30kDa | GTF2F2 | NM_004128 | BTF4 // RAP30 // TF2F2 // TFIIF | 3488094 | chr13 | + | 44592617 | 44756234 | 6.60 |
| RAS p21 protein activator (GTPase activating protein) 1 | RASA1 | NM_002890 // NM_022650 | CM-AVM // CMAVM // DKFZp434N071 // GAP // PKWS // RASA // RASGAP // p120GAP | 2819044 | chr5 | + | 86599391 | 86724647 | 6.60 |
| chromosome 8 open reading frame | C8orf53 | NM_032334 | MGC14595 | 3112543 | chr8 | + | 117847923 | 117930859 | 6.60 |
| activator of basal transcription 1 | ABT1 | NM_013375 | hABT1 | 2899519 | chr6 | + | 26661709 | 26708945 | 6.59 |
| DEAD (Asp-Glu-Ala-Asp) box polypeptide 50 | DDX50 | NM_024045 | GU2 // GUB // MGC3199 // RH- II/GuB | 3250019 | chr10 | + | 70330615 | 70377158 | 6.58 |
| component of oligomeric golgi complex 1 | COG1 | NM_018714 | DKFZp762L1710 // KIAA1381 // LDLB | 3733938 | chr17 | + | 68695573 | 68739952 | 6.58 |
| adaptor-related protein complex 1, gamma 1 subunit | AP1G1 | NM_001030007 // NM_001128 | ADTG // CLAPG1 // MGC18255 | 3697799 | chr16 | - | 70320417 | 70400594 | 6.57 |
| UTP15, U3 small nucleolar ribonuclooprotein, homolog (S. cerevisiae) | UTP15 | NM_032175 | FLJ12787 | 2815455 | chr5 | + | 72897308 | 72915109 | 6.57 |
| PR domain containing 4 // protein kinase, cAMP-dependent, regulatory, type II, beta | PRDM4 // PRKAR2B | NM_012406 // NM_002736 | MGC45046 // PFM1 // PRKAR2 // RII- BETA | 3470037 | chr12 | - | 106650784 | 106679235 | 6.56 |
| nuclear transcription factor, X-box binding-like 1 | NFXL1 | NM_152995 | FLJ16294 // HOZFP | 2768273 | chr4 | - | 47543715 | 47612453 | 6.55 |
| YTH domain family, member 3 | YTHDF3 | NM_152758 | FLJ31657 | 3100909 | chr8 | + | 64213088 | 64392289 | 6.55 |
| MAP/microtubule affinity-regulating kinase 2 | MARK2 | NM_001039468 // NM_001039469 // NM_004954 // NM_017490 | EMK1 // MGC99619 // PAR-1 | 3334025 | chr11 | + | 63362634 | 63435067 | 6.55 |
| GC-rich promoter binding protein 1 | GPBP1 | NM_022913 | DKFZp761C169 // GPBP // MGC126339 | 2810458 | chr5 | + | 56504841 | 56597388 | 6.55 |
| CTD (carboxy-terminal domain, RNA polymerase II, polypeptide A) phosphatase, subunit 1 | CTDP1 | NM_004715 // NM_048368 | CCFDN // FCP1 | 3795312 | chr18 | + | 75540799 | 75617601 | 6.54 |
| protein tyrosine phosphatase, non-receptor type 12 | PTPN12 | NM_002835 | PTP-PEST // PTPG1 | 3009959 | chr7 | + | 77004361 | 77123074 | 6.54 |
| ubiquitin-conjugating enzyme E2M (UBC12 homolog, yeast) // ubiquitin-conjugating enzyme E2M pseudogene 1 | UBE2M // UBE2MP1 | NM_003969 // NR_002837 | UBC-RS2 // UBC12 // hUbc12 // - | 3872999 | chr19 | - | 63758732 | 63762147 | 6.54 |
| wingless-type MMTV integration site family, member 11 | WNT11 | NM_004626 | HWNT11 // MGC141946 // MGC141948 | 3382523 | chr11 | - | 75542387 | 75599441 | 6.53 |
| chromosome 14 open reading frame 32 | C14orf32 | NM_144578 | MGC23138 // MISS // c14_5346 | 3536663 | chr14 | + | 54588114 | 54606655 | 6.53 |
| signal sequence receptor, beta (translocon-associated protein beta) | SSR2 | XM_001128341 // NM_003145 | DKFZp686F19123 // HSD25 // TLAP // TRAPB | 2437871 | chr1 | - | 154245480 | 154257371 | 6.52 |
| RAS and EF-hand domain containing | RASEF | NM 152573 | FLJ31614 // RAB45 | 3211938 | chr9 | - | 84772206 | 84937805 | 6.52 |
| cold shock domain protein A | CSDA | NM_003651 | CSDA1 // DBPA // ZONAB | 3444252 | chr12 | - | 10742960 | 10775038 | 6.51 |
| leucine-rich PPR-motif containing | LRPPRC | NMJ_133259 | CLONE-23970 // GP130 // LRP130 // LSFC | 2550790 | chr2 | - | 43961504 | 44085128 | 6.51 |
| oxysterol binding protein | OSBP | NM_002556 | OSBP1 | 3374698 | chr11 | - | 59098455 | 59140193 | 6.51 |
| MAP/microtubule affinity-regulating kinase 3 | MARK3 | NM_002376 | CTAK1 // KP78 // PAR1A | 3553690 | chr14 | + | 102921419 | 103370656 | 6.50 |
| oxidation resistance 1 | OXR1 | NM_181354 | FLJ10125 // FLJ38829 // FLJ41673 // FLJ42450 // FLJ45656 // Nbla00307 | 3110999 | chr8 | + | 107351659 | 107834092 | 6.50 |
| ATG16 autophagy related 16-like 1 (S. cerevisiae) | ATG16L1 | NM_198890 // NM_017974 // NM_030803 | APG16L // ATG16L // FLJ00045 // FLJ10035 // FLJ10828 // FLJ22677 // WDR30 | 2532793 | chr2 | + | 233825030 | 233869050 | 6.50 |
| ankyrin repeat domain 17 | ANKRD17 | NM_032217 // NM_198889 | FLJ22206 // GTAR // KIAA0697 // NY- BR-16 | 2773023 | chr4 | - | 74158547 | 74343428 | 6.50 |
| small nuclear ribonucleoprotein polypeptide B" | SNRPB2 | NM_003092 // NM_198220 | MGC24807 // MGC45309 | 3877776 | chr20 | + | 16645421 | 16670916 | 6.49 |
| protein kinase, cAMP-dependent, regulatory, type 1, alpha (tissue specific extinguisher 1) | PRKAR1A | NM_002734 // NM_212471 // NM_212472 | CAR // CNC // CNC1 // DKFZp779L0468 // MGC17251 // PKR1 | 3732885 | chr17 | + | 64019700 | 64059052 | 6.49 |
| chromosome 20 open reading frame 20 | C20orf20 | NM_018270 | Eaf7 // FLJ10914 // MRG 15SP // MRGBP | 3892918 | chr20 | + | 60898107 | 60903524 | 6.49 |
| ADP-ribosylation-like factor 6 interacting protein 4 | ARL6IP4 | NM_001002251 // NM_001002252 // NM_016638 // NM_018694 | MGG814 // SR-25 // SRp25 | 3435681 | chr12 | + | 122030886 | 122033406 | 6.48 |
| pre-mRNA cleavage factor 1, 59 kDa subunit | FLJ12529 | NM_024811 | FLJ39024 // MGC9315 | 3375340 | chr11 | - | 60926704 | 60954030 | 6.48 |
| RNA binding motif protein 17 | RBM17 | NM_032905 | MGC14439 // SPF45 | 3233547 | chr10 | + | 6170982 | 6225410 | 6.47 |
| actin binding LIM protein family, member 3 | ABL1M3 | NM_014945 | HMFN1661 | 2834863 | chr5 | + | 148497619 | 148620192 | 6.47 |
| asparagine synthetase domain containing 1 | ASNSD1 | NM_019048 | FLJ20752 // NBLA00058 // NS3TP1 | 2519860 | chr2 | + | 190198920 | 190244286 | 6.46 |
| poly(A) polymerase alpha | PAPOLA | NM_032632 | MGC5378 // PAP | 3550392 | chr14 | + | 96038038 | 96104627 | 6.46 |
| transmembrane protein 87A | TMEM87A | NM_015497 | DKFZP564G2022 | 3620515 | chr15 | - | 40289650 | 40353024 | 6.46 |
| KIAA0907 | KIAA0907 | NM_014949 | RP11-336K24.1 | 2437753 | chr1 | - | 154149414 | 154174836 | 6.45 |
| DEAD (Asp-Glu-Ala-Asp) box | DDX10 | NM_004398 | HRH-J8 | 3347831 | chr11 | + | 108041010 | 108316858 | 6.45 |
| polypeptide 10 protein tyrosine phosphatase, non- | PTPN1 | NM_002827 | PTP1B | 3888721 | chr20 | + | 48560298 | 48634700 | 6.45 |
| receptor type 1 insulin-like growth factor 2 mRNA | IGF2BP2 | NM_001007225 // NM 006548 | IMP-2 // IMP2 // VICKZ2 // p62 | 2708922 | chr3 | - | 186844235 | 187025506 | 6.45 |
| binding protein 2 zinc finger protein 592 | ZNF592 | NM_014630 | KIAA0211 // MGC138437 // MGC138439 | 3605832 | chr15 | + | 83092594 | 83150653 | 6.44 |
| HIV-1 Rev binding protein | HRB | XM_941338 // NM_004504 | MGC116938 // MGC116940 // RAB // RIP | 2530599 | chr2 | + | 228045122 | 228134167 | 6.43 |
| mitogen-activated protein kinase kinase kinase 4 | MAP3K4 | NM_005922 // NM_006724 | FLJ42439 // KIAA0213 // MAPKKK4 // MEKK4 // MTK1 // PRO0412 | 2934801 | chr6 | + | 161332723 | 161458399 | 6.42 |
| ubiquitin specific peptidase 15 | USP15 | NM_006313 | KIAA0529 // MGC131982 // MGC149838 // MGC74854 // UNPH4 | 3419147 | chr12 | + | 60940328 | 61086166 | 6.41 |
| cullin 1 | CUL1 | NM_003592 | MGC149834 // MGC149835 | 3030285 | chr7 | + | 148026213 | 148129128 | 6.41 |
| family with sequence similarity 104, | FAM104A | NM_032837 | FLJ14775 | 3769969 | chr17 | - | 68715100 | 68740203 | 6.41 |
| member A | NUP88 | NM_002532 | MGC8530 | 3742652 | chr17 | - | 5204992 | 5264204 | 6.41 |
| nucleoporin 88kDa kinesin family member 22 | KIF22 | NM_007317 | KID // KNSL4 // OBP // OBP-1 // | 3655628 | chr16 | + | 29708911 | 29724778 | 6.40 |
| zinc finger protein 313 3 | ZNF313 | NM_018683 | RNF114 | 3888474 | chr20 | + | 47986321 | 48003818 | 6.39 |
| ATG3 autophagy related 3 homolog (S. cerevisiae) | ATG3 | NM_022488 | APG3 // APG3L // DKFZp564M1178// FLJ22125 // MGC15201 // PC3-96 | 2688759 | chr3 | - | 113726408 | 113769332 | 6.39 |
| PTPRF interacting protein, binding protein 1 (liprin beta 1) | PPFIBP1 | NM_003622 // NM_177444 | L2 // hSGT2 // hSgt2p | 3409211 | chr12 | + | 27567649 | 27739981 | 6.39 |
| flotillin 1 | FLOT1 | NM_005803 | - | 2948587 | chr6 | - | 30796156 | 30818490 | 6.38 |
| isoleucyl-tRNA synthetase | IARS | NM_002161 // NM_013417 | FLJ20736 // IARS1 // ILRS // PRO0785 | 3214668 | chr9 | - | 94012330 | 94096287 | 6.38 |
| TM2 domain containing 2 | TM2D2 | NM_001024380 // NM_001024381 // NM_031940 // NM_078473 | BLP1 // MGC125813 // MGC125814 | 3132333 | chr8 | - | 38965485 | 38973467 | 6.38 |
| hypothetical protein MGC22014 | MGC22014 | XM_371501 // XM_942026 | KIAA0401 | 2489071 | chr2 | + | 74066761 | 74188804 | 6.37 |
| THO complex 1 | THOC1 | NM_005131 | HPR1 // P84 // P84N5 | 3795680 | chr18 | - | 201783 | 258461 | 6.36 |
| chromosome 14 open reading frame 129 | C14orf129 | NM_016472 | HSPC210 // MGC4945 | 3550328 | chr14 | + | 95899075 | 95923378 | 6.35 |
| vascular endothelial zinc finger 1 | VEZF1 | NM_007146 | DB1 // ZNF161 | 3764066 | chr17 | - | 53359568 | 53420600 | 6.34 |
| heat shock 70kDa protein 8 // Fc fragment of lgG, low affinity IIIb, receptor(CD16b) | HSPA8 // FCGR3B | NM_006597 // NM_153201 // NM_000570 | HSC54 // HSC70 // HSC71 // HSP71 // HSP73 // HSPA10 // LAP1 // MGC131511 // MGC29929 // NIP71 // CD16 // CD16b // FCG3 // FCGR3 | 3395416 | chr11 | - | 122429309 | 122438895 | 6.33 |
| cullin-associated and neddylation-dissociated 1 | CAND1 | NM_018448 | DKFZp434M1414 // FLJ10114 // FLJ10929 // FLJ38691 // FLJ90441 // KIAA0829 // TIP120 // TIP120A | 3420713 | chr12 | + | 65946048 | 65994719 | 6.32 |
| kelch repeat and BTB (POZ) domain containing 4 | KBTBD4 | NM_016506 // NM_018095 | BKLHD4 // FLJ10450 // HSPC252 | 3372337 | chr11 | - | 47550326 | 47557362 | 6.32 |
| karyopherin alpha 4 (importin alpha 3) | KPNA4 | NM_002268 | IPOA3 // MGC12217 // MGC26703 // QIP1 // SRP3 | 2703217 | chr3 | - | 161663997 | 161766070 | 6.31 |
| chromosome 12 open reading frame 52 | C12orf52 | NM_032848 | FLJ14827 | 3432641 | chr12 | + | 112107758 | 112114543 | 6.31 |
| aurora kinase A // serine/threonine kinase 6 pseudogene | AURKA // STK6P | NM_003600 // NM_198433 // NM_198434 // NM_198435 // NM_198436 // NM_198437 // NR_001587 | AIK // ARK1 // AURA // AURORA2 // BTAK // MGC34538 // STK15 // STK6 // STK7 // | 3910785 | chr20 | - | 54377855 | 54402553 | 6.30 |
| eukaryotic translation initiation factor 2, subunit 1 alpha, 35kDa | EIF2S1 | NM_004094 | EIF-2 // EIF-2A // EIF-2alpha // EIF2 // EIF2A | 3541137 | chr14 | + | 66896518 | 66922983 | 6.30 |
| ubiquitin specific peptidase 7 (herpes virus-associated) | USP7 | NM_003470 | HAUSP // TEF1 | 3679564 | chr16 | - | 8893458 | 8964832 | 6.30 |
| SH3 domain containing ring finger 2 | SH3RF2 | NM_152550 | FLJ23654 // MGC149788 // MGC149789 // MGC90410 // | 2833924 | chr5 | + | 145296335 | 145441529 | 6.30 |
| hydroxymethylbilane synthase | HMBS | NM_000190 // NM_001024382 | PBG-D // PBGD // UPS | 3351841 | chr11 | + | 118460803 | 118470689 | 6.30 |
| eukaryotic translation initiation factor 4E | EIF4E | M_001968 | CBP // E1F4E1 // EIF4EL1 // EIF4F // MGC111573 | 2778980 | chr4 | - | 99888790 | 100070801 | 6.30 |
| splicing factor, arginine/serine-rich 15 | SFRS15 | NM_020706 | DKFZP434E098 // FLJ23364 // KIAA1172 // SRA4 | 3928866 | chr21 | - | 31965222 | 32077764 | 6.30 |
| gem (nuclear organelle) associated protein 5 | GEMIN5 | NM_016465 | DKFZP586M1824 // MGC142174 | 2882897 | chr5 | - | 154247066 | 154309713 | 6.30 |
| TNF receptor-associated factor 6 | TRAF6 | NM_004620 // NM 145803 | MGC:3310 // RNF85 | 3369890 | chr11 | - | 36465173 | 36488940 | 6.29 |
| neurofibromin 2 (bilateral acoustic neuroma) | NF2 | NM_000268 // NM_016418 // NM_181825 // NM_181828 // NM_181829 // NM_181830 // NM_181831 // NM_181832 // NM_181833 | ACN // BANF // Merlin // SCH | 3942062 | chr22 | + | 28329446 | 28424577 | 6.29 |
| structural maintenance of chromosomes 3 | SMC3 | NM_005445 | BAM // BMH // CSPG6 // HCAP // SMC3L1 | 3263790 | chr10 | + | 112317039 | 112365483 | 6.28 |
| dynamin binding protein | DNMBP | NM_015221 | KIAA1010 // TUBA | 3303165 | chr10 | - | 101624846 | 101759656 | 6.28 |
| cystatin B (stefin B) | CSTB | NM_000100 | CST6 // EPM1 // PME // STFB | 3934245 | chr21 | - | 44016298 | 44020687 | 6.28 |
| mucin 20, cell surface associated | MUC20 | NM_152673 | FLJ14408 // KIAA1359 | 2659039 | chr3 | + | 196867150 | 196953662 | 6.27 |
| tuberous sclerosis 1 | TSC1 | NM**_**000368 // NM_001008567 | KIAA0243 // LAM // MGC86987 // TSC | 3228373 | chr9 | - | 134756560 | 134809831 | 6.27 |
| abl interactor 2 | AB12 | XM_001126750// NM_005759 | ABI-2 // ABI2B // AIP-1 // AbIBP3 // SSH3BP2 // argBPIA // argBPIB | 2523689 | chr2 | + | 203901161 | 204005128 | 6.27 |
| golgi reassembly stacking protein 2, 55kDa // dehydrogenase/reductase (SDR family) member 9 | GORASP2 // DHRS9 | NM_015530 // NM_005771 // NM_199204 | DKFZP434D156 // FLJ13139 // GOLPH6 // GRASP55 // GRS2 // p59 // 3alpha-HSD // **RDH15** // RDHL // RETSDR8 | 2515050 | chr2 | + | 171457150 | 171551619 | 6.27 |
| ATPase, Ca++ transporting, cardiac muscle, slow twitch 2 | ATP2A2 | NM_001681 // NM_170665 | ATP2B // DAR // DD //MGC45367// SERCA2 | 3431483 | chr12 | + | 109203332 | 109352495 | 6.26 |
| hypothetical protein HSPC148 | HSPC148 | NM_016403 | - | 3387171 | chr11 | - | 94322815 | 94346692 | 6.26 |
| EH-domain containing 1 | EHD1 | NM_006795 | FLJ42622 // FLJ44618 // H-PAST // HPAST1 // PAST // PAST1 | 3377226 | chr11 | - | 64376790 | 64404202 | 6.26 |
| X-box binding protein 1 | XBP1 | NM_001079539 // NM 005080 | TREB5 // XBP2 | 3956589 | chr22 | - | 27520140 | 27526564 | 6.25 |
| enhancer of zeste homolog 2 (Drosophila) | EZH2 | NM_004456 // NM 152998 | ENX-1 // EZH1 // MGC9169 | 3078348 | chr7 | - | 148135436 | 148212647 | 6.25 |
| SEC24 related gene family, member B (S. cerevisiae) | SEC24B | XM_001130118 // NM_001042734 // NM_006323 | MGC48822 // SEC24 | 2739079 | chr4 | + | 110510304 | 110681811 | 6.24 |
| RAB, member RAS oncogene family-like 5 | RAB L5 | NM_022777 | DKFZp761N0823 // FLJ13225 // FLJ14117 | 3064638 | chr7 | - | 100742601 | 100751793 | 6.24 |
| RCD1 required for cell differentiation homolog (S. pombe) // phospholipase C, delta 4 | RQCD1 // PLCD4 | NM_005444 // NM_032726 | CNOT9 // RCD1 // RCD1+ // MGC12837 | 2527856 | chr2 | + | 219141552 | 219170027 | 6.24 |
| poly(rC) binding protein 2 | PCBP2 | NM_005016 // NM_031989 | HNRPE2 // MGC110998 // hnRNP-E2 | 3416036 | chr12 | + | 52132173 | 52161417 | 6.23 |
| solute carrier family 30 (zinc transporter), member 9 | SLC30A9 | NM_006345 | C4orf1 // GAC63 // HUEL // ZNT9 | 2725381 | chr4 | + | 41684531 | 41812215 | 6.23 |
| zinc finger protein 192 | ZNF192 | NM_006298 | LD5-1 // ZKSCAN8 | 2900299 | chr6 | + | 28217285 | 28232872 | 6.23 |
| jumonji domain containing 2A | JMJD2A | NM**_**014663 | JHDM3A // JMJD2 // KIAA0677 | 2333429 | chr1 | + | 43888407 | 43943994 | 6.23 |
| HtrA serine peptidase 2 | HTRA2 | NM_013247 // NM 145074 | OMI // PARK13 // PRSS25 | 2489411 | chr2 | + | 74609783 | 74614240 | 6.23 |
| epidermal growth factor receptor pathway substrate 8 | EPS8 | NM_004447 | - | 3445908 | chr12 | - | 15664367 | 15833589 | 6.23 |
| glutamine and serine rich 1 | QSER1 | NM_024774 // NM_001076786 | FLJ21924 | 3325768 | chr11 | + | 32871321 | 32971438 | 6.22 |
| phosphomannomutase 2 | PMM2 | NM_000303 | CDG1 // CDG1a // CDGS | 3647504 | chr16 | + | 8798971 | 8850664 | 6.22 |
| DEAH (Asp-Glu-Ala-His) box polypeptide 15 | DHX15 | NM_001358 | DBP1 // DDX15 // HRH2 // PRP43 // PRPF43 // PrPp43p | 2763805 | chr4 | - | 24128159 | 24250940 | 6.22 |
| transforming growth factor, beta receptor II (70/80kDa) | TGFBR2 | NM_001024847 // NM_003242 | AAT3 // FAA3 // HNPCC6 // MFS2 // RIIC // TAAD2 // TGFR-2 // TGFbeta-RII | 2615360 | chr3 | + | 30492214 | 30710745 | 6.21 |
| tropomyosin 1 (alpha) | TPM1 | NM_000366 // NM_001018004 // NM_001018005 // NM_00J018006 // NM_001018007 // NM_001018008 // NM_001018020 | HTM-alpha // TMSA // TPM1-alpha // TPM1-kappa | 3597338 | chr15 | + | 61105711 | 61151151 | 6.21 |
| HLA-B associated transcript 4 | BAT4 | NM_033177 | ANKRD59 // D6S54E // G5 // GPANK1 // GPATCH10 | 2949210 | chr6 | - | 31736995 | 31742029 | 6.21 |
| COX4 neighbour | COX4NB | NM_006067 | C16orf4 // NOC4 | 3703164 | chr16 | - | 84362936 | 84391266 | 6.21 |
| RNA binding motif protein 7 | RBM7 | NM_016090 | FLJ11153 | 3349918 | chr11 | + | 113776539 | 113790335 | 6.20 |
| suppressor of Ty 4 homolog 1 (S. cerevisiae) | SUPT4H1 | NM_003168 | SPT4H // SUPT4H | 3764384 | chr17 | - | 53777538 | 53784705 | 6.19 |
| PRP4 pre-mRNA processing factor 4 homolog B (yeast) | PRPF4B | NM_003913 | KlAA0536 // PR4H // PRP4 // PRP4H // PRP4K // dJ1013A10.1 | 2892738 | chr6 | + | 3966500 | 4010215 | 6.19 |
| phosphoglycerate kinase 1 | PGK1 | NM_000291 | MGC117307 // MGC142128 // MGC8947 // MIG10 // PGKA | 3982462 | chrX | + | 77207320 | 77269191 | 6.19 |
| ATP-binding cassette, sub-family C (CFTR/MRP), member 1 | ABCC1 | NM_004996 // NM_019862 // NM_019898 // NM_019899 // NM_019900 // NM_019901 // NM_019902 | ABC29 // ABCC // DKFZp686N04233 // DKFZp781G125 // GS-X // MRP // MRP1 | 3649890 | chr16 | + | 15950935 | 16144419 | 6.18 |
| transmembrane protein 123 | TMEM123 | NM_052932 | KCT3 // PORIMIN // PORMIN | 3388631 | chr11 | - | 101772285 | 101828945 | 6.18 |
| SEH1-like (S. cerevisiae) | SEH1L | NM_001013437 // NM 031216 | SEC13L // SEH1A // SEH1B // Seh1 | 3779756 | chr18 | + | 12886655 | 12981306 | 6.17 |
| ferritin, heavy polypeptide-like 7 // ferritin, heavy polypeptide 1 // ferritin, heavy polypeptide-like 3 // ferritin, heavy polypeptide pseudogene 1 // ferritin, heavy polypeptide-like 12 // ferritin, heavy polypeptide-like 11 // ferritin, heavy | FTHL7 // FTH1 // FTHL3 // FTHP1 // FTHL12 // FTHL11 // FTHL8 | NR_002202 // NM_002032 // NR_002201 // - // NR_002205 // NR_002204 // NR_002203 | - // FTH // FTHL6 // MGC104426 // PIG15 // PLIF // FTHL5 | 3375648 | chr11 | - | 61488337 | 61492603 | 6.16 |
| DNA polymerase-transactivated protein 6 | DNAPTP6 | NM_015535 | DKFZp564A2416 | 2522094 | chr2 | + | 200879035 | 201053805 | 6.15 |
| serologically defined colon cancer antigen 10 | SDCCAG10 | NM_005869 | NY-CO-10 | 2812120 | chr5 | + | 64100099 | 64417210 | 6.15 |
| SHC (Src homology 2 domain containing) transforming protein 1 | SHC1 | NM_003029 // NM_183001 | FLJ26504 // SHC // SHCA // p52SHC // p66 // p66SHC | 2436985 | chr1 | - | 153201334 | 153213510 | 6.15 |
| tetratricopeptide repeat domain 17 | TTC17 | NM_018259 | DKFZp686D20222 // FLJ10890 // FLJ13099 // FLJ23673 | 3327948 | chr11 | + | 43337004 | 43473027 | 6.13 |
| polymerase (RNA) III (DNA directed) E (80kD) | POLR3E | NM_018119 | RPC5 // SIN | 3652489 | chr16 | + | 22216140 | 22253905 | 6.13 |
| polypeptide bromodomain containing 1 | BRD1 | NM_014577 | BRL // BRPF1 // BRPF2 // DKFZp686F0325 | 3965314 | chr22 | - | 48552694 | 48636916 | 6.12 |
| SET translocation (myeloid leukemia-associated) | SET | NM_003011 | 2PP2A // 12PP2A // IGAAD // PHAPII // TAF-IBETA | 3190659 | chr9 | + | 130485775 | 130498483 | 6.12 |
| glutamate-cysteine ligase, catalytic subunit | GCLC | NM_001498 | GCS // GLCL // GLCLC | 2957700 | chr6 | - | 53438876 | 53589510 | 6.12 |
| SCY1-like 2 (S. cerevisiae) | SCYL2 | NM_017988 | CVAK104 // FLJ10074 // | 3428131 | chr12 | + | 99185070 | 99260564 | 6.12 |
| ribosomal protein L8 | RPLB | NM_000973 // NM_033301 | - | 3159040 | chr8 | - | 145985994 | 145988615 | 6.11 |
| protein tyrosine phosphatase type IVA, member 1 | PTP4A1 | NM_003463 | DKFZp779M0721 // HH72 // PRL-1 // PRL1 // PTP(CAAX1) // PTPCAAX1 | 2911903 | chr6 | + | 64289625 | 64351448 | 6.10 |
| Rac GTPase activating protein 1 | RACGAP1 | NM_013277 | HsCYK-4 // ID-GAP // MgcRacGAP | 3454223 | chr12 | - | 48657002 | 48705537 | 6.09 |
| cold shock domain containing E1, RNA-binding // neuroblastoma RAS viral (v-ras) oncogene homolog | CSDE1 // NRAS | NM_001007553 // NM_007158 // NM_002524 | D1S155E // DKFZp779B0247 // DKFZp779J1455 // FLJ26882 // RP5- 1000E10.3 // UNR // N-ras // NRAS1 | 2429277 | chr1 | - | 115061068 | 115102225 | 6.09 |
| translocation protein 1 | TLOC1 | NM_003262 | Dtrp1 // FLJ32803 // HTP1 // SEC62 | 2651782 | chr3 | + | 171166446 | 171194653 | 6.09 |
| muscleblind-like (Drosophila) | MBNL1 | NM_021038 // NM_207292 // NM_207293 // NM_207294 // NM_207295 // NM_207296 // NM 207297 | DKFZp686P06174 // EXP // EXP35 // EXP40 // EXP42 // KIAA0428 // MBNL | 2648141 | chr3 | + | 153444327 | 153670549 | 6.08 |
| chromosome X open reading frame 40A // chromosome X open reading frame 40B | CXorf40A // CXorf40B | NM-76124 // NM_001013845 | CXorf40 // EOLAI // | 3994451 | chrX | + | 148413672 | 148439412 | 6.08 |
| ubiquitin B // chromosome 17 open reading frame 45 | UBB // C17orf45 | NM_018955 // NM_152350 | FLJ25987 // MGC8385 // FLJ25777 // MGC40157 | 3712041 | chr17 | + | 16223047 | 16228977 | 6.08 |
| solute carrier family 7 (cationic amino acid transporter, y+ system), 1 | SLC7A1 | M_003045 | ATRC1 // CAT-1 // ERR // HCATI // REC1L | 3507710 | chr13 | - | 28981563 | 29067721 | 6.07 |
| member smu-1 suppressor of mec-8 and unc-52 homolog (C. elegans) | SMU1 | NM_018225 | BWD // FLJ10805 // FLJ11970 // MGC117363 // RP11- 54K16.3 // SMU-1 | 3203382 | chr9 | - | 33031772 | 33072208 | 6.06 |
| annexin A2 | ANXA2 | NM_001002857 // NM_001002858 // NM_004039 | ANX2 // ANX2L4 // CAL1 H // LIP2 // LPC2 // LPC2D // P36 // PAP-IV | 3627248 | chr15 | - | 58247806 | 58482354 | 6.06 |
| COP9 constitutive photomorphogenic homology subunit 7B (Arabidopsis) | COPS7B | NM_022730 | FLJ12612 // MGC111077 | 2532064 | chr2 | + | 232354256 | 232382342 | 6.06 |
| chromosome 1 open reading frame 174 | C1orf174 | NM_207356 | RP13-531 C17.2 | 2393816 | chr1 | - | 3791939 | 3812760 | 6.06 |
| v-ets erythroblastosis virus E26 | ETS2 | NM_005239 | - | 3921068 | chr21 | + | 39059147 | 39118744 | 6.06 |
| oncogene homolog 2 (avian) actinin, alpha 4 | ACTN4 | NM_004924 | DKFZp686K23158 // FSGS // FSGS1 | 3832643 | chr19 | + | 43830130 | 43913003 | 6.06 |
| protein phosphatase 2 (formerly 2A), subunit, alpha isoform | PPP2CA | NM_002715 | PP2Ac // PP2CA // RP-C | 2876046 | chr5 | - | 133557588 | 133589841 | 6.05 |
| catalytic glycogen synthase kinase 3 beta | GSK3B | NM_002093 | - | 2691014 | chr3 | - | 121018519 | 121297142 | 6.05 |
| hypothetical protein FLJ14154 | FLJ14154 | NM-024845 | - | 3645901 | chr16 | + | 3433714 | 3476953 | 6.04 |
| poliovirus receptor-related 2 (herpesvirus entry mediator B) | PVRL2 | NM_001042724 // NM 002856 | CD112 // HVEB // PRR2 // PVRR2 | 3835814 | chr19 | + | 50041409 | 50084314 4 | 6.04 |
| DnaJ homology subfamily A member 5 | DNAJA5 | NM 001012339 // NM 194283 | - | 2806256 | chr5 | + | 34965441 | 34994822 | 6.03 |
| zinc finger, RAN-binding domain containing 2 | ZRANB2 | NM_005455 // NM_203350 | DKFZp686J1831 // DKFZp686N09117 // FLJ41119 // ZIS // ZIS1 // ZIS2 // ZNF265 | 2418000 | chr1 | - | 71292434 | 71355820 | 6.03 |
| GDP dissociation inhibitor 2 | GDI2 | NM_001494 | FLJ16452 // FLJ37352 // RABGDIB | 3275132 | chr10 | - | 5844502 | 5924081 | 6.03 |
| transmembrane protein 138 | TMEM138 | NM 016464 | HSPC196 | 3332886 | chr11 | + | 60885939 | 60900898 | 6.02 |
| CDC28 protein kinase regulatory subunit 2 | CKS2 | NM_001827 | CKSHS2 | 3178583 | chr9 | + | 91050736 | 91121542 | 6.01 |
| ADAM metallopeptidase domain 10 | ADAM10 | NM_001110 | CD156c // HsT18717 // MADM // kuz | 3626555 | chr15 | - | 56651514 | 56829469 | 6.00 |
| telomeric repeat binding factor 2 | TERF2 | NM 005652 | TRBF2 // TRF2 | 3696571 | chr16 | - | 67946973 | 68001605 | 6.00 |
| elongation factor, RNA polymerase II, | ELL2 | NM 012081 | | 2867873 | chr5 | - | 95246561 | 95323733 | 5.99 |
| proteasome maturation protein | POMP | NM_015932 | 013orf12 // HSPCB014 // PNAS-110 // UMP1 | 3483348 | chr13 | + | 28125188 | 28151050 | 5.99 |
| mitochondrial ribosomal protein L16 | MRPL16 | NM_017840 | FLJ20484 // L16mt // PNAS-111 | 3374856 | chr11 | - | 59330154 | 59334921 | 5.99 |
| glucosaminyl (N-acetyl) transferase 3, mucin type | GCNT3 | NM_004751 | C2/4GnT // C2GnT- M // C2GnT2 // | 3596147 | chr15 | + | 57628756 | 57719710 | 5.98 |
| KIAA0409 | KIAA0409 | NM_015324 | RRP8 | 3360982 | chr11 | - | 6492639 | 6582206 | 5.98 |
| tankyrase 1 binding protein 1, 182kDa | TNKS18P1 | NM_033396 | FLJ45975 // KfAA1741 // TAB182 | 3373675 | chr11 | - | 56823694 | 56848992 | 5.98 |
| polymerase (RNA) I polypeptide E, 53kDa | POLR1E | NM_022490 | FLJ13390 // FLJ13970 // PAF53 // PRAF1 // RP11- 405L18.3 | 3168938 | chr9 | + | 37475945 | 37500379 | 5.97 |
| E3 ubiquitin protein ligase, HECT domain containing, 1 | EDD1 | NM_015902 | DD5 // EDD // FLJ11310 // HYD // KIAA0896 // MGC57263 | 3147321 | chr8 | - | 103333634 | 103493671 | 5.97 |
| G protein-coupled receptor, family C, group 5, member A | GPRC5A | NM_003979 | GPCR5A // RA13 // RAIG 1 | 3405587 | chr12 | + | 12929011 | 12986488 | 5.96 |
| fragile X mental retardation 1 | FMR1 | NM_002024 | FMRP // FRAXA // MGC87458 | 3994100 | chrX | + | 146801098 | 146840329 | 5.96 |
| thyroid hormone receptor associated protein 2 | THRAP2 | NM_015335 | DKFZp781D0112 // FLJ21627 // KIAA1025 // MED13L // PROSIT240 // TRAP240L | 3473083 | chr12 | - | 114868953 | 115419724 | 5.96 |
| epithelial membrane protein 1 | EMP1 | NM_001423 | CL-20 // EMP-1 // TMP | 3405748 | chr12 | + | 13230082 | 13264499 | 5.95 |
| SUMO1/sentrin specific peptidase 5 | SENP5 | NM_152699 | FLJ42398 // MGC27076 | 2659631 | chr3 | + | 198079101 | 198143960 | 5.94 |
| FUS interacting protein (serine/arginine-rich) 1 | FUSIP1 | NM_006625 // NM_054016 | FUSIP2 // NSSR // SFRS13 // SRp38 // SRrp40 // TASR // TASR1 // TASR2 | 4045780 | chr1_ran dom | - | 1493804 | 1514582 | 5.93 |
| UTP14, U3 small nucleolar ribonuclooprotein, homolog A (yeast) | UTP14A | NM_006649 | KIAA0266 // NY-CO- 16 // SDCCAG16 // dJ537K23.3 | 3990566 | chrX | + | 128832975 | 128912087 | 5.93 |
| cell division cycle associated 3 | CDCA3 | NM_031299 | GRCCB // MGC2577 // TOME-1 | 3442322 | chr12 | - | 6824231 | 6831482 | 5.93 |
| zinc finger, AN1-type domain 3 | ZFAND3 | NM_021943 | FLJ13222 // TEX27 | 2905664 | chr6 | + | 37895285 | 38231400 | 5.93 |
| glucocorticoid receptor DNA binding factor 1 | GRLF1 | NM_004491 | GRF-1 // KLAA1722 // MGC10745 // P190-A // P190A // p190RhoGAP | 3837001 | chr19 | + | 52113779 | 52200169 | 5.92 |
| GPI-anchored membrane protein 1 | GPIAP1 | NM_005898 // NM_203364 | GP1P137 // M11S1 // p137GPI | 3326183 | chr11 | + | 34009711 | 34081946 | 5.92 |
| AT hook containing transcription factor 1 | AHCTF1 | XM_001126456 // M_015446 | DKFZp434N093 // ELYS // MST108 // MSTP108 // TMBS62 | 2465324 | chr1 | - | 245069027 | 245162066 | 5.92 |
| aurora kinase B | AURKB | NM_004217 | AIK2 // AIM-1 // AIM1 // ARK2 // AurB // IPL1 // STK12 // STK5 | 3744263 | chr17 | - | 8034636 | 8054649 | 5.92 |
| ariadne homolog 2 (Drosophila) | ARIH2 | NM_006321 | ARI2 // FLJ10938 II FLJ33921 // TRIAD 1 | 2621827 | chr3 | + | 48930742 | 48997971 | 5.91 |
| chromosome 11 open reading frame 68 | C11orf68 | NM_031450 | Bles03 // P5326 | 3378007 | chr11 | - | 65440893 | 65443107 | 5.91 |
| nuclear factor of kappa light polypeptide gene enhancer in B-cells inhibitor, alpha | NFKBIA | NM_020529 | IKBA // MAD-3 // NFKBI | 3561039 | chr14 | - | 34940473 | 34944214 | 5.90 |
| calcyclin binding protein | CACYBP | NM_001007214 // NM_014412 | GIG5 // MGC87971 // PNAS-107 // RP1-102G20.6 // S100A6BP // S1P | 2368198 | chr1 | + | 173235214 | 173247469 | 5.90 |
| zinc finger protein 593 | ZNF593 | NM_015871 | ZT86 | 2326311 | chr1 | + | 26368813 | 26371688 | 5.90 |
| proteasome (prosome, macropain) 265 subunit, non-ATPase, 2 | PSMD2 | NM_002808 | MGC14274 // P97 // S2 // TRAP2 | 2655650 | chr3 | + | 185499199 | 185509504 | 5.89 |
| YY1 transcription factor | YY1 | NM_003403 | DELTA // NF-E1 // UCRBP // YIN- YANG-1 | 3551677 | chr14 | + | 99750383 | 99818868 | 5.89 |
| Smith-Magenis syndrome chromosome region, candidate 7-like | SMCR7L | NM_019008 | FLJ20232 // HSU79252 // dJ1104E15.3 | 3945877 | chr22 | + | 38226061 | 38244079 | 5.88 |
| CDC-like kinase 1 | CLK1 | NM_001024646 // NM_004071 | CLK // CLK/STY // STY | 2594497 | chr2 | - | 201425997 | 201437659 | 5.88 |
| phosphatidylinositol transfer protein, beta | PITPNB | NM_012399 | PI-TP-beta // PtdInsTP // VIB1B B | 3956290 | chr22 | - | 26577440 | 26645487 | 5.87 |
| A kinase (PRKA) anchor protein 8-like | AKAP8L | NM_014371 | DKFZp434L0650 // HAP95 // NAKAP // NAKAP95 | 3853345 | chr19 | - | 15351869 | 15390819 | 5.87 |
| chromosome 17 open reading frame 70 | C17orf70 | NM_025161 | FLJ22175 // FLJ30151 | 3773980 | chr17 | - | 77117366 | 77130137 | 5.87 |
| ring finger protein 34 | RNF34 | NM-025126 // NM_194271 | FLJ21786 // RFI // RIF // RIFF | 3434823 | chr12 | + | 120322070 | 120352805 | 5.87 |
| solute carrier family 7 (cationic amino acid transporter, y+ system), member 6 | SLC7A6 | NM_001076785 // NM_003983 | DKFZp686K15246 // KlAA0245 // LAT-2 // LAT3 // y+LAT-2 | 3666146 | chr16 | + | 66855932 | 66893223 | 5.86 |
| solute carrier family 39 (zinc transporter), member 14 | SLC39A14 | NM_015359 | KIAA0062 // LZT-Hs4 // ZIP14 // cig19 | 3089360 | chr8 | + | 22280765 | 22347587 | 5.86 |
| PI-3-kinase-related kinase SMG-1 | SMG1 | NM_015092 | 61E3.4 // ATX // KIAA0421 // LIP | 3683050 | chr16 | - | 18719381 | 18845318 | 5.86 |
| zinc finger protein 259 | ZNF259 | NM 003904 | MGC110983 // ZPR1 | 3392871 | chr11 | - | 116153652 | 116163944 | 5.86 |
| NADH dehydrogenase (ubiquinone) 1 beta subcomplex, 11, 17.3kDa | NDUFB11 | NM_019056 | ESSS // FLJ20494 // MGC111182 // NP17.3 // Np15 // | 4007009 | chrX | - | 46886563 | 46902114 | 5.86 |
| heterogeneous nuclear ribonucleoprotein U (scaffold attachment factor A) | HNRPU | NM_004501 // NM_031844 | HNRNPU // SAF-A // U21.1 | 2464499 | chr1 | - | 243078713 | 243094575 | 5.85 |
| BCL2-like 1 | BCL2L1 | NM_001191 // NM_138578 | BCL-XL/S // BCL2L // BCLX // Bcl-X // DKFZp781 P2092 // bcl-xL // bcl-xS | 3902489 | chr20 | - | 29715925 | 29775453 | 5.85 |
| stem-loop (histone) binding protein | SLBP | NM_006527 | HBP | 2757319 | chr4 | - | 1664276 | 1684080 | 5.85 |
| retinitis pigmentosa 2 (X-linked recessive) | RP2 | NM_006915 | KIAA0215 // TBCCD2 | 3975869 | chrX | + | 46581319 | 46637601 | 5.85 |
| cullin 4A | CUL4A | NM_001008895 // NM_003589 | - | 3502497 | chr13 | + | 112905835 | 112967356 | 5.85 |
| tripartite motif-containing 28 | TRIM28 | NM_005762 | FLJ29029 // KAP1 // RNF96 // TF1B // TIF1B | 3844238 | chr19 | + | 63740597 | 63753931 | 5.84 |
| DEAD (Asp-Glu-Ala-Asp) box polypeptide 39 | DDX39 | NM_005804 | BAT1 // BAT1L // DDXL // MGC18203 // MGC8417 // URH49 | 3852691 | chr19 | - | 14380562 | 14404078 | 5.84 |
| lectin, galactoside-binding, soluble, 8 (galectin 8) | LGALS8 | NM_006499 // NM_201543 // NM_201544 // NM_201545 | Gal-8 // PCTA-1 // PCTA1 // Po66-CBP | 2386867 | chr1 | + | 234717161 | 234782890 | 5.84 |
| ankyrin repeat domain 10 | ANKRD10 | NM_017664 | DKFZp686B07190 // FLJ20093 | 3525679 | chr13 | - | 110322146 | 110365417 | 5.83 |
| zinc finger and BTB domain containing 9 // synaptic Ras GTPase activating protein 1 homolog (rat) | ZBTB9 // SYNGAP1 | NM_152735 // NM_006772 | MGC23166 // DKFZp761 G1421 // KIAA1938 // RASA1 // RASA5 // | 2903703 | chr6 | + | 33494813 | 33533290 | 5.82 |
| transmembrane protein 70 | TMEM70 | NM_001040613 // NM_017866 | FLJ20533 | 3103494 | chr8 | + | 75046321 | 75057572 | 5.82 |
| aftiphilin | AFTPH | NM_001002243 // NM_017657 // NM_203437 | FLJ20080 // FLJ23793 // MGC33965 // Nbla 10388 | 2485433 | chr2 | + | 64604969 | 64714437 | 5.82 |
| polymerase (RNA) polypeptide A, 194kDa | POLR1A | NM_015425 | DKFZP586M0122 // FLJ21915 // MGC87965 // RPA1 // RPO1-4 | 2562605 | chr2 | - | 86103197 | 86186509 | 5.81 |
| general transcription factor IIIC, polypeptide 5, 63kDa | GTF3C5 | NM_012087 | FLJ20857 // TFIIIC63 // TFIIICepsilon // TFiiiC2-63 | 3192653 | chr9 | + | 134895917 | 134923708 | 5.81 |
| tumor necrosis factor receptor superfamily, member 21 | TNFRSF21 | NM_014452 | BM-018 // DR6 // MGC31965 | 2956052 | chr6 | - | 47307230 | 47385639 | 5.80 |
| sema domain, immunoglobulin domain (Ig), transmembrane domain (TM) and short cytoplasmic domain, (semaphorin) 4B | SEMA48 | NM_020210 // NM_198925 | KIAA1745 // MGC131831 // SEMAC // SemC | 3607927 | chr15 | + | 88529156 | 88573879 | 5.80 |
| mitochondrial ribosomal protein S11 | MRPS11 | NM_022839 // NM_176805 | FLJ22512 // FLJ23406 // HCC-2 | 3607183 | chr15 | + | 86811688 | 86822612 | 5.80 |
| nuclear factor of activated T-cells 5, tonicity-responsive | NFAT5 | NM_006599 // NM_138713 // NM_138714 // NM_173214 // NM 173215 | KIAA0827 // NF-AT5 // NFATL1 // NFATZ // OREBP // TONEBP | 3666779 | chr16 | + | 68156498 | 68296054 | 5.80 |
| nuclear receptor subfamily 2, group F, member 2 | NR2F2 | NM_021005 | ARP1 // COUP-TFII // COUPTFB // MGC117452 // SVP40 // TFCOUP2 | 3610110 | chr15 | + | 94448164 | 94683620 | 5.79 |
| SH3 domain containing ring finger 1 | SH3RF1 | NM_020870 | FLJ21602 // KIAA1494 // POSH // RNF142 // | 2793137 | chr4 | - | 170220651 | 170428725 | 5.78 |
| splicing factor 3b, subunit 3, 130kDa | SF3B3 | NM_012426 | KIAA0017 // RSE1 // SAP130 // SF3b130 // STAF130 | 3667281 | chr16 | + | 69115212 | 69169072 | 5.78 |
| PTC7 protein phosphatase homolog (S. cerevisiae) | PPTC7 | NM_139283 | DKFZp686M07120 // MGC133072 // TA- PP2C | 3471300 | chr12 | - | 109455446 | 109514152 | 5.77 |
| homeodomain interacting protein kinase 1 | HIPK1 | NM_152696 // NM_181358 // NM_198268 // NM_198269 | KIAA0630 // MGC26642 // MGC33446 // MGC33548 // Myak // Nbak2 | 2352758 | chr1 | + | 114273467 | 114322014 | 5.77 |
| nuclear receptor binding protein 1 | NRBP1 | NM_013392 | BCON3 // FLJ27109 // FLJ35541 // MADM // MUDPNP // NRBP | 2474527 | chr2 | + | 27504171 | 27518620 | 5.77 |
| cleavage stimulation factor, 3' pre-RNA, subunit 1, 50kDa | CSTF1 | NM_001033521 // NM_001 033522 // NM_001324 | CstF-50 // CstFp50 | 3890154 | chr20 | + | 54393411 | 54413257 | 5.77 |
| pre-B-cell colony enhancing factor 1 // pre-B cell enhancing factor 1 pseudogene | PBEF1 // RP11- 92J19.4 | NM_005746 // - | 1110035014Rik // DKFZP666B131 // MGC117256 // NAMPT // PBEF // LOC646309 | 3066818 | chr7 | - | 105625846 | 105713266 | 5.77 |
| transmembrane protein 11 | TMEM11 | NM_003876 | C17orf35 // PM1 // PMI | 3749767 | chr17 | - | 21041205 | 21058509 | 5.77 |
| transmembrane protein 115 | TMEM115 | NM 007024 | PL6 | 2675304 | chr3 | - | 50367198 | 50372046 | 5.76 |
| catenin (cadherin-associated protein), alpha-like 1 | CTNNAL1 | NM_003798 | CLLP // alpha-CATU | 3219621 | chr9 | - | 110744011 | 110815625 | 5.76 |
| clathrin, heavy chain (Hc) | CLTC | NM_004859 | CHC17 // CLH-17 // CLTCL2 // Hc // KIAA0034 | 3729172 | chr17 | + | 55052021 | 55127299 | 5.76 |
| cyclin H | CCNH | NM_001239 | CAK // p34 // p37 | 2865860 | chr5 | - | 86707385 | 86782317 | 5.74 |
| SECIS binding protein 2 | SECISBP2 | NM_024077 | DKFZp686C09169 // SBP2 | 3178611 | chr9 | + | 91122988 | 91146214 | 5.74 |
| WD repeat domain 26 | WDR26 | NM 025160 | FLJ21016 // MIP2 | 2458082 | chr1 | - | 222639474 | 222691338 | 5.74 |
| stomatin (EPB72)-like 2 // Fanconi anemia, complementation group G | STOML2 // FANCG | NM_013442 // NM_004629 | HSPC108 // SLP-2 // FAG // XRCC9 | 3204534 | chr9 | - | 35089540 | 35093157 | 5.74 |
| nucleolar and coiled-body phosphoprotein 1 | NOLC1 | NM_004741 | KIAA0035 // NOPP130 // NOPP140 // NS5ATP13 // P130 | 3261492 | chr10 | + | 103901962 | 103913597 | 5.73 |
| ADP-ribosylhydrolase like 2 | ADPRHL2 | NM_017825 | ARH3 // FLJ20446 // dJ665N4.2 | 2330253 | chr1 | + | 36327093 | 36332113 | 5.73 |
| chromosome 1 open reading frame 164 | C1 orf164 | NM_018150 | FLJ10597 | 2333907 | chr1 | + | 44643293 | 44889968 | 5.72 |
| platelet-activating factor acetylhydrolase, isoform Ib, beta subunit 30kDa | PAFAH1B2 | NM_002572 | - | 3350749 | chr11 | + | 116520229 | 116552810 | 5.72 |
| DPH2 homolog (S. cerevisiae) // UDP-Gal:betaGlcNAc beta 1,4-galactosyltransferase, polypeptide 2 // ATPase, H+ transporting, lysosomal 21 kDa, V0 subunit b | DPH2 // B4GALT2 // ATP6V0B | NM_001039589 // NM_001384 // NM_001 005417 // NM_003780 // NM_001039457 // NM_004047 | DPH2L2 // B4Gal-T2 // B4Gal-T3 // beta4Gal-T2 // ATP6F // HATPL // VMA16 | 2333635 | chr1 | + | 44208244 | 44211909 | 5.72 |
| chromosome 4 open reading frame 14 | C4orf14 | NM_032313 | MGC3232 // hAtNOS1 // | 2770427 | chr4 | - | 57496489 | 57539746 | 5.72 |
| ST3 beta-galactoside alpha-2,3-sialyltransferase 4 | ST3GAL4 | NM_006278 | CGS23 // FLJ11867 // NANTA3 // SAT3 // S1AT4 // SIAT4C // ST3Gal IV // ST3GallV // STZ | 3355145 | chr11 | + | 125730175 | 126063667 | 5.71 |
| myeloid differentiation primary response gene (88) | MYD88 | NM_002468 | - | 2617563 | chr3 | + | 38155029 | 38159513 | 5.71 |
| interferon-related developmental regulator 2 | IFRD2 | NM_006764 | FLJ40446 // IFNRP // SKMcl5 // SM15 | 2675088 | chr3 | - | 50300134 | 50305282 | 5.71 |
| TNF receptor-associated factor 4 | TRAF4 | NM_145751 // NM_004295 | CART1 // MLN62 // RNF83 | 3715839 | chr17 | + | 24095168 | 24102103 | 5.71 |
| Kruppel-like factor 5 (intestinal) | KLF5 | NM_001730 | BTEB2 // CKLF // IKLF | 3493543 | chr13 | + | 72527115 | 72549671 | 5.70 |
| melanophilin | MLPH | NM_001042467 // NM_024101 | MGC2771 // MGC59733 // SLAC2-A | 2534252 | chr2 | + | 238025804 | 238135783 | 5.70 |
| pleckstrin homology, Sec7 and coiled-coil domains 2 (cytohesin-2) | PSCD2 | NM_004228 // NM_017457 | ARNO // CTS18 // CTS18.1 // PSCD2L // SEC7L // Sec7p-L // Sec7p-like | 3837836 | chr19 | + | 53664134 | 53690474 | 5.69 |
| 5'-3' exoribonuclease 2 | XRN2 | NM_012255 | - | 3879467 | chr20 | + | 21219705 | 21322172 | 5.69 |
| large subunit GTPase 1 homolog (S. cerevisiae) | LSG1 | NM_018385 | FLJ11301 | 2711818 | chr3 | - | 195842815 | 195874226 | 5.69 |
| ubiquitin protein ligase E3C | UBE3C | NM 014671 | KIAA0010 // KIAA10 | 3033924 | chr7 | + | 158624381 | 156754823 | 5.68 |
| Rho GTPase-activating protein | RICS | NM_014715 | GC-GAP // GRIT // KIAA0712 // MGC1892 // p200RhoGAP // p250GAP | 3397877 | chr11 | - | 128343040 | 128567297 | 5.68 |
| v-raf murine sarcoma viral oncogene homolog B1 | BRAF | NM_004333 | B-raf 1 // BRAF1 // MGC 126806 // MGC138284 // | 3076340 | chr7 | - | 139994192 | 140274640 | 5.68 |
| heterogeneous nuclear ribonucleoprotein H3 (2H9) | HNRPH3 | NM_012207 // NM_021644 | 2H9 | 3249738 | chr10 | + | 69760947 | 69772949 | 5.68 |
| NudC domain containing 1 | NUDCD1 | NM_032869 | CML66 // FLJ14991 | 3148796 | chr8 | - | 110322324 | 110415934 | 5.68 |
| GATA binding protein 6 | GATA6 | NM_005257 | - | 3781245 | chr18 | + | 18003412 | 18060817 | 5.67 |
| preimplantation protein 3 // heat shock 10kDa protein 1 (chaperonin 10) | PREI3 // HSPE1 | NM_015387 11 NM_199482 // NM_002157 | 2C4D // CGI-95 // MGC12264 // MOB1 // MOB3 // CPN10 // GROES // HSP10 | 2521500 | chr2 | + | 198088565 | 198125849 | 5.67 |
| chromosome 14 open reading frame 151 | C14orf151 | NM_032714 | MGC13251 | 3554282 | chr14 | + | 104227008 | 104244902 | 5.67 |
| ADAM metallopeptidase domain 9 (meltrin gamma) | ADAM9 | NM_001005845 // NM 003816 | KIAA0021 // MCMP // MDC9 // Mltng | 3095002 | chr8 | + | 38973642 | 39082264 | 5.67 |
| target of EGR1, member 1 (nuclear) | TOE1 | NM_025077 | FLJ13949 | 2334319 | chr1 | + | 45577929 | 45582381 | 5.67 |
| acidic (leucine-rich) nuclear phosphoprotein 32 family, member B | ANP32B | NM_006401 | APRIL // PHAPI2 // SSP29 | 3181417 | chr9 | + | 99784907 | 99823167 | 5.66 |
| cleavage and polyadenylation specific factor 4, 30kDa | CPSF4 | NM_001081559 // NM_006693 | CPSF30 // NAR // NEB1 | 3014764 | chr7 | + | 98874515 | 98905314 | 5.66 |
| IK cytokine, down-regulator of HLA II | IK | NM_006083 | CSA2 // IK protein // MGC59741 // RED | 2831932 | chr5 | + | 140006890 | 140022229 | 5.66 |
| phosphoribosyl pyrophosphate amidotransferase | PPAT | NM_002703 | ATASE // GPAT | 2770242 | chr4 | - | 56954288 | 57029053 | 5.65 |
| proteasome (prosome, macropain) 26S subunit, ATPase, 2 | PSMC2 | NM_002803 | MGC3004 // MSS1 // Nbla10058 // S7 | 3017206 | chr7 | + | 102771969 | 102797076 | 5.65 |
| N-acetyltransferase 5 | NAT5 | NM_016100 // NM_181527 // NM_181528 | NAT3 // dJ1002M8.1 | 3878934 | chr20 | + | 19935711 | 19962808 | 5.65 |
| USP6 N-terminal like | USP6NL | XM_374768 // XM_927409 // XM_938665 // XM_943800 // NM 001080491 | KIAA0019 // RNTRE // TRE2NL | 3277468 | chr10 | - | 11523200 | 11725919 | 5.65 |
| EMG1 nucleolar protein homolog (S. cerevisiae) | EMG1 | NM_006331 | C2F // Grcc2f // NEP1 | 3403140 | chr12 | + | 6949871 | 6966191 | 5.65 |
| glutaminase | GLS | NM_014905 | DKFZp686015119 // FLJ10358 // GLS1 // KIAA0838 | 2520291 | chr2 | + | 191453802 | 191538513 | 5.65 |
| pleiomorphic adenoma gene-like 2 | PLAGL2 | NM 002657 | FLJ23283 | 3902682 | chr20 | - | 30243975 | 30259284 | 5.63 |
| TM2 domain containing 3 | TM2D3 | NM_025141 // NM_078474 | BLP2 | 3642358 | chr15 | - | 99977972 | 100010609 | 5.62 |
| SEC24 related gene family, member C (S. cerevisiae) | SEC24C | NM_004922 // NM_198597 | KIAA0079 | 3251848 | chr10 | + | 75174138 | 75210008 | 5.62 |
| ubiquitin-conjugating enzyme E2D 3 (UBC4/5 homolog, yeast) | UBE2D3 | NM_003340 // NM_181886 // NM_161867 // NM_181888 // NM_181889 // NM_181890 // NM_181891 // NM_181892 // NM_181893 | E2(17)KB3 // MGC43926 // MGC5416 // UBC4/5 // UBCH5C | 2779992 | chr4 | - | 103934623 | 104009473 | 5.61 |
| Rho guanine nucleotide exchange factor (GEF) 7 | ARHGEF7 | NM_003899 // NM_145735 | BETA-PIX // COOL1 // DKFZp761K1021 // KIAA0142 // KIAA0412 // Nbla10314 // P50 // P50BP // P85 // P85COOL1 // P85SPR // PAK3 // | 3501661 | chr13 | + | 110565635 | 110756075 | 5.61 |
| ring finger protein 139 | RNF139 | NM_007218 | HRCA1 // MGC31961 // RCA1 // TRC8 | 3114618 | chr8 | + | 125556189 | 125570389 | 5.59 |
| valosin-containing protein // Fanconi anemia, complementation group G | VCP // FANCG | NM_007126 // NM_004629 | IBMPFD // MGC131997 // MGC148092 // MGC8560 // TERA // p97 // FAG // | 3204404 | chr9 | - | 35026106 | 35062648 | 5.58 |
| ADP-ribosylation factor 1 | ARF1 | NM_001024226 // NM_001024227 // NM_001024228 // NM_001658 | - | 2383726 | chr1 | + | 226313262 | 226353506 | 5.58 |
| bladder cancer associated protein | BLCAP | NM_006698 | BC10 | 3904928 | chr20 | - | 35554050 | 35589711 | 5.57 |
| heterogeneous nuclear ribonucleoprotein H1 (H) | HNRPH1 | NM_005520 | DKFZp686A15170 // HNRPH // hnRNPH | 2890148 | chr5 | - | 178970155 | 178993890 | 5.57 |
| programmed cell death 2-like | PDCD2L | NM_032346 | MGC13096 | 3829751 | chr19 | + | 39587143 | 39608910 | 5.57 |
| TNF receptor-associated factor 2 | TRAF2 | NM_021138 | MGC:45012 // TRAP // TRAP3 | 3194896 | chr9 | + | 138896205 | 138943484 | 5.56 |
| ring finger protein 43 | RNF43 | NM_017763 | DKFZp781H02126 // DKFZp781H0392 // FLJ20315 // MGC125630 // RNF124 // URCC | 3764399 | chr17 | - | 53785272 | 53849935 | 5.56 |
| tousled-like kinase 1 | TLK1 | NM_012290 | KIAA0137 // PKU- BETA | 2586603 | chr2 | - | 171538552 | 171796060 | 5.55 |
| suppressor of zeste 12 homolog (Drosophila) | SUZ12 | NM_015355 | CHET9 // JJAZ1 // KIAA0160 | 3717395 | chr17 | + | 27258855 | 27352177 | 5.55 |
| dynactin 4 (p62) | DCTN4 | NM_016221 | - | 2881554 | chr5 | - | 150065753 | 150135890 | 5.54 |
| leucine rich repeat containing 1 // chromosome 20 open reading frame 44 | LRRC1 // C20orf44 | NM_018214 // NM_018244 // NM_199487 // NM_199513 | FLJ10775 // FLJ11834 // LANO // dJ523E19.1 // BFZB // CBP3 // MGC104353 // MGC141902 | 2910680 | chr6 | + | 53695560 | 53897416 | 5.54 |
| GTPase activating protein (SH3 domain) binding protein 2 // NMDA receptor regulated 2 // RAR-related orphan receptor A | G3BP2 // NARG2 // RORA | NM_012297 // NM_203504 // NM_203505 // NM_001018089 // NM_024611 // NM_002943 // NM_134260 // NM_134261 // NM 134262 | - // MGC119326 // MGC119329 // NR1F1 // ROR1 // ROR2 // ROR3 // RZRA | 2773756 | chr4 | - | 76786807 | 76869171 | 5.54 |
| ubiquitin specific peptidase 3 | USP3 | NM_006537 | MGC129678 // MGC129879 // SIH003 // UBP | 3597603 | chr15 | + | 61554385 | 61673885 | 5.54 |
| peptidylprolyl isomerase (cyclophilin)-like 4 // solute carrier family 25 (mitochondrial carrier; phosphate carrier), member 24 | PPIL4 // SLC25A24 | NM_139126 // NM_013386 // NM_213651 | HDCME13P // APC1 // DKFZp686G0123 // SCAMC-1 | 2978876 | chr6 | - | 149811664 | 149908884 | 5.54 |
| structural maintenance of chromosomes 1A | SMC1A | NM_006306 | DKFZp686L19178 // DXS423E // KIAA0178 // MGC138332 // SB1.8 // SMC1 // SMC1L1 // SMC1 alpha // | 4009238 | chrX | - | 53417797 | 53466400 | 5.53 |
| NM23-LV // non-metastatic cells 1, protein (NM23A) expressed in | NME1-NME2 // NME1 | NM_001018136 // NM_000269 // | NME2 // AWD // GAAD // NDPKA // NM23 // NM23-H1 | 3726934 | chr17 | + | 46564693 | 46594905 | 5.52 |
| taxilin alpha // doublecortin domain containing 2B | TXLNA // DCDC2B | NM_198175 NM_175852 // XM_926306 // XM_940631 | DKFZp451J0118 // IL14 // MGC118870 // MGC118871 // RP4-622L5.4 // TXLN // - | 2328713 | chr1 | + | 32417884 | 32436467 | 5.52 |
| cysteine-rich, angiogenic inducer, 61 | CYR61 | NM_001554 | CON1 // GIG1 // IGFBP10 | 2344888 | chr1 | + | 85816676 | 85867516 | 5.52 |
| prothymosin, alpha (gene sequence 28) // family with sequence similarity | PTMA // FAM22G | NM_002823 // XM_001127718 // NM_001045477 | MGC104802 // TMSA // - | 2532021 | chr2 | + | 232280604 | 232286493 | 5.52 |
| 22, member G casein kinase 1, epsilon // KIAA1660 protein | CSNK1E // KIAA1660 | NM_001894 // NM_152221 // XM_929784 // XM_940170 | HCKIE // MGC10398 //- | 3960478 | chr22 | - | 37010115 | 37150916 | 5.51 |
| MCM3 minichromosome maintenance deficient 3 (S. cerevisiae) | MCM3 | NM_002388 | HCC5 // MGC1157 // P1-MCM3 // P1.h // RLFB | 2957126 | chr6 | - | 52236739 | 52356643 | 5.50 |
| solute carrier family 25 (mitochondrial carrier, phosphate carrier), member 3 | SLC25A3 | NM_213612 // NM_002635 // NM_005888 // NM_213611 | OK/SW-cl.48 // PHC | 3427820 | chr12 | + | 97511471 | 97519906 | 5.50 |
| potassium channel tetramerisation 5 | KCTD5 | NM_018992 | FLJ20040 | 3645204 | chr16 | + | 2640820 | 2700477 | 5.50 |
| domain containing STE20-like kinase (yeast) | SLK | NM_014720 | KIAA0204 // MGC133067 // STK2 // bA16H23.1 // se20-9 | 3262433 | chr10 | + | 105716666 | 105778975 | 5.49 |
| glutathione reductase | GSR | NM 000637 | MGC78522 | 3130161 | chr8 | - | 30655000 | 30705038 | 5.49 |
| par-3 partitioning defective 3 homolog (C. elegans) | PARD3 | NM_019619 | ASIP // Baz // Bazooka // FLJ21015 // PAR3 // PAR3alpha // PARD3A // SE2-5L16 // SE2-5LT1 // SE2- | 3284596 | chr10 | - | 34413632 | 35144249 | 5.49 |
| UTP18. small subunit (SSU) processome component, homolog (yeast) | UTP18 | NM_016001 | CGI-48 // WDR50 | 3726992 | chr17 | + | 46692081 | 46730289 | 5.49 |
| serine/arginine repetitive matrix 1 // chromosome 1 open reading frame 130 | SRRM1 // C1orf130 | NM_005839 // NM_001010980 | 160-KD // MGC39488 // POP101 // SRM160 // FLJ42528 | 2325526 | chr1 | + | 24830814 | 24929968 | 5.49 |
| conserved nuclear protein NHN1 | NHN1 | NM_144604 | FLJ22664 // FLJ34530 // FLJ36075 | 3673515 | chr16 | + | 87152329 | 87225870 | 5.49 |
| UBX domain containing 8 | UBXD8 | NM_014613 | ETEA // KIAA0887 | 2842570 | chr5 | + | 175807217 | 175870117 | 5.48 |
| elongation protein 4 homolog (S. cerevisiae) | ELP4 | NM_019040 | C11orf19 // FLJ20498 // PAX6NEB // PAXNEB // dJ68P15A.1 | 3325307 | chr11 | + | 31487883 | 31764940 | 5.48 |
| hypothetical protein FLJ20366 | FLJ20366 | NM_017786 | GOLSYN | 3148871 | chr8 | - | 110655383 | 110964912 | 5.48 |
| SYF2 homolog, RNA splicing factor (S. cerevisiae) // MAX interactor 1 // chromosome 1 open reading frame 63 | SYF2 // MXI1 // C1 orf63 | NM_015484 // NM_207170 // NM_001008541 // NM_005962 // NM_130439 // NM_020317 | CBPIN // DKFZp56402082 // NTC31 // P29 // MAD2 // MGC43220 // MXD2 // MXI // DJ465N24.2.1 // NPD014 // RP3- 465N24.4 | 2402068 | chr1 | - | 25324131 | 25431599 | 5.47 |
| exostoses (multiple) 1 | EXT1 | NM_000127 | EXT // ttv | 3150060 | chr8 | - | 118880792 | 119193382 | 5.46 |
| polymerase (RNA) II (DNA directed) polypeptide D // WD repeat domain 33 | POLR2D // WDR33 | NM_004805 // NM_001006622 // NM_001006623 // NM_018383 | HSRBP4 // HSRPB4 // RBP4 // FLJ11294 // WDC146 | 2575134 | chr2 | - | 128317597 | 128334003 | 5.45 |
| ankyrin repeat domain 13 family, member D | ANKRD13D | XM_001129739 // NM 207354 | MGC50828 | 3336857 | chr11 | + | 66812660 | 66826524 | 5.45 |
| serum amyloid A-like 1 | SAAL1 | NM_138421 | FLJ41463 | 3365249 | chr11 | - | 18048076 | 18084203 | 5.45 |
| C1q domain containing 1 | C1QDC1 | NM_001002259 // NM_023925 // NM_032156 | EEG-1 // EEG1 // FLJ11391 // FLJ22569 // MGC102894 // MGC134847 // MGC134848 | 3449368 | chr12 | - | 30753755 | 30799712 | 5.44 |
| chromosome 11 open reading frame 30 | C 11 orf30 | NM_020193 | EMSY // FLJ90741 // GL002 | 3340913 | chr11 | + | 75803897 | 75941697 | 5.44 |
| ubiquilin 1 // death inducer-obliterator 1 | UBQLN1 // DIDO1 | NM_013438 // NM_053067 // NM_022105 // NM_033081 // NM_080796 // NM_080797 | DA41 // DSK2 // FLJ90054 // PLIC-1 // XDRP1 // BYE1 // C20orf158 // DATFI // DIDO2 // DIDO3 // DIO-1 // DIO1 // DKFZp434P1115 // FLJ11265 // KIAA0333 // MGC16140 // | 3212143 | chr9 | - | 85464717 | 85512928 | 5.44 |
| similar to ribosomal protein P0 // ribosomal protein, large, P0 | RPLP0-like // RPLPO | XR-015741 // XR_017813 // NM_001002 // NM_053275 | BLOCK 23 // L10E // MOC1 11226 // MOC88175 // P0 // PRLPO // RPPO | 3474344 | chr12 | - | 119118908 | 119123401 | 5.44 |
| zinc finger, NFX1-type containing 1 // gasdermin-like | ZNFX1 // GSDML | NM_02 1 035 // NM_001042471 // NM_018530 | FLJ39275 // MGC131926 // PP4052 // PRO2521 | 3908831 | chr20 | - | 47233911 | 47420063 | 5.44 |
| exportin 4 | XPO4 | NM_022459 | FLJ13046 // KIAA1721 | 3504434 | chr13 | - | 20251014 | 20375187 | 5.43 |
| centrosomal protein 72kDa | CEP72 | NM_018140 | FLJ10565 // KIAA1519 // MGC5307 | 2798777 | chr5 | + | 608522 | 720187 | 5.43 |
| basic transcription factor 3 | BTF3 | NM_001037637 // NM_001207 | BETA-NAC // BTF3a // BTF3b // NACB | 2815331 | chr5 | + | 72704665 | 72850839 | 5.43 |
| c-Maf-inducing protein | CMIP | NM_030629 // NM_198390 | KIAA1694 | 3670772 | chr16 | + | 80030441 | 80323852 | 5.42 |
| cerebellar degeneration-related protein 2. 62kDa | CDR2 | NM_001802 | CDR62 // Yo | 3684782 | chr16 | - | 22264766 | 22356527 | 5.42 |
| ubiquitin-like 3 | UBL3 | M_007106 | DKFZP434K151 // FLJ32018 // HCG-1 // PNSC1 | 3507798 | chr13 | - | 29200782 | 29371040 | 5.42 |
| ATP-binding cassette, sub-family F (GCN20), member 1 | ABCF1 | NM_001025091 // NM_001090 | ABC27 // ABC50 | 2901687 | chr6 | + | 30647103 | 30667268 | 5.42 |
| RAB22A, member RAS oncogene family | RAB22A | NM_020673 | MGC16770 | 3890870 | chr20 | + | 56318177 | 56375962 | 5.42 |
| axin 1 | AXIN1 | NM_003502 // NM_181050 | AXIN // MGC52315 | 3675047 | chr16 | - | 277442 | 351633 | 5.41 |
| mitochondrial ribosomal protein L44 | MRPL44 | NM_022915 | FLJ12701 // FLJ13990 | 2529782 | chr2 | + | 224530378 | 224540666 | 5.41 |
| zinc finger protein 146 // zinc finger protein 268 | ZNF146 // ZNF268 | NM_007145 // NM_152943 // NM_003415 | MGC125660 // MGC125661 // OZF // HZF3 // MGC126498 | 3831260 | chr19 | + | 41397873 | 41421503 | 5.40 |
| golgi SNAP receptor complex member 1 | GOSR1 | NM_001007024 // NM_001007025 // NM 004871 | GOS28 // GOS28/P28 // GS28 // P28 | 3716481 | chr17 | + | 25828452 | 25893594 | 5.40 |
| zinc finger protein 512 | ZNF512 | NM_032434 | KIAA1805 // MGC111046 | 2474651 | chr2 | + | 27659393 | 27699586 | 5.40 |
| formin binding protein 4 | FNBP4 | NM_015308 | DKFZp77911064 // FBP30 // FLJ41904 // KIAA1014 | 3372459 | chr11 | - | 47694235 | 47745657 | 5.39 |
| zinc finger protein 707 | ZNF707 | NM_173831 | - | 3119765 | chr8 | + | 144771273 | 144849532 | 5.39 |
| FERM domain containing 5 | FRMD5 | NM_032892 | FLJ41022 // MGC14161 | 3621728 | chr15 | - | 41950261 | 42274751 | 5.39 |
| serum/glucocorticoid regulated | SGK | NM_005627 | SGK1 | 2975014 | chr6 | - | 134532088 | 134680889 | 5.38 |
| hypoxia-inducible factor 1, alpha subunit (basic helix-loop-helix transcription factor) | HIF1A | NM_001530 // NM_181054 | HIF-1 alpha // HIF1- ALPHA // MOP1 // PASD8 | 3539070 | chr14 | + | 61225495 | 61285222 | 5.38 |
| deoxyhypusine synthase | DHPS | NM_001930 // NM_013406 // NM_013407 | MIG13 | 3851603 | chr19 | - | 12647534 | 12653680 | 5.37 |
| thioredoxin-like 1 | TXNL1 | NM_004786 | TRP32 // TXL-1 // TXNL // TxI | 3809324 | chr18 | - | 52334063 | 52469773 | 5.37 |
| RAN, member RAS oncogene family | RAN | NM_006325 | ARA24 // Gsp1 // TC4 | 3438027 | chr12 | + | 129922411 | 129928164 | 5.36 |
| dynein, cytoplasmic 1, light intermediate chain 2 | DYNC1LI2 | NM_006141 | DNCLI2 // LIC2 | 3695199 | chr16 | - | 65312304 | 65343212 | 5.36 |
| enhancer of rudimentary homolog (Drosophila) | ERH | XM_001130537 // NM_004450 | DROER // FLJ27340 | 3570049 | chr14 | - | 68916605 | 68935306 | 5.35 |
| transmembrane 4 L six family member 1 | TM4SF1 | M_014220 | H-L6 // L6 // M3S1 // TAAL6 | 2700365 | chr3 | - | 150567718 | 150578270 | 5.34 |
| lipase, endothelial | UPG | NM_006033 | EDL // EL // | 3787855 | chr18 | + | 45341077 | 45481323 | 5.34 |
| exportin 6 | XPO6 | NM_015171 | EXP6 // FLJ22519 // KIAA0370 // RANBP20 | 3686339 | chr16 | - | 28016804 | 28130734 | 5.34 |
| hypothetical protein FLJ11171 | FLJ11171 | NM_018348 | - | 3697563 | chr16 | - | 69872813 | 69881003 | 5.34 |
| UPF1 regulator of nonsense transcripts homolog (yeast) | UPF1 | NM_002911 | FLJ43809 // FLJ46894 // HUPF1 // KIAA0221 // NORF1 // RENT1 // pNORF1 | 3825383 | chr19 | + | 18803757 | 18840030 | 5.34 |
| surfeit 6 | SURF6 | NM 006753 | FLJ30322 | 3228621 | chr9 | - | 135186667 | 135193036 | 5.32 |
| neural precursor cell expressed, developmentally down-regulated 4-like // sema domain, immunoglobulin domain (Ig), transmembrane domain (TM) and short cytoplasmic domain, (semaphorin) 4G | NEDD4L // SEMA4G | NM_015277 // NM_017893 | FLJ33870 // KIAA0439 // RSP5 // hNedd4-2 // FLJ20590 // KIAA1619 // MGC102867 | 3789947 | chr18 | + | 53862627 | 54232131 | 5.30 |
| tetraspanin 14 | TSPAN14 | NM_030927 | DC-TM4F2 // MGC11352 // TM4SF14 | 3254521 | chr10 | + | 82203922 | 82272901 | 5.30 |
| core-binding factor, beta subunit | CBFB | NM_001755 // NM_022845 | PEBP2B | 3665116 | chr16 | + | 65620522 | 65692457 | 5.30 |
| baculoviral IAP repeat-containing 5 (survivin) | BIRC5 | NM_001012270 // NM_001012271 // NM_001168 | API4 // EPR-1 | 3736290 | chr17 | + | 73721882 | 73733311 | 5.29 |
| grainyhead-like 2 (Drosophilia) | GRHL2 | NM_024915 | BOM // D FNA28 // FLJ11172 // FLJ13782 // MGC149294 // MGC149295 // TFCP2L3 | 3109687 | chr8 | + | 102573566 | 102751110 | 5.29 |
| ecdysoneless homolog (Drosophila) | ECD | NM_007265 | GCR2 // HSGT1 | 3294242 | chr10 | - | 74559939 | 74598411 | 5.29 |
| RNA binding motif protein 13 | RBM13 | NM_032509 | MAK16 // MAK16L | 3093259 | chr8 | + | 33462192 | 33478316 | 5.28 |
| cell division cycle associated 2 | CDCA2 | NM_152562 | FLJ25804 // MGC129906 // MGC129907 // Repo- Man | 3090697 | chr8 | + | 25362573 | 25431420 | 5.28 |
| myeloid/lymphoid or mixed-lineage leukemia (trithorax homolog, 4 | MLLT4 | NM_001040000 // NM_001040001 // NM_005936 | AF-6 // AF6 // AFADIN // FLJ34371 // RP3-431 P23.3 | 2936857 | chr6 | + | 167970473 | 168115537 | 5.28 |
| Drosophila); translocated to, nuclear receptor subfamily 1, group H, member 2 | NR1H2 | NM_007121 | LXR-b // LXRB // NER // NER-I // RIP15 // UNR | 3839276 | chr19 | + | 55524771 | 55578457 | 5.28 |
| tight junction protein 2 (zona | TJP2 | NM_004817 // NM_201629 | MGC26306 // X104 // ZO-2 // ZO2 | 3173880 | chr9 | + | 70925894 | 71059931 | 5.27 |
| occludens 2) eukaryotic translation initiation factor 2A, 65kDa | EIF2A | NM_032025 | CDA02 // EIF-2A // MST089 // MSTP004 // MSTP089 | 2647742 | chr3 | + | 151747168 | 151788822 | 5.27 |
| histone acetyltransferase 1 | HAT1 | NM_001033085 // NM_003642 | - | 2515369 | chr2 | + | 172487173 | 172556842 | 5.26 |
| ring finger protein 138 | RNF138 | NM_016271 // NM_198128 | HSD-4 // MGC8758 // NARF // STRIN | 3783749 | chr18 | + | 27925835 | 28030948 | 5.26 |
| phosphatase and tensin homolog (mutated in multiple advanced | PTEN | NM_000314 | BZS // MGC11227 // MHAM // MMAC1 // PTEN1 // TEP1 | 3256689 | chr10 | + | 89598814 | 89722145 | 5.25 |
| cancers 1) Ran GTPase activating protein 1 | RANGAP1 | NM_002883 | Fug1 // KIAA1835 // MGC20266 // SD | 3961842 | chr22 | - | 39969442 | 40028027 | 5.24 |
| zinc finger and BTB domain containing 7A | ZBTB7A | NM_015898 | DKFZp5470146 // FBI-1 // FBI1 // LRF // MGC99631 // ZBTB7 // pokemon | 3846594 | chr19 | - | 3990713 | 4033507 | 5.24 |
| methionyl aminopeptidase 2 // hypothetical protein FLJ11292 | METAP2 // FLJ11292 | NM_006838 // NM_018382 | MNPEP // p67 // p67elF2 // - | 3426917 | chr12 | + | 94391437 | 94696442 | 5.23 |
| small nuclear ribonucleoprotein polypeptides B and B1 | SNRPB | NM_003091 // NM_198216 | COD // SNRPB1 // SmB/SmB' // snRNP-B | 3894995 | chr20 | - | 2369715 | 2399499 | 5.23 |
| CD151 molecule (Raph blood group) // FK506 binding protein 9-like | CD151 // FKBP9L | NM_001039490 // NM_004357 // NM_139029 // NM_139030 // NM_182827 | GP27 // MER2 // PETA-3 // RAPH // SFA1 // TSPAN24 // FKBP9 // MGC20531 | 3316344 | chr11 | + | 822755 | 828824 | 5.23 |
| Wolf-Hirschhom syndrome candidate 1 | WHSC1 | NM_001042424 // NM_007331 // NM_133330 // NM_133331 // NM_133334 // NM_133335 // NM_133336 | FLJ23286 // KIAA1090 // MMSET // NSD2 // REIIBP // TRX5 // WHS | 2715076 ' | chr4 | + | 1842909 | 1953717 | 5.23 |
| heterogeneous nuclear ribonucleoprotein U-like 1 | HNRPUL1 | NM_144733 // NM_144734 // NM_007040 // NM_44732 | E1B-AP5 // E1BAP5 // FLJ12944 | 3834089 | chr1 9 | + | 46460006 | 46505508 | 5.22 |
| ubiquitin-conjugating enzyme E2C // p21 (CDKN1A)-activated kinase 3 | UBE2C // PAK3 | NM_007019 // NM_81799 // NM_181800 // NM_181801 // NM_181802 // NM_181803 // NM_002578 | UBCH10 // dJ447F3.2 // CDKN1A // MRX30 // MRX47 // OPHN3 // PAK3beta // bPAK // hPAK3 | 3887049 | chr20 | + | 43874662 | 43878994 | 5.22 |
| ribosomal L1 domain containing 1 | RSL1D1 | NM_015659 | CSIG // DKFZP564M182 // L12 // MGC138433 // MGC142259 // | 3680583 | chur16 | - | 11835208 | 11852988 | 5.22 |
| Sjogren's syndrome nuclear autoantigen 1 | SSNA1 | NM_003731 | N14 // NA-14 // NA14 | 3195296 | chr9 | + | 139202850 | 139204636 | 5.22 |
| zinc finger protein 227 // zinc finger protein 155 | ZNF227 // ZNF155 | NM_82490 // NM_003445 // NM_198089 | - // MGC161655 // pHZ-96 | 3835544 | chr19 | + | 49408524 | 49433254 | 5.21 |
| splicing factor, arginine/serine-rich 6 | SFRS6 | NM_006275 | B52 // MGC5045 // SRP55 | 3886050 | chr20 | + | 41519932 | 41526301 | 5.21 |
| transmembrane protein 5 | TMEM5 | NM_014254 | HP10481 | 3419585 | chr12 | + | 62440396 | 62489599 | 5.20 |
| peptide deformylase (mitochondrial) // component of oligomeric golgi complex 8 | PDF // COG8 | NM_022341 // NM_032382 | - // DOR1 // FLJ22315 | 3696524 | chr16 | - | 67914755 | 67931014 | 5.20 |
| membrane-associated ring finger (C3HC4) 6 | 6-Mar | NM_005885 | KIAA0597 // MARCH-VI // | 2801608 | chr5 | + | 10406824 | 10490959 | 5.20 |
| Sp3 transcription factor // Sp3 transcription factor pseudogene | SP3 // RP11- 114G1.1 | NM_001017371 // NM_003111 // - | DKFZp68601631 // SPR-2 // LOC160824 | 2587520 | chr2 | - | 174445149 | 174610682 | 5.20 |
| ubiquitin specific peptidase like 1 | USPL1 | NM_005800 | C13orf22 // D13S106E // DKFZp781K2286 // FLJ32952 // RP11- 121019.1 // bA121019.1 | 3484005 | chr13 | + | 30028543 | 30132897 | 5.20 |
| nucleolin | NCL | NM_005381 | C23 // FLJ45706 | 2603460 | chr2 | - | 232027713 | 232080578 | 5.19 |
| myotubularin related protein 3 | MTMR3 | NM_021090 // NM_153050 // NM_153051 | FYVE-DSP1 // KIAA0371 // ZFYVE10 | 3942179 | chr22 | + | 28609182 | 28762301 | 5.19 |
| Sec61 gamma subunit | SEC61 G | NM_001012456 // NM_014302 | SSS1 | 3051395 | chr7 | - | 54753676 | 54847174 | 5.19 |
| signal recognition particle 72kDa | SRP72 | NM_006947 | - | 2728224 | chr4 | + | 57028214 | 57064596 | 5.19 |
| chromosome 1 open reading frame 77 | C1orf77 | NM_015607 | DKFZP547E1010 // MGC131924 // MGC86949 // RP1- 178F15.2 // pp7704 | 2359736 | chr1 | + | 151872948 | 151885444 | 5.18 |
| zinc finger protein 787 | ZNF787 | NM_001002836 | - | 3871730 | chr19 | - | 61261349 | 61355028 | 5.18 |
| TAF5-like RNA polymerase 11. p300/CBP-associated factor (PCAF)-associated factor, 65kDa // mucin 4, cell surface associated | TAF5L // MUC4 | NM_001025247 // NM_014409 // XM_001125749 // NM_004532 // NM_018406 // NM 138297 | PAF65B // HSA276359 | 2459971 | chr1 | - | 227795491 | 227828457 | 5.18 |
| cyclin T1 | CCNT1 | NM_001240 | CCNT // CYCT1 | 3453218 | chr1 2 | - | 47372840 | 47397048 | 5.18 |
| A kinase (PRKA) anchor protein 1 | AKAP1 | NM_003488 | AKAP // AKAP121 // AKAP149 // AKAP84 // D-AKAP1 // MGC1807 // PRKA1 // SAKAP84 | 3728097 | chr17 | + | 52517583 | 52667254 | 5.18 |
| proteasome (prosome, macropain) 26S subunit, ATPase, 5 | PSMC5 | NM_002805 | S8 // SUG1 // TBP10 // TRIP1 // p45 // p45/SUG | 3730941 | chr17 | + | 59258523 | 59263555 | 5.18 |
| protein phosphatase 1, regulatory (inhibitor) subunit 15B // phosphoinositide-3-kinase, class 2, beta polypeptide | PPP1R15B // PIK3C2B | NM_032833 // NM_002646 | FLJ14744 // C2-P13K // DKFZp686G16234 | 2452049 | chr1 | - | 202639152 | 202647598 | 5.17 |
| myristoylated alanine-rich protein kinase C substrate | MARCKS | NM_002356 | 80K-L // FLJ14368 // FLJ90045 // MACS // MRACKS // PKCSL // PRKCSL | 2922215 | chr6 | + | 114284762 | 114292853 | 5.17 |
| M-phase phosphoprotein 10 (U3 small nucleolar ribonucleoprotein) | MPHOSPH1 0 | NM_005791 | MPP10 // MPP10P | 2488078 | chr2 | + | 71210952 | 71237711 | 5.16 |
| transmembrane protein 170 | TMEM170 | NM_145254 | FLJ37611 | 3699581 | chr16 | - | 74034456 | 74107659 | 5.15 |
| YTH domain family, member 2 | YTHDF2 | NM_016258 | HGRG8 // NY-REN-2 | 2327630 | chr1 | + | 28935740 | 28968859 | 5.15 |
| breast cancer anti-estrogen resistance 3 | BCAR3 | NM_003567 | KIAA0554 // NSP2 // SH2D3B | 2423422 | chr1 | - | 93790445 | 94085633 | 5.15 |
| KH domain containing, RNA binding, signal transduction associated 1 | KHDRBS1 | NM_006559 | FLJ34027 // Sam68 // p62 | 2328465 | chr1 | + | 32212249 | 32299034 | 5.15 |
| reticulon 4 | RTN4 | NM_007008 // NM_020532 // NM_153828 // NM_207520 // NM_207521 | ASY // NI220/250 // NOGO // NOGO-A // NSP // NSP-CL // Nbla00271 // Nbla10545 // RTN-X // RTN4-A // RTN4- B1 // RTN4-B2 // RTN4-C | 2553576 | chr2 | - | 55051075 | 55161279 | 5.15 |
| calcitonin gene-related peptide-receptor component protein | RCP9 | NM_001040647 // NM_001040648 // NM_014478 | CGRP-RCP // CRCP // MGC111194 // RCP | 3005332 | chr7 | + | 65131004 | 65262097 | 5.15 |
| solute carrier family 3 (activators of dibasic and neutral amino acid transport), member 2 | SLC3A2 | NM_001012661 // NM_001 012662 // NM_001012663 // NM_001012664 // NM_001013251 // NM_002394 | 4F2 // 4F2HC // 4T2HC // CD98 // CD98HC // MDU1 // NACAE | 3333711 | chr11 | + | 62380117 | 62412872 | 5.15 |
| zinc finger, DHHC-type containing 5 | ZDHHC5 | NM_015457 | DKFZP586K0524 // KIAA1748 // ZNF375 | 3331433 | chr11 | + | 57192053 | 57225319 | 5.14 |
| Smg-5 homolog, nonsense mediated mRNA decay factor (C. elegans) | SMG5 | NM_015327 | EST1B // FLJ34864 // KIAA1089 // LPTS-RP1 // LPTSRP1 // RP11- 54H19.7 // SMG-5 | 2438042 | chr1 | - | 154485643 | 154519254 | 5.14 |
| alk8, alkylation repair homolog 2 (E. coli) | ALKBH2 | NM_001001655 | ABH2 // MGC90512 // hABH2 | 3470689 | chr12 | - | 108010254 | 108015656 | 5.13 |
| BUD31 homolog (S. cerevisiae) | BUD31 | NM_003910 | EDG-2 // EDG2 // G10 // MGC111202 // YCR063W | 3014742 | chr7 | + | 98844221 | 98855171 | 5.13 |
| MIS12, MIND kinetochore complex component, homolog (yeast) | MIS12 | NM_024039 | 2510025F08Rik // KNTC2AP // MGC2488 // MTW1 // hMis12 | 3707759 | chr17 | + | 5330450 | 5334853 | 5.13 |
| tripartite motif-containing 26 | TRIM26 | NM_003449 | AFP // RNF95 // ZNF173 | 2948259 | chr6 | - | 30260217 | 30290029 | 5.13 |
| similar to FRG1 protein (FSHD region gene 1 protein) // FSHD region gene 1 | MGC72104 // FRG1 | NM_207350 // NM_004477 | - // FSG1 | 2756029 | chr4 | + | 191051868 | 191138306 | 5.13 |
| ATPase family, AAA domain containing 1 | ATAD1 | NM_032810 | AFDC1 // FLJ14600 // FNP001 | 3299255 | chr10 | - | 89500784 | 89591413 | 5.12 |
| BTB (POZ) domain containing 3 | BTBD3 | NM_014962 // NM_181443 | KIAA0952 // MGC130038 // MGC 130039 // dJ742J24.1 | 3876645 | chr20 | + | 11722386 | 11855239 | 5.12 |
| sterol regulatory element binding transcription factor 2 | SREBF2 | NM_004599 | SREBP2 | 3947123 | chr22 | + | 40559049 | 40633247 | 5.12 |
| splicing factor 3a, subunit 3, 60kDa | SF3A3 | NM_006802 | PRP9 // PRPF9 // SAP61 // SF3a60 | 2407439 | chr1 | - | 38195241 | 38229180 | 5.11 |
| ATP synthase, H+ transporting, mitochondrial F1 complex, beta polypeptide | ATP5B | NM_001686 | ATPMB // ATPSB // MGC5231 | 3458033 | chr12 | - | 55318243 | 55326110 | 5.11 |
| mitochondrial ribosomal protein L55 | MRPL55 | NM_181441 // NM_181454 // NM_181455 // NM_181456 // NM_181462 // NM_181463 // NM_181464 // NM_181465 | AAVG5835 // DKFZp686D1387 // MGC61802 // PRO19675 | 2459438 | chr1 | - | 226360734 | 226367172 | 5.11 |
| SMAD specific E3 ubiquitin protein ligase 1 | SMURF1 | NM_020429 // NM_181349 | KIAA1625 | 3063083 | chr7 | - | 98449133 | 98579664 | 5.11 |
| myeloid/lymphoid or mixed-lineage leukemia (trithorax homolog, Drosophila) | MLL | NM_005933 | ALL-1 // CXXC7 // HRX // HTRX1 // MLL/GAS7 // MLL1A // TRX1 | 3351385 | chr11 | + | 117811029 | 117902749 | 5.10 |
| family with sequence similarity 89. member 8 | FAM89B | NM_152832 | MTVR1 | 3335338 | chr11 | + | 65096490 | 65098230 | 5.10 |
| alkB, alkylation repair homolog 3 (E. coli) | ALKBH3 | NM_139178 | ABH3 // DEPC-1 // DEPC1 // MGC118790 // MGC118792 // | 3328214 | chr11 | + | 43858633 | 43898486 | 5.10 |
| SET domain containing 2 | SETD2 | NM_014159 | FLJ16420 // FLJ22472 // FLJ23184 // FLJ45883 // HIF-1 // HSPC069 // HYPB // KIAA1732 | 2672532 | chr3 | - | 47032936 | 47180418 | 5.09 |
| p53 and DNA damage regulated 1 | PDRG1 | NM_030815 | C20orf126 // PDRG | 3902609 | chr20 | - | 29995826 | 30005314 | 5.09 |
| ubiquitin carboxyl-terminal esterase L3 (ubiquitin thiolesterase) | UCHL3 | NM_006002 | - | 3494102 | chr13 | + | 75021620 | 75078250 | 5.08 |
| hepatocyte growth factor-regulated tyrosine kinase substrate | HGS | NM_004712 | HRS // ZFYVE8 | 3738138 | chr17 | + | 77260785 | 77279549 | 5.08 |
| proteasome (prosome, macropain) 26S subunit, ATPase, 4 | PSMC4 | NM_006503 // NM_153001 | MGC13687 // MGC23214 // MGC8570 // MIP224 // S6 // TBP7 | 3833291 | chr19 | + | 45168772 | 45179181 | 5.08 |
| tubulin, beta | TUBB | NM_178014 | M40 // MGC117247 // MGC16435 // OK/SW-cl.56 // TUBB1 // TUBB5 | 2901913 | chr6 | + | 30795977 | 30801165 | 5.08 |
| kinesin family member 20A | KIF20A | NM_005733 | FLJ21151 // MKLP2 // RAB6KIFL | 2830638 | chr5 | + | 137542289 | 137552275 | 5.08 |
| arginyl-tRNA synthetase | RARS | NM_002887 | ArgRS // DALRD1 // MGC8641 | 2839671 | chr5 | + | 167845940 | 167878885 | 5.08 |
| calpain 2, (m/II) large subunit | CAPN2 | NM_001748 | CANPL2 // CANPml // FLJ39928 // mCANP | 2382117 | chr1 | + | 221955990 | 222031806 | 5.08 |
| ribosomal protein S15a | RPS15A | NM_001019 // NM_001030009 | FLJ27457 // MGC111208 // S15a | 3683018 | chr16 | - | 18700121 | 18709175 | 5.07 |
| topoisomerase (DNA) II binding protein 1 | TOPBP1 | NM_007027 | TOP2BP1 | 2695941 | chr3 | - | 134799729 | 134863527 | 5.07 |
| calcium homeostasis endoplasmic reticulum protein // cofactor required for Sp1 transcriptional activation, subunit 7, 70kDa | CHERP // CRSP7 | NM_006387 // NM_004831 | DAN16 // SCAF6 // SRA1 // CRSP70 // MED26 | 3853942 | chr19 | - | 16489710 | 16517113 | 5.06 |
| echinoderm microtubule associated protein like 4 | EML4 | NM_019063 | C2orf2 // DKFZp686P18118 // ELP120 // FLJ10942 // FLJ32318 // ROPP120 | 2478748 | chr2 | + | 42250017 | 42418636 | 5.06 |
| spicing factor 3b, subunit 4, 49kDa | SF3B4 | NM_005850 | MGC10828 // SAP49 // SF3b49 | 2434159 | chr1 | - | 148161628 | 148167125 | 5.05 |
| rho/rac guanine nucleotide exchange factor (GEF) 18 | ARHGEF18 | NM_015318 | KIAA0521 // MGC15913 | 3818732 | chr19 | + | 7319892 | 7443363 | 5.05 |
| pescadillo homolog 1, containing BRCT domain (zebrafish) | PES1 | NM_014303 | PES | 3957445 | chr22 | - | 29302623 | 29333107 | 5.05 |
| Ras association (RalGDS/AF-6) domain family 7 | RASSF7 | NM_003475 | C11orf13 // HRAS1 // HRC1 // MGC126069 // MGC126070 | 3315952 | chr11 | + | 550321 | 554016 | 5.05 |
| Wilms tumor 1 associated protein // acetyl-Coenzyme A acetyltransferase 2 (acetoacetyl Coenzyme A thiolase) | WTAP // ACAT2 | NM_004906 // NM_152857 // NM_152858 // NM_005891 | DKFZp686F20131 // KIAA0105 // MGC3925 // - | 2934089 | chr6 | + | 160066607 | 160097332 | 5.04 |
| origin recognition complex, subunit 5-like (yeast) | ORC5L | NM_002553 // NM 181747 | ORC5 // ORC5P // ORC5T | 3065963 | chr7 | - | 103553146 | 103719295 | 5.04 |
| chromodomain protein, Y-like | CDYL | NM_004824 // NM_170751 // NM 170752 | CDYL1 // DKFZP586C1622 // MGC131936 | 2892979 | chr6 | + | 4651344 | 4900768 | 5.03 |
| aprataxin | APTX | NM_017692 // NM_175069 // NM_175071 // NM_175072 // NM 175073 | AOA // AOA1 // AXA1 // EAOH // EOAHA // FHA-HIT // FLJ20157 // MGC1072 | 3203311 | chr9 | - | 32962360 | 33015096 | 5.03 |
| phosphatidylinositol 4-kinase, catalytic, beta polypeptide // regulatory factor X, 5 (influences HLA class II expression) | PIK4CB // RFX5 | NM_002651 // NM_000449 // NM_001025603 | P14K-BETA // PI4KIIIbeta // PI4Kbeta // - | 2434925 | chr1 | - | 149530589 | 149567792 | 5.03 |
| serine palmitoyltransferase, long chain base subunit 2 | SPTLC2 | NM_004863 | KIAA0526 // LCB2 // SPT2 | 3573152 | chr4 | - | 77042100 | 77202740 | 5.02 |
| cell division cycle associated 5 | CDCA5 | NM 080668 | MGC16386 | 3377423 | chr11 | - | 64592075 | 64608249 | 5.02 |
| myeloid/lymphoid or mixed-lineage leukemia 5 (trithorax homolog, Drosophila) | MLL5 | NM_018682 // NM_182931 | FLJ10078 // FLJ14026 // HDCMC04P // MGC70452 | 3017547 | chr7 | + | 104436029 | 104542698 | 5.02 |
| F-box protein 28 | FBXO28 | NM_015176 | FLJ10766 // Fbx28 // KIAA0483 | 2382336 | chr1 | + | 222368455 | 222416368 | 5.02 |
| eukaryotic translation initiation factor 3, subunit 1 alpha, 35kDa | EIF3S1 | NM_003758 | elF3-alpha // elF3- p35 // elF3j | 3591963 | chr15 | + | 42616580 | 42644124 | 5.02 |
| polymerase (RNA) III (DNA directed) polypeptide C (62kD) | POLR3C | NM_006468 | RPC3 // RPC62 | 2432647 | chr1 | - | 144297192 | 144333220 | 5.01 |
| bromodomain adjacent to zinc finger domain, 1B | BAZ1B | NM_023005 // NM_032408 | WBSCR10 // WBSCR9 // WSTF | 3056044 | chr7 | - | 72492710 | 72574552 | 5.01 |
| isocitrate dehydrogenase 3 (NAD+) beta | IDH3B | NM_006899 // NM_174855 // NM_174856 | FLJ11043 // H-IDHB // MGC903 | 3895075 | chr20 | - | 2586736 | 2592862 | 5.01 |
| basic transcription factor 3-like 4 | BTF3L4 | NM_152265 | MGC23908 // MGC88389 // RP4- 800M22.5 | 2336271 | chr1 | + | 52293292 | 52339895 | 5.01 |
| Src homology 2 domain containing adaptor protein B // cytoskeleton associated protein 2 // mitochondrial carrier triple repeat 1 | SHB // CKAP2 // MCART1 | NM_003028 // NM_018204 // NM_033412 | RP11-3J10.8 // bA3J10.2 // FLJ10749 // LB1 // TMAP // se20-10 // CG7943 // | 3205659 | chr9 | - | 37901375 | 38059198 | 5.00 |

### Example 9: Analysis with microarray (3)

Using the PBMC samples in Example 4(2), genes of which introns exhibited increased expression were identified. Ribosomal RNA was removed from 1 µg of each total RNA of control cells (n=3) and cells with the treatment (n=3) using RiboMinus Human/Mouse Transcriptome Isolation Kit (Invitrogen). Single stranded cDNA synthesis, double stranded cDNA synthesis, cRNA synthesis, second single stranded cDNA synthesis, cDNA fragmentation, and cDNA labeling in order were carried out for total RNA from which ribosomal RNA was removed, using GeneChip Whole Transcript Sense Target Labeling and Control Reagents (Affymetrix) to make a cDNA probe. Subsequently, the cDNA probe was used for hybridization with Human Exon 1.0 ST Array (Affymetrix). The array was washed and stained, and luminescence intensity was measured by a scanner.

The expression level of the probe set and the gene was quantified by using Expression Console Ver. 1.0 (Affymetrix) with Summarization Method and Normalization Method being set to "Median polish as used in RMA" and "None", respectively. The expression level of the probe was normalized with that of the gene.

### (1) Identification of a gene of which introns exhibit increased expression in a group with the treatment at a concentration of 3 nM

Probe sets showing that the normalized change in the level expression of the probe set in the group with the treatment at 3 nM was more than 5 times; the expression level of the probe set and the gene in the group with the treatment at 3 nM was more than 100; and probe set annotation was "extended, full, free", were picked out. Welch's t-test was conducted with the probe sets extracted for the group with the treatment at 3 nM and the control group to determine a p value and a q value. A gene containing the probe sets with the q value of less than 5% was determined as a candidate gene with increased expression level of the intron regions (Table 4).

In Table 4, for the gene evaluated, gene name (Gene Name), abbreviated name (Gene Symbol), accession number (Accession), alias name (Synonym), transcription cluster number in Human Exon 1.0 ST Array (Human Exon 1.0 ST Array Transcript Cluster ID), chromosome number (Chromosome), type of strand (Strand), start region (Start), stop region (Stop), and change in expression level (Fold Change) were respectively shown.

**[Table 4]**

| Gene Name | Gene Symbol | Accession | Synonym | Human Exon ST 1.0 | Human Genome hg18 | | | | Fold Change |
|---|---|---|---|---|---|---|---|---|---|
| | | | | Transcript Cluster ID | Chromo some | Strand | Start | Stop | |
| TIMP metallopeptidase inhibitor 1 | TIMP1 | NM_003254 | CLGI // EPA // EPO // FLJ90373 // HCl // TIMP | 3976341 | chrX | + | 47326654 | 47332978 | 14.98 |
| ADAM llopeptidase domain 19 (meltrin beta) | ADAM19 | NM_023038 // NM_033274 | FKSG34 // MADDAM // MLTNB | 2883440 | chr5 | - | 156755122 | 156935351 | 9.71 |
| phosphodiesterase 4A, cAMP-specific (phosphodiesterase E2 dunce homolog, | PDE4A | NM_006202 | DPDE2 // PDE4 | 3820501 | chr19 | + | 10388560 | 10451531 | 9.64 |
| Rap guanine nucleotide exchange factor (GEF) 1 | RAPGEF1 | NM_005312 // NM_198679 | C3G // DKFZp781P1719 // GRF2 | 3227696 | chr9 | - | 133441988 | 133605262 | 9.27 |
| myosin, heavy chain 9, non-muscle | MYH9 | NM_002473 | DFNA17 // EPSTS // FTNS // MHA // NMHC-II-A // NMMHCA | 3959451 | chr22 | - | 35007278 | 35113982 | 7.90 |
| serpin peptidase inhibitor, clade B (ovalbumin), member 9 // serpin peptidase inhibitor, clade B (ovalbumin), member 6 | SERPINB9 // SERPINB6 | NM_004155 // NM_004568 | CAP-3 // CAP3 // PI9 // CAP // DKFZp686I0422 2 // MGC111370 // MSTP057 // | 2939034 | chr6 | - | 2832402 | 2858242 | 7.75 |
| RNA binding motif protein 23 | RBM23 | NM_0010773 51 // NM_0010773 52 // NM_018107 | CAPERbeta // FLJ10482 // MGC4458 // PP239 // RNPC4 | 3556888 | chr14 | - | 22439714 | 22458231 | 7.63 |
| polyhomeotic homolog 3 (Drosophila) | PHC3 | NM_024947 | DKFZp313K1221 // EDR3 // FLJ12729 // FLJ12967 // HPH3 // MGC88144 | 2704894 | chr3 | - | 171287994 | 171381537 | 7.30 |
| splicing factor proline/glutamine-rich (polypyrimidine tract binding protein associated) | SFPQ | NM_005066 | POMP100 // PSF | 2406064 | chr1 | - | 35351696 | 35506894 | 7.08 |
| BTB (POZ) domain containing 11 | BTBD11 | NM_0010175 23 // NM_0010180 72 // NM_152322 | FLJ33957 // FLJ42845 | 3430462 | chr12 | + | 106237825 | 106577543 | 7.07 |
| v-src sarcoma (Schmidt-Ruppin A-2) viral oncogene homolog (avian) | SRC | NM_005417 // NM_198291 | ASV // SRC1 // c-SRC // p60-Src | 3884191 | chr20 | + | 35406502 | 35467847 | 6.98 |
| signal-induced proliferation-associated 1 like 1 | SIPA1L1 | NM_015556 | DKFZp686G134 4 // E6TP1 // KIAA0440 | 3542847 | chr14 | + | 70857592 | 71468470 | 6.93 |
| MYST histone acetyltransferase 2 | MYST2 | NM_007067 | HB01 // HBOA | 3725779 | chr17 | + | 45196447 | 45261455 | 6.84 |
| GPI-anchored membrane protein 1 | GPIAP1 | NM_005898 // NM_203364 | GPIP137 // M11S1 // p137GPI | 3326183 | chr11 | + | 34009711 | 34081946 | 6.78 |
| integrin, alpha X (complement component 3 receptor 4 subunit) | ITGAX | XM_0011278 69 // NM_000887 | CD11C | 3657041 | chr16 | + | 31274012 | 31301819 | 6.76 |
| microtubule-associated protein, RP/EB family, member 1 | MAPRE1 | NM_012325 | EB1 // MGC117374 // MGC129946 | 3882069 | chr20 | + | 30871304 | 30901864 | 6.65 |
| RAB6 interacting protein 1 | RAB6IP1 | NM_015213 | FLJ22354 // FLJ33829 // FLJ43455 // KIAA1091 | 3362263 | chr11 | - | 9116953 | 9243468 | 6.61 |
| testis derived transcript (3 LIM domains) | TES | NM_015641 // NM_152829 | DKFZP586B202 2 // MGC1146 // TESS // TESS-2 // | 3020192 | chr7 | + | 115637811 | 115743800 | 6.22 |
| t-complex 1 | TCP1 | NM_0010088 97 // NM_030752 | CCT-alpha // CCT1 // CCTa // D6S230E // TCP-1-alpha | 2982381 | chr6 | - | 160119521 | 160130731 | 6.19 |
| CHMP family, member 7 | CHMP7 | NM_152272 | MGC29816 | 3089853 | chr8 | + | 23157105 | 23176288 | 6.08 |
| toll-like receptor 4 | TLR4 | NM_138554 | CD284 // TOLL // hToll | 3186966 | chr9 | + | 119506334 | 119670150 | 6.00 |
| fibronectin type III domain containing 3B | FNDC3B | NM_022763 | DKFZp686D141 70 // DKFZp762K137 // FAD104 // FLJ23399 // MGC10002 // PRO4979 // YVTM2421 | 2652410 | chr3 | + | 173240112 | 173601176 | 5.97 |
| serine/threonine kinase 38 like | STK38L | NM_015000 | KL4A0965 // NDR2 | 3409081 | chr12 | + | 27288343 | 27370157 | 5.97 |
| eukaryotic translation initiation factor 4 gamma, 2 | EIF4G2 | NM_0010425 59 // NM_001418 | AAG1 // DAP5 // FLJ41344 // NAT1 // p97 | 3362719 | chr11 | - | 10487574 | 10788746 | 5.97 |
| aminopeptidase puromycin sensitive | NPEPPS | XM_0011285 88 // NM_006310 | MP100 // PSA | 3724698 | chr17 | + | 42955347 | 43057168 | 5.96 |
| membrane protein, palmitoylated 1, 55kDa | MPP1 | NM_002436 | AAG12 // DXS552 // DXS552E // EMP55 // MRG1 // PEMP | 4027585 | chrX | - | 153658739 | 154075618 | 5.94 |
| eukaryotic translation initiation factor 4 gamma, 1 | EIF4G1 | NM_004953 // NM_182917 // NM_198241 // NM_198242 // NM_198244 | DKFZp686A1451 // EIF4F // EIF4G // p220 | 2655688 | chr3 | + | 185514970 | 185535829 | 5.83 |
| leucine rich repeat containing 8 family, member C | LRRC8C | NM_032270 | AD158 // DKFZp5B6J1119 // FAD158 // MGC138551 | 2345929 | chr1 | + | 89871229 | 90008050 | 5.81 |
| EH-domain containing 1 | EHD1 | NM_006795 | FLJ42622 // FLJ44618 // H- PAST // HPAST1 // PAST // PAST1 | 3377226 | chr11 | - | 64376790 | 64404202 | 5.78 |
| 3-hydroxy-3-methylglutaryl-Coenzyme A synthase 1 (soluble) | HMGCS1 | NM_002130 | HMGCS // MGC90332 | 2855501 | chr5 | - | 43324231 | 43349337 | 5.74 |
| insulin induced gene 1 | INSIG1 | NM_005542 // NM_198336 // NM 198337 | CL-6 // MGC1405 | 3033209 | chr7 | + | 154649208 | 154732868 | 5.72 |
| chromodomain helicase DNA binding protein 2 | CHD2 | NM_0010425 72 // NM 001271 | DKFZp781D172 7 | 3609138 | chr15 | + | 91227096 | 91387351 | 5.70 |
| DEAD (Asp-Glu-Ala-Asp) box polypeptide 17 | DDX17 | NM_006386 // NM_030881 | DKFZp761H2016 // P72 // RH70 | 3960629 | chr22 | - | 37202800 | 37232288 | 5.70 |
| hypothetical protein KIAA1434 | RP5-1022P6.2 | NM_019593 | FLJ11085 // K1AA1434 // MGC26147 | 3896370 | chr20 | - | 5473033 | 5546357 | 5.68 |
| nuclear factor of kappa light polypeptide gene enhancer in B-cells 2 (p49/p100) | NFKB2 | NM_0010774 93 // NM_0010774 94 // NM_002502 | LYT-10 // LYT10 | 3261643 | chr10 | + | 104144262 | 104152716 | 5.61 |
| ubiquitin-activating enzyme E1-like 2 | UBE1L2 | NM_018227 | FLJ10808 // FLJ23367 | 2771718 | chr4 | - | 68130308 | 68249472 | 5.57 |
| ras homolog gene family, member A | RHOA | NM_001664 | ARH12 // ARHA // RHO12 // | 2674242 | chr3 | - | 49371587 | 49424514 | 5.54 |
| HECT, UBA and WWE domain containing 1 | HUWE1 | NM_031407 | ARF-BP1 // HECTH9 // HSPC272 // Ib772 // KIAA0312 // LASU1 // MULE // | 4009315 | chrX | - | 53575796 | 53740871 | 5.54 |
| cyclin-dependent kinase 8 | CDK8 | NM_001260 | K35 // MGC126074 // MGC126075 | 3482498 | chr13 | + | 25701360 | 25877335 | 5.53 |
| E3 ubiquitin protein ligase, HECT domain containing, 1 | EDD1 | NM_015902 | DD5 // EDD // FLJ11310 // HYD // KIAA0896 // MGC57263 | 3147321 | chr8 | - | 103333634 | 103493671 | 5.49 |
| dedicator of cytokinesis 4 | DOCK4 | NM_014705 | FLJ34238 // KIAA0716 // MGC134911 // MGC134912 | 3068097 | chr7 | - | 111152902 | 111633744 | 5.42 |
| sterol regulatory element binding transcription factor 2 | SREBF2 | NM_004599 | SREBP2 | 3947123 | chr22 | + | 40559049 | 40633247 | 5.40 |
| heterogeneous nuclear ribonucleoprotein M | HNRPM | NM_005968 // NM_031203 | DKFZp547H118 // HNRNPM // HNRNPM4 // HNRPM4 // HTGR1 // NAGR1 | 3819543 | chr19 | + | 8415651 | 8459993 | 5.34 |
| SH3 domain binding glutamic acid-rich protein like 3 // coiled-coil domain containing 21 | SH3BC3RL3 // CCDC21 | NM_031286 // NM_022778 | SH3BP-1 // TIP-B1 // DKFZP434L011 7 // DKFZp434P232 // FLJ13976 // FLJ22000 | 2326448 | chr1 | + | 26478669 | 26480591 | 5.34 |
| exportin 4 | XPO4 | NM_022459 | FLJ13046 // KIAA1721 | 3504434 | chr13 | - | 20251014 | 20375187 | 5.33 |
| squalene epoxidase | SQLE | NM_003129 | | 3114832 | chr8 | + | 126063738 | 126105522 | 5.19 |
| peptidylprolyl isomerase F (cyclophilin F) | PPIF | NM_005729 | CYP3 // Cyp-D // FLJ90798 // MGC117207 | 3253880 | chr10 | + | 80777230 | 80785093 | 5.19 |
| serum response factor (c-fos serum response element-binding transcription factor) | SRF | NM_003131 | MCM1 | 2907730 | chr6 | + | 43246765 | 43257189 | 5.15 |
| c-mer proto-oncogene tyrosine kinase | MERTK | NM_006343 | MER // MGC133349 // c-mer | 2500550 | chr2 | + | 112372656 | 112513624 | 5.12 |
| echinoderm microtubule associated protein like 4 | EML4 | NM_019063 | C2orf2 // DKFZp686P1811 8 // ELP120 // FLJ10942 // FLJ32318 // ROPP120 | 2478748 | chr2 | + | 42250017 | 42418636 | 5.02 |

### (2) Identification of the gene of which introns exhibit increased expression in a dose-dependent fashion in groups with the treatment at a concentration of 3 nM, 10 nM, and 30 nM.

Probe sets showing that the normalized change in the level expression of the probe set in the group with the treatment at 30 nM was more than 5 times; the expression level of the probe set and the gene in the group with the treatment at 30 nM was more than 100; and probe set annotation was "extended, full, free", were picked out. Regression analysis was performed with the probe sets extracted for the control group, the group with the treatment at 3 nM, the group with the treatment at 10 nM, and the group with the treatment at 30 nM to determine a p and q value for the slope of regression formula. A gene containing the probe sets with the q value of less than 5% was determined as a candidate gene with increased expression level of the intron regions (Table 5).

In Table 5, for the gene evaluated, gene name (Gene Name), abbreviated name (Gene Symbol), accession number (Accession), alias name (Synonym), transcription cluster number in Human Exon 1.0 ST Array (Human Exon 1.0 ST Array Transcription Cluster ID), chromosome number (Chromosome), type of strand (Strand), start region (Start), stop region (Stop), and change in expression level per 1 nM (Fold Change/nM) were respectively shown.

**[Table 5]**

| Gene Name | Gene Symbol | Accession | Synonym | Human Exon ST 1.0 | Human Genome hg18 | | | | Fold Change /nM |
|---|---|---|---|---|---|---|---|---|---|
| | | | | Transcript Cluster ID | Chrom osome | Strand | Start | Stop | |
| epiregulin | EREG | NM_001432 | ER | 2731513 | chr4 | + | 75440309 | 75473341 | 1.08 |
| pellino homolog 1 (Drosophila) | PELI1 | NM_020651 | DKFZp686C18116 // MGC50990 | 2556302 | chr2 | - | 64173290 | 64225291 | 1.08 |
| integrin, alpha V (vitronectin receptor, alpha polypeptide, antigen CD51) | ITGAV | NM_002210 | CD51 // MSK8 // VNRA | 2519229 | chr2 | + | 187163045 | 187253869 | 1.08 |
| heat shock 70kDa protein 5 (glucose-regulated protein, 78kDa) | HSPA5 | NM_005347 | BIP // FLJ26106 // GRP78 // MIF2 | 3225398 | chr9 | - | 127038953 | 127064590 | 1.08 |
| villin 2 (ezrin) | VIL2 | NM_003379 | CVIL // CVL // DKFZp762H157 // FLJ26216 // MGC1584 | 2981912 | chr6 | - | 159106770 | 159160432 | 1.08 |
| UDP-Gal:betaGlcNAc beta 1,4-galactosyltransferase, polypeptide 5 | B4GALT5 | NM_004776 | B4Gal-T5 // BETA4-GALT-N // MGC138470 // beta4Gal-T5 // beta4GaIT-V // gt- | 3908963 | chr20 | - | 47682889 | 47809709 | 1.08 |
| pleckstrin homology, Sec7 and coiled-coil domains 1(cytohesin 1) | PSCD1 | NM_004762 // NM_017456 | B2-1 // CYTOHESIN-1 // D17S811E // FLJ34050 // FLJ41900 // SEC7 | 3772525 | chr17 | - | 74181731 | 74289973 | 1.08 |
| EH-domain containing 4 | EHD4 | NM_139265 | PAST4 | 3620276 | chr15 | - | 39977674 | 40052063 | 1.08 |
| TIMP metallopeptidase inhibitor 1 | TIMP1 | NM_003254 | CLGI // EPA // EPO // FLJ90373 // HCI // TIMP | 3976341 | chrX | + | 47326654 | 47332978 | 1.08 |
| interleukin 1 receptor antagonist | IL1RN | NM_000577 // NM_173841 // NM_173842 // NM_173843 | ICIL-1RA // IL- 1ra3 // IL1F3 // IL1RA // IRAP // MGC10430 | 2501204 | chr2 | + | 113573383 | 113608060 | 1.08 |
| PRP40 pre-mRNA processing factor 40 homolog A (S. cerevisiae) | PRPF40A | XM_371575 // XM_938514 | FBP-11 // FBP11 // FLAF1 // FLJ20585 // FNBP3 // HIP10 // HYPA // NY-REN- | 2581548 | chr2 | - | 153212414 | 153283546 | 1.08 |
| ATP-binding cassette, sub-family A (ABC1), member 1 | ABCA1 | NM_005502 | ABC-1 // ABC1 // CERP // FLJ14958 // HDLDT1 // TGD | 3218528 | chr9 | - | 106583121 | 106748222 | 1.08 |
| RA85A, member RAS oncogene family | RAB5A | NM 004162 | RAB5 | 2613386 | chr3 | + | 19963571 | 20002852 | 1.07 |
| protein phosphatase 2 (formerly 2A), catalytic subunit, alpha isoform | PPP2CA | NM_002715 | PP2Ac // PP2CA // RP-C | 2876046 | chr5 | - | 133557588 | 133589841 | 1.07 |
| membrane protein, palmitoylated 7 (MAGUK p55 subfamily member 7) | MPP7 | NM_173496 | FLJ32798 | 3282601 | chr10 | - | 28377816 | 28631969 | 1.07 |
| RAB6 interacting protein 1 | RAB6IP1 | NM_015213 | FLJ22354 // FLJ33829 // FLJ43455 // KIAA1091 | 3362263 | chr11 | - | 9116953 | 9243468 | 1.07 |
| GRB2-associated binding protein 2 | GAB2 | NM_012296 // NM_080491 | KIAA0571 | 3383227 | chr11 | - | 77603886 | 77989843 | 1.07 |
| chloride intracellular channel 4 // adducin 3 (gamma) | CLIC4 // ADD3 | NM_013943 // NM_001121 // NM_016824 // NM_019903 | CUC4L // DKFZP566G223 // FLJ38640 // H1 // huH1 // p64H1 // ADDL | 2325593 | chr1 | + | 24943202 | 25071376 | 1.07 |
| TBC1 domain family, member 23 | TBC1D23 | NM_018309 | DKFZp667G062 // FLJ11046 // NS4ATP1 | 2633587 | chr3 | + | 101462368 | 101526762 | 1.07 |
| solute carrier family 16, member 3 (monocarboxylic acid transporter 4) | SLC16A3 | NM_001042422 // NM_001042423 // NM 004207 | MCT3 // MCT4 // MGC138472 // MGC138474 | 3738629 | chr17 | + | 77775274 | 77812305 | 1.07 |
| sestrin 3 | SESN3 | NM_144665 | MGC29667 // SEST3 | 3387259 | chr11 | - | 94545767 | 94605309 | 1.07 |
| nuclear RNA export factor 1 | NXF1 | NM_001081491 // NM_006362 | DKFZp66700311 // MEX67 // TAP | 3376155 | chr11 | - | 62316178 | 62329529 | 1.07 |
| myristoylated alanine-rich protein kinase C substrate | MARCKS | NM_002356 | 80K-L // FLJ14368 // FLJ90045 // MACS // MRACKS // PRKCSL // PRKCSL | 2922215 | chr6 | + | 114284762 | 114292853 | 1.07 |
| retinitis pigmentosa 2 (X-linked recessive) | RP2 | NM_006915 | KlAA0215 // TBCCD2 | 3975869 | chrX | + | 46581319 | 46637601 | 1.07 |
| DnaJ (Hsp40) homolog, subfamily C, member 3 | DNAJC3 | NM_006260 | HP58 // P58 // P581PK // PRKRI | 3497270 | chr13 | + | 95125276 | 95245596 | 1.07 |
| interleukin 1, alpha | IL1A | NM_000575 | IL-1A // IL1 // IL1-ALPHA // | 2571483 | chr2 | - | 113247977 | 113259446 | 1.07 |
| TANK-binding kinase 1 | TBK1 | NM_013254 | FLJ11330 // NAK // T2K | 3419849 | chr12 | + | 63132014 | 63202097 | 1.07 |
| adaptor-related protein complex 3, beta 1 subunit | AP3B1 | NM_003664 | ADTB3 // ADTB3A // HPS // HPS2 // PE | 2863730 | chr5 | - | 77318390 | 77671471 | 1.07 |
| ATPase type 13A3 | ATP13A3 | XM_931948 // XM_942079 | AFURS1 // DKFZp686K16189 // FLJ90613 | 2711644 | chr3 | - | 195604703 | 195757213 | 1.07 |
| coiled-coil domain containing 131 | CCDC131 | NM_144982 | DKFZp686A0722 // KIAA0546 // MGC23401 // MGC90200 // PSRC2 | 3462094 | chr12 | - | 70269806 | 70344289 | 1.07 |
| calmodulin 2 (phosphorylase kinase, delta) | CALM2 | NM_001743 | CAMII // PHKD // PHKD2 | 2551924 | chr2 | - | 47232159 | 47286178 | 1.07 |
| ubiquitously transcribed tetratricopeptide repeat gene, Y-linked // ubiquitously transcribed tetratricopeptide repeat, X chromosome | UTY // UTX | NM_007125 // NM_182659 // NM_182660 // NM_021140 | DKFZp686L12190 // UTY1 // DKFZp686A03225 // MGC141941 // bA386N14.2 | 4035017 | chrY | - | 13772846 | 14101944 | 1.07 |
| brain abundant, membrane attached signal protein 1 | BASP1 | NM_006317 | CAP-23 // CAP23 // MGC8555 // NAP-22 // NAP22 | 2803329 | chr5 | + | 17118727 | 17381190 | 1.07 |
| GTP cyclohydrolase 1 (dopa-responsive dystonia) | GCH1 | NM_000161 // NM_001024024 // NM_001024070 // NM_001024071 | DYT5 // GCH // GTP-CH-1 // GTPCH1 | 3565524 | chr14 | - | 54375952 | 54439279 | 1.07 |
| proteasome (prosome, macropain) 26S subunit, non-ATPase, 3 | PSMD3 | NM_002809 | P58 // RPN3 // S3 | 3720636 | chr17 | + | 35390438 | 35407732 | 1.07 |
| acyl-CoA synthetase long-chain family member 1 | ACSL1 | NM_001995 | ACS1 // FACL1 // FACL2 // LACS // LACS1 // LACS2 | 2796553 | chr4 | - | 185913758 | 186002178 | 1.07 |
| numb homolog (Drosophila) | NUMB | NM_001005743 // NM_001005744 // NM_001005745 // NM_003744 | S171 | 3571347 | chr14 | - | 72811631 | 73000081 | 1.07 |
| protein kinase C, epsilon | PRKCE | NM_005400 | MGC125656 // MGC125657 // PKCE // nPKC- epsilon | 2480168 | chr2 | + | 45731934 | 46343070 | 1.07 |
| quaking homolog, KH domain RNA binding (mouse) | QK1 | XM_945803 // NM_006775 // NM_206853 // NM_206854 // NM_206855 | DKFZp58610923 // Hqk // QK // QK3 | 2935475 | chr6 | + | 163684268 | 163960462 | 1.07 |
| eukaryotic translation initiation factor 2C, 2 | EIF2C2 | NM_012154 | AGO2 // MGC3183 //Q10 | 3156193 | chr8 | - | 141599440 | 141726037 | 1.07 |
| CD2 molecule | CD2 | M_001767 | SRBC // T11 | 2353669 | chr1 | + | 117098550 | 117149098 | 1.07 |
| nuclear factor of kappa light polypeptide gene enhancer in B-cells 1 (p105) | NFKB1 | NM_003998 | DKFZp686C01211 // EBP-1 // KBF1 // MGC54151 // NF-kappa-B // NFKB-p105 // | 2737717 | chr4 | + | 103568522 | 103757506 | 1.07 |
| transcription elongation factor B (SIII), polypeptide 3 (110kDa, elongin A) | TCEB3 | NM_003198 | SIII // TCEB3A | 2325251 | chr1 | + | 23942459 | 23961135 | 1.07 |
| COP9 constitutive photomorphogenic homolog subunit 2 (Arabidopsis) | COPS2 | NM_004236 | ALIEN // CSN2 // SGN2 // TRIP15 | 3623424 | chr15 | - | 47203877 | 47235198 | 1.07 |
| pyridoxal (pyridoxine, vitamin B6) kinase // chromosome 21 open reading frame 124 | PDXK // C21orf124 | NM_003681 // NM_032920 | C21 orf97 // PKH // PNK // FLJ31940 // MGC15873 // PRED79 | 3923257 | chr21 | + | 43963416 | 44006601 | 1.07 |
| nucleoporin 98kDa | NUP98 | NM_005387 // NM-016320 // NM_139131 // NM_139132 | ADIR2 // NUP196 | 3359910 | chr11 | - | 3652836 | 3775353 | 1.07 |
| muskelin 1, intracellular mediator containing kelch motifs | MKLN1 | NM_013255 | FLJ11162 | 3024275 | chr7 | + | 130445315 | 130837940 | 1.07 |
| ceramide kinase | CERK | NM_022766 // NM_182661 | DKFZp434E02H // FLJ21430 // FLJ23239 // KIAA1646 // LK4 // MGC131878 // dA59H18.2 // dA59H18.3 // hCERK | 3964154 | chr22 | - | 45454343 | 45537349 | 1.07 |
| non-POU domain containing, octamer-binding | NONO | NM_007363 | NMT55 // NRB54 // P54 // P54NRB | 3980887 | chrX | + | 70420006 | 70437726 | 1.07 |
| neuroepithelial cell transforming gene 1 | NET1 | NM_001047160 // NM_005863 | ARHGEF8 // NET1A | 3233182 | chr10 | + | 5444535 | 5491001 | 1.07 |
| mannosyl (alpha-1,3-)-glycoprotein beta-1,4-N-acetylglucosaminyltransferase, Isozyme A // hypothetical protein MaC52110 | MGAT4A // MGC52110 | NM_012214 // NM_001008215 | GNT-IV // GNT- IVA // 6330578E17Rik | 2566414 | chr2 | - | 98600132 | 98714000 | 1.07 |
| protein phosphatase 1, regulatory (inhibitor) subunit 10 | PPP1R10 | NM_002714 | CAT53 // FB19 // PNUTS | 2948425 | chr6 | - | 30676177 | 30694348 | 1.07 |
| inositol polyphosphate-5-phosphatase, 40kDa | INPP5A | XM_001133189 // NM_005539 | 5PTASE // DKFZp434A1721 // MGC116947 // MGC116949 | 3272205 | chr10 | + | 134201308 | 134446967 | 1.07 |
| plasminogen activator, urokinase receptor | PLAUR | NM_001005376 // NM_001005377 // NM_002659 | CD87 // UPAR // URKR | 3864551 | chr19 | - | 48841865 | 48866539 | 1.06 |
| PHD finger protein 12 | PHF12 | NM_001033561 // NM 020889 | KIAA1523 MGC131914 // PF1 | 3751184 | chr17 | - | 24256404 | 24302905 | 1.06 |
| synapse associated protein 1, SAP47 homolog (Drosophila) | SYAP1 | NM_032796 | DKFZp686K221 // FLJ14495 // FLJ44185 // PRO3113 | 3970130 | chrX | + | 16639861 | 16690708 | 1.06 |
| chromosome 1 open reading frame 152 // phosphodiesterase 4D interacting protein (myomegalin) | Clorf152 // PDE4DIP | NR_003242 // XM_943179 // NM_001 002810 // NM_001D02811 // NM_001002812 // NM_014644 // NM_022359 | COAS3 // CMYA2 // DKFZp781J054 // MGC75440 // MMGL | 2431886 | chr1 | - | 142444924 | 143804054 | 1.06 |
| homocysteine-inducible, endoplasmic reticulum stress-inducible, ubiquitin-like domain member 1 | HERPUD1 | NM_001010989 // NM_001010990 // NM_014685 | HERP // KIAA0025 // Mif1 // SUP | 3662387 | chr16 | + | 55523269 | 55552122 | 1.06 |
| TNF receptor-associated factor 1 | TRAF1 | NM_005658 | EBI6 // MGC:10353 | 3223738 | chr9 | - | 122699696 | 122731521 | 1.06 |
| pumilio homolog 2 (Drosophila) | PUM2 | NM_015317 | FLJ36528 // KIAA0235 // MGC138251 // MGC138253 // PUMH2 // PUML2 | 2542816 | chr2 | - | 20291440 | 20450197 | 1.06 |
| Dmx-like 2 | DMXL2 | NM_015263 | FLJ26672 // KIAA0856 // RC3 | 3624145 | chr15 | - | 49527223 | 49702258 | 1.06 |
| RAB8B, member RAS oncogene family | RAB8B | NM_016530 | FLJ38125 | 3597476 | chr15 | + | 61268620 | 61347018 | 1.06 |
| tight junction protein 2 (zona occludens 2) | TJP2 | NM_004817 // NM_201629 | MGC26306 // X104 // ZO-2 // ZO2 | 3173880 | chr9 | + | 70925894 | 71059931 | 1.06 |
| BCL2/adenovirus E1B 19kDa interacting protein 2 | BNIP2 | NM_004330 | BNIP-2 // NIP2 | 3627076 | chr15 | - | 57742025 | 57768916 | 1.06 |
| sorting nexin 9 // synaptojanin 2 | SNX9 // SYNJ2 | NM_016224 // NM_003898 | MST155 // MSTP155 // SDP1 // SH3PX1 // SH3PXD3A // WISF // INPP5H // KIAA0348 // MGC44422 | 2933331 | chr6 | + | 158164282 | 158286097 | 1.06 |
| programmed cell death 4 (neoplastic transformation inhibitor) | PDCD4 | NM_014456 // NM_145341 | H731 // MGC33046 // MGC33047 | 3263944 | chr10 | + | 112621575 | 112649753 | 1.06 |
| leupaxin | LPXN | NM 004811 | LDPL | 3374402 | chr11 | - | 58050925 | 58102459 | 1.06 |
| solute carrier family 7 (cationic amino acid transporter, y+ system), member 5 // SLC7A5 pseudogene | SLC7A5 // LAT1-3TM | NM_003486 // NR_002593 | 4F2LC // CD98 // D16S469E // E16 // LAT1 // MPE16 // hLAT1 // DC49 // MLAS // hLAT1-3TM | 3703885 | chr16 | - | 86421130 | 86460615 | 1.06 |
| heat shock protein 90kDa beta (Grp94), member 1 // thymine-DNA glycosylase // heat shock protein 90kDa beta (Grp94), member 2 (pseudogene) | HSP90B1 // TDG // HSP90B2P | NM_003299 // NM_003211 // - | ECGP // GP96 // GRP94 // TRA1 // - // GRP94P1 // GRP94b // HSP // HSPCP2 // HsGrp94b // TRA1P1 // TRAP1 | 3429312 | chr12 | + | 102848291 | 102871551 | 1.06 |
| TNFAIP3 interacting protein 1 | TNIP1 | NM_006058 | ABIN-1 // KIAA0113 // NAF1 // VAN | 2881672 | chr5 | - | 150389707 | 150446947 | 1.06 |
| NECAP endocytosis associated 2 | NECAP2 | NM_018090 | FLJ10420 // RP4- 798A10.1 | 2322389 | chr1 | + | 16639774 | 16659570 | 1.06 |
| proteasome (prosome, macropain) subunit, alpha type, 5 | PSMA5 | NM_002790 | MGC117302 // MGC125802 // MGC125803 // MGC125B04 // PSC5 // ZETA | 2427074 | chr1 | - | 109742573 | 109770611 | 1.06 |
| guanine nucleotide binding protein-like 2 (nucleolar) | GNL2 | NM_013285 | FLJ40906 // HUMAUANTIG // NGP1 // Ngp-1 // dJ423B22.6 | 2407191 | chr1 | - | 37805043 | 37834120 | 1.06 |
| GRIP and coiled-coil domain containing 2 | GCC2 | NM_014635 // NM 181453 | GCC185 // KIAA0336 | 2498977 | chr2 | + | 108431315 | 108492470 | 1.06 |
| RAD50 homolog (S. cerevisiae) | RAD50 | NM_005732 // NM_133462 | RAD50-2 // hRad50 | 2828564 | chr5 | + | 131774479 | 132009928 | 1.06 |
| fibronectin type III domain containing 3B | FNDC3B | NM_022763 | DKFZp686D14170 // DKFZp762K137 // FAD104 // FLJ23399 // MGC10002 // PRO4979 // YVTM2421 | 2652410 | chr3 | + | 173240112 | 173601176 | 1.06 |
| son of sevenless homolog 2 (Drosophila) | SOS2 | NM 006939 | - | 3563734 | chr14 | - | 49654812 | 49768331 | 1.06 |
| heat shock protein 90kDa alpha (cytosolic), class A member 1 // heat shock protein 90kDa alpha (cytosolic), class A member 2 // heat shock protein 90kDa alpha (cytosolic), class A member 6 (pseudogene) | HSP90AA1 // HSP90AA2 // HSP90AA6P | NM_001017963 // NM_005348 // NM_001040141 // - | FLJ31884 // HSP86 // HSP90A // HSP90N // HSPC1 // HSPCA // HSPCAL1 // HSPCAL4 // HSPN // Hsp89 // Hsp90 // LAP2 // HSP90ALPHA // HSPCAL3 // HSP90Af | 3580179 | chr14 | - | 101616842 | 101676327 | 1.06 |
| phosphodiesterase 4B, cAMP-specific (phosphodiesterase E4 dunce homolog, Drosophila) | PDE4B | NM_001037339 // NM_001037340 // NM_001037341 // NM_002600 | DKFZp686F2182 // DPDE4 // MGC126529 // PDEIVB | 2340529 | chr1 | + | 66030487 | 66612844 | 1.06 |
| interleukin-1 receptor-associated kinase 2 | IRAK2 | NM_001570 | IRAK-2 // MGC150550 | 2610359 | chr3 | + | 10181579 | 10260427 | 1.06 |
| chromosome 14 open reading frame 32 | C14orf32 | NM_144578 | MGC23138 // MISS // c14 5346 | 3536663 | chr14 | + | 54588114 | 54606655 | 1.06 |
| nucleoside phosphorylase | NP | NM_000270 | MGC117396 // MGC125915 // MGC125916 // PNP // PRO1837 // PUNP | 3527514 | chr14 | + | 20007398 | 20015985 | 1.06 |
| proline-serine-threonine phosphatase interacting protein 2 | PSTPIP2 | NM_024430 | MAYP // MGC34175 | 3806211 | chr18 | - | 41817502 | 41906221 | 1.06 |
| growth factor receptor-bound protein 2 | GRB2 | NM_002086 // NM_203506 | ASH // EGFRBP- GRB2 // Grb3-3 // MST084 // MSTP084 | 3770743 | chr17 | - | 70805871 | 70913384 | 1.06 |
| signal-induced proliferation-associated 1 like 1 | SIPA1L1 | NM_015556 | DKFZp686G1344 // E6TP1 // KIAA0440 | 3542847 | chr14 | + | 70857592 | 71468470 | 1.06 |
| exportin 1 (CRM1 homolog, yeast) // thyroid hormone receptor, beta (erythroblastic leukemia viral (v-erb-a) oncogene homolog 2, avian) | XPO1 // THRB | NM_003400 // NM_000461 | CRM1 // DKFZp686B1823 // ERBA-BETA // ERBA2 // GRTH // MGC126109 // MGC126110 // NR1A2 // THR1 // THR81 // THR82 | 2555490 | chr2 | - | 61558490 | 61619343 | 1.06 |
| centromere protein C 1 | CENPC1 | NM_001812 | CENP-C // CENPC // MIF2 // hcp-4 | 2771654 | chr4 | - | 68020183 | 68093837 | 1.06 |
| v-abl Abelson murine leukemia viral oncogene homolog 2 (arg, Abelson-related gene) | ABL2 | NM_005158 // NM 007314 | ABLL // ARG | 2446047 | chr1 | - | 177335093 | 177528267 | 1.06 |
| ring finger protein 10 | RNF10 | NM_014868 | KIAA0262 // MGC126758 // MGC126764 // | 3434413 | chr12 | + | 119456489 | 119500226 | 1.06 |
| Notch homolog 2 (Drosophila) // neuroblastoma breakpoint family, member 14 | NOTCH2 // NBPF14 | XM_001133349 // NP_024408 // NM_015383 | AGS2 // hN2 // DJ328E19.C1.1 // FLJ35032 // NBPF | 2431112 | chr1 | - | 120205744 | 120426930 | 1.06 |
| endothelin converting enzyme 1 // amyloid beta (A4) precursor protein-binding, family B, member 2 (Fe65-like) | ECE1 // APBB2 | NM_001397 // NM_173075 | ECE // FE65L // FE65L1 // MGC35575 | 2400518 | chr1 | - | 21394307 | 21544720 | 1.06 |
| phosphoinositide-3-kinase adaptor protein 1 | PIK3AP1 | NM_152309 | BCAP // RP11- 34E5.3 | 3301914 | chr10 | - | 98342808 | 98470259 | 1.06 |
| BTB and CNC homology 1, basic leucine zipper factor 2 | BACH2 | NM_021813 | - | 2964553 | chr6 | - | 90692975 | 91063182 | 1.06 |
| transcription ATPase, H+ transporting, lysosomal 56/58kDa, V1 subunit B2 | ATP6V1B2 | NM_001693 | ATP6B1B2 // ATP6B2 // HO57 // VATB // VPP3 // Vma2 | 3088544 | chr8 | + | 20098984 | 20143098 | 1.06 |
| TRAF2 and NCK interacting kinase | TNIK | NM_015028 | AD 2 | 2705266 | chr3 | - | 172258898 | 172660964 | 1.06 |
| AT-hook transcription factor | AKNA | NM_030767 | KIAA1968 // RP11- 8211.4 | 3221916 | chr9 | - | 116138238 | 116200584 | 1.06 |
| Fas (TNF receptor superfamily, member 6) | FAS | NM_000043 // NM_152871 // NM_152872 // NM_152873 // NM_152874 // NM_152875 // NM_152876 // NM_152877 | ALPS1A // APO-1 // APT1 // CD95 // FAS1 // FASTM // TNFRSF6 | 3257098 | chr10 | + | 90721519 | 90765521 | 1.06 |
| ankyrin repeat domain 17 | ANKRD17 | NM_032217 // NM_198889 | FLJ22206 // GTAR // KIAA0697 // NY-BR-16 | 2773023 | chr4 | - | 74158547 | 74343428 | 1.06 |
| clathrin interactor 1 | CLINT1 | NM_014666 | CLINT // ENTH // EPN4 // EPNR // EPSINR // KIAA0171 | 2883609 | chr5 | - | 157145339 | 157284818 | 1.06 |
| PRP8 pre-mRNA processing factor 8 homolog (S. cerevisiae) | PRPF8 | NM_006445 | HPRP8 // PRP8 // PRPC8 // RP13 | 3740479 | chur17 | - | 1500681 | 1534906 | 1.06 |
| AF4/FMR2 family, member 4 | AFF4 | NM_014423 | AF5Q31 // MCEF // MGC75036 | 2875555 | chr5 | - | 132238768 | 132327205 | 1.06 |
| v-rel reticuloendotheliosis viral oncogene homolog (avian) | REL | NM_002908 | C-Rel | 2484358 | chr2 | + | 60962266 | 61004124 | 1.06 |
| excision repair cross-complementing rodent repair deficiency, complementation group 1 (includes overlapping antisense sequence) | ERCC1 | NM_001983 // NM_202001 | UV20 | 3865378 | chr19 | - | 50602433 | 50673926 | 1.06 |
| ubiquitously transcribed tetratricopeptide repeat, X chromosome // ubiquitously transcribed tetratricopeptide repeat gene, Y-linked | UTX // UTY | NM_021140 // NM_007125 // NM_182659 // NM_182660 | DKFZp686A03225 // MGC141941 // bA386N14.2 // DKFZp686L12190 // UTY1 | 3975467 | chrX | + | 44590716 | 44896185 | 1.06 |
| ATPase, Ca++ transporting, plasma membrane 1 | ATP2B1 | NM_001001323 // NM 001682 | PMCA1 | 3464983 | chr12 | - | 88505965 | 88620030 | 1.06 |
| v-ets erythroblastosis virus E26 oncogene homolog 2 (avian) | ETS2 | NM_005239 | - | 3921068 | chr21 | + | 39059147 | 39118744 | 1.06 |
| zinc finger protein 91 homolog (mouse) // ciliary neurotrophic factor | ZFP91 // CNTF | NM_053023 // NM_170768 // NM 000614 | FKSG11 // PZF // ZNF757 // HCNTF | 3331730 | chr11 | + | 58101868 | 58149782 | 1.06 |
| integrin, alpha 5 (fibronectin receptor, alpha polypeptide) | ITGA5 | NM_002205 | CD49e // FNRA // VLA5A | 3456732 | chr12 | - | 53075332 | 53099491 | 1.06 |
| IBR domain containing 3 | IBRDC3 | NM_153341 | FLJ90005 | 2405312 | chr1 | - | 33172120 | 33203343 | 1.06 |
| cofactor required for Sp1 transcriptional activation, subunit 2, 150kDa | CRSP2 | XM_001126052 // NM_004229 | CRSP150 // CSRP // CXorf4 // DRIP150 // EXLM1 // MED14 // MGC104513 // RGR1 // TRAP170 | 4005644 | chrX | - | 40392502 | 40480045 | 1.06 |
| aryl hydrocarbon receptor | AHR | NM 001621 | - | 2991233 | chr7 | + | 17304771 | 17363729 | 1.06 |
| solute carrier family 43, member 2 | SLC43A2 | NM_152346 | FLJ23848 // LAT4 // MGC34680 | 3740367 | chr17 | - | 1423948 | 1478920 | 1.06 |
| chemokine (C-C motif) ligand 20 | CCL20 | NM_004591 | CKb4 // LARC // MIP-3a // MIP3A // SCYA20 // | 2530713 | chr2 | + | 228386802 | 228427090 | 1.06 |
| serpin peptidase inhibitor, clade B (ovalbumin), member 8 | SERPINB8 | NM_001031848 // NM_002640 // NM_198833 | CAP2 // PI8 | 3791996 | chr18 | + | 59787618 | 59823238 | 1.06 |
| chaperonin containing TCP1, subunit 4 (delta) | CCT4 | NM_006430 | Cctd // MGC126164 // MGC126165 // SRB | 2555630 | chr2 | - | 61939693 | 61970469 | 1.06 |
| AP2 associated kinase 1 | AAK1 | NM_014911 | KIAA1048 // MGC138170 | 2558150 | chr2 | - | 69538639 | 69783824 | 1.06 |
| musculin (activated B-cell factor-1) | MSC | NM_005098 | ABF-1 // ABF1 // MYOR | 3140213 | chr8 | - | 72843959 | 73059105 | 1.06 |
| Yip1 domain family, member 5 | YIPF5 | NM_001024947 // NM_030799 | DKFZp313L2216 // FinGER5 // SB140 // SMAP-5 // SMAP5 // YIP1A | 2879509 | chr5 | - | 143463451 | 143565514 | 1.06 |
| START domain containing 7 | STARD7 | NM_020151 // NM_139267 | GTT1 | 2565143 | chr2 | - | 96214334 | 96238296 | 1.06 |
| ADP-ribosylation factor guanine nucleotide-exchange factor 1(brefeldin A-inhibited) | ARFGEF1 | NM_006421 | ARFGEP1 // BIG1 // D730028018Rik // DKFZP434L057 // P200 | 3139035 | chr8 | - | 68250009 | 68418446 | 1.06 |
| myeloid/lymphoid or mixed-lineage leukemia 5 (trithorax homolog, Drosophila) | MLL5 | NM_018682 // NM_182931 | FLJ10078 // FLJ14026 // HDCMC04P // MGC70452 | 3017547 | chr7 | + | 104436029 | 104542698 | 1.06 |
| DEAD (Asp-Glu-Ala-Asp) box polypeptide 3, X-linked | DDX3X | XM_001129278 // NM_001356 | DBX // DDX14 // DDX3 // HLP2 | 3974838 | chrX | + | 41055591 | 41108668 | 1.06 |
| nibrin | NBN | NM-001024688 // NM_002485 | AT-V1 // AT-V2 // ATV // FLJ10155 // MGC87362 // NBS | 3143970 | chr8 | - | 91014745 | 91066433 | 1.06 |
| AT rich interactive domain 4B (RBP1-like) // RNA binding motif protein 34 | ARID4B // RBM34 | NM_016374 // NM_031371 // NM_015014 | BCAA // BRCAA1 // DKFZp313M2420 // MGC163290 // RBBP1L1 // RBP1L1 // SAP180 // KIAA0117 | 2461786 | chr1 | - | 233396677 | 233558145 | 1.06 |
| ankyrin repeat domain 44 | ANKRD44 | NM_153697 | MGC21968 // MGC70444 | 2593464 | chr2 | - | 197512324 | 197901798 | 1.06 |
| RB1-inducible coiled-coil 1 | RB1CC1 | NM_014781 | OCI // DRAGOU14 // FIP200 | 3135184 | chr8 | - | 53641628 | 53789545 | 1.06 |
| ubiquitin specific peptidase 12 | USP12 | NM 182488 | USP12L1 | 3506738 | chr13 | | 26518485 | 26644264 | 1.06 |
| apoptosis inhibitor 5 | API5 | NM_006595 | AAC-11 // AAC11 // AP15L1 | 3327906 | char11 | + | 43290109 | 43322649 | 1.06 |
| HIR histone cell cycle regulation defective homolog A (S. cerevisiae) | HIRA | NM_003325 | DGCR1 // TUP1 // TUPLE1 | 3952637 | chr22 | - | 17696576 | 17799452 | 1.06 |
| guanylate binding protein 7 // guanylate binding protein 2, interferon-inducible // guanylate binding protein 4 | GBP7 // GBP2 // GBP4 | NM_207398 // NM_004120 // NM_052941 | FLJ38822 // GBP4L // - // Mpa2 | 2421925 | chr1 | - | 89343675 | 89496682 | 1.06 |
| CASP8 and FADD-like apoptosis regulator | CFLAR | NM_003879 | CASH // CASP8AP1 // CLARP // Casper // FLAME // FLAME-1 // FLIP // I-FLICE // MRIT // USURPIN // c- FLIP // c-FLIPL // | 2522616 | chr2 | + | 201689135 | 201744701 | 1.06 |
| human immunodeficiency virus type I enhancer binding protein 2 | HIVEP2 | NM_006734 | HIV-EP2 // MBP-2 // MIBP1 | 2977265 | chr6 | - | 143065515 | 143308841 | 1.06 |
| serpin peptidase inhibitor, clade B (ovaibumin), member 9 // serpin peptidase inhibitor, clade B (ovalbumin), member 6 | SERPINB9 // SERPINB6 | NM_004155 // NM_004568 | CAP-3 // CAP3 // P19 // CAP // DKFZp686104222 // MGC111370 // MSTP057 // P16 // PTI | 2939034 | chr6 | - | 2832402 | 2858242 | 1.06 |
| myxovirus (influenza virus) resistance 2 (mouse) | MX2 | NM_002463 | MXB | 3922037 | chr21 | + | 41655820 | 41703177 | 1.06 |
| solute carrier family 25, member 37 | SLC25A37 | XM_001128238 // XM_001128248 // XM_001128255 // XM_001128269 // NM_016612 | HT015 // MFRN // MSC // MSCP // PR01278 // PR01584 // PR02217 | 3090006 | chr8 | + | 23439117 | 23485313 | 1.06 |
| WW domain binding protein 11 | WBP11 | NM_016312 | DKFZp779M1063 // NPWBP // SIPP1 | 3445670 | chr12 | - | 14829481 | 14848171 | 1.06 |
| bromodomain adjacent to zinc finger domain, 1A | BAZIA | NM_013448 // NM_182648 | ACF1 // DKFZP586E0518 // FLJ14383 // WALp1 // WCRF180 // hACF1 | 3560711 | chr14 | - | 34291692 | 34414604 | 1.06 |
| aquaporin 9 | AQP9 | NM_020980 | HsT17287 // SSC1 | 3595594 | chr15 | + | 56217766 | 56280468 | 1.06 |
| solute carrier family 39 (zinc transporter), member 8 | SLC39A8 | NM_022154 | BIGM 103 // LZT- Hs6 | 2779823 | chr4 | - | 103393022 | 103486067 | 1.06 |
| FYN oncogene related to SRC, FGR, YES | FYN | NM_002037 // NM_153047 // NM_153048 | MGC45350 // SLK // SYN | 2969886 | chr6 | - | 112088237 | 112301348 | 1.06 |
| elongation factor, RNA polymerase II, 2 | ELL2 | NM_012081 | | 2867873 | chr5 | - | 95246561 | 95323733 | 1.06 |
| Janus kinase 1 (a protein tyrosine kinase) | JAK1 | NM_002227 | JAK1A // JAK1B | 2416522 | chr1 | - | 65071504 | 65278035 | 1.06 |
| v-rel reticuloendotheliosis viral oncogene homolog B, nuclear factor of kappa light polypeptide gene enhancer in B-cells 3 (avian) | RELB | NM_006509 | I-REL | 3835966 | chr19 9 | + | 50196537 | 50233287 | 1.06 |
| chemokine (C-C motif) ligand 2 | CCL2 | NM_002982 | GDCF-2 // GDCF- 2 HC11 // HC11 // HSMCR30 // MCAF // MCP-1 // MCP1 // MGC9434 // SCYA2 // SMC-CF | 7385547 | chr17 | + | 29513430 | 29608325 | 1.06 |
| hexokinase 2 | HK2 | NM_000189 | DKFZp686M1669 // HKII // HXK2 | 2489545 | chr2 | + | 74913290 | 74974310 | 1.06 |
| ELK3, ETS-domain protein (SRF accessory protein 2) | ELK3 | NM_005230 | ERP // NET // SAP2 | 3427098 | chr12 | + | 95112269 | 95187725 | 1.06 |
| SNF1-like kinase | SNF1LK | NM_173354 | MSK // SIK | 3934111 | chr21 | - | 43637578 | 43705100 | 1.06 |
| lipopolysaccharide-induced TNF factor | LITAF | NM_004862 | CMT1C // FLJ38636 // MGC116698 // MGC116700 // MGC116701 // MGC125274 // MGC125275 // MGC125276 // PIG7 // SIMPLE // TP5317 | 3680434 | chr16 | - | 11549088 | 11599620 | 1.06 |
| tRNA splicing endonuclease 54 homolog (S. cerevisiae) | TSEN54 | NM_207346 | FLJ37147 // SEN54L // sen54 | 3734865 | chr17 | + | 71022989 | 71032409 | 1.06 |
| chromodomain helicase DNA binding protein 6 | CHD6 | NM_032221 | CHD5 // K1AA1335 // RIGB | 3906160 | chr20 | - | 39464169 | 39680547 | 1.06 |
| chemokine (C-X-C motif) ligand 1 (melanoma growth stimulating activity, alpha) | CXCL1 | NM_001511 | GRO1 // GROa // MGSA // MGSA alpha // MGSA-a // NAP-3 // | 2731381 | chr4 | + | 74953973 | 74983954 | 1.06 |
| myotubularin related protein 3 | MTMR3 | NM_021090 // NM_153050 // NM_153051 | FYVE-DSP1 // KIAA0371 // ZFYVE10 | 3942179 | chr22 | + | 28609182 | 28762301 | 1.06 |
| adaptor-related protein complex 1, beta 1 subunit | AP1B1 | NM_001127 // NM_145730 | ADTB1 // AP105A // BAM22 // CLAPB2 | 3956781 | chr22 | - | 28053592 | 28206955 | 1.06 |
| actinin, alpha 1 | ACTN1 | NM_001102 | FLJ40884 | 3569814 | chr14 | - | 68409817 | 68515815 | 1.06 |
| mitogen-activated protein kinase kinase kinase kinase 4 | MAP4K4 | NM_004834 // NM_145686 // NM_145687 | FLH21957 // FLJ10410 // FLJ20373 // FLJ90111 // HGK // K1AA0687 // NIK | 2496727 | chr2 | + | 101600496 | 101877880 | 1.06 |
| activated leukocyte cell adhesion molecule | ALCAM | NM_001627 | CD166 // FLJ38514 // MEMD // MGC71733 | 2634494 | chr3 | + | 106304077 | 106816151 | 1.06 |
| valosin-containing protein // Fanconi anemia, complementation group G | VCP // FANCG | NM_007126 // NM_004629 | IBMPFD // MGC131997 // MGC148092 // MGC8560 // TERA // p97 // FAG // XRCC9 | 3204404 | chr9 | - | 35026106 | 35062648 | 1.06 |
| heat shock 105kDa/110kDa protein 1 | HSPH1 | NM_006644 | DKFZp686M05240 // HSP105 // HSP105A // HSP1058 // KIAA0201 // NY- CO-25 | 3508330 | chr13 | - | 30558258 | 30651692 | 1.06 |
| WAS protein family, member 2 | WASF2 | NM_006990 | SCAR2 // WAVE2 // dJ393P12.2 | 2403111 | chr1 | - | 27603331 | 27689256 | 1.06 |
| RAP1 interacting factor homolog (yeast) | RIF1 | NM_018151 | DKFZp781N1478 // FLJ12870 | 2510485 | chr2 | + | 151974674 | 152072768 | 1.06 |
| chromosome 20 open reading frame 77 | C20orf77 | NM_021215 | CREPT // DKFZp434P0735 // FLJ44520 // dJ1057B20.2 | 3884450 | chr20 | + | 36095232 | 36189576 | 1.06 |
| Wilms tumor 1 associated protein // acetyl- Coenzyme A acetyltransforase 2 (acetoacetyl Coenzyme A thiolase) | WTAP // ACAT2 | NM_004906 // NM_152857 // NM_152858 // NM_005891 | DKFZp686F20131 // KLAA0105 // MGC3925 // - | 2934089 | chr6 | + | 160066607 | 160097332 | 1.06 |
| proline-rich nuclear receptor coactivator 1 | PNRC1 | NM_006813 | B4-2 // PNAS-145 // PROL2 // PRR2 // RP11-63L7.5 | 2916716 | chr6 | + | 89847048 | 89852045 | 1.06 |
| jumonji domain containing 1C | JMJD1C | NM_004241 // NM_032776 | DKFZp761F0118 // FLJ14374 // KIAAL380 // RP11- 10C13.2 // TRIP8 | 3291682 | chr10 | - | 64589487 | 64951821 | 1.06 |
| ELL associated factor 1 | EAF1 | NM_033083 | - | 2612371 | chr3 | + | 15443374 | 15466824 | 1.06 |
| mitogen-activated protein kinase-activated protein kinase 2 | MAPKAPK2 | NM_004759 // NM_032960 | - | 2376922 | chr1 | + | 204924896 | 204974249 | 1.O6 |
| importin 7 | 1P07 | NM_006391 | FLJ14581 // MGC138673 // RANBP7 | 3319840 | chr11 | + | 9362702 | 9426296 | 1.06 |
| eukaryotic translation initiation factor 2, subunit 1 alpha, 35kDa | EIF2S1 | NM_004094 | EIF-2 // EIF-2A // EIF-2alpha // EIF2 // EIF2A | 3541137 | chr14 | + | 66896518 | 66922983 | 1.06 |
| CD82 molecule | CD82 | NM_001024844 // NM_002231 | 4F9 // C33 // GR15 // IA4 // KAI1 // R2 // SAR2 // ST6 // | 3328520 | chr11 | + | 44543723 | 44598484 | 1.06 |
| Wotf-Hirschhorn syndrome candidate 1-like 1 | WHSC1L1 | NM_017778 // NM_023034 | DKFZp667H044 // FLJ20353 // MGC126766 // MGC142029 // NSD3 // pp14328 | 3131916 | chr8 | - | 38251717 | 38359646 | 1.06 |
| ADP-ribosylation factor-like 8B | ARL8B | NM_018184 | ARL10C // FLJ10702 // Gie1 | 2608765 | chr3 | + | 5138874 | 5197592 | 1.06 |
| Rap guanine nucleotide exchange factor (GEF) 2 | RAPGEF2 | XM_376350 // XM_934714 // XM_934717 // XM_934718 // XM_944403 // XM_944410 // XM_944412 | CNrasGEF // PDZ- GEF1 // PDZGEF1 // RA-GEF // Rap GEP // Rap-GEP | 2749699 | chr4 | + | 160171745 | 160501468 | 1.06 |
| guanine nucleotide binding protein (G protein), gamma 2 | GNG2 | NM_053064 | - | 3535628 | chr14 | + | 51383702 | 51506715 | 1.06 |
| influenza virus NS1A binding protein | IVNS1ABP | NM_006469 // NM_016389 | DKFZp686K06216 // FLARA3 // FLJ10069 // FLJ10411 // FLJ10962 // FLJ35593 // FLJ36593 // HSPC068 // KIAA0850 // ND1 // NS-1 // NS1- BP // NS1BP | 2448073 | chr1 | - | 183532162 | 183553081 | 1.06 |
| ancient ubiquitous protein 1 // DEAQ box polypeptide 1 (RNA-dependent ATPase) | AUP1 // DQX1 | NM_181575 // NM_133637 | - // FLJ23757 | 2560254 | chr2 | - | 74607294 | 74610455 | 1.06 |
| TERF1 (TRF1)-interacting nuclear factor 2 | TINF2 | NM 012461 | TIN2 | 3558012 | chr14 | - | 23776465 | 23781950 | 1.06 |
| N-myc downstream regulated gene 1 | NDRG1 | NM_006096 | CAP43 // CMT4D // DRG1 // GC4 // HMSNL // NDR1 // NMSL // PROXY1 // RIT42 // RTP // TARG1 // TDD5 | 3154317 | chr8 | - | 134318628 | 134379306 | 1.06 |
| kinesin 2 | KNS2 | NM_005552 // NM_182923 | KLC // KLC1 // KNS2A // MGC15245 | 3553872 | chr14 | + | 103160147 | 103247456 | 1.06 |
| CD44 molecule (Indian blood group) // mitogen-activated protein kinase 10 | CD44 // MAPK10 | NM_000610 // NM_001001389 // NM_001001390 // NO_001001391 // NM_001001392 // NM_002753 // NM_138980 // NM_138981 // NM_138982 | CDW44 // CSPG8 // ECMR-lll // HCELL // IN // LHR // MC56 // MDU2 // MDU3 // MGC10468 // MIC4 // MUTCH-I // Pgp1 // FLJ12099 // FLJ33785 // JNK3 // JNK3A // MGC50974 // PRKM10 // p493F12 // | 3326635 | chr11 | + | 35117003 | 35210509 | 1.06 |
| mitogen-activated protein kinase kinase 1 | MAP2K1 | NM_002755 | MAPKK1 // MEK1 // MKK1 // PRKMK1 | 3598662 | chr15 | + | 64466253 | 64571690 | 1.06 |
| chromosome 16 open reading frame 72 | C16orf72 | NM_014117 | FLJ41272 // PRO0149 | 3647632 | chr16 | + | 9092682 | 9121055 | 1.06 |
| tumor necrosis factor receptor superfamily, member 1B | TNFRSF1B | NM_001066 | CD120b // TBPII // TNF-R-II // TNF- R75 // TNFBR // TNFR2 // TNFR80 // p75 // p75TNFR | 2320727 | chr1 | + | 12149647 | 12192323 | 1.06 |
| protein phosphatase 2, regulatory subunit B', gamma isoform | PPP2R5C | NM_002719 // NM_178586 // NM_178587 // NM_178588 | B56G // MGC23064 // PR61 G | 3552729 | chr14 | + | 101296771 | 101464066 | 1.06 |
| TSPY-like 2 // paraoxonase 3 | TSPYL2 // PON3 | XM_001128695 // NM_022117 // NM_000940 | CDA1 // CINAP // CTCL // DENTT // HRIHFB2216 // SE20-4 // - | 3978169 | chrX | + | 53124038 | 53134445 | 1.06 |
| serine/threonine kinase 17a | STK17A | NM_004760 | DRAK1 | 2999485 | chr7 | + | 43573961 | 43642649 | 1.06 |
| ELAV (embryonic lethal, abnormal vision, Drosophila)-like 1 (Hu antigen R) // translocase of inner mitochondrial membrane 44 homolog (yeast) | ELAVL1 // TIMM44 | NM_001419 // NM_006351 | ELAV1 // HUR // Hua // MelG // DKFZp686H05241 // TIM44 | 3848689 | chr19 | - | 7929463 | 7999655 | 1.06 |
| basic leucine zipper and W2 domains 1 | BZW1 | XM_001126385 // NM_014670 | BZAP45 // KIAA0005 // NbIa10236 | 2522439 | chr2 | + | 201384456 | 201418386 | 1.06 |
| fibronectin type III domain containing 3A // cancer/testis antigen CT45-3 | FNDC3A // RP13-36C9.3 | NM_001079673 // NM_014923 // NM_001017435 | FLJ31509 // FNDC3 // KIAA0970 // bA203116.1 // bA203I16.5 // CT45-3 // RP13- | 3489212 | chr13 | + | 48428770 | 48681909 | 1.06 |
| MAX dimerization protein 1 | MXD1 | NM_002357 | MAD // MAD1 // MGC104659 | 2487549 | chr2 | + | 69995330 | 70023575 | 1.06 |
| TIA1 cytotoxic granule-associated RNA binding protein-like 1 | TIAL1 | NM_001033925 // NM_003252 | MGC33401 // TCBP // TIAR | 3309629 | chr10 | - | 121291481 | 121346521 | 1.06 |
| nuclear receptor subfamily 4, group A, member 3 | NR4A3 | NM_006981 // NM_173198 // NM_173199 // NM_173200 | CHN // CSMF // MINOR // NOR1 // TEC | 3181976 | chr9 | + | 101623410 | 101669157 | 1.06 |
| SAM domain, SH3 domain and nuclear localization signals 1 | SAMSN1 | NM_022136 | HACS1 // NASH1 | 3925473 | chr21 | - | 14779427 | 14877594 | 1.06 |
| sperm specific antigen 2 | SSFA2 | NM-006751 | CS-1 // CS1 // DKFZp313O1039 // DKFZp779G0129 // FLJ45996 // KIAA1927 // KRAP // SPAG13 | 2518428 | chr2 | + | 182464815 | 182516732 | 1.06 |
| zinc finger protein 317 // zinc finger protein 658 | ZNF317 // ZNF658 | NM_020933 // XM_001125688 // NM-033160 | KIAA1588 // DKFZp572C163 // FLJ32813 // MGC35232 | 3819880 | chr19 | + | 9112073 | 9135082 | 1.06 |
| vacuolar protein sorting 35 homolog (S. cerevisiae) | VPS35 | NM_018206 | DKFZp434E1211 // DKFZp434P1672 // FLJ10752 // FLJ13588 // FLJ20388 // MEM3 | 3689922 | chr16 | - | 45220566 | 45280598 | 1.06 |
| CD8a molecule | CD8A | NM_001768 // NM_171827 | CD8 // Leu2 // MAL // p32 | 2562932 | chr2 | - | 86857923 | 86889020 | 1.06 |
| general transcription factor IIB | GTF2B // RP11-82K18.3 | NM_001514 | TF2B // TFIIB | 2421753 | chr1 | - | 89090909 | 89130195 | 1.06 |
| ring finger and WD repeat domain 2 | RFWD2 | NM_001001740 // NM_022457 | COP1 // FLJ10416 // RNF200 | 2445141 * | chr1 | - | 174150358 | 174443232 | 1.06 |
| formin binding protein 1 | FNBP1 | NM_015033 | FBP17 // KlAA0554 // MGC126804 | 3227159 | chr9 | - | 131689314 | 131845274 | 1.06 |
| transmembrane protein 23 | TMEM23 | NM_147156 | MGC17342 // MOB // MOB1 // SMS1 | 3289235 | chr10 | - | 51706090 | 52054733 | 1.06 |
| hypoxia-inducible factor 1, alpha subunit (basic helix-loop-helix transcription factor) | HIF1A | NM_001530 // NM_181054 | HIF-1alpha // HIF1-ALPHA // MOP1 // PASD8 | 3539070 | chr14 | + | 61225495 | 61285222 | 1.06 |
| annexin A5 | ANXA5 | NM_001154 | ANX5 // ENX2 // PP4 | 2784027 | chr4 | - | 122695163 | 122838353 | 1.06 |
| T-cell activation GTPase activating protein | TAGAP | NM_054114 // NM_138810 // NM_152133 | FKSG15 // FLJ32631 // FLJ39771 // MGC133247 // MGC27381 // TAGAP1 | 2982076 | chr6 | - | 159375488 | 159517834 | 1.06 |
| dolichyl-phosphate mannosyltransferase polypeptide 1, catalytic subunit | DPM1 | NM_003859 | CDGIE // MPDS | 3909395 | chr20 | - | 48984632 | 49008494 | 1.06 |
| TIA1 cytotoxic granule-associated RNA binding protein | TIA1 | NM_022037 // NM_022173 | - | 2558511 | chr2 | - | 70290081 | 70329315 | 1.06 |
| CD83 molecule | CD83 | NM_001040280 // NM 004233 | BL11 // HB15 | 2895841 | chr6 | + | 14118209 | 14485106 | 1.06 |
| serine/arginine repetitive matrix 2 | SRRM2 | NM_016333 | 300-KD // DKFZp686O15166 // FLJ21926 // FLJ22250 // KIAA0324 // MGC40295 // SRL300 // SRm300 | 3645253 | chr16 | + | 2742650 | 2761908 | 1.06 |
| mitogen-activated protein kinase kinase 3 | MAP2K3 | XM_001130488 // NM_145110 // NM_002756 // NM 145109 | MAPKK3 // MEK3 // MKK3 // PRKMK3 | 3714729 | chr17 | + | 21128581 | 21159113 | 1.06 |
| mitogen-activated protein kinase kinase 1 interacting protein 1 | MAP2K1IP1 | NM_021970 | MP1 | 2779408 | chr4 | - | 101018538 | 101034726 | 1.06 |
| hypothetical protein FLJ31951 | FLJ31951 | NM_144726 | DKFZp686M11215 | 2884216 | chr5 | - | 158516997 | 158569129 | 1.06 |
| capping protein (actin filament) muscle Z-line, alpha 1 | CAPZA1 | NM_006135 | CAPPA1 // CAPZ // CAZI | 2352228 | chr1 | + | 112945824 | 113015736 | 1.06 |
| chemokine (C-X-C motif) ligand 10 | CXCL10 | NM_001565 | C7 // IFI10 // INP10 // IP-10 // SCYB10 // crg-2 // gIP-10 // mob-1 | 2773958 | chr4 | - | 77151393 | 77163693 | 1.06 |
| v-raf murine sarcoma 3611 viral oncogene homolog | ARAF | NM_001654 | A-RAF // ARAF1 // PKS2 // RAFA1 | 3976299 | chrX | + | 47300826 | 47316249 | 1.06 |
| ADAM metallopeptidase domain 17 (tumor necrosis factor, alpha, converting enzyme) | ADAM17 | NM_003183 | CD156b // MGC71942 // TACE // cSVP | 2539821 | chr2 | - | 9546070 | 9630637 | 1.06 |
| protein tyrosine phosphatase type IVA, member 1 | PTP4A1 | NM_003463 | DKFZp779M0721 // HH72 // PRL-1 // PRL1 // PTP(CAAX1) // PTPCAAX1 | 2911903 | chr6 | + | 64289625 | 64351448 | 1.06 |
| poly (ADP-ribose) polymerase family, member 9 | PARP9 | NM_031458 | BAL // BAL1 // DKFZp666B0810 // DKFZp686M15238 // FLJ26637 // FLJ41418 // MGC:7868 | 2692060 | chr3 | - | 123729469 | 123766193 | 1.06 |
| WD repeat domain 74 | WDR74 | XM_001125771 // NM_018093 | FLJ10439 // FLJ21730 | 3376235 | chr11 | - | 62356627 | 62365857 | 1.06 |
| KLAA0256 gene product // trafficking protein particle complex 5 | KIAA0256 // TRAPPC5 | NM_014701 // NM_001042461 // NM_001042462// NM_174894 | - // MGC52424 | 3623320 | chr15 | - | 47055671 | 47126043 | 1.06 |
| CD47 molecule | CD47 | NM_001025079 // NM_001777 // NM_198793 | IAP // MER6 // OA3 | 2687739 | chr3 | - | 109143203 | 109306052 | 1.06 |
| inositol polyphosphate-5-phosphatase, 145kDa | INPP5D | XM_929960 // NM_001017915 // NM_005541 | MGC104855 // MGC142140 // MGC142142 // SHIP // SHIP1 // SIP-145 // hp51CN | 2532699 | chr2 | + | 233632835 | 233823600 | 1.06 |
| slingshot homolog 2 (Drosophila) | SSH2 | NM_033389 | KIAA1725 // MGC78588 // SSH- 2 | 3751625 | chr17 | - | 24977091 | 25281397 | 1.06 |
| oxysterol binding protein-like 8 | OSBPL8 | NM_001003712 // NM_020841 | DKFZp686A11164 // MGC126578 // MGC133203 // ORP8 // OSBP10 | 3462949 | chr12 | - | 75269250 | 75477720 | 1.06 |
| RAS p21 protein activator 3 | RASA3 | NM_007368 | GAP1IP4BP // GAPIII // MGC46517 // | 3526831 | chr13 | - | 113765177 | 113916197 | 1.06 |
| stromal antigen 2 | STAG2 | NM_001042749 // NM_001042750 // NM_001042751 // NM_006603 | DKFZp686P168 // DKFZp781H1753 // FLJ25871 // SA-2 // SA2 // bA517O1.1 | 3989721 | chrX | + | 122922013 | 123384079 | 1.06 |
| NIMA (never in mitosis gene a)-related kinase 7 | NEK7 | NM 133494 | - | 2373736 | chr1 | + | 196225008 | 196558827 | 1.06 |
| protein tyrosine phosphatase, receptor type, E | PTPRE | NM_006504 // NM_130435 | DKFZp313F1310 // HPTPE // PTPE // R-PTP-EPSILON | 3270270 | chr10 | + | 129595315 | 129774152 | 1.06 |
| solute carrier organic anion transporter family, member 4A1 | SLCO4A1 | NM_016354 | OATP-E // OATP1 // OATP4A1 // OATPRP1 // POAT // SLC21A12 | 3892812 | chr20 | + | 60744242 | 60787582 | 1.06 |
| v-src sarcoma (Schmidt-Ruppin A-2) viral oncogene homolog (avian) | SRC | NM_005417 // NM_198291 | ASV // SRC1 // c- SRC // p60-Src | 3884191 | chr20 | + | 35406502 | 35467847 | 1.06 |
| phosphoinositide-3-kinase, regulatory subunit 5, p101 | PIK3R5 | NM_014308 | F730038115Rik // FOAP-2 // P101- PI3K | 3744680 | chr17 | - | 8718740 | 8809747 | 1.06 |
| HECT, UBA and WWE domain containing 1 // paraoxonase 1 // paraoxonase 3 | HUWE1 // PON1 // PON3 | NM_031407 // NM_000446 // NM_000940 | ARF-BP1 // HECTH9 // HSPC272 // 1b772 // KIAA0312 // LASU1 // MULE // UREB1 // ESA // PON // - | 4009315 | chrX | - | 53575796 | 53740871 | 1.06 |
| antizyme inhibitor 1 | AZIN 1 | NM_015878 // NM_148174 | MGC3832 // MGC691 // OAZI // OAZIN // | 3147591 | chr8 | - | 103907725 | 103953967 | 1.06 |
| A kinase (PRKA) anchor protein 13 | AKAP13 | NM_006738 // NM_007200 // Nm_144767 | AKAP-Lbc // BRX // FLJ11952 // FLJ43341 // HA-3 // Ht31 // LBC // PROTO-LB // PROTO-LBC // c- Ibc | 3606304 | chr15 | + | 83681343 | 84102785 | 1.06 |
| eukaryotic translation initiation factor 4 gamma, 2 | EIF4G2 | NM_001042559 // NM_001418 | AAG1 // DAP5 // FLJ41344 // NAT1 // p97 | 3362719 | chr11 | - | 10487574 | 10788746 | 1.06 |
| suppressor of Ty 6 homolog (S. cerevisiae) | SUPT6H | NM_003170 | KIAA0162 // MGC87943 // SPT6 // SPT6H // emb-5 | 3715703 | chr17 | + | 24013260 | 24053809 | 1.06 |
| solute carrier family 36 (proton/amino acid symporter), member 4 | SLC36A4 | NM_152313 | FLJ38932 // PAT4 | 3386638 | chr11 | - | 92485640 | 92570746 | 1.06 |
| DOT1-like, histone H3 methyltransferase (S. cerevisiae) | DOT1 L | NM_032482 | DOT1 // KIAA1814 | 3816264 | chr19 | + | 2114945 | 2183576 | 1.06 |
| epithelial stromal interaction 1 (breast) // junctophilin 3 | EPSTI1 // JPH3 | NM_001 002264 // NM_033255 // NM_020655 | BRESI1 // MGC29634 // CAGL237 // FLJ44707 // HDL2 // JP-3 // JP3 // TNRC22 | 3511698 | chr13 | - | 42309603 | 42464520 | 1.06 |
| promyelocytic leukemia | PML | XM_001132060 // NM_002675 // NM_033238 // NM_033239 // NM_033240 // NM_033244 // NM_033246 // NM_033247 // NM_033249 // NM_033250 | MYL // PP8675 // RNF71 // TRIM19 | 3601387 | chr15 | + | 72073603 | 72126162 | 1.06 |
| DnaJ (Hsp40) homolog, subfamily A, member 1 | DNAJA1 | NM_001539 | DJ-2 // DjA1 // HDJ2 // HSDJ // HSJ2 // HSPF4 // hDJ-2 | 3166718 | chr9 | + | 33015173 | 33029946 | 1.06 |
| TSC22 domain family, member 3 | TSC22D3 | NM 001015881 // NM_004089 // NM_198057 | DIP // DKFZp313A1123 // DSIPI // GILZ // TSC-22R // hDIP | 4017381 | chrX | - | 106803586 | 106916423 | 1.06 |
| RNA binding motif protein 4 | RBM4 | NM_002896 | DKFZp547K0918 // LARK // MGC75138 // RBM4A // ZCCHC21 // ZCRB3A | 3336422 | chr11 | + | 66161951 | 66190547 | 1.06 |
| Smug-7 homolog, nonsense mediated mRNA decay factor (C. elegans) | SMG7 | NM_014837 // NM_173156 // NM_201568 // NM_201569 | C1orf16 // EST1C // FLJ23717 // KLAA0250 // SGA56M // SMG-7 | 2371255 | chr1 | + | 181708036 | 181834004 | 1.06 |
| baculoviral IAP repeat-containing 2 | BIRC2 | NM_001166 | AP11 // HIAP2 // Hiap-2 // MIHB // RNF48 // cIAP1 | 3346584 | chr1 | + | 101722704 | 101754720 | 1.06 |
| DEAH (Asp-Glu-Ala-His) box polypeptide 15 | DHX15 | NM_001358 | DBP1 // DDX15 // HRH2 // PRP43 // PRPF43 // PrPp43p | 2763805 | chr4 | - | 24128159 | 24250940 | 1.06 |
| echinoderm microtubule associated protein like 4 | EML4 | NM_019063 | C2orf2 // DKFZp686P18118 // ELP120 // FLJ10942 // FLJ32318 // ROPP120 | 2478748 | chr2 | + | 42250017 | 42418636 | 1.06 |
| protein phosphatase 6, catalytic subunit | PPP6C | NM_002721 | - | 3225348 | chr9 | - | 126948675 | 126991992 | 1.06 |
| bromodomain and WD repeat domain containing 1 | BRWD1 | NM_001007246 // NM_018963 // NM_033656 | C21orf107 // FLJ43918 // N143 // WDR9 | 3932148 | chr21 | - | 39479279 | 39615332 | 1.06 |
| amyloid beta (A4) precursor-like protein 2 | APLP2 | NM_001642 | APPH // APPL2 // CDEBP | 3356115 | chr11 | + | 129444931 | 129519895 | 1.06 |
| sperm associated antigen 9 | SPAG9 | NM_003971 // NM_172345 | FLJ13450 // FLJ14006 // FLJ34602 // HLC4 // HSS // JLP // KIAA0516 // MGC117291 // MGC14967 // MGC74461 // PHET // PIG6 | 3762519 | chr17 | - | 46394554 | 46553215 | 1.06 |
| Rap guanine nucleotide exchange factor (GEF) 6 // folliculin interacting protein 1 | RAPGEF6 // FNIP1 | NM_016340 // NM_001008738 // NM_133372 | DKFZp667N084 // DKFZp686115116 // K1A001 LB // PDZ- GEF2 // PDZGEF2 // RA-GEF-2 // DKFZp686E18167 // DKFZp781 P0215 // KIAA1961 // MGC667 | 2874794 | chr5 | - | 130760272 | 131160628 | 1.06 |
| ATPase, Na+/K+ transporting, beta 3 polypeptide | ATP1B3 | XM_001133533 // XM_001133534 // NM_001679 | ATPB-3 // CD298 // FLJ29027 | 2645764 | chr3 | + | 143077611 | 143128338 | 1.06 |
| leukocyte-associated immunoglobulin-like receptor 1 | LAIR1 | NM_002287 // NM 021706 | CD305 // LAIR-1 | 3870824 | chr19 | - | 59557066 | 59578582 | 1.06 |
| Interleukin enhancer binding factor 2. 45kDa | ILF2 | NM_004515 | MGC8391 // NF45 // PRO3063 | 2436132 | chr1 | - | 151900372 | 151910103 | 1.06 |
| solute carrier family 38, member 1 | SLC38A1 | NM_001077484 // NM_030674 | ATA1 // NAT2 // SAT1 // SNAT1 | 3452231 | chr12 | - | 44863120 | 44952390 | 1.06 |
| tudor domain containing 7 | TDRD7 | NM_014290 | KIAA1529 // PCTAIRE2BP // RP11-508D10.1 // TRAP | 3181193 | chr9 | + | 99202471 | 99298225 | 1.06 |
| thioredoxin domain containing 14 // catenin (cadherin-associated protein), delta 1 | TXNDC14 // CTNND1 | NM_015959 // XM_001 126721 // XM_001126756 // XM_001126767 // XM_001126781 // XM_001128793 // XM_001126823 // NM_001331 | CGI-31 // DKFZp781O2021 // MGC111151 // PIG26 // TMX2 // CAS // CTNND // KIAA0384 // P120CAS // P120CTN // p120 | 3331487 | chr11 | + | 57235839 | 57391291 | 1.06 |
| zinc finger protein 207 | ZNF207 | NM_001032293 // NM_003457 | DKFZp761N202 | 3717635 | chr17 | + | 27701159 | 27759429 | 1.06 |
| zinc finger, AN1-type domain 3 | ZFAND3 | NM_021943 | FLJ13222 // TEX27 | 2905664 | chr6 | + | 37895285 | 38231400 | 1.06 |
| catenin (cadherin-associated protein), beta 1, 88kDa | CTNNB1 | XM_001133660 // XM_001133664 // XM_001133673 // XM_001133675 // NM_001904 | CTNNB // FLJ25606 | 2618940 | chr3 | + | 41211356 | 41256934 | 1.06 |
| NCK-associated protein 1-like | NCKAP1L | NM_005337 | HEM1 | 3416577 | chr12 | + | 53177769 | 53224156 | 1.06 |
| upstream binding protein 1 (LBP-1a) | UBP1 | NM_014517 | DKFZp686L1745 // LBP-1B // LBP-1a // LBP1A // | 2668351 | chr3 | - | 33404835 | 33456874 | 1.06 |
| cytoskeleton-associated protein 4 | CKAP4 | NM_006825 | CLIMP-63 // ERGIC-63 // MGC99554 // p63 | 3469687 | chr12 | - | 105155796 | 105222139 | 1.06 |
| ubiquitin B // chromosome 17 open reading frame 45 | UBB // C17orf45 | NM_018955 // NM_152350 | FLJ25987 // MGC8385 // FLJ25777 // MGC40157 | 3712041 | chr17 | + | 16223047 | 16228977 | 1.06 |
| ATPase, Class V, type 10D | ATP10D | NM_020453 | ATPVD // | 2726072 | chr4 | + | 47179942 | 47290260 | 1.06 |
| cytoplasmic polyadenylation element binding protein 4 | CPEB4 | NM_030627 | KIAA1673 | 2841699 | chr5 | + | 173177911 | 173320580 | 1.06 |
| zinc finger protein 451 | ZNF451 // KIAA1702 | NM_001031623 // NM_015555 | COASTER // FLJ90693 // KIAA0576 // MGC26701 // dJ417I1.1 | 2911303 | chr6 | + | 57062631 | 57143064 | 1.06 |
| superoxide dismutase 2, mitochondrial | SOD2 // MGC5618 | NM_000636 // NM_001024465 // NM_001024466 | IPO-B // MNSOD // Mn-SOD | 2982319 | chr6 | - | 160020086 | 160103541 | 1.06 |
| optic atrophy 1 (autosomal dominant) | OPA1 | NM_015560 // NM_130831 // NM_30832 // NM_130833 // NM_130834 // NM_130835 // NM_130836 // NM_130837 | FLJ12460 // KIAA0567 // NPG // NTG // largeG | 2658346 | chr3 | + | 194793673 | 194902403 | 1.06 |
| Rho-associated, coiled-coil containing protein kinase 1 | ROCK1 | NM_005406 | MGC131603 // MGC43611 // P160ROCK | 3800619 | chr18 | - | 16783710 | 17017478 | 1.06 |
| GNAS complex locus | GNAS | NM_000516 // NM_001077488 // NM_001077489 // NM_001077490 // NM_016592 // NM_080425 // NM_080426 // NR_003259 | AHO // C20orf45 // GNAS1 // GPSA // GSA // GSP // MGC33735 // PHP1A // PHP1B // POH // dJ309F20.1.1 // dJ806M20.3.3 | 3891163 | chr20 | + | 56848165 | 56919604 | 1.06 |
| myxovirus (influenza virus) resistance 1, | MX1 | NM_002462 | IFI-78K // IFI78 // MX // MxA | 3922100 | chr21 | + | 41714183 | 41752955 | 1.06 |
| interferon-inducible protein p78 (mouse) SP110 nuclear body protein | SP110 | NM_004509 // NM_004510 // NM_080424 | FLJ22835 // IF141 // IF175 // VODI | 2603051 | chr2 | - | 230740259 | 230797812 | 1.06 |
| AP1 gamma subunit binding protein 1 | AP1GBP1 | NM_007247 // NM_080550 | MGC104959 // SYNG | 3754677 | chr17 | - | 32949033 | 33043613 | 1.06 |
| TAR DNA binding protein | TARDBP | NM_007375 | TDP-43 | 2320048 | chr1 | + | 10995025 | 11013170 | 1.06 |
| methionine adenosyltransferase II, alpha | MAT2A | NM_005911 | MATA2 // MATII // SAMS2 | 2491615 | chr2 | + | 85597809 | 85625908 | 1.06 |
| RNA binding motif protein 17 | RBM17 | NM_032905 | MGC14439 // SPF45 | 3233547 | chr10 | + | 6170982 | 6225410 | 1.06 |
| asparaginyl-tRNA synthetase // sideroflexin 3 | NARS // SFXN3 | NM_004539 // NM_030971 | ASNRS // BA108L7.2 // SFX3 | 3809671 | chr18 | - | 53418770 | 53448841 | 1.06 |
| PAN3 polyA specific ribonuclease subunit homolog (S. cerevisiae) | PAN3 | NM_175854 | - | 3483159 | chr13 | + | 27572646 | 27773671 | 1.06 |
| SMAD specific E3 ubiquitin protein ligase 2 | SMURF2 | NM_022739 | DKFZp686F0270 // MGC138150 | 3766960 | chr17 | - | 59968891 | 60088896 | 1.06 |
| ankyrin repeat domain 15 | ANKRD15 | NM_015158 // NM_153186 | DKFZp451 G231 // KANK // KIAA0172 // MGC43128 | 3159483 | chr9 | + | 460301 | 746375 | 1.06 |
| structural maintenance of chromosomes flexible hinge domain containing 1 | SMCHD1 | XM_113962 // XM_938891 | DKFZp68600631 // KIAA0650 | 3776193 | chr18 | + | 2688138 | 2794997 | 1.06 |
| transcription factor 12 (HTF4, helix-loop-helix transcription factors 4) | TCF12 | NM_003205 // NM_207036 // NM_207037 // NM_207038 // NM_207040 | HEB // HTF4 // HsT17266 | 3595096 | chr15 | + | 54963208 | 55402126 | 1.06 |
| ATPase, Ca++ transporting, cardiac muscle, slow twitch 2 | ATP2A2 | NM_001681 // NM_170665 | ATP2B // DAR // DD // MGC45367 // SERCA2 | 3431483 | chr12 | + | 109203332 | 109352495 | 1.06 |
| ubiquitin associated protein 1 | UBAP1 | NM_016525 | MGC119669 // MGC8710 // NAG20 // UAP // | 3167325 | chr9 | + | 34168995 | 34243205 | 1.06 |
| heparan sulfate (glucosamine) 3-O-sulfotransferase 3B1 | HS3ST3B1 | NM_006041 | 30ST3B1 // 3OST3B1 | 3711262 | chr17 | + | 14145125 | 14193444 | 1.06 |
| arrestin domain containing 2 | ARRDC2 | NM_001025604 // NM_015683 | CLONE24945 // PP2703 | 3824713 | chr19 | + | 17972961 | 17985905 | 1.06 |
| casein kinase 1, alpha 1 | CSNK1A1 | NM_001025105 // NM_001892 | CK1 // HLCDGP1 // PRO2975 | 2880932 | chr5 | - | 148800168 | 148922811 | 1.06 |
| CCR4-NOT transcription complex, subunit 2 | CNOT2 | NM_014515 | CDC36 // HSPC131 // NOT2 | 3421897 | chr12 | + | 68901823 | 69035035 | 1.06 |
| mitogen-activated protein kinase kinase kinase 8 | MAP3K8 | NM_005204 | COT // EST // ESTF // FLJ10486 // TPL2 // TpI-2 // c-COT | 3240987 | chr10 | + | 30762872 | 30791283 | 1.06 |
| mitogen-activated protein kinase kinase kinase 2 | MAP3K2 | XM_001128799 // NM_006609 | MEKK2 // MEKK2B | 2574798 | chr2 | - | 127772731 | 127863022 | 1.06 |
| protein phosphatase 4, regulatory subunit 2 | PPP4R2 | NM_174907 | MGC131930 | 2629693 | chr3 | + | 73019946 | 73201035 | 1.06 |
| topoisomerase (DNA) I | TOP1 | NM_003286 | TOPI | 3885464 | chr20 | + | 39090891 | 39186535 | 1.06 |
| ubiquitin specific peptidase 7 (herpes virus-associated) | USP7 | NM_003470 | HAUSP // TEF1 | 3679564 | chr16 | - | 8893458 | 8964832 | 1.06 |
| RAB21, member RAS oncogene family | RAB21 | NM_014999 | KIAA0118 | 3422269 | chr12 | + | 70434927 | 70470787 | 1.06 |
| MAX interactor 1 | MX1 | NM_001008541 // NM_005962 // NM_130439 | MAD2 // MGC43220 // MXD2 // MXI | 3263624 | chr10 | + | 111944059 | 112037108 | 1.06 |
| NGFI-A binding protein 1 (EGR1 binding protein | NAB1 | NM_005966 | - | 2520225 | chr2 | + | 191205069 | 191437200 | 1.06 |
| cytochrome P450, family 1, subfamily B, polypeptide 1 | CYP1B1 | NM_000104 | CP1 B // GLC3A | 2548699 | chr2 | - | 38138730 | 38196009 | 1.06 |
| chromosome 1 open reading frame 63 | C1orf63 | NM_020317 | DJ465N24.2.1 // NPD014 // RP3- 465N24.4 | 2402111 | chr1 | - | 25440667 | 25538017 | 1.06 |
| nucleoporin 153kDa | NUP153 | NM_005124 | HNUP153 // N153 | 2943808 | chr6 | - | 17723255 | 17855638 | 1.06 |
| protein phosphatase 1K (PP2C domain containing) | PPM1K | NM_152542 | DKFZp667B084 // DKFZp761G058 // PTMP // UG0882E07 | 2777333 | chr4 | - | 89401891 | 89424935 | 1.06 |
| phosphoinositide-3-kinase, class 3 | PIK3C3 | NM_002647 | MGC61518 // | 3786039 | chr18 | + | 37534028 | 37985705 | 1.05 |
| CCHC-type zinc finger, nucleic acid binding protein | CNBP | NM_003418 | CNBP1 // DM2 // PROMM // RNF163 // ZCCHC22 // ZNF9 | 2694644 | chr3 | - | 130369369 | 130385467 | 1.05 |
| ubiquitin specific peptidase 9, X-linked // ubiquitin specific peptidase 9, Y-linked (fat facets-like, Drosophila) | USP9X // USP9Y | NM_001039590 // NM_001039591 // NM_004654 | DFFRX // FAF // AZF // AZFA // DFFRY // SP3 | 3974708 | chrX | + | 40829542 | 40980776 | 1.05 |
| baculoviral IAP repeat-containing 6 (apollon) | BIRC6 | NM_016252 | APOLLON // BRUCE // FLJ13726 // FLJ13786 // | 2476219 | chr2 | + | 32435095 | 32697447 | 1.05 |
| chromosome 11 open reading frame 30 | C11orf30 | NM_020193 | EMSY // FLJ90741 // GL002 | 3340913 | chr11 | + | 75803897 | 75941697 | 1.05 |
| pleckstrin homology domain containing, family M (with RUN domain) member 1 | PLEKHM1 | XM_001128220 // NM 014798 | AP162 // B2 // KIAA0356 | 3759849 | chr17 | - | 40869050 | 40923923 | 1.05 |
| autism susceptibility candidate 2 | AUTS2 | NM_015570 | KIAA0442 // MGC13140 | 3006572 | chr7 | + | 68695979 | 69896399 | 1.05 |
| YME1-like 1 (S. cerevisiae) | YME1 L1 | NM_014263 // NM_139312 // NM_139313 | FTSH // MEG4 // PAMP // YME1 L | 3282213 | chr10 | - | 27439066 | 27484191 | 1.05 |
| toll-like receptor 4 | TLR4 | NM_138554 | CD284 // TOLL // hToll | 3186966 | chr9 | + | 119506334 | 119670150 | 1.05 |
| splicing factor 3b, subunit 1, 155kDa | SF3B1 | NM_001005526 // NM_012433 | PRP10 // PRPF10 // SAP155 // SF3b155 | 2593670 | chr2 | - | 197962756 | 198039327 | 1.05 |
| E3 ubiquitin protein ligase, HECT domain containing, 1 | EDD1 | NM_015902 | DD5 // EDD // FLJ11310 // HYD // KIAA0896 // MGC57263 | 3147321 | chr8 | - | 103333634 | 103493671 | 1.05 |
| ATP-binding cassette, sub-family C (CFTR/MRP), member 1 | ABCC1 | NM_004996 // NM_019862 // NM_019898 // NM_019899 // NM_019900 // NM_019901 // NM_019902 | ABC29 // ABCC // DKFZp686N04233 // DKFZp781G125 // GS-X // MRP // MRP1 | 3649890 | chr16 | + | 15950935 | 16144419 | 1.05 |
| pleckstrin | PLEK | NM_002664 | FLJ27168 // P47 | 2486811 | chr2 | + | 68415853 | 68526386 | 1.05 |
| farnesyltransferase, CAAX box, alpha | FNTA | NM_001018676 // NM_001018677 // NM_002027 | FPTA // MGC99680 // PGGT1A | 3096428 | chr8 | + | 43030413 | 43060080 | 1.05 |
| centrosomal protein 170kDa // centrosomal protein 170kDa-like | CEP170 // CEP170L | XM_001125768 // NM_001042404 // NM_001042405 // NM_014812 // XR_015931 // XR_017916 // NR_003135 | FAM68A // KAB // KIAA0470 // FAM68B // KIAA0470L // MGC26143 | 2463864 | chr1 | - | 241354364 | 241485236 | 1.05 |
| forkhead box K2 | FOXK2 | XM_001134363 // XM_001134364 // NM_004514 | ILF // ILF-1 // ILF1 | 3738969 | chr17 | + | 78070883 | 78195807 | 1.05 |
| B-cell CLL/iyvnphoma 9-like | BCL9L | NM_182557 | BCL9-2 // DLNB11 | 3393993 | chr11 | - | 118272076 | 118304947 | 1.05 |
| cell division cycle 2-like 5 (cholinesterase-related cell division controller) | CDC2L5 | NM_003718 // NM_031267 | CDC2L // CHED // FLJ35215 // KIAA1791 | 2998536 | chr7 | + | 39897668 | 40103253 | 1.05 |
| proteasome (prosome, macropain) 26S subunit, non-ATPase, 11 | PSMD11 | NM_002815 | MGC3844 // S9 // p44.5 | 3717737 | chr17 | + | 27795087 | 27834449 | 1.05 |
| calreticulin | CALR | NM_004343 | FLJ26680 // RO // SSA // cC1qR | 3822049 | chr19 | + | 12910423 | 12916480 | 1.05 |
| schlafen family member 11 | SLFN11 | NM_152270 | FLJ34922 // SLFN8/9 | 3753500 | chr17 | - | 30696595 | 30724798 | 1.05 |
| nuclear receptor subfamily 3, group C, member 1 (glucocorticoid receptor) | NR3C1 | NM_000176 // NM_001018074 // NM_001018075 // NM_001018076 // NM_001018077 // NM_001020825 // NM_001024094 | GCCR//GCR// GR // GRL | 2879312 | chr5 | - | 142566614 | 142794517 | 1.05 |
| dual specificity phosphatase 16 | DUSP16 | NM_030640 | KIAA1700 // MGC129701 // MGC129702 // MKP-7 // MKP7 | 3444958 | chr12 | - | 12510545 | 12606611 | 1.05 |
| glycoprotein V (platelet) // zinc finger protein 394 | GP5 // ZNF394 | NM_004488 // NM 032164 | CD42d // FLJ12298 // ZKSCAN14 | 3063337 | chr7 | - | 98922007 | 98935856 | 1.05 |
| nuclear protein, ataxia-telangiectasia locus | NPAT | NM 002519 | E14 | 3390067 | chr11 | - | 107533176 | 107599902 | 1.05 |
| splicing factor, arginine/serine-rich 3 | SFRS3 | NM_003017 | SRp20 | 2905118 | chr6 | + | 36669658 | 36741293 | 1.05 |
| guanine nucleotide binding protein (G protein) alpha 12 | GNA12 | NM_007353 | MGC 104623 // MGC99644 // NNX3 // RMP // gep | 3035892 | chr7 | - | 2724003 | 2910140 | 1.05 |
| chromosome 13 open reading frame 18 | C13orf18 | NM_025113 | FLJ21562 // FLJ43762 | 3512948 | chr13 | - | 45814156 | 45917111 | 1.05 |
| nuclear factor of kappa light polypeptide gene enhancer in B-cells 2 (p49/p100) | NFKB2 | NM_001077493 // NM_001077494 // NM_002502 | LYT-10//LYT10 | 3261643 | chr10 | + | 104144262 | 104152716 | 1.05 |
| chromosome 22 open reading frame 28 | C22orf28 | NM_014306 | DJ149A16.6 // HSPC117// RP1- 149A16.6 | 3958253 | chr22 | - | 31113563 | 31138223 | 1.05 |
| ubiquitin specific peptidase 4 (proto-oncogene) | USP4 | NM_003363 // NM_199443 | MGC149848// MGC149849 // UNP // Unph | 2674179 | chr3 | - | 49290730 | 49353046 | 1.05 |
| F-box protein 11 | FBXO11 | NM_012167 // NM_018693 // NM_025133 | FBX11 // FLJ12673 // MGC44383 // PRMT9 // UG063H01 // VIT1 | 2552153 | chr2 | - | 47869608 | 47988296 | 1.05 |
| PRP4 pre-mRNA processing factor 4 homolog B (yeast) | PRPF4B | NM_003913 | K1AA0536 // PR4H // PRP4 // PRP4H // PRP4K // dJ1013A10.1 | 2892738 | chr6 | + | 3966500 | 4010215 | 1.05 |
| ATPase, aminophospholipid transporter (APLT), Class I, type 8A, member 1 // protocadherin LKC | ATP8A1 // PCLKC | NM_006095 // NM_017675 | ATPASEII // ATPIA // ATPP2 // MGC130042 // MGC130043 // MGC26327 // FLJ20124 // FLJ20383 // MGC163154 | 2767378 | chr4 | - | 42105160 | 42353857 | 1.05 |
| zinc finger protein 267 | ZNF267 | NM_003414 | HZF2 | 3657367 | chr16 | + | 31730255 | 31836159 | 1.05 |
| BAT2 domain containing 1 | BAT2D1 | NM 015172 | BAT2-iso // XTP2 | 2367154 | chr1 | + | 169716078 | 169829262 | 1.05 |
| DEAD (Asp-Glu-Ala-Asp) box polypeptide 18 // wingless-type MMTV integration site family, member 88 | DDX18 // WNT8B | NM_006773 // NM_003393 | FLJ33908 // MrDb // - | 2502300 | chr2 | + | 118243834 | 118323562 | 1.05 |
| Ran GTPase activating protein 1 | RANGAP1 | NM_002883 | Fug1 // KIAA1835 // MGC20266 // | 3961842 | chr22 | - | 39969442 | 40028027 | 1.05 |
| protein kinase, cAMP-dependent, regulatory, type I, alpha (tissue specific extinguisher 1) | PRKAR1A | NM_002734 // NM_212471 // NM_212472 | CAR // CNC // CNC1 // DKFZp779L0468 // MGC17251 // PKR1 // PRKAR1 // | 3732885 | chr17 | + | 64019700 | 64059052 | 1.05 |
| ubiquitin specific peptidase 38 | USP38 | NM_032557 | FLJ35970 // HP43.BKD // KIAA1891 | 2745499 | chr4 | + | 144325529 | 144364549 | 1.05 |
| damage-regulated autophagy modulator | DRAM | NM 018370 | FLJ11259 | 3428783 | chr12 | + | 100795270 | 100930011 | 1.05 |
| SET domain containing 5 | SETD5 | XM_926615 // XM_931376 // XM_931417 // XM_931444 // XM_931447 // XM_931455 // XM_931478 // XM_939712 // XM_944260 // XM_944276 // XM_944280 // XM_944295 // XM_944298 // XM_944303 // NM 001080517 | DKFZp686J18276 // FLJ10707 // KIAA1757 | 2609608 | chr3 | + | 9414309 | 9495916 | 1.05 |
| myeloid differentiation primary response gene | MYD88 | NM_002468 | - | 2617563 | chr3 | + | 38155029 | 38159513 | 1.05 |
| transmembrane protein 1 | TMEM1 | NM_001001723 // NM_003274 | EHOC-1 // EHOC1 // GT334 // MGC126777 | 3923436 | chr21 | + | 44256650 | 44350842 | 1.05 |
| RNA binding motif protein 23 // chromosome 9 open reading frame 102 | RBM23 // C9orf102 | NM_001077351 // NM_001077352 // NM_018107 // NM_001034155 // NM_020207 | CAPERbeta // FLJ10482 // MGC4468 // PP239 // RNPC4 // FLJ37706 // SR278 | 3556888 | chr14 | - | 22439714 | 22458231 | 1.05 |
| myeloid/lymphoid or mixed-lineage leukemia (trithorax homology, Drosophila); translocated to, | MLLT3 | NM_004529 | AF9 // FLJ2035 // YEATS3 | 3200982 | chr9 | - | 20334968 | 20612522 | 1.05 |
| eukaryotic translation initiation factor 3, subunit 10 theta, 150/170kDa | E1F3S10 | NM_003750 | EIF3 // EIF3A // KIAA0139 // P167 // elF3-p170 // elF3-theta // p180 // p185 | 3309215 | chr10 | - | 120621551 | 120830522 | 1.05 |
| chromosome 3 open reading frame 63 | C3orf63 | NM_015224 | DKFZp686C2456 // KIAA1105 // RAP140 // se89-1 | 2677653 | chr3 | - | 56629203 | 56692517 | 1.05 |
| adenosine monophosphate deaminase (isoform E) // peroxisome proliferator-activated receptor alpha // tRNA 5-methylaminomethyl-2-thiouridylate methyltransferase // G-2 and S-phase expressed 1 | AMPD3 // PPARA // TRMU // GTSE1 | NM_001025390 NM_005036 // NM_018006 // NM016426 NM_000480 // NM_001025389 // NM_001025390 // NM_001001929 // NM_001001930 // NM_032644 // NM_001001928 // NM_005038 // NM_018006 // NM_016426 | - // MGC2237 // MGC2452 // NRIC1 // PPAR // hPPAR // MGC99627 // MTO2 // MTU1 // TRMT // TRMT1 // TRNT1 // B99 | 3320169 | chr11 | + | 10428461 | 10485703 | 1.05 |
| retinoic acid receptor, alpha | RARA | NM_001033603 // NM_001024809 NM_001033603 // NM_000O964 // NM_001024809 | NR1B1 // RAR | 3720921 | chr17 | + | 35718982 | 35767411 | 1.05 |
| p21 (CDKNIA)-activated kinase 2 | PAK2 | XM_001126110 // NM_002577 | PAK65 // PAKgamma | 2659577 | chr3 | + | 197951145 | 198043895 | 1.05 |
| splicing factor 3b, subunit 2, 145kDa | SF3B2 | NM_006842 | SAP145 // SF3B145 // SF3b1 // SF3b150 | 3335907 | chr11 | + | 65573499 | 65593343 | 1.05 |
| eukaryotic translation initiation factor 4A, isoform 1 // SUMO1/sentrin/SMT3 specific peptidase 3 | E1F4A1 // SENP3 | NM_015670 NM_001416 // NM_015670 | DDX2A // EIF-4A // EIF4A // DKFZP586K0919 // DKFZp762A152 // SMT3IP1 // SSP3 | 3708826 | chr17 | + | 7416768 | 7423040 | 1.05 |
| syntaxin 6 | STX6 | NM_005819 NM_005819 | - | 2446567 | chr1 | - | 179095658 | 179258656 | 1.05 |
| carbohydrate (chondroitin 4) sulfotransferase 1t | CHST11 | NM_018413 | C4ST // C4ST-1 // C4ST1 // HSA269537 | 3429566 | chr12 | + | 103374529 | 103679918 | 1.05 |
| protein phosphatase 1, catalytic subunit, beta isoform | PPP1CB | NM_206877 // NM_208876 NM_206877 // NM_002709 // NM_208876 | MGC3672 // PP-1B // PPP1CD | 2475209 | chr2 | + | 28828128 | 28879300 | 1.05 |
| mitogen-activated protein kinase 6 | MAPK6 | NM_002748 | DKFZp686F03189 // ERK3 // HsT17250 // PRKM6 // | 3594129 | | + | 50098740 | 50146174 | 1.05 |
| GTP binding protein 4 | GTPBP4 | NM_012341 NM_012341 | CRFG // FLJ10686 // FLJ10690 // FLJ39774 // NGB | 3231774 | chr10 | + | 942450 | 1055862 | 1.05 |
| forkhead box O3A | FOXO3A | NM_001455 // NM_201559 | AF6q21 // DKFZp781A0677 // FKHRL1 // FKHRL1P2 // MGC12739 // MGC31925 | 2920475 | chr6 | + | 108987684 | 109112650 | 1.05 |
| transmembrane protein 2 | TMEM2 | NM 013390 | - | 3209384 | chr9 | - | 73452177 | 73573229 | 1.05 |
| cyclin G associated kinase | GAK | XM_001127411 // NM_005255 | FLJ40395 // MGC99654 | 2756673 | chr4 | - | 833110 | 916149 | 1.05 |
| tumor necrosis factor (ligand) superfamily, member 8 | TNFSF8 | NM_001244 | CD153 // CD30L // CD30LG // MGC138144 | 3222144 | chr9 | - | 116695445 | 116732620 | 1.05 |
| presenilin 1 (Alzheimer disease 3) | PSEN1 | NM_000021 | AD3 // FAD // PS1 // S182 | 3543481 | chr14 | + | 72672571 | 72760151 | 1.05 |
| D4, zinc and double PHD fingers family 2 | DPF2 | NM_006268 | MGC10180 // REQ // UBID4 // ubi-d4 | 3335089 | chr11 | + | 64857703 | 64877276 | 1.05 |
| splicing factor proline/glutamine-rich (polypyrimidine tract binding protein associated) | SFPQ | NM_005066 | POMP100 // PSF | 2406064 | chr1 | - | 35351696 | 35506894 | 1.05 |
| development and differentiation enhancing factor 1 | DDEF1 | NM_018482 | AMAP1 // ASAP1 // KIAA1249 // PAG2 // PAP // | 3153428 | chr8 | - | 131133540 | 131525100 | 1.05 |
| TNF receptor-associated factor 3 | TRAF3 | NM_003300 // NM_145725 // NM_145726 | CAP-1 // CD40bp // CRAF1 // LAP1 | 3553337 | chr14 | + | 102313566 | 102447585 | 1.05 |
| thyroid hormone receptor associated protein 1 | THRAP1 | NM_005121 | ARC250 // DRIP250 // HSPC221 // K1AA0593 // | 3765689 | chr17 | - | 57374750 | 57498031 | 1.05 |
| general transcription factor IIIC, polypeptide 4, 90kDa | GTF3C4 | NM_012204 | FLJ21002 // MGC138450 // TFIII90 // TFIIIC90 // TFIICdelta // TFiiiC2-90 | 3192525 | chr9 | + | 134535243 | 134581784 | 1.05 |
| praja 2, RING-H2 motif containing | PJA2 | NM_014819 | K1AA0438 // Neurodap1 // RNF131 | 2870397 | chr5 | - | 108614328 | 108773545 | 1.05 |
| suppressor of var1, 3-like 1 (S. cerevisiae) | SUPV3L1 | NM_003171 | SUV3 | 3250204 | chr10 | + | 70609946 | 70638996 | 1.05 |
| ADP-ribosylation factor 1 | ARF1 | NM_001024226 // NM_001024227 // NM_001024228 // M_001658 | - | 2383726 | chr1 | + | 226313262 | 226353506 | 1.05 |
| cullin-associated and neddylation-dissociated 1 | CAND1 | NM_018448 | DKFZp434M1414 // FLJ10114 // FLJ10929 // FLJ38691 // FLJ90441 // KIAA0829 // TIP120 // TIP120A | 3420713 | chr12 | + | 65946048 | 65994719 | 1.05 |
| ankylosis, progressive homolog (mouse) | ANKH | XM_001132013 // NM_054027 | ANK // CCAL2 // CMDJ // CPPDD // FLJ27166 // HANK // MANK | 2849469 | chr5 | - | 14757920 | 14924876 | 1.05 |
| UBX domain containing 2 | UBXD2 | NM_014607 | FLJ23318 // KIAA0242 // UBXDC1 // erasin | 2507495 | chr2 | + | 136215526 | 136259081 | 1.05 |
| interferon gamma receptor 1 | IFNGR1 | NM_000416 | CD119 // FLJ45734 // IFNGR | 2976113 | chr6 | - | 137560317 | 137582279 | 1.05 |
| GDP dissociation inhibitor 1 | GDI1 | NM_001493 | FLJ41411 // GDIL // MRX41 // MRX48 // OPHN2 // RABGD1A // RABGDIA // XAP-4 | 3996404 | chrX | + | 153318480 | 153324978 | 1.05 |
| myotubularin related protein 6 | MTMR6 | NM_004685 | - | 3506153 | chr13 | - | 24700317 | 24774271 | 1.05 |
| KIAA0226 | KIAA0226 | XM_032901 // XM_936700 | - | 2713555 | chr3 | - | 198863016 | 198960684 | 1.05 |
| Nipped-B homolog (Drosophila) // nuclear receptor binding protein 1 | NIPBL // NRBP1 | NM_015384 // NM_133433 // NM_013392 | CDLS // DKFZp434L1319 // FLJ11203 // FLJ12597 // FLJ13354 // FLJ13648 // FLJ44854 // IDN3 // IDN3-B // BCON3 // FLJ27109 // FLJ35541 // MADM // MUDPNP // NRBP | 2806799 | chr5 | + | 36894163 | 37115932 | 1.05 |
| DENN/MADD domain containing 4A | DENND4A | NM_005848 | FLJ33949 // IRLB // KIAA0476 // MYCPBP | 3629811 | chr15 | - | 63737458 | 63871676 | 1.05 |
| MK167 (FHA domain) interacting nucleolar phosphoprotein | MKI67IP | NM_032390 | NIFK // Nopp34 | 2573786 | chr2 | - | 122132489 | 122231052 | 1.05 |
| myeloid/lymphoid or mixed-lineage leukemia 3 // ADAM metallopeptidase with thrombospondin type 1 motif, 2 // leucine-rich repeat kinase 1 // B melanoma antigen family, member 3 | MLL3 // ADAMTS2 // LRRK1 // BAGE3 | NM_021230 // NM_170606 // NM_014244 // NM_021599 // NM_024652 // NM_182481 | DKFZp686C08112 // FLJ12625 // FLJ38309 // HALR // KIAA1506 // MGC119851 // MGC119852 // MGC119B53 // ADAM-TS2 // ADAMTS-3 // NPI // PCINP II PCPNI // hPCPNI // FLJ23119 // FLJ27465 // KIAA1790 // RIPK6 // Roco1 // - | 3080033 | chr7 | - | 151462947 | 151763974 | 1.05 |
| cyclin D2 | CCND2 | NM_001759 | KIAK0002 // MGC102758 | 3401704 | chr12 | + | 4232432 | 4284764 | 1.05 |
| isopentenyl-diphosphate delta isomerase 1 | MID11 | NM_004508 | IPP1 // IPPI1 | 3273601 | chr10 | - | 1075869 | 1092634 | 1.05 |
| ST8 alpha-N-acetyl-neuraminide alpha-2,8-sialyltransferase 4 | ST8SIA4 | NM_005668 // NM_175052 | MGC34450 // MGC61459 // PST // PST1 // SIAT8D // ST8SIA-IV | 2868904 | chr5 | - | 100116166 | 100293502 | 1.05 |
| Kruppel-like factor 9 | KLF9 | NM_001206 | BTEB // BTEB1 | 3208995 | chr9 | - | 72189344 | 72219360 | 1.05 |
| splicing factor, arginine/serine-rich 2, interacting protein | SFRS2IP | NM_004719 | CASP11 // SIP1 // SRRP129 | 3452145 | chr12 | - | 44599187 | 44672160 | 1.05 |
| ubiquitin specific peptidase 47 | USP47 | M_017944 | DKFZp686C13257 // FLJ20727 // | 3320604 | chr11 | + | 11819556 | 11937446 | 1.05 |
| low density lipoprotein receptor (familial hypercholesterolemia) | LDLR | NM_000527 | FH // FHC | 3821015 | chr19 | + | 11061114 | 11115050 | 1.05 |
| Rho GDP dissociation inhibitor (GDI) beta | ARHGDIB | NM_001175 | D4 // GDIA2 // GDID4 // LYGDI // Ly-GDI // | 3445786 | chr12 | - | 14986174 | 15005951 | 1.05 |
| polymerase (RNA) III (DNA directed) polypeptide E (80kD) | POLR3E | NM_018119 | RPC5 // SIN | 3652489 | chr16 | + | 22216140 | 22253905 | 1.05 |
| DEAD (Asp-Glu-Ala-Asp) box polypeptide 3, Y-linked | DDX3Y | NM_004660 | DBY | 4030162 | chrY | + | 13525423 | 13555808 | 1.05 |
| adenosine deaminase, MRNA-specific | ADAR | NM_001025107 // NM_001111 // NM_015840 // NM_015841 | ADAR1 // DRADA // DSH // DSRAD // G1P1 // 1FI-4 // IFI4// K88dsRBP // p136 | 2436754 | chr1 | - | 152821187 | 152867095 | 1.05 |
| actin binding UM protein 1 | ABLIM1 | NM_001003407 // NM_001003408 // NM_002313 // NM_006720 | ABLIM // DKFZp781D0148 // FLJ14564 // KIAA0059 // LIMAB1 // LIMATIN // MGC1224 | 3307939 | chr10 | - | 116180867 | 116571234 | 1.05 |
| IBR domain containing 2 | IBRDC2 | NM_182757 | KIAA0161 // MGC71786 // bA528A10.3 // p53RFP | 2897172 | chr6 | + | 18385558 | 18650038 | 1.05 |
| IK cytokine, down-regulator of HLA II | IK | NM_006083 | CSA2 // IK protein // MGC59741 // RED | 2831932 | chr5 | + | 140006890 | 140022229 | 1.05 |
| RAD21 homolog (S. pombe) | RAD21 | NM_006265 | FLJ25655 // FLJ40596 // HR21 // HRAD21 // KIAA0078 // MCD1 // NXP1 // SCC1 // hHR21 | 3149843 | chr8 | - | 117909861 | 117956284 | 1.05 |
| eukaryotic translation initiation factor 4 gamma, 1 | EIF4G1 | NM_004953 // NM_182917 // NM_198241 // NM_198242 // NM_198244 | DKFZp686A1451 // EIF4F // EIF4G // p220 | 2655688 | chr3 | + | 185514970 | 185535829 | 1.05 |
| lactate dehydrogenase A | LDHA | NM_005566 | LDH-M // LDH1 // PIG19 | 3322775 | chr11 | + | 18372531 | 18386528 | 1.05 |
| ubiquitin protein ligase E3A (human papilloma virus E6-associated protein, Angelman syndrome) | UBE3A | NM_000462 // NM_130838 // NM_130839 | ANCR // AS // E6- AP // EPVE6AP // FLJ26981 // HPVE6A | 3614087 | chr15 | - | 23006655 | 23355108 | 1.05 |
| nuclear receptor subfamily 4, group A, member 1 | NR4A1 | NM_002135 // NM_173157 // NM_173158 | GFRP1 // HMR // MGC9485 // N10 // NAK-1 // NGFIB // NP10 // NUR77 // TR3 | 3415229 | chr12 | + | 50717248 | 50739539 | 1.05 |
| diaphanous homolog 1 (Drosophila) | DIAPH1 | NM_001079812 // NM_005219 | DFNA1 // DRF1 // FLJ25265 // LFHL1 // hDIA1 | 2878662 | chr5 | - | 140874773 | 140978866 | 1.05 |
| zinc finger CCCH-type, antiviral 1 | ZC3HAV1 | NM_020119 // NM_024625 | DKFZp686F2052 // DKFZp686H1869 // DKFZp686019171 // FL86421 // FLJ13288 // MGC48898 // ZAP // ZC3H2 // ZC3HDC2 | 3075566 | chr7 | - | 138378808 | 138445046 | 1.05 |
| SEC24 related gene family, member C (S. cerevisiae) | SEC24C | NM_004922 // NM_198597 | KIAA0079 | 3251848 | chr10 | + | 75174138 | 75210008 | 1.05 |
| calpain 2, (m/ll) large subunit | CAPN2 | NM_001748 | CANPL2 // CANPml // FLJ39928 // | 2382117 | chr1 | + | 221955990 | 222031806 | 1.05 |
| OTU domain containing 4 | OTUD4 | NM_017493 // NM_199324 | DKFZp434I0721 // HIN1 // HSHIN1 // KIAA1046 | 2788195 | chr4 | - | 146274170 | 146471942 | 1.05 |
| Nedd4 binding protein 1 | N4BP1 | NM_153029 | FLJ31821 // KIAA0615 | 3690597 | chr16 | - | 47130140 | 47324384 | 1.05 |
| 6-phosphofructo-2-kinase/fructose-2,8-biphosphatase 3 | PFKFB3 | NM_004566 | IPFK2 // PFK2 | 3233605 | chr10 | + | 6226893 | 6336597 | 1.05 |
| ATPase, H+ transporting, lysosomal 50/57kDa, V1 subunit H | ATP6V1H | NM_015941 // NM_213619 // NM_213620 | CGI-11 // MSTP042 // SFD // SFDalpha // SFDbeta // VMA13 | 3135452 | chr8 | - | 54790669 | 54918651 | 1.05 |
| ferritin, heavy polypeptide-like 7 // ferritin, heavy polypeptide 1 // ferritin, heavy polypeptide-like 3 // ferritin, heavy polypeptide pseudogene 1 // ferritin, heavy polypeptide-like 12 // ferritin, heavy polypeptide-like 11 // ferritin, heavy polypeptide-like 8 | FTHL7 // FTH1 // FTHL3 // FTHP1 // FTHL12 // FTHL11 // FTHL8 | NR_002202 // NM_002032 // NR_002201 // - // NR_002205 // NR_002204 // NR_002203 | - // FTH // FTHL6 // MGC104426 // P1G15 // PLIF // FTHL5 | 3375648 | chr11 | - | 61488337 | 61492603 | 1.05 |
| protein inhibitor of activated STAT, 1 | PIAS1 | NM_016166 | DDXBP1 // GBP // GU/RH-II // MGC141 878 // MGC141879 // ZMIZ3 | 3599280 | chr15 | + | 65898917 | 66277975 | 1.05 |
| interferon-induced protein with tetratricopeptide repeats 3 | IFIT3 | NM_001031683 // NM_001549 | CIG-49 // GARG- 49 // IFI60 // IFIT4 // IRG2 // ISG60 // RIG-G | 3257204 | chr10 | + | 91059126 | 91090705 | 1.05 |
| cullin 4A | CUL4A | NM_001008895 // NM_003589 | - | 3502497 | chr13 | + | 112905835 | 112967356 | 1.05 |
| SH2 domain protein 2A | SH2D2A | NM_003975 | F2771 // TSAd | 2438575 | chr1 | - | 155038957 | 155053637 | 1.05 |
| EH-domain containing 1 | EHD1 | NM_006795 | FLJ42622 // FLJ44618 // H- PAST // HPAST1 // PAST // PAST1 | 3377226 | chr11 | - | 64376790 | 64404202 | 1.05 |
| SH2B adaptor protein 3 | SH2B3 | NM_005475 | LNK | 3431892 | chr12 | + | 110328135 | 110373802 | 1.05 |
| tropomodulin 3 (ubiquitous) | TMOD3 | NM_014547 | UTMOD | 3594066 | chr15 | + | 49909087 | 50026784 | 1.05 |
| SMAD family member 3 | SMAD3 | NM_005902 | DKFZP586N0721 // DKFZp686J10186 // HSPC193 // HsT17436 // JV15- 2 // MADH3 // MGC60396 // Smad 3 | 3598959 | chr15 | + | 65145043 | 65296818 | 1.05 |
| vacuolar protein sorting 53 homolog (S. cerevisiae) | VPS53 | NM_018289 | FLJ10979 // MGC39512 // hVps53L // | 3739679 | chr17 | - | 375959 | 564837 | 1.05 |
| TRAF and TNF receptor associated protein | TTRAP | NM_016614 | AD022 // EAP2 // MGC111021 // MGC9099 // dJ30M3.3 | 2945645 | chr6 | - | 24758144 | 24775240 | 1.05 |
| MAP/microtubule affinity-regulating kinase 2 | MARK2 | NM_001039468 // NM_001039469 // NM_004954 // NM_017490 | EMK1 // MGC99619 // PAR-1 | 3334025 | chr11 | + | 63362634 | 63435067 | 1.05 |
| helicase with zinc finger | HELZ | NM_014877 | DRHC // HUMORF5 // KIAA0054 // MGC 163454 | 3768015 | chr17 | - | 62485987 | 62672547 | 1.05 |
| golgi-specific brefeldin A resistance factor 1 | GBF1 | NM_004193 | FLJ21263 // FLJ21500 // KIAA0248 // MGC134877 // MGC 134878 | 3261544 | chr10 | + | 103993260 | 104132642 | 1.05 |
| serine/threonine kinase 38 like | STK38L | NM_015000 | KIAA0965 // NDR2 | 3409081 | chr12 | + | 27288343 | 27370157 | 1.05 |
| BCL2-related protein A1 | BCL2A1 | NM_004049 | ACC-1 // ACC-2 // BCL2L5 // BFL1 // GRS // HBPA1 | 3635198 | chr15 | - | 78040295 | 78138851 | 1.05 |
| membrane-associated ring finger (C3HC4) 7 | MARCH7 | NM_022826 | AXO // AXOT // DKFZP586F1122 // MARCH-VII // RNF177 | 2512330 | chr2 | + | 160277236 | 160333585 | 1.05 |
| periphilin 1 | PPHLN1 | NM_016488 // NM_201438 // NM_01439 // NM_201440 // NM_201515 | HSPC206 // HSPC232 // MGC48786 | 3412008 | chr12 | + | 40917957 | 41128677 | 1.05 |
| tankyrase, TRF1-interacting ankyrin-related ADP-ribose polymerase 2 | TNKS2 | NM_025235 | PARP-5b // PARP- 5c // PARP5B // PARP5C // TANK2 // TNKL | 3257850 | chr10 | + | 93548049 | 93633726 | 1.05 |
| kelch-like 6 (Drosophila) | KLHL6 | NM_130446 | FLJ00029 | 2708066 | chr3 | - | 184688052 | 184789879 | 1.05 |
| Kruppel-like factor 10 | KLF10 | NM_001032282 // NM 005655 | EGRA // TIEG // TIEG1 | 3147508 | chr8 | - | 103682306 | 103767558 | 1.05 |
| neugrin, neurite outgrowth associated | NGRN | NM_001033088 // NM_016845 | DSC92 // NEUGRIN | 3607998 | chr15 | + | 88586640 | 88670084 | 1.05 |
| DEAD (Asp-Glu-Ala-Asp) box polypeptide 5 | DDX5 | NM_004396 | DKFZp686J01190 // G17P1 // HLR1 // HUMP68 // p68 | 3766893 | chr17 | - | 59924843 | 59935796 | 1.05 |
| SMEK homolog 2, suppressor of mek1 (Dictyostelium) | SMEK2 | NM_020463 | FLFL2 // FLJ31474 // KIAA1387 // PSY2 // smkl | 2553911 | chr2 | - | 55627936 | 55699519 | 1.05 |
| thioredoxin reductase 1 | TXNRD1 | NM_003330 // NM_182729 // NM_182742 // NM 182743 | GRIM-12 // MGC9145 // TR // TR1 // TRXR1 // TXNR | 3429460 | chr12 | + | 103130628 | 103354079 | 1.05 |
| Ras and Rab interactor 3 | RIN3 | NM_024832 | DKFZp782H1813 // FLJ11700 // FLJ22439 | 3548929 | chr14 | + | 92049804 | 92225160 | 1.05 |
| itchy homolog E3 ubiquitin protein ligase (mouse) | ITCH | NM_031483 | AIF4 // AIP4 // NAPP1 // dJ46801.1 | 3882854 | chr20 | + | 32414702 | 32562859 | 1.05 |
| KIAA0174 | KIAA0174 | NM 014761 | MGC117220 | 3667766 | chr16 | + | 70481983 | 70522196 | 1.05 |
| formin-like 3 | FMNL3 | NM_175738 // NM_198900 | DKFZp762B245 // FHOD3 // FLJ45265 // MGC45819 // | 3454006 | chr12 | - | 48316548 | 48387433 | 1.05 |
| ARP3 actin-related protein 3 homolog (yeast) | ACTR3 | NM_005721 | ARP3 | 2501697 | chr2 | + | 114360575 | 114502125 | 1.05 |
| cyclin T2 // aminocarboxymuconate semialdehyde decarboxylase | CCNT2 // ACMSD | NM_001241 // NM_058241 // NM_138326 | FLJ90560 // MGC134840 // - | 2507209 | chr2 | + | 135392283 | 135432746 | 1.05 |
| topoisomerase (DNA) II beta 180kDa | TOP2B | NM_001068 | TOPIIB // top2beta | 2666478 | chr3 | - | 25614141 | 25681392 | 1.05 |
| RNA binding motif protein 16 // T-cell lymphoma invasion and metastasis 2 | RBM16 // TIAM2 | NM_014892 // NM_001010927 // NM_012454 | KIAA1116 // FLJ41865 // STEF | 2932360 | chr6 | + | 155029299 | 155324283 | 1.05 |
| solute carrier family 3 (activators of dibasic and neutral amino acid transport), member 2 | SLC3A2 | NM_001012661 // NM_001012662 // NM_001012663 // NM_001012664 // NM_001013251 // NM 002394 | 4F2 // 4F2HC // 4T2HC // CD98 // CD98HC // MDU1 // NACAE | 3333711 | chr11 | + | 62380117 | 62412872 | 1.05 |
| Der1-like domain family, member 1 | DERL1 | NM_024295 | DER-1 // DER1 // FLJ13784 // FLJ42092 // MGC3067 // PRO2577 | 3151401 | chr8 | - | 124094605 | 124123781 | 1.05 |
| phosphodiesterase 7A | PDE7A | NM_002603 // NM_002604 | HCP1 // PDE7 | 3138464 | chr8 | - | 66789124 | 67101393 | 1.05 |
| formin-like 1 | FMNL1 | NM_005892 | C17orf1 // C17orf1B // FHOD4 // FMNL // KW-13 // MGC133052 // MGC1894 // | 3723378 | chr17 | + | 40654831 | 40680464 | 1.05 |
| Rap guanine nucleotide exchange factor (GEF) 1 | RAPGEF1 | NM_005312 // NM_198679 | C3G // DKFZp781P1719 // GRF2 | 3227696 | chr9 | - | 133441988 | 133605262 | 1.05 |
| transmembrane BAX inhibitor motif containing 4 | TMBIM4 | NM_016056 | CGI-119 // S1R // ZPRO | 3460593 | chr12 | - | 64816984 | 64850064 | 1.05 |
| B double prime 1, subunit of RNA polymerase III transcription initiation factor IIIB | BDP1 | NM_018429 | DKFZp686C01233 // DKFZp688K0831 // HSA238520 // KIAA1241 // KIAA1689 // TAF3BI // TFC5 // TFIIIB" // TFIIB150 // TFIIIB90 // TFNR | 2814527 | chr5 | + | 70699177 | 70899402 | 1.05 |
| MYST histone acetyltransferase 2 | MYST2 | NM_007067 | HBO1 // HBOA | 3725779 | chr17 | + | 45196447 | 43261455 | 1.05 |
| KIAA0247 | KIAA0247 | NM_014734 | | 3542145 | chr14 | + | 69145907 | 69250533 | 1.05 |
| ubiquitin specific peptidase 36 | USP36 | NM_025090 | DUB1 | 3772581 | chr17 | - | 74295075 | 74348574 | 1.05 |
| protein kinase, AMP-activated, gamma 2 non-catalytic subunit | PRKAG2 | NM_001040633 // NM_016203 // NM 024429 | AAKG // AAKG2 // CMH6 // H91620p // WPWS | 3079803 | chr7 | - | 150865031 | 151205160 | 1.05 |
| RAB18, member RAS oncogene family | RAB18 | NM_021252 | RAB18LI1 | 3240095 | chr10 | + | 27791811 | 27930577 | 1.05 |
| WW domain containing adaptor with coiled-coil | WAC | NM_016628 // NM_100264 // NM_100486 | BM-016 // MGC10753 // PRO1741 //Wwp4 // bA48B24 // bA48B24.1 | 3240340 | chr10 | + | 28828043 | 29005095 | 1.05 |
| stromal interaction molecule 2 | STIM2 | NM_020860 | FLJ39527 // KIAA1 482 | 2722377 | chr4 | + | 26415653 | 26636427 | 1.05 |
| actin, gamma 1 | ACTG1 | NM_001614 | ACT // ACTG // DFNA20 // DNA26 | 3773532 | chr17 | - | 77091456 | 77105797 | 1.05 |
| ash 1 (absent, small, or homeotic)-like (Drosophila) | ASHIL | NM_018489 | ASH1 // ASH1L1 // FLJ10504 // KIAA1420 | 2437417 | chr1 | - | 153571685 | 153798937 | 1.05 |
| FLJ36874 protein | FLJ36874 | NM_152716 | MGC125671 // MGC125672 | 3374746 | chr11 | - | 59160770 | 59193099 | 1.05 |
| thyroid hormone receptor associated protein 2 | THRAP2 | NM_015335 | DKFZp781D0112 // FLJ21627 // KIAA 1025 // MED13L // PROSIT240 // TRAMP240L | 3473083 | chr12 | - | 114868853 | 115419724 | 1.05 |
| B-cell CLL/lymphoma 10 | BCL10 | NM_003921 | CARMEN // CIPER // CLAP // c-E10 // mE10 | 2420808 | chr1 | - | 85504067 | 85516171 | 1.05 |
| collagen, type IV, alpha 3 (Goodpasture antigen) binding protein | COL4A3BP | NM_005713 // NM_031361 | CERT // CERTL // FLJ20597 // GPBP // STARD11 | 2862950 | chr5 | - | 74700067 | 74843717 | 1.05 |
| wings apart-like homolog (Drosophila) | WAPAL | NM_015045 | FOE // KIAA0261 // WAPL | 3298738 | chr10 | - | 88184999 | 88286267 | 1.05 |
| SFRS protein kinase 2 | SRPK2 | NM_82691 // NM_182692 | FLJ36101 // SFRSK2 | 3066297 | chr7 | - | 104537547 | 104842466 | 1.05 |
| RNA binding motif protein 22 | RBM22 | NM_018047 | FLJ10290 // ZC3H16 | 2881521 | chr5 | - | 150050460 | 150060885 | 1.05 |
| chromodomain helicase DNA binding protein 1 | CHD1 | NM_001270 | DKFZp686E2337 | 2868523 | chr5 | - | 98218502 | 98293051 | 1.05 |
| G1 to S phase transition 1 | GSPT1 | NM_002094 | 551 G9.2 // ETF3A // GST1 // eRF3a | 3680610 | chr16 | - | 11865619 | 11917408 | 1.05 |
| ubiquilin 1 // death inducer-obliterator 1 | UBQLN1 // DIDO1 | NM_013438 // NM_053067 // NM_022105 // NM_033081 // NM_080796 // NM_080797 | DA41 // DSK2 // FLJ90054 // PUC- 1 // XDRP1 // BYE1 // C20orf158 // DATF1 // DIDO2 // DID03 // DIO-1 // DIO1 // DKFZp434P1115 // FLJ11265 // KIAA0333 // MGC16140 // dJ885L7.8 | 3212143 | chr9 | - | 85464717 | 85512928 | 1.05 |
| golgi associated, gamma adaptin ear containing, ARF binding protein 2 | GGA2 | NM_015044 | FLJ20966 // KIAA1080 // VEAR | 3685183 | chr16 | - | 23381059 | 23429320 | 1.05 |
| solute carrier family 2 (facilitated glucose transporter), member 3 | SLC2A3 | NM_006931 | FLJ90380 // GLUT3 | 3442854 | chr12 | - | 7917549 | 7980138 | 1.05 |
| protein tyrosine phosphatase, non-receptor type 1 | PTPN1 | NM_002827 | PTP1B | 3888721 | chr20 | + | 48560298 | 48634700 | 1.05 |
| megakaryoblastic leukemia (translocation) 1 | MKL1 | NM_020831 | BSAC // MAL // MRTF-A | 3961496 | chr22 | - | 39136248 | 39362660 | 1.05 |
| sequestosome 1 | SQSTM1 // OSIL | NM_003900 | A170 // OSIL // PDB3 // ZIP3 // p60 // p62 // p62B | 2844479 | chr5 | + | 179166014 | 179198853 | 1.05 |
| family with sequence similarity 78, member A | FAM78A | NM_033387 | C9orf59 // FLJ00024 | 3227574 | chr9 | - | 133123292 | 133147120 | 1.05 |
| coenzyme Q2 homolog, prenyltransferase | COQ2 | NM_015697 | CL640 // FLJ26072 | 2775965 | chr4 | - | 84404005 | 84424988 | 1.05 |
| small nuclear ribonucleoprotein polypeptide B" | SNRPB2 | NM_003092 // NM_198220 | MGC24807 // MGC45309 | 3877776 | chr20 | + | 16645421 | 16670916 | 1.05 |
| bromodomain adjacent to zinc finger domain, 2A | BAZ2A | NM_013449 | DKFZp781B109 // FLJ13768 // FLJ13780 // FLJ45876 // KIAA0314 // TIP5 // WALp3 | 3457947 | chr12 | - | 55275319 | 55316417 | 1.05 |
| RNA binding motif protein 25 | RBM25 | NM_021239 | MGC105088 // MGC117168 // RNPC7 // S164 | 3543411 | chr14 | + | 72594776 | 72658577 | 1.05 |
| integrator complex subunit 6 | INTS6 | NM_001039937 // NM_001039938 // NM_012141 | DBI-1 // DDX26 // DDX26A // DICE1 // DKFZP434B105 // HDB // INT6 // Notch12 | 3514488 | chr13 | - | 50826009 | 50927014 | 1.05 |
| cyclin M3 | CNNM3 | XM_001127059 // NM_017623 // NM_199078 | ACDP3 // DKFZp43411016 // FLJ20018 | 2494749 | chr2 | + | 96843277 | 96864841 | 1.05 |
| Mdm2, transformed 3T3 cell double minute 2, p53 binding protein (mouse) | MDM2 | NM_002392 // NM_006878 // NM_006879 // NM_006881 // NM_006882 | HDMX // MGC71221 // hdm2 | 3421300 | chr12 | + | 67488176 | 67525479 | 1.05 |
| colony stimulating factor 1 receptor, formerly McDonough feline sarcoma viral (v-fms) oncogene homolog | CSF1R | NM_005211 | C-FMS // CD115 // CSFR // FIM2 // FMS | 2881187 | chr5 | - | 149412410 | 149473128 | 1.05 |
| thioredoxin-like 1 | TXNL1 | NM_004786 | TRP32 // TXL-1 // TXNL // Txl | 3809324 | chr18 | - | 52334063 | 52469773 | 1.05 |
| ELOVL family member 5, elongation of long chain fatty acids (FEN1/Elo2, SUR4/Elo3-like, yeast) | ELOVL5 | NM_021814 | HELO1 // RP3- 483K16.1 // dJ483K16.1 | 2957596 | chr6 | - | 53179349 | 53321902 | 1.05 |
| PFTAIRE protein kinase 1 | PFTK1 | NM_012395 | KIAA0834 // PFTAIRE1 | 3012064 | chr7 | + | 90002707 | 90677837 | 1.05 |
| nuclear mitotic apparatus protein 1 | NUMA1 | NM 006185 | NUMA | 3380901 | chr11 | - | 71389909 | 71469367 | 1.05 |
| sparc/osteonectin, cwcv and kazal-like domains proteoglycan (testican) 2 | SPOCK2 | NM_014767 | testican-2 | 3293840 | chr10 | - | 73457514 | 73518527 | 1.05 |
| clathrin, heavy chain (Hc) | CLTC | NM_004859 | CHC17 // CLH-17 // CLTCL2 // Hc // KIAA0034 | 3729172 | chr17 | + | 55052021 | 55127299 | 1.05 |
| F-box and leucine-rich repeat protein 11 | FBXL11 | NM_012308 | CXXC8 // DKFZP434M1735 // FBL11 // FBL7 // FLJ00115 // FLJ46431 // JHDM1A // KIAA1004 // LILINA | 3336696 | chr11 | + | 66643299 | 66782124 | 1.05 |
| signal-induced proliferation-associated gene 1 | SIPA1 | NM_006747 // NM_153253 | MGC102688 // MGC17037 // SPA1 | 3335465 | chr11 | + | 65162171 | 65174965 | 1.05 |
| kelch-like 24 (Drosophila) // YEATS domain containing 2 | KLHL24 // YEATS2 | NM_017644 // NM_018023 | DRE1 // FLJ25796 // FLJ10201 // FLJ12841 // FLJ13308 // KIAA1197 | 2655113 | chr3 | + | 184836105 | 184882100 | 1.05 |
| thymopoietin | TMPO | NM_001032283 // NM_001032284 // NM_003276 | CMD1T // LAP2 // MGC61508 // PRO0868 // TP | 3427767 | chr12 | + | 97395927 | 97502543 | 1.05 |
| STT3, subunit of the oligosoccharyltransferase complex, homolog B (S. cerevisiae) | STT3B | NM_178862 | FLJ90106 // SIMP // STT3-B | 2615600 | chr3 | + | 31544326 | 31662283 | 1.05 |
| ATP-binding cassette, sub-family F (GCN20), member 1 | ABCF1 | NM_001025091 // NM 001090 | ABC27 // ABC50 | 2901687 | chr6 | + | 30647103 | 30667268 | 1.05 |
| CUG triplet repeat, RNA binding protein 2 | CUGBP2 | NM_001025076 // NM_001025077 // NM 006561 | BRUNOL3 // ETR- 3 // NAPOR | 3234760 | chr10 | + | 10544225 | 11418680 | 1.05 |
| major histocompatibility complex, class II, DR | HLA-DRA | NM_019111 | HLA-DRA1 | 2903189 | chr6 | + | 32496972 | 32520923 | 1.05 |
| RAN binding protein 2 | RANBP2 // RGPD4 | NM_006267 | NUP358 // TRP1 // TRP2 | 2499158 | chr2 | + | 108702376 | 108768725 | 1.05 |
| G protein-coupled receptor kinase 5 | GRK5 | NM_005308 | GPRK5 | 3267036 | chr10 | + | 120957193 | 121256088 | 1.05 |
| FRY-like | FRYL | XM_001134434 // XM_001134456 // NM_015030 | DKFZp686E205 // FLJ16177 // KIAA0826 | 2768468 | chr4 | - | 48194140 | 48272015 | 1.05 |
| bromodomain containing 1 | BRD1 | NM_014577 | BRL // BRPF1 // BRPF2 // DKFZp686F0325 | 3965314 | chr22 | - | 48552694 | 48636916 | 1.05 |
| nuclear factor of activated T-cells, cytoplasmic, calcineurin-dependent 2 | NFATC2 | NM_012340 // NM_173091 | KIAA0611 // NFAT1 // NFATP | 3909553 | chr20 | - | 49436909 | 49603743 | 1.05 |
| TATA element modulatory factor 1 | TMF1 | NM_007114 | ARA160 | 2681157 | chr3 | - | 69151671 | 69184154 | 1.05 |
| ral guanine nucleotide dissociation stimulator | RALGDS | NM_001042368 NM_006266 | FLJ20922 // RGF // RaIGEF | 3228463 | chr9 | - | 134962931 | 135014381 | 1.05 |
| GATA binding protein 2 | GATA2 | NM_032638 | MGC2306 // NFE1B | 2694314 | chr3 | - | 129675850 | 129700720 | 1.05 |
| forty-two-three domain containing 1 | FYTTD1 | NM_001011537 // NM_032288 | DKFZp76181514 | 2659887 | chr3 | + | 198960821 | 198996244 | 1.05 |
| splicing factor, arginine/serine-rich 1 (splicing factor 2, alternate splicing factor) | SFRS1 | NM_001078166 // NM_006924 | ASF // MGC5228 // SF2 // SF2p33 // SRp30a | 3764103 | chr17 | - | 53421412 | 53439635 | 1.05 |
| transcription elongation regulator 1 | TCERG1 | NM_001040006 // NM_006706 | CA150 // MGC133200 // TAF2S | 2834093 | chr5 | + | 145754916 | 145871713 | 1.05 |
| 3-hydroxy-3-methylglutaryl-Coenzyme A synthase 1 (soluble) | HMGCS1 | NM_002130 | HMGCS // MGC90332 | 2855501 | chr5 | - | 43324231 | 43349337 | 1.05 |
| inositol 1,4,5-trisphosphate 3-kinase B | ITPKB | NM_002221 | IP3K // IP3K-B // PIG37 | 2458921 | chr1 | - | 224865889 | 225014518 | 1.05 |
| insulin-like growth factor 1 receptor | IGF1R | NM_000875 | CD221 // IGFIR // JTK13 // MGC142170 // MGC142172 // MGC18216 | 3610804 | chr15 | + | 97010298 | 97320602 | 1.05 |
| ribulose-5-phosphate-3-epimerase // chemokine binding protein 2 | RPE // CCBP2 | NM_006916 // NM_199229 // NM_001296 | MGC2636 // RPE2- 1 // CCR10 // CCR9 // CMKBR9 // D6 // MGC126678 // MGC138250 // hD6 | 2525852 | chr2 | + | 210575559 | 210637315 | 1.05 |
| KIAA1429 | K1AA1429 | NM_015496 // NM_183009 | DKFZP4341116 // DKFZp781B2117 // MGC138493 // MGC141940 // MSTP054 | 3145020 | chr8 | - | 95569109 | 95634851 | 1.05 |
| dual-specificity tyrosine-(Y)-phosphorylation regulated kinase 1A | DYRK1A | NM_001396 // NM_101395 // NM_30436 // NM_130437 // NM 130438 | DYRK // DYRK1 // HP86 // MNB // MNBH | 3920566 | chr21 | + | 37661116 | 37857051 | 1.05 |
| coronin 7 | CORO7 | NM_024535 | 0610011B16Rik // CRN7 // FLJ22021 // FLJ44188 | 3678083 | chr16 | - | 4343041 | 4406954 | 1.05 |
| ribosomal protein S6 kinase, 70kDa, polypeptide 1 | RPS6KB1 | NM_003161 | PS6K // S6K // S6K1 // STK14A // p70(S6K)-alpha // p70-S6K // p70- alpha | 3729294 | chr17 | + | 55325239 | 55429105 | 1.05 |
| MAK10 homolog, amino-acid N-acetyltransferase subunit, (S. cerevisiae) | MAK10 | NM_024635 | FLJ21613 // FLJ22643 // bA379P1.1 | 3177563 | chr9 | + | 87733620 | 87828205 | 1.05 |
| family with sequence similarity 62 (C2 domain containing) member B | FAM62B | NM_020728 | CHR2SYT // ESYT2 // KIAA1228 | 3082248 | chr7 | - | 158205107 | 158355198 | 1.05 |
| guanylate binding protein 4 // guanylate binding protein 7 | GBP4 // GBP7 | NM_052941 // NM 207398 | Mpa2 // FLJ38822 // GBP4L | 2421995 | chr1 | - | 89419435 | 89437211 | 1.05 |
| 5-methyltatrahydrofolate-homocysteine methyltransferase reductase | MTRR | NM_002454 // NM_024010 | MGC129643 // MSR | 2800906 | chr5 | + | 7922217 | 7954221 | 1.05 |
| tripeptidyl peptidase II | TPP2 | NM_003291 | FLJ40359 | 3499453 | chr13 | + | 101981581 | 102131173 | 1.05 |
| acyl-CoA synthetase short-chain family member 1 | ACSS1 | NM_032501 | ACAS2L // AceCS2L // FLJ45659 // MGC33843 | 3901696 | chr20 | - | 24934882 | 24987616 | 1.05 |
| tousled-like kinase 1 | TLK1 | NM_012290 | KIAA0137 // PKU- BETA | 2586603 | chr2 | - | 171538552 | 171796060 | 1.05 |
| secretory carrier membrane protein 1 | SCAMP1 | NM_052822 // NM_004866 | SCAMP // SCAMP37 | 2817053 | chr5 | + | 77691978 | 77812317 | 1.05 |
| tripartite motif-containing 28 | TRIM28 | NM_005762 | FLJ29029 // KAP1 // RNF96 // TF1 B // TIF1B | 3844238 | chr19 | + | 63740597 | 63753931 | 1.05 |
| chromosome 11 open reading frame 58 | C11orf58 | NM_014267 | IMAGE145052 // MGC117265 // SMAP | 3322048 | chr11 | + | 16716744 | 16734468 | 1.05 |
| serine incorporator 1 | SERINC1 | NM_020755 | KIAA1253 // TDE1L // TDE2 // TMS-2 | 2972310 | chr6 | - | 122804811 | 122948968 | 1.05 |
| karyopherin (importin) beta 1 | KPNB1 | NM_002265 | IMB1 // IPOB // Impnb // MGC2155 // MGC2156 // MGC2157 // NTF97 | 3724782 | chr17 | + | 43082272 | 43117868 | 1.05 |
| hypothetical FLJ46363 // ataxin 2-like | FLJ46363 // ATXN2L | NM_207434 // XM_001127543 // NM_007245 // NM_017492 // NM_145714 // NM_148414 // NM_48415 // NM_148416 | - // A2D // A2LG // A2LP // A2RP | 3654859 | chr16 | + | 28817718 | 28756043 | 1.05 |
| quiescin Q6 | QSCN6 // FLJ23867 | NM_001004128 // NM_002826 | Q6 // QSOX1 | 2369950 | chr1 | + | 178353067 | 178436483 | 1.05 |
| splicing factor, arginine/serine-rich 6 | SFRS6 | NM_006275 | B52 // MGC5045 // SRP55 | 3886050 | chr20 | + | 41519932 | 41526301 | 1.05 |
| moesin | MSN | NM_002444 | - | 3979659 | chrX | + | 64804248 | 64878488 | 1.05 |
| intersectin 2 | ITSN2 | NM_006277 // NM_019595 // NM_147152 | KIAA1256 // SH3D1B // SH3P18 // SWA // SWAP | 2544238 | chr2 | - | 24279241 | 24436906 | 1.05 |
| SAFB-like, transcription modulator | SLTM | NM_001013843 // NM_017968 // NM_024755 | DKFZp762G052 // FLJ10005 // FLJ13213 // Met | 3626704 | chr15 | - | 56949072 | 57013564 | 1.05 |
| transcription factor 4 | TCF4 | NM_003199 | E2-2 // ITF2 // MGC149723 // MGC149724 // SEF2 // SEF2-1 // SEF2-1A // SEF2- 1B | 3808854 | chr18 | - | 51010981 | 51506795 | 1.05 |
| DEAH (Asp-Glu-Ala-His) box polypeptide 9 | DHX9 | NM_001357 | DDX9 // LKP // NDH II // NDHII // RHA | 2370991 | chr1 | + | 181075106 | 181123738 | 1.05 |
| chromosome 10 open reading frame 119 | C10orf119 | NM_024834 | FLJ13081 // FLJ36756 | 3309755 | chr10 | - | 121568302 | 121642742 | 1.05 |
| SET domain containing 2 | SETD2 | NM_014159 | FLJ16420 // FLJ22472 // FLJ23184 // FLJ45883 // HIF-1 // HSPC069 // HYPB // KIAA1732 | 2672532 | chr3 | - | 47032936 | 47180418 | 1.05 |
| golgi reassembly stacking protein 2, 55kDa // dehydrogenase/reductase (SDR family) member 9 | GORASP2 // DHRS9 | NM_015530 // NM_005771 // NO_199204 | DKFZP434D156 // FLJ13139 // GOLPH6 // GRASP55 // GRS2 // p59 // 3alpha- HSD // RDH15 // RDHL // RETSDR8 | 2515050 | chr2 | + | 171457150 | 171551619 | 1.05 |
| target of myb1 (chicken) | TOM1 | NM 005488 | FLJ33404 | 3944084 | chr22 | + | 34025845 | 34073962 | 1.05 |
| ATPase, H+ transporting, lysosomal 42kDa, V1 subunit C1 | ATP6V1C1 | NM_001007254 // NM_001695 | ATP6C // ATP6D // FLJ20057 // VATC // Vma5 | 3110171 | chr8 | + | 104102441 | 104171800 | 1.05 |
| potassium channel tetramerisation domain containing 20 | KCTD20 | NM_173562 | C6orf69 // MGC14254 // dJ108K11.3 | 2905069 | chr6 | + | 36518349 | 36566874 | 1.05 |
| | STK10 | NM_005990 | LOK // PRO2729 | 2887048 | chr5 | - | 171401689 | 171547855 | 1.05 |
| serine/threonine kinase 10 GA binding protein transcription factor, alpha subunit 60kDa // GA binding protein transcription factor, alpha subunit pseudogene | GABPA // GABPAP | NM_002040 // - // NR_002723 | E4TF1-60 // E4TF1A // NFT2 // NRF2 // NRF2A // E4TF1 // E4TF1B // GABPB1 | 3916576 | chr21 | + | 26028762 | 26066641 | 1.05 |
| centaurin, beta 2 | CENTB2 | NM_012287 | ACAP2 // CNT-B2 // KIAA0041 | 2712040 | chr3 | - | 196476779 | 196645492 | 1.05 |
| fibrosin 1 | FBS1 | NM_022452 | FBS // FLJ11618 | 3656362 | chr16 | + | 30577810 | 30590035 | 1.05 |
| G protein-coupled receptor kinase interactor 2 | GIT2 | NM_014776 // NM_057169 // NM_057170 // NM_139201 | CAT-2 // DKFZp686G01261 // KIAA0148 // MGC760 | 3471005 | chr12 | - | 108852000 | 108918536 | 1.05 |
| vimentin | VIM | NM 003380 | FLJ36605 | 3236958 | chr10 | + | 17296705 | 17357733 | 1.05 |
| nuclear receptor coactivator 2 | NCOA2 | NM_006540 | GRIP1 // MGC138808 // NCoA-2 // T1F2 | 3139722 | chr8 | - | 71184397 | 71478626 | 1.05 |
| thrombaspondin 1 | THBS1 | NM_003246 | THBS // TSP // TSP1 | 3589458 | chr15 | + | 37614946 | 37678406 | 1.05 |
| trinucleotide repeat containing 6B | TNRC6B | NM_001024843 // NM_015088 | KIAA1093 | 3946192 | chr22 | + | 38770332 | 39061757 | 1.05 |
| GTPase activating protein (SH3 domain) binding protein 2 // NMDA receptor regulated 2 // RAR-related orphan receptor A | G3BP2 // NARG2 // RORA | NM_012297 // NM_203504 // NM_203505 // NM_001018089 // NM_024611 // NM_002943 // NM_134260 // NM_134261 // NM_134262 | - // MGC119326 // MGC119329 // NR1F1 // ROR1 // ROR2 // ROR3 // RZRA | 2773756 | chr4 | - | 76786807 | 76869171 | 1.05 |
| calcium/calmodulin-dependent protein kinase IV | CAMK4 | NM_001744 | CaMK-GR // MGC36771 | 2823880 | chr5 | + | 110587242 | 110854177 | 1.05 |
| oxidative-stress responsive 1 | OXSR1 | NM_005109 | KIAA1101 // OSR1 | 2617579 | chr3 | + | 38182030 | 38271979 | 1.05 |
| ATPase, Na+/K+ transporting, alpha 1 polypeptide | ATP1A1 | NM_000701 // NM_001001586 | MGC3285 // MGC51750 | 2353477 | chr1 | + | 116706775 | 116754386 | 1.05 |
| transportin 1 | TNPO1 | NM_002270 // NM 153188 | IPO2 // KPNB2 // MIP // MIP1 // | 2815043 | chr5 | + | 72148176 | 72245960 | 1.05 |
| heterogeneous nuclear ribonucleoprotein U-like 1 | HNRPUL1 | NM_144733 // NM_1 44734 // NM_007040 // NM_144732 | E1B-AP5 // E1BAP5 // FLJ12944 | 3834089 | chr19 | + | 46460006 | 46505508 | 1.05 |
| CD300e molecule // fibrillin 2 (congenital contractural arachnodactyly) | CD300E // FBN2 | NM_181449 // NM_001999 | CD300LE // CLM2 // IREM2 // CCA | 3770345 | chr17 | - | 70117621 | 70131474 | 1.05 |
| CD55 molecule, decay accelerating factor for complement (Cromer blood group) | CD55 | NM_000574 | CR // DAF // TO | 2377229 | chr1 | + | 205561488 | 205607671 | 1.05 |
| TBC1 domain family, member 15 | TBC1D15 | NM_022771 | DKFZp686M1379 // DKFZp761D0223 // FLJ12085 | 3422326 | chr12 | + | 70519754 | 70604360 | 1.05 |
| v-raf murine sarcoma viral oncogene homolog B1 | BRAF | NM_004333 | B-raf 1 // BRAF1 // MGC126806 // MGC138284 // RAFB1 | 3076340 | chr7 | - | 139994192 | 140274640 | 1.05 |
| splicing factor, arginine/serine-rich 5 | SFRS5 | NM_001039465 // NM_006925 | HRS // SRP40 | 3542207 | chr14 | + | 69263363 | 69308465 | 1.05 |
| GRAM domain containing 1A | GRAMD1A | NM_020895 | FLJ22411 // FLJ90346 // KIAA1533 | 3830002 | chr19 | + | 40161899 | 40210121 | 1.05 |
| mediator of RNA polymerase II transcription, subunit 28 homolog (S. cerevisiae) | MED28 | NM_025205 | 1500003D12Rik // DKFZP434N185 // EG1 // magicin | 2720181 | chr4 | + | 17225344 | 17244808 | 1.05 |
| jumonji, AT rich interactive domain 1A | JARJD1A | NM_001042603 // NM 005056 | RBBP2 // RBP2 | 3439603 | chr12 | - | 259504 | 368944 | 1.05 |
| activating transcription factor 6 | ATF6 | NM 007348 | - | 2363919 | chr1 | + | 160002678 | 160208836 | 1.05 |
| matrin 3 | MATR3 | NM_018834 // NM_199189 | DKFZp686K0542 // DKFZp688K23100 // KIAA0723 // MGC9105 | 2831124 | chr5 | + | 138505686 | 138695245 | 1.05 |
| granzyme B (granzyme 2, cytotoxic T-lymphocyte-associated serine esterase 1) | GZMB | NM_004131 | CCP1 // CGL-1 // CGL1 // CSP-B // CSPB // CTLA1 // CTSGL1 // HLP // SECT | 3558375 | chr14 | - | 24169951 | 24173308 | 1.05 |
| cyclin L1 | CCNL1 | NM_020307 | BM-001 // PRO1073 // ania- | 2702307 | chr3 | - | 158305077 | 158361233 | 1.05 |
| neighbor of BRCA1 gene 1 | NBR1 | XM_001124650 // XM_001124827 // XM_001127781 // NM_005899 // NM_031858 // NM 031862 | 1A1-3B // KIAA0049 // M17S2 // MIG19 | 3722417 | chr17 | + | 38563761 | 38719231 | 1.05 |
| tyrosyl-rtRNA synthetase | YARS | NM_003680 | CMTDIC // TYRRS // YRS // YTS | 2405192 | chr1 | - | 33012453 | 33056331 | 1.05 |
| solute carrier family 25 (mitochondrial carrier, phosphate carrier), member 3 | SLC25A3 | NM_213612 // NM_002635 // NM_005888 // NM_213611 | OK/SW-cl.48 // PHC | 3427820 | chr12 | + | 97511471 | 97519906 | 1.05 |
| CCR4-NOT transcription complex, subunit 1 | CNOT1 | NM_016284 // NM_206999 | AD-005 // CDC39 // DKFZp686E0722 // FLJ90644 // KIAA1007 // NOT1 // NOT1H | 3693673 | chr16 | - | 57111094 | 57236550 | 1.05 |
| ankyrin repeat domain 13 family, member D | ANKRD13D | XM_001129739 // NM_207354 | MGC50828 | 3336857 | chr11 | + | 66812660 | 66826524 | 1.05 |
| PHD finger protein 10 | PHF10 | NM_018288 // NM_133325 | FLJ10975 // MGC111009 // XAP135 | 2986084 | chr6 | - | 169845929 | 169866056 | 1.05 |
| hypothetical protein KIAA1434 | RP5-1022P6.2 | NM_019593 | FLJ11085 // KIAA1434 // MGC26147 | 3896370 | chr20 | - | 5473033 | 5546357 | 1.05 |
| ribosomal protein 56 kinase, 90kDa, polypeptide 3 | RPS6KA3 | NM_004586 | CLS // HU-3 // ISPK-1 // MAPKAPK1B // MRX19 // RSK // RSK2 // S6K- alpha3 // p90- RSK2 // pp90RSK2 | 4002173 | chrX | - | 20077954 | 20254253 | 1.05 |
| UPF2 regulator of nonsense transcripts homolog (yeast) | UPF2 | NM_015542 // NM_080599 | DKFZP434D222 // HUPF2 // KIAA1408 // MGC138834 // MGC138835 // | 3277662 | chr10 | - | 11984615 | 12125155 | 1.05 |
| thioredoxin interacting protein | TXNIP | NM_006472 | EST01027 // HHCPA78 // THIF // VDUP1 | 2356115 | chr1 | + | 144149846 | 144164251 | 1.05 |
| protein phosphatase 1, regulatory (inhibitor) subunit 16B | PPP1R16B | NM_015568 | ANKRD4 // KIAA0823 // TIMAP | 3884830 | chr20 | + | 36866338 | 36985066 | 1.05 |
| peptidylprolyl isomerase F (cyclophilin F) | PPIF | NM_005729 | CYP3 // Cyp-D // FLJ90798 // MGC117207 | 3253880 | chr10 | + | 80777230 | 80785093 | 1.05 |
| 3-hydroxy-3-methylglutaryl-Coenzyme A reductase | HMGCR | NM_000859 | - | 2815965 | chr5 | + | 74668800 | 74700136 | 1.05 |
| chloride intracellular channel 1 | CUC1 | NM 001288 | G6 // NCC27 | 2949330 | chr6 | - | 31806365 | 31815144 | 1.05 |
| HBS1-like (S. cerevisiae) // aldehyde dehydrogenase 8 family, member A1 | HBS1L // ALDH8A1 | NM_006620 // NM_022568 // NM_170771 | DKFZp686L13262 // EF-1 a // ERFS // HBS1 // HSPC276 // ALDH12 // DJ352A20.2 // DKFZp779D2315 // | 2975287 | chr6 | - | 135314409 | 135417715 | 1.05 |
| vav 3 oncogene | VAV3 | NM.001079874 // NM_006113 | FLJ40431 | 2426385 | chr1 | - | 107914594 | 108309371 | 1.05 |
| Rho guanine nucleotide exchange factor (GEF) 7 | ARHGEF7 | NM_003899 // NM_145735 | BETA-PIX // COOL1 // DKFZp761K1021 // KIAA0142 // KIAA0412 // Nbla10314 // P50 // P50BP // P85 // P85COOL1 // P85SPR // PAK3 // PIXB | 3501661 | chr13 | + | 110565635 | 110756075 | 1.05 |
| SWI/SNF related, matrix associated, actin dependent regulator of chromatin, subfamily a, member 5 | SMARCA5 | NM_003601 | ISWI // SNF2H // WCRF135 // hISWI // hSNF2H | 2745646 | chr4 | + | 144616165 | 144697227 | 1.05 |
| proteasome maturation protein | POMP | NM_015932 | C13orf12 // HSPC014 // PNAS-110 // UMP1 | 3483348 | chr13 | + | 28125188 | 28151050 | 1.05 |
| DnaJ (Hsp40) homolog, subfamily B, member 6 | DNAJB6 | NM_005494 // NM_058246 | DKFZp566D0824 // FLJ42837 // HHDJ1 // HSJ-2 // HSJ2 // MGC1152 // MGC117297 II | 3034027 | chr7 | + | 156799667 | 156902887 | 1.05 |
| zinc finger RNA binding protein | ZFR | NM_016107 | FLJ41312 | 2852333 | chr5 | - | 32390101 | 32537319 | 1.05 |
| tetraspanin 14 | TSPAN14 | NM_030927 | DC-TM4F2 // MGC11352 // TM4SF14 | 3254521 | chr10 | + | 82203922 | 82272901 | 1.05 |
| myelin basic protein | MBP | NM_001025081 // NM_001025090 // NM_001025092 // NM_001025094 // NM_001025098 // NM_001025100 // NM_001025101 // NM 002385 | MGC99675 | 3814063 | chr18 | - | 72853742 | 73023063 | 1.05 |
| testis derived transcript (3 LIM domains) | TES | NM_015641 // NM_152829 | DKFZP58682022 // MGC1146 // TESS // TESS-2 // TESTIN | 3020192 | chr7 | + | 115637811 | 115743800 | 1.05 |
| microtubule-associated protein 1 light chain 3 beta | MAP1LC3B | NM_022818 | MAP1A/1BLC3 | 3672830 | chr16 | + | 85982917 | 85996864 | 1.05 |
| transcription factor binding to IGHM enhancer 3 | TFE3 | NM_006521 | RCCP2 // TFEA | 4007734 | chrX | - | 48771186 | 48787973 | 1.05 |
| CHMP family, member 7 | CHMP7 | NM_152272 | Mac2981 | 3089853 | chr8 | + | 23157105 | 23176288 | 1.05 |
| potassium channel tetramerisation domain containing 10 | KCTD10 | NM_031954 | FLJ41739 // MSTP028 // ULRO61 | 3470793 | chr12 | - | 108370845 | 108399528 | 1.05 |
| ets variant gene 6 (TEL oncogene) | ETV6 | NM_001987 | TEL // TEL/ABL | 3405032 | chr12 | + | 11645490 | 11939588 | 1.05 |
| programmed cell death 6 interacting protein | PDCD61P | NM_013374 | AIP1 // Alix // DRIP4 // HP95 // MGC17003 | 2616317 | chr3 | + | 33743952 | 33886195 | 1.05 |
| ras homolog gene family, member F (in filopodia) | RHOF | NM_019034 | ARHF // FLJ20247 // RIF | 3475324 | chr12 | - | 120698553 | 120716624 | 1.05 |
| lymphocyte cytosolic protein 2 (SH2 domain containing leukocyte protein of 76kDa) | LCP2 | NM_005565 | SLP-76 // SLP76 | 2886595 | chr5 | - | 169607157 | 169657789 | 1.05 |
| vitamin D (1,25- dihydroxyvitamin D3) receptor | VDR | NM_000376 II NM_001017535 | NR111 | 3452818 | chr12 | - | 46521596 | 46606823 | 1.05 |
| diablo homolog (Drosophila) | DIABLO | NM_019887 // NM_138929 // NM_138930 | DIABLO-S // FLJ10537 // FLJ25049 // SMAC // SMAC3 | 3475511 | chr12 | - | 121267461 | 121277963 | 1.05 |
| B-cell translocation gene 1, anti-proliferative | BTG1 | NM_001731 | - | 3465409 | chr12 | - | 90902887 | 91063960 | 1.05 |
| CD53 molecule | CD53 | NM_000560 // NM_001040033 | MOX44 // TSPAN25 | 2351572 | chr1 | + | 111215352 | 111296983 | 1.05 |
| damage-specific DNA binding protein 1, 127kDa | DDB1 | XM_001128974 // XM_001128983 // NM_001923 | DDBA // UV-DDB1 // XAP1 // XPCE // XPE // XPE-BF | 3375245 | chr11 | - | 60823513 | 60857553 | 1.05 |
| ariadne homolog 2 (Drosophila) | ARIH2 | NM_006321 | AR12 // FLJ10938 // FLJ33921 // TRIAD1 | 2621827 | chr3 | + | 48930742 | 48997971 | 1.05 |
| protein tyrosine phosphatase, non-receptor type 12 | PTPN12 | NM_002835 | PTP-PEST // PTPG1 | 3009959 | chr7 | + | 77004361 | 77123074 | 1.05 |
| meteorin, glial cell differentiation regulator-like // mediator of RNA polymerase Il transcription, subunit 25 homolog (S. cerevisiae) | METRNL // MED25 | XM_941466 11 NM_001004431 // NM_030973 | MGC99788 // ACID1 // ARC92 // DKFZp434K0512 // MGC70671 // P78 // TCBAP0758 | 3739431 | chr17 | + | 78630769 | 78653225 | 1.05 |
| trinucleotide repeat containing 6A | TNRC6A | NM_020847 // NM_014494 | CAGH26 // OKFZp666E117 // FLJ22043 // GW1 // GW182 // KIAA1460 // MGC75384 // TNRC6 | 3653398 | chr16 | + | 24583665 | 24748478 | 1.05 |
| ubiquitin specific peptidase 10 | USP10 | NM_005153 | K1AA0190 // MGC2621 // UBPO | 3671873 | chr16 | + | 83291080 | 83371023 | 1.05 |
| DnaJ (Hsp40) homolog, subfamily A, member 2 | DNAJA2 | NM_005880 | CPR3 // DJA2 // DNAJ // DNJ3 // HIRIP4 PRO3015 // RDJ2 | 3690084 | chr16 | - | 45546783 | 45565155 | 1.05 |
| MYC binding protein 2 | MYCBP2 | NM_015057 | DKFZp686M08244 // FLJ10106 // FLJ13826 // FLJ21597 // FLJ21646 // KIAA0916 // PAM | 3518496 | chr13 | - | 76500672 | 76799212 | 1.05 |
| SEC24 related gene family, member B (S. cerevisiae) | SEC24B | XM 001130118 // NM_001042734 II NM_006323 | MGC48822 // SEC24 | 2739079 | chr4 | + | 110510304 | 110681811 | 1.05 |
| interferon gamma receptor 2 (interferon gamma transducer 1) | IFNGR2 | NM_005534 | AF-1 // IFGR2 // IFNGT1 | 3918635 | chr21 | + | 33696906 | 33774607 | 1.05 |
| VAMP (vesicle-associated membrane protein)-associated protein A, 33kDa | VAPA | NM_003574 // NM_194434 | MGC3745 // VAP- 33 // VAP-A // VAP33 // hVAP-33 | 3778601 | chr18 | + | 9903984 | 9950012 | 1.05 |
| SP100 nuclear antigen | SP100 | NM_001080391 // NM_003113 | DKFZp686E07254 // FLJ00340 // FLJ34579 | 2531377 | chr2 | + | 230985008 | 231142598 | 1.05 |
| transforming growth factor beta regulator 1 | TBRG1 | No 032811 | FLJ14621 // FLJ25020 // FLJ90113 // MGC129890 // NIAM // TB-5 | 3354174 | chr11 | + | 123997906 | 124011654 | 1.05 |
| growth arrest and DNA-damage-inducible, beta | GADD45B | NM_015675 | DKFZP566B133 // GADD45BETA // MYD118 | 3816509 | chr19 | + | 2425718 | 2469133 | 1.05 |
| alanyl (membrane) aminopeptidase (aminopeptidase N, aminopeptidase M, microsomal aminopeptidase, CD13, p150) | ANPEP | NM_001150 | APN // CD13 // LAP1 // PEPN // gp150 | 3638607 | chr15 | - | 88129142 | 88159617 | 1.05 |
| proteasome (prosome, macropain) activator subunit 4 | PSME4 | NM_014614 | FLJ21864 // KIAA0077 // MGC138374 // MGC142228 // PA200 | 2553282 | chr2 | - | 53944710 | 54160919 | 1.05 |
| microtubule-associated protein, RP/EB family, member 1 | MAPRE1 | NM_012325 | EB1 // MGC117374 // MGC129946 | 3882069 | chr20 | + | 30871304 | 30901864 | 1.05 |
| BM11 polycomb ring finger oncogene | BMI1 | NM_005180 | MGC12685 // PCGF4 // RNF51 | 3238491 | chr10 | + | 22650103 | 22660820 | 1.05 |
| CUG triplet repeat, RNA binding protein 1 | CUGBP1 | NM_001025596 // NM_006560 // NM_198700 | BRUNOL2 // CUG- BP // CUGBP // NAB50 // hNab50 | 3372253 | chur11 | - | 47444068 | 47543613 | 1.05 |
| transforming, acidic coiled-coil containing protein 3 | TACC3 | NM_006342 | ERIC1 // MGC117382 // MGC133242 | 2714955 | chr4 | + | 1692616 | 1716693 | 1.05 |
| zinc finger, CCHC domain containing 6 II hypothetical protein MGC13114 | ZCCHC6 // MGC13114 | NM_024617 // NM_001040160 // NM_ 001040161 // NM_001040162 // NM_001040163 // NM_001040164 // NM_001040165 // NM_001040166 // NM_032366 | DKFZp666B142 // DKFZp686C11112 // DKFZp686F119 // OKFZp68611269 // PAPD6 // JFP2 | 3212976 | chr9 | - | 88092469 | 88159805 | 1.05 |
| C-type lectin domain family 4, member E | CLEC4E | NM_014358 | CLECSF9 // MINCLE | 3443183 | chr12 | - | 8577026 | 8584811 | 1.05 |
| interferon, gamma-inducible protein 16 | IF116 | NM_005531 | 1FNGIP1 // MGC9466 // PYHIN2 | 2362394 | chr1 | + | 157215279 | 157291561 | 1.05 |
| LMBR1 domain containing 1 | LMBRD1 | NM_018368 | C6orf209 // FLJ11240 // RP11- 810122.1 // bA810122.1 | 2960010 | chr6 | - | 70361077 | 70634121 | 1.05 |
| protein kinase D3 | PRKD3 | NM_005813 | EPK2 // PKC-NU // PKD3 // PRKCN // nPKC-NU | 2548500 | chr2 | - | 37331165 | 37405413 | 1.05 |
| CCR4-NOT transcription complex, subunit 7 | CNOT7 | NM_013354 // NM_054026 | CAF1 // hCAF-1 | 3125775 | chr8 | - | 17128912 | 17148758 | 1.05 |
| heterogeneous nuclear ribonucleoprotein M | HNRPM | NM_005968 // NM_031203 | DKFZp547H118 // HNRNPM // HNRNPM4 // HNRPM4 // HTGR1 // NAGR1 | 3819543 | chr19 | + | 8415651 | 8459993 | 1.05 |
| heterogeneous nuclear ribonucleoprotein H1 (H) | HNRPH1 | NM_005520 | DKFZp686A15170 // HNRPH // | 2890148 | chr5 | - | 178970155 | 178993890 | 1.05 |
| adenosine A2a receptor | ADORA2A | NM_000675 | ADORA2 // RDC8 // hA2aR | 3940099 | chr22 | + | 23143709 | 23200243 | 1.05 |
| PPAR binding protein | PPARBP | NM_004774 | CRSP1 // CRSP200 // DR1P205 // DRIP230 // MED1 // MGC71488 // PBP // PPARGBP // RB18A // | 3755714 | chr17 | - | 34801544 | 34861069 | 1.05 |
| serine/threonine kinase 17b | STK17B | NM_004226 | DRAK2 | 2593159 | chr2 | - | 196706552 | 196744596 | 1.05 |
| adhesion regulating molecule 1 | ADRM1 | NM_007002 // NM_175573 | GP110 // MGC29536 // | 3892607 | chr20 | + | 60305485 | 60317305 | 1.05 |
| splicing arginine/serine-rich 9 | SFRS9 | NM_003769 | SRp30c | 3474502 | chr12 | - | 119372834 | 119407274 | 1.05 |
| factor, E2F transcription factor 4, p107/p130-binding | E2F4 | NM_001950 | E2F-4 | 3665288 | chr16 | + | 65783266 | 65790299 | 1.05 |
| chromodomain helicase DNA binding protein 2 | CHD2 | NM_001042572 // NM_001271 | DKFZp781D1727 | 3609138 | chr15 | + | 91227096 | 91387351 | 1.05 |
| serpin peptidase inhibitor, clade B (ovalbumin), member 2 | SERPINB2 | NM_002575 | HsT1201 // PAI // PAI-2 // PAI2 // PLANH2 | 3791935 | chr18 | + | 59704265 | 59722138 | 1.05 |
| phosphatidylinositol 3,4,5-trisphosphate-dependent RAC exchanger 1 | PREX1 | NM_020820 | KIAA1415 | 3908631 | chr20 | - | 46674205 | 46938090 | 1.05 |
| pre-B-cell colony enhancing factor 1 // pre-B cell enhancing factor 1 pseudogene | PBEF1 // RP11-92J19.4 | NM_005746 // - | 1110035014Rik // DKFZP666B131 // MGC117256 // NAMPT // PBEF // LOC646309 | 3066818 | chr7 | - | 105625846 | 105713266 | 1.05 |
| interferon regulatory factor 1 | IRF1 | NM_00219B | IRF-1 // MAR | 2875348 | chr5 | - | 131846280 | 131893043 | 1.05 |
| nuclear receptor coactivator 4 | NCOA4 | NM_005437 | ARA70 // DKFZp762E1112 // ELE1 // PTC3 // RFG | 3246372 | chr10 | + | 51202191 | 51260947 | 1.05 |
| adrenergic, beta, receptor kinase 1 | ADRBK1 | M_001619 | BARK1 // BETA- ARK1 // GRK2 | 3336801 | chr11 | + | 66790523 | 36810933 | 1.05 |
| BTAF1 RNA polymerase II, B-TFUD transcription factor-associated, 170kDa (Mot1 homolog, S. cerevisiae) | BTAF1 | NM_003972 | KIAA0940 // MGC138406 // MOT1 // TAF(II)170 // TAF172 // TABII170 | 3257938 | chr10 | + | 93673509 | 93821994 | 1.05 |
| golgi autoantigen, golgin subfamily a, 4 | GOLGA4 | NM_002078 | GCP2 // GOLG // MU-RMS-40.18 // p230 | 2617041 | chr3 | + | 37259518 | 37452233 | 1.05 |
| ras homolog gene family, member A | RHOA | NM_001664 | ARH12 // ARHA // RHO12 // RHOH12 | 2674242 | chr3 | - | 49371587 | 49424514 | 1.05 |
| regulatory factor X, 5 (influences HLA class II | RFX5 | NM_000449 // NM_001025603 | - | 2434971 | chr1 | - | 149578882 | 149586373 | 1.05 |
| expression) protein phosphatase 2 (formerly 2A), regulatory subunit B, alpha isoform | PPP2R2A | NM_002717 | B55A // FLJ26613 // MGC52248 // PR52A // PR55A | 3090922 | chr8 | + | 26156860 | 26286261 | 1.05 |
| chromosome 20 open reading frame 11 | C20orf11 | NM_017896 | TWA1 | 3893072 | chr20 | + | 61039681 | 61050570 | 1.05 |
| PCI domain containing 2 | PCID2 | NM_018386 | F10 // FLJ11305 // MGC 16774 | 3526378 | chr13 | - | 112877970 | 112912799 | 1.05 |
| lymphocyte antigen 9 // SLAM family member 7 | LY9 // SLAMF7 | NM_001033667 11 // NM_002348 // NM_021181 | CD229 // SLAMF3 // hly9 // mLY9 // 19A // CD319 // CRACC // GS1 | 2363248 | chr1 | + | 159032527 | 159064855 | 1.05 |
| arrestin domain containing 3 | ARRDC3 | NM_020801 | KIAA1376 // TLIMP | 2866704 | chr5 | - | 90700311 | 90782084 | 1.05 |
| chromosome 14 open reading frame 103 | C14orf103 | NM_018036 | FLJ10242 | 3578278 | chr14 | - | 95817349 | 95900740 | 1.05 |
| myosin IXB | MYO9B | NM_004145 | CELIAC4 // MYR5 | 3823982 | chr19 | + | 17047591 | 17185088 | 1.05 |
| t-complex 1 | TCP1 | NM_001008897 // NM_030752 | CCT-alpha // CCT1 // CCTa // D6S230E // TCP- 1-alpha | 2982381 | chr6 | - | 160119521 | 160130731 | 1.05 |
| translocation protein 1 | TLOC1 | NM_003262 | Dtrp1 // FLJ32803 // HTP1 // SEC62 | 2651782 | chr3 | + | 171166446 | 171194653 | 1.05 |
| far upstream element (FUSE) binding protein 1 | | | FBP // FUBP | 2419235 | chr1 | - | 78182330 | 78217881 | 1.05 |
| mannosidase, alpha, class 1A, member 2 // urothelial cancer associated 1 | FUBP1 MAN1A2 // UCA1 | NM_003902 NM_006699 // - | MAN1B //- | 2353881 | chr1 | + | 117689854 | 117931990 | 1.05 |
| engulfment and cell motility 2 | ELMO2 | NM_133171 // NM_182764 | CED-12 // CED12 // ELMO-2 // FLJ11656 // KIAA1834 | 3907830 | chr20 | - | 44426906 | 44495031 | 1.05 |
| erbb2 interacting protein // caspase 6, apoptosis-related cysteine peptidase | ERBS21P // CASP6 | NM_001006600 // NM_018695 // NM_001226 // NM_032992 | ERBIN // LAP2 // MCH2 | 2812435 | chr5 | + | 65258079 | 65451372 | 1.05 |
| heterogeneous nuclear ribonucleoprotein R | HNRPR | NM_005826 | FLJ25714 // HNRNPR // hnRNP-R | 2401275 | chr1 | - | 23486545 | 23543926 | 1.05 |
| membrane-bound transcription factor peptidase, site 1 | MBTPS1 | NM_003791 // NM_201268 | KIAA0091 // MGC138711 // MGC 138712 // PCSK8 // S1P // SKI-1 | 3702293 | chr16 | - | 82644885 | 82708018 | 1.05 |
| RAB35, member RAS oncogene family | RAB35 | NM_006861 | H-ray // RAB1C // RAY | 3474228 | chr12 | - | 119017289 | 119039689 | 1.05 |
| famesyl-diphosphate farnesyltransferase 1 | FDFT1 | NM_004462 | DGPT // ERG9 // SQS // SS | 3086206 | chr8 | + | 11690497 | 11740987 | 1.05 |
| SATB homeobox 1 | SATB1 | NM_002971 | | 2665199 | chr3 | - | 18364435 | 18462086 | 1.05 |
| RAS guanyl releasing protein 1 (calcium and DAG-regulated) | RASGRP1 | NM_005739 | CALDAG-GEFI// CALDAG-GEFII // MGC129998 // MGC129999 // RASGRP // V // hRasGRP1 | 3618736 | chr15 | - | 36567598 | 36809973 | 1.05 |
| interferon-related developmental regulator 1 | IFRD 1 | NM_001007245 // NM_001550 | PC4 // TIS7 | 3019519 | chr7 | + | 111835465 | 111903788 | 1.05 |
| coatomer protein complex, subunit alpha // peroxisomal biogenesis factor 19 | COPA // PEX19 | NM_004371 // NM_002857 | FLJ26320 // HEP- COP // D1S2223E // HK33 // PMP1 // PMPI // PXF // PXMP1 | 2440143 | chr1 | - | 158525015 | 158580073 | 1.05 |
| SCY1-like 2 (S. cerevisiae) | SCYL2 | NM_017988 | CVAK104 // FLJ10074 // KIAA1360 | 3428131 | chr12 | + | 99185070 | 99260564 | 1.05 |
| Ctr9, Paf1/RNA polymerase II complex component, homolog (S. cerevisiae) // tRNA 5-methylaminomethyl-2-thiouridylate methyltransferase | CTR9 // TRMU | NM_014633 // NM_018006 | KIAA0155 // SH2BP1 // TSBP // p15D // p150TSP // MGC99627 // MTO2 // MTU1 // TRMT // TRMT1 // | 3320301 | chr11 | + | 10684027 | 10774985 | 1.05 |
| serine/threonine kinase 40 // erythrocyte membrane protein band 4.1 (elliptocytosis 1, RH-linked) | STK40 // EPB41 | NM_032017 // NM_004437 // NM_ 203342 // NM_ 203343 | MGC4796 // RP11- 268J15.4 // SHIK // SgK495 // 4.1R // EL1 // HE | 2406677 | chr1 | - | 36577822 | 36626062 | 1.05 |
| heterogeneous nuclear ribonucleoprotein U (scaffold attachment factor A) | HNRPU | NM_ 004501 // NM_031844 | HNRNPU // SAF-A // U21.1 | 2464499 | chr1 | - | 243078713 | 243094575 | 1.05 |
| REST corepressor 1 | RCOR1 | NM_015156 | COREST // KIAA0071 // RCOR | 3553228 | chr14 | + | 102053947 | 102266629 | 1.05 |
| CREB binding protein (Rubinstein-Taybi syndrome) | CREBBP | NM_001079846 // NM_004380 | CBP // RSTS // RTS | 3677795 | chr16 | - | 3715075 | 3870713 | 1.05 |
| golgi apparatus protein 1 // CDC42 small effector 1 // sema domain, transmembrane domain (TM), and cytoplasmic domain, (semaphorin) 6C | GLG1 // CDC42SE1 // SEMA6C | NM_012201 // NM_001038707 // NM_020239 // NM_030913 | CFR-1 // ESL-1 // FLJ23319 // FLJ23967 // MG- 160 // MG160 // SCIP1 // SPEC1 // SEMAY // m-Sema Y // m-Sema Y2 | 3698919 | chr16 | - | 73015604 | 73209803 | 1.05 |
| PHD finger protein 3 | PHF3 | NM_015153 | KIAA0244 // MGC142210 // MGC142212 | 2911944 | chr6 | + | 64403666 | 64547188 | 1.05 |
| WDR45-like | WDR45L | NM 019613 | WIPI-3 // WIPI3 | 3775157 | chr17 | - | 78165748 | 78199803 | 1.05 |
| HMG-box transcription factor 1 | HBP1 | NM_012257 | FLJ16340 | 3018420 | chr7 | + | 106596679 | 106630194 | 1.05 |
| chromosome 2 open reading frame 25 | C2orf25 | M_015702 | CL25022 | 2580635 | chr2 | - | 150101637 | 150152607 | 1.05 |
| heat shock 70kDa protein 8 // Fc fragment of IgG, low affinity IIIb, receptor (CD16b) | HSPA8 // FCGR3B | NM_006597 // NM_153201 // NM_000570 | HSC54 // HSC70 // HSC71 // HSP71 // HSP73 // HSPA10 // LAP1 // MGC131511 // MGC29929 // NIP71 // CD16 // | 3395416 | chr11 | - | 122429309 | 122438895 | 1.05 |
| chromosome 6 open reading frame 62 | C6orf62 | NM_030939 | DKFZP564G182 // FLJ12619 // Nbla00237 // XTP12 // dJ30M3.2 | 2945677 | chr6 | - | 24798431 | 24851423 | 1.05 |
| leptin receptor overlapping transcript-like 1 | LEPROTL1 | NM_015344 | HSPC112 // Vps55 // my047 | 3092276 | chr8 | + | 30059542 | 30115386 | 1.05 |
| ubiquitin-conjugating enzyme E21 (UBC9 homolog, yeast) | UBE21 | NM_003345 // NM_194259 // NM_194260 // NM_194261 | C35887,1 // P18 // UBC9 | 3643703 | chr16 | + | 1289451 | 1317016 | 1.05 |
| nucleoporin 155kDa | NUP155 | NM_004298 // NM_153485 | KIAA0791 // N155 | 2853768 | chr5 | - | 37325714 | 37407016 | 1.05 |
| lysosomal trafficking regulator | LYST | NM_000081 // NM_001005736 | CHS // CHS1 | 2461999 | chr1 | - | 233878999 | 234113611 | 1.05 |
| mitogen-activated protein kinase 14 | MAPK14 | NM_001315 // NM_139012 // NM_139013 // NM_139014 | CSBP1 // CSBP2 // CSPB1 // EXIP // Mxi2 // PRKM14 // PRKM15 // RK // SAPK2A // p38 // p38ALPHA | 2904877 | chr6 | + | 36103487 | 36186989 | 1.05 |
| nuclear factor (erythroid-derived 2)-like 2 // hypothetical protein DKFZp451M2119 | NFE2L2 // DKFZp451 M21 19 | NM_006164 // NM_182585 | NRF2 // - | 2588827 | chr2 | - | 177794809 | 177837753 | 1.05 |
| WD repeat domain 26 | WDR26 | NM_025160 | FLJ21016 // MIP2 | 2458082 | chr1 | - | 222639474 | 222691338 | 1.05 |
| heterogeneous nuclear ribonucleoprotein K | HNRPK | NM_002140 // NM_031262 // NM_031263 | CSBP // FLJ41122 // HNRNPK // TUNP | 3212294 | chr9 | - | 85772377 | 85785355 | 1.05 |
| transducin-like enhancer of split 4 (E(sp1) homolog, Drosophila) | TLE4 | NM_007005 | BCE-1 // E(sp1) // ESG // ESG4 // GRG4 | 3176209 | chr9 | + | 81269283 | 81552230 | 1.05 |
| bromodomain containing 2 | BRD2 | NM_005104 | D6S113E // DKFZp686N0336 // FLJ31942 // FSRG1 // KIAA9001 // NAT // RING3 // RNF3 | 2903343 | chr6 | + | 33044392 | 33057253 | 1.05 |
| signal transducer and activator of transcription 3 (acute-phase response factor) | STAT3 | NM_003150 // NM_139276 // NM_213662 | APRF // FLJ20882 // MGC16063 | 3757840 | chr17 | - | 37718873 | 37794201 | 1.05 |
| ariadne homolog, ubiquitan-conjugating enzyme E2 binding protein, 1 (Drosophila) | ARIH1 | NM_005744 | ARI // HARI // HHARI // | 3600744 | chr15 | + | 70553731 | 70666726 | 1.05 |
| zinc finger, NFX1-type containing 1 // gasdermin-like | ZNFX1 // GSDML | NM_021035 // NM_001042471 // NM 018530 | FLJ39275 // MGC131926 // PP4052 // | 3908831 | chr20 | - | 47233911 | 47420063 | 1.05 |
| SUMO1 / sentrin specific peptidase 6 | SENP6 | NM_015571 | FLJ11355 // FLJ11887 // KIAA0389 // KIAA0797 // SSP1 // SUSP1 | 2913983 | chr6 | + | 76367955 | 76484717 | 1.05 |
| DEAD (Asp-Glu-Ala-Asp) box polypeptide 17 | DDX17 | NM_006386 // NM_030881 | DKFZp761 H2016 // P72 // RH70 | 3960629 | chr22 | - | 37202800 | 37232288 | 1.05 |
| ubiquitin specific peptidase 15 | USP15 | NM_006313 | KWA0529 // MGC131982 // MGC149838 // MGC74854 // UNPH4 | 3419147 | chr12 | + | 60940328 | 61086166 | 1.05 |
| Ras association (RaIGDS/AF-6) domain family 2 | RASSF2 | NM_014737 // NM 170774 | DKFZp78101747 // KIAA0168 | 3896034 | chr20 | - | 4655529 | 4752291 | 1.05 |
| dual specificity phosphatase 6 | DUSP6 | NM_001946 // NM_022652 | MKP3 // PYST1 | 3464860 | chr12 | - | 88223376 | 88273467 | 1.05 |
| E1A binding protein p300 | EP300 | NM_001429 | p300 | 3946615 | chr22 | + | 39817736 | 39906025 | 1.05 |
| solute carrier family 7 (cationic amino acid transporter, y+ system), member 6 | SLC7A6 | NM_001076785 // NM_003983 | DKFZp686K15246 // KIAA0245 // LAT-2 // LAT3 // y+LAT-2 | 3666146 | chr16 | + | 66855932 | 66893223 | 1.05 |
| RNA binding motif protein 5 | RBM5 | NM_005778 | FLJ39876 // G15 // H37 // LUCA15 // RMB5 | 2622469 | chr3 | + | 50101372 | 50134000 | 1.05 |
| nuclear protein localization 4 homolog (S. cerevisiae) | NPLOC4 | NM_017921 | FLJ20657 // FLJ23742 // KIAA 1499 // NPL4 | 3774029 | chr17 | - | 77134363 | 77214526 | 1.05 |
| cyclin L2 // aurora kinase A interacting protein 1 | CCNL2 // AURKAIP1 | NM 001039577 // NM_030937 // NM_017900 | ANIA-6B // DKFZp761A1210 // DKFZp7620195 // HCLA-ISO // HLA- ISO // PCEE // SB138 // AIP // AKIP // FLJ20608 | 4041923 | chr1_ra ndom | + | 359569 | 372958 | 1.05 |
| poly(rC) binding protein 2 | PCBP2 | NM_005016 // NM_031989 | HNRPE2 // MGC110998 // hnRNP-E2 | 3416036 | chr12 | + | 52132173 | 52161417 | 1.05 |
| aftiphilin | AFTPH | NM_001002243 // NM_017657 // NM_203437 | FLJ20080 // FLJ23793 // MGC33965 // Nbla10388 | 2485433 | chr2 | + | 64604969 | 64714437 | 1.05 |
| GTP binding protein 1 | GTPBP1 | NM_004286 | GP-1 // GP1 // HSPC018 // MGC20069 | 3945396 | chr22 | + | 37431694 | 37459514 | 1.05 |
| protein kinase C, eta | PRKCH | NM_006255 | MGC26269 // MGC5363 // PKC- L // PKCL // PRKCL // nPKC- | 3538893 | chr14 | + | 60816958 | 61087440 | 1.05 |
| glutaminase | GLS | NM_014905 | DKFZp686015119 // FLJ 10358 // GLS1 // KIAA0838 | 2520291 | chr2 | + | 191453802 | 191538513 | 1.05 |
| tubulin, beta | TUBB | NM_178014 | M40 // MGC117247 // MGC16435 // OK/SW-cl.56 // TUBB1 TUBB5 | 2901913 | chr6 | + | 30795977 | 30801165 | 1.05 |
| neuroguidin, EIF4E binding protein | NGDN | XM_033371 // XM_932898 // XM_932900 // XM_932903 // XM_932906 // XM_941350 // XM_945161 // XM_945163 // XM_945166 // XM 945170 // NM_ 001042635 // NM_015514 | C14orf120 // DKFZP5640092 // LCP5 // NGD // Ipd-2 | 3529156 | chr14 | + | 23008821 | 23048896 | 1.05 |
| zinc finger, RAN-binding domain containing 2 | ZRANB2 | NM_005455 // NM_203350 | DKFZp686J1831 DKFZp686N09117 // FLJ41119 // ZIS // ZIS1 // ZIS2 // ZNF265 | 2418000 | chr1 | - | 71292434 | 71355820 | 1.05 |
| DEAD (Asp-Glu-Ala-Asp) box polypeptide 21 | DDX21 | NM_004728 | DKFZp686F21172 // GUA // GURDB // RH-II/GU // RH-II/GuA | 3250055 | chr10 | + | 70380584 | 70414821 | 1.05 |
| sorting nexin 19 | SNX19 | NM_014758 | CHET8 // DKFZp6671205 // KIAA0254 | 3398482 | chr11 | - | 130250320 | 130291615 | 1.05 |
| phosphatidylinositol binding clathrin assembly protein | PICALM | NM_001008660 // NM_007166 | CALM // CLTH // LAP | 3385175 | chr11 | - | 85345353 | 85458481 | 1.05 |
| PI-3-kinase-related kinase SMG-1 | SMG1 | NM_015092 | 61E3.4 // ATX // KIAA0421 // LIP | 3683050 | chr16 | - | 18719381 | 18845318 | 1.05 |
| MON2 homolog (S. cerevisiae) | MON2 | NM_015026 | KIAA1040 // MGC35493 | 3419239 | chr12 | + | 61146751 | 61280506 | 1.05 |
| signal recognition particle receptor ('docking protein') | SRPR | NM_003139 | DP // MGC17355 // MGC3650 // MGC9571 // SRP- alpha // Sralpha | 3396916 | chr11 | - | 125636504 | 125644013 | 1.05 |
| heat shock protein 90kDa alpha (cytosolic), class B member 1 | HSP90AB1 | NM_007355 | D6S182 // FLJ26984 // HSP90-BETA // HSP90B // HSPC2 // HSPCB | 2908474 | chr6 | + | 44319983 | 44330383 | 1.05 |
| eukaryotic translation initiation factor 5 | EIF5 | NM_001969 // NM_183004 | EIF-5A | 3553607 | chr14 | + | 102743652 | 102881108 | 1.05 |
| patatin-like phospholipase domain containing 10 pseudogene // patatin-like phospholipase domain containing 8 | PNPLA10P // PNPLA8 | XM_927062 // XM_938392 // NM_015723 | - // IPLA2(GAMMA) // IPLA2-2 // IPLA2G | 3067592 | chr7 | - | 107880325 | 107956012 | 1.05 |
| 2'-5'-oligoadenylate synthetase 3, 100kDa | OAS3 | NM_006187 | MGC133260 // | 3432467 | chr12 | + | 111860550 | 111895663 | 1.05 |
| aminopeptidase puromycin sensitive | NPEPPS | XM_001128588 // NM_006310 | MP100 // PSA | 3724698 | chr17 | + | 42955347 | 43057168 | 1.05 |
| SAP30 binding protein // cardiotrophin-like cytokine factor 1 | SAP30BP // CLCF1 | NM_013260 // NM_013246 | DKFZp586L2022 // HCNGP // HTRG // HTRP // BSF3 // CISS2 // CLC // NNT1 // NR6 | 3735107 | chr17 | + | 71174801 | 71215729 | 1.05 |
| bromodomain adjacent to zinc finger domain, 1B | BAZ1B | NM_023005 // NM_032408 | WBSCR10 // WBSCR9 // WSTF | 3056044 | chr7 | - | 72492710 | 72574552 | 1.05 |
| cullin 3 | CUL3 | NM_003590 | | 2601544 | chr2 | - | 225025520 | 225158348 | 1.05 |
| DEAD (Asp-Glu-Ala-Asp) box polypeptide 42 | DDX42 | NM_007372 // NM_203499 | FLJ43179 // RHELP // RNAHP // SF3b125 | 3730899 | chr17 | + | 59204975 | 59250510 | 1.05 |
| SWI/SNF related, matrix associated, actin dependent regulator of chromatin, subfamily a, member 2 | SMARCA2 | NM_003070 // NM_139045 | BAF190 // BRM // FLJ36757 // MGC74511 // SNF2 // SNF2L2 // SNF2LA // SWI2 // Sth1p // hBRM // hSNF2a | 3159946 | chr9 | + | 1968220 | 2253060 | 1.05 |
| stress-induced-phosphoprotein 1 (Hsp70/Hsp90-organizing protein) | STIP1 | NM_006819 | HOP // IEF-SSP- 3521 // P60 // STI1L | 3334224 | chr11 | + | 63709341 | 63728586 | 1.05 |
| splicing factor, arginine/serine-rich 7, 35kDa | SFRS7 | NM_001031684 | 9G8 // AAG3 // HSSG1 // RBM37 // ZCCHC20 // ZCRB2 | 2548970 | chr2 | - | 38824253 | 38832154 | 1.05 |
| ribosomal protein L14 // ribosomal protein L14-like | RPL14 // RPL14L | NM_001034996 // NM_003973 // XR_017789 // XR-017790 | CAG-ISL-7 // CTG-B33 // L14 // MGC88594 // RL14 // hRL14 // bcm1298 | 2618640 | chr3 | + | 40473807 | 40485068 | 1.05 |
| acidic (leucine-rich) nuclear phosphoprotein 32 family, member C // acidic (leucine-rich) nuclear phosphoprotein 32 family, member A | ANP32C // ANP32A | NM_012403 // NM_006305 | PP32R1 // C15orf1 // IIPP2A // LANP // MAPM // MGC119787 // MGC150373 // PHAP1 // PHAPI // PP32 | 3630912 | chr15 | - | 66857944 | 66900452 | 1.05 |
| endoglin (Osler-Rendu-Weber syndrome 1) | ENG | NM_000118 | CD105 // END // FLJ41744 // HHT1 // ORW // ORW1 | 3226097 | chr9 | - | 129615329 | 129660473 | 1.05 |
| lysosomal associated multispanning membrane protein 5 | LAPTM5 | NM_006762 | MGC125860 // MGC125861 | 2404158 | chr1 | - | 30977913 | 31095913 | 1.05 |
| family with sequence similarity 108, member A2 // family with sequence similarity 108, member A1 | FAM108A2 // FAM108A1 | XM_039721 // XM_933830 // NM_001080422 // NM_031213 | C1orf47 // C19orf27 // MGC5244 | 3845581 | chr19 | - | 1827984 | 1836490 | 1.05 |
| interleukin 16 (lymphocyte chemoattractant factor) | IL16 | NM_004513 // NM_172217 | FLJ16806 // FLJ42735 // FLJ44234 // HsT19289 // IL-16 // LCF // prIL-16 | 3604287 | chr15 | + | 79262148 | 79392377 | 1.05 |
| SEC14 and spectrin domains 1 | SESTD1 | NM_178123 | DKFZp43400515 // SOLO | 2589929 | chr2 | - | 179644888 | 180002479 | 1.05 |
| coatomer protein complex, subunit beta 2 (beta prime) | COPB2 | NM_004766 | beta-COP | 2697792 | chr3 | - | 140555482 | 140591192 | 1.05 |
| Finkel-Biskis-Reilly murine sarcoma virus (FBR-MuSV) ubiquitously expressed (fox derived); ribosomal protein S30 | FAU | NM_001997 | FAU1 // RPS30 | 3377463 | chr11 | - | 64644697 | 64646234 | 1.05 |
| AHA1, activator of heat shock 90kDa protein ATPase homolog 1 (yeast) | AHSA1 | M_012111 | AHA1 //C14 or f3 // p38 | 3545466 | chr14 | + | 76993989 | 77005560 | 1.05 |
| SLU7 splicing factor homolog (S. cerevisiae) | SLU7 | NM_006425 | 9G8 // MGC9280 // hSlu7 | 2884658 | chr5 | - | 159761226 | 159778959 | 1.05 |
| multiple C2 domains, transmembrane 1 | MCTP1 | NM_001002796 // NM_024717 | FLJ22344 | 2867443 | chr5 | - | 94066156 | 94646035 | 1.05 |
| ubiquitin-conjugating enzyme E2Z (putative) | UBE2Z | NM 023079 | FLJ13855 // | 3725481 | chr17 | + | 44340808 | 44361870 | 1.05 |
| hypothetical protein MGC40489 | MGC40489 | XR_015622 // XR_016048 | FLJ30780 | 3767053 | chr17 | - | 60176255 | 60263758 | 1.05 |
| ATG9 autophagy related 9 homolog A (S. cerevisiae) // ATP-binding cassette, sub-family B (MDR/TAP), member 6 | ATG9A // ABCB6 | NM_ 001077198 // NM_024085 // NM_005689 | APG9L1 // MGD3208 // ABC // ABC14 // EST45597 // FLJ22414 // MTABC3 // PRP // umat | 2599955 | chr2 | - | 219781799 | 219802587 | 1.05 |
| isoleucyl-tRNA synthetase | LARS | NM_002161 // NM_013417 | FLJ20736 // IARS1 // ILRS // PRO0785 | 3214668 | chr9 | - | 94012330 | 94096287 | 1.05 |
| LIM domain binding 1 | LDB1 | NM 003893 | CUM2 // NLI | 3304215 | chr10 | - | 103830793 | 103883389 | 1.05 |
| YY1 transcription factor | YY1 | NM_003403 | DELTA // NF-E1 // UCRBP // YIN- YANG-1 | 3551677 | chr14 | + | 99750383 | 99818868 | 1.05 |
| WD repeat domain, phosphoinositide interacting 2 | WIP12 | NM_001033518 // NM_001033519 // NM_001033520 // NM_015610 // NM_ 016003 | Atg21 // CGI-50 // DKFZP434J154 // DKFZp686P02188 // FLJ12979 // FLJ14217 // FLJ42984 // WIPI-2 | 2988536 | chr7 | + | 5196360 | 5239975 | 1.05 |
| BCL2-associated transcription factor 1 | BCLAF1 | NM_001077440 // NM_001077441 // NM 014739 | BTF // MAA0164 // bK211L9.1 | 2975680 | chr6 | - | 136619703 | 136652847 | 1.05 |
| ADAM metallopeptidase domain 19 (meltrin beta) | ADAM19 | NM_023038 // NM_033274 | FKSG34 // MADDAM // | 2883440 | chr5 | - | 156755122 | 156935351 | 1.05 |
| RAE1 RNA export 1 homolog (S. pombe) | RAE1 | NM_ 001015885 // NM_003610 | FLJ30608 // MGC117333 // MGC126076 // MGC126077 // MIG14 // MRNP41 // Mnrp41 // dJ481 F12.3 // dJ800J21.1 | 3890555 | chr20 | + | 55359493 | 55386992 | 1.05 |
| cyclin L2 // aurora kinase A interacting protein 1 | CCNL2 // AURKAIP1 | NM_001039577 // NM_030937 // NM_017900 | ANIA-6B // DKFZp761A1210 // DKFZp7620195 // HCLA-ISO // HLA- ISO // PCEE // SB138 // AIP // AKIP // FLJ20608 | 2391532 | chr1 | - | 1314609 | 1324575 | 1.05 |
| nucleolar and coiled-body phosphoprotein 1 | NOLC1 | NM_004741 | KIAA0035 // NOPP130 // NOPP140 // NS5ATP13 // P130 | 3261492 | chr10 | + | 103901962 | 103913597 | 1.05 |
| B-cell CLL/lymphoma 11B (zinc finger protein) | BCL11B | NM_022898 // NM_138576 | CTIP-2 // CTIP2 // RIT1 // hRIT1- | 3579114 | chr14 | - | 98705377 | 98931503 | 1.05 |
| proteasome (prosome, macropain) 26S subunit, non-ATPase, 7 (Mov34 homology) | PSMD7 | NM_002811 | MOV34 // P40 // S12 | 3668617 | chr16 | + | 72888182 | 72901528 | 1.05 |
| chromosome 14 open reading frame 118 | C14orf118 | NM_017926 // NM_017972 | FLJ10033 // FLJ20689 // MGC61896 | 3544905 | chr14 | + | 75688015 | 75800924 | 1.05 |
| translocase of inner mitochondrial membrane 23 homolog (yeast) | TIMM23 | XM_001133798 // NM_006327 | MGC22767 // PRO11197 // TIM23 // TIMM23B | 3289031 | chr10 | - | 51180787 | 51293382 | 1.05 |
| proteasome (prosome, macropain) 26S subunit, non-ATPase, 2 | PSMD2 | NM_002808 | MGC14274 // P97 // S2 // TRAP2 | 2655650 | chr3 | + | 185499199 | 185509504 | 1.05 |
| LSM14A, SCD6 homolog A (S. cerevisiae) | LSM14A | NM_015578 | C19 or f 13 // DKFZP434D1335 // FAM61 A // RAP55 | 3829575 | chr19 | + | 39355192 | 39416345 | 1.05 |
| phosphatidylinositol transfer protein, alpha // myosin IC | PITPNA // MYO1C | NM_006224 // NM_001080779 // NM_001080950 // NM_033375 | MGC99649 // PITPN // VIB1A // FLJ23903 // MMI- beta // MMIb // NMI // myr2 | 3740304 | chr17 | - | 1367883 | 1412932 | 1.05 |
| centaurin, delta 1 | CENTD1 | NM_015230 // NM_139182 | ARAP2 // FLJ13675 // FLJ44916 // | 2765590 | chr4 | - | 35626248 | 35922356 | 1.05 |
| structural maintenance of chromosomes 1 A | SMC1A | NM_006306 | DKFZp686L19178 // DXS423E // KIAA0178 // MGC138332 // SB1.8//SMC1 // SMC1L1 // SMC1alpha // | 4009238 | chrX | - | 53417797 | 53466400 | 1.05 |
| solute carrier family 43, member 3 | SLC43A3 | NM_014096 // NM_017611 // NM_199329 | DKFZp762A227// EEG1 // FOAP-13 // PRO1659 // SEEEG-1 | 3373845 | chr11 | - | 56931008 | 56951629 | 1.04 |
| ninjurin 1 | NINJ1 | NM_004148 | NIN1 // NINJURIN | 3215146 | chr9 | - | 94923613 | 94939639 | 1.04 |
| CDC-like kinase 3 | CLK3 | NM_001292 // NM_003992 | FLJ22858 | 3601741 | chr15 | + | 72677906 | 72719105 | 1.04 |
| sortilin-related receptor, L(DLR class) A repeats-containing | SORL1 // C11orf32 | NM_003105 | LR11 // LRP9 // SORLA // SorLA-1 // gp250 | 3352948 | chr11 | + | 120828130 | 121077997 | 1.04 |
| KIAA0831 | KIAA0831 | NM_014924 | MGC126291 // MGC126292 | 3565739 | chr14 | - | 54902008 | 54948457 | 1.04 |
| CD6 molecule | CD6 | NM_006725 | TP120 | 3332663 | chr11 | + | 60495619 | 60544422 | 1.04 |
| pre-mRNA cleavage factor 1, 59 kDa subunit | FLJ12529 | NM_024811 | FLJ39024 // MGC9315 | 3375340 | chr11 | - | 60926704 | 60954030 | 1.04 |
| SEC23 interacting protein | SEC231P | NM_007190 | MSTP053 // P125 | 3267455 | chr10 | + | 121642213 | 121691984 | 1.04 |
| cell division cycle 40 homolog (S. cerevislae) | CDC40 | NM_015891 | EHB3 // FLJ10564 // MGC102802 // PRP17 // PRPF17 | 2921086 | chr6 | + | 110606544 | 110682171 | 1.04 |
| calnexin | CANX | NM 001024649 // NM_001746 | CNX // FLJ26570 // IP90 // P90 | 2844203 | chr5 | + | 179058077 | 179091243 | 1.04 |
| chromosome 20 open reading frame 112 | C20orf112 | NM_080616 | DKFZP566G1424 // dJ1184F4.2 | 3902764 | chr20 | - | 30498759 | 30636537 | 1.04 |
| splicing factor 3b, subunit 3, 130kDa | SF3B3 | N M_012426 | KIAA0017 // RSE1 // SAP130 // SF3b130 // STAF130 | 3667281 | chr16 | + | 69115212 | 69169072 | 1.04 |
| family with sequence similarity 44, member A | FAM44A | NM_148894 | FLJ33215 // KIAA1327 | 2761285 | chr4 | - | 13179462 | 13207890 | 1.04 |
| casein kinase 2, alpha 1 polypeptide // casein kinase 2, alpha 1 polypeptide pseudogene | CSNK2A1 II CSNK2A1P | NM_001895 // NM_177559 11 NM 177560 // NR_002207 | CK2A1 // CKII // CKH alpha // - | 3894228 | chr20 | - | 402069 | 472534 | 1.04 |
| eukaryotic translation initiation factor 3, subunit 7 zeta, 66/67kDa | EIF3S7 | NM_003753 | MGC126526 // MGCI7258 // eIF3- p66 // eIF3-zeta // eIF3d | 3959631 | chr22 | - | 35236857 | 35255429 | 1.04 |
| GTP binding protein 2 | GTPBP2 | NM.019096 | MGC74725 | 2954771 | chr6 | - | 43681031 | 43704877 | 1.04 |
| fusion (involved in t(12:16) in malignant liposarcoma) | FUS | NM_001010850 // NM_004960 | CHOP // FUS- CHOP // FUS1 // TLS // TLS/CHOP | 3656904 | chr16 | + | 31093395 | 31111003 | 1.04 |
| KIAA0430 | KIAA0430 | NM_014647 | A-362G6.1 // LKAP | 3681956 | chr16 | - | 15595757 | 15654441 | 1.04 |
| lymphocyte cytosolic protein 1 (L-plastin) | LCP1 | NM_002298 | CP64 // DKFZp781A23186 // FLJ25423 // FLJ26114 // FLJ39956 // L- PLASTIN // LC64P // PLS2 | 3512874 | chr13 | - | 45595067 | 45684007 | 1.04 |
| DENN/MADD domain containing 3 | DENND3 | NM_014957 | K1AA0870 | 3118651 | chr8 | + | 142207943 | 142289494 | 1.04 |
| K1AA0317 | KIAA0317 | NM_001039479 | - | 3572041 | chr14 | - | 74189599 | 74249655 | 1.04 |
| ninein (GSK3B interacting protein) | NIN | NM_016350 // NM_020921 // NM_182944 // NM_182945 // NM_182946 | KIAA1565 | 3564071 | chr14 | - | 50256234 | 50403020 | 1.04 |
| transmembrane protein 161 B // integrin, alpha 2b (platelet glycoprotein IIb of IIb/IIa complex, antigen CD41 ) | TMEM161B // ITGA2B | NM_153354 // NM_000419 | FLB3342 // MGC3321 4 // PR01313 // CD41 // CD41 B // GP2B // GPllb // GTA // HPA3 | 2866045 | chr5 | - | 87526693 | 87600565 | 1.04 |
| Ewing sarcoma breakpoint region 1 | EWSR1 | NM_005243 // NM_013986 | EWS | 3941907 | chr22 | + | 27994201 | 28026501 | 1.04 |
| YTH domain containing 1 | YTHDC1 | NM_001031732 // NM_133370 | KIAA1966 // YT521 // YT521-B | 2772017 | chr4 | - | 68858700 | 68934802 | 1.04 |
| squamous cell carcinoma antigen recognized by T cells 3 | SART3 | NM_014706 | KIAA0156 // MGC138188 // RP11-13G14 // TIP110 // p110(nrb) | 3470253 | chr12 | - | 107440140 | 107479296 | 1.04 |
| cytoplasmic linker associated protein 1 | CLASP1 | NM_015282 | DKFZp686D1968 // DKFZp686H2039 // FLJ33821 // FLJ41222 // KIAA0622 // MAST1 // | 2573641 | chr2 | - | 121811825 | 122169321 | 1.04 |
| nuclear interacting protein 1 | NRIP1 | NM_003489 | RIP140 | 3925639 | chr21 | - | 15255125 | 15359182 | 1.04 |
| receptor protein phosphatase 1, regulatory (inhibitor) | PPP1R12A | NM_002480 | MBS // MGC133042 // | 3463571 | chr12 | - | 78690959 | 78853356 | 1.04 |
| subunit 12A STE20-like kinase (yeast) | SLK | NM_014720 | KIAA0204 // MGC133067 // STK2 // bA16H23.1 // se20-9 | 3262433 | chr10 | + | 105716666 | 105778975 | 1.04 |
| polymerase (RNA) II (DNA directed) polypeptide B, 140kDa | POLR2B | NM_000938 | POL2RB // RPB2 II hRPB140 II hsRPB2 | 2728448 | chr4 | + | 57539163 | 57592513 | 1.04 |
| HIV-1 Rev binding protein | HRB | XM_941338 // NM_004504 | MG01 16938 II MGC116940 // RAB II RIP | 2530599 | chr2 | + | 228045122 | 228134167 | 1.04 |
| zinc finger CCCH-type containing 12A | ZC3H12A | NM_025079 | FLJ23231 // MCPIP // RP3- 423B22.1 // | 2330687 | chr1 | + | 37712740 | 37722550 | 1.04 |
| transforming growth factor, beta 1 | TGF81 | NM_000660 | CED // DPD1 // TGFB | 3863021 | chr19 | - | 46499343 | 46551636 | 1.04 |
| ADP-ribosylation factor 4 | ARF4 | XM_001132763 // NM_001660 | - | 2678090 | chr3 | - | 57532172 | 57558635 | 1.04 |
| pleiomorphic adenoma gene-like 2 | PLAGL2 | NM_002657 | FLJ23283 | 3902682 | chr20 | - | 30243975 | 30259284 | 1.04 |
| jumonji domain containing 2C | JMJD2C | NM_015061 | FLJ25949 // GASC1 // JHDM3C // KIAA0780 // bA146B14.1 | 3161566 | chr9 | + | 6747651 | 7165647 | 1.04 |
| transmembrane BAX inhibitor motif containing 1 | TMBIM1 | NM_022152 | PP1201 // RECS1 | 2599371 | chr2 | - | 218847185 | 218865515 | 1.04 |
| GPI-anchored membrane protein 1 | GPIAPI | NM_005898 // NM_203364 | GP1P137 // M11S1 // p137GPI | 3326183 | chr11 | + | 34009711 | 34081946 | 1.04 |
| DEAD (Asp-Glu-Ala-Asp) box polypeptide 47 // apolipoprotein L domain containing 1 | DDX47 // APOLD1 | NM_016355 // NM_201224 // NM_030817 | DKFZp5640176 // E4-DBP // FLJ30012 // HQ0256 // MSTP162 // DKFZP434F0318 // FLJ25138 | 3405531 | chr12 | + | 12856462 | 12874176 | 1.04 |
| SERPINE1 mRNA binding protein 1 | SERBP1 | NM_001018067 // NM_001018068 // NM_001018069 // NM_015640 | CGI-55 // CHD31P // DKFZp564M2423 // FLJ90489 // HABP4L // PAI- RBP1 // PAIRSP1 | 2417174 | chr1 | - | 67646101 | 67669020 | 1.04 |
| coiled-coil domain containing 47 | CCDC47 | NM_020198 | GK001 // MSTP041 | 3766334 | chr17 | - | 59176353 | 59204726 | 1.04 |
| lamin B receptor | LBR | NM_002296 // NM_194442 | DHCR14B // LMN2R // MGC9041 // PHA | 2458289 | chr1 | - | 223655840 | 223683230 | 1.04 |
| caspase 3, apoptosis-related cysteine peptidase | CASP3 | NM_004346 // NM_032991 | CPP32 // CPP32B // SCA-1 | 2796484 | chr4 | - | 185782536 | 185807608 | 1.04 |
| Burkitt lymphoma receptor 1, GTP binding protein (chemokine (C-X-C motif) receptor 5) | BLR1 | NM_001716 // NM_032966 | CD185 // CXCR5 // MOR15 // MGC117347 | 3351675 | chrl1 | + | 118259777 | 118272180 | 1.04 |
| major histocompatibility complex, class I, E | HLA-E | NM-005516 | DKFZp686P19218 // EA1.2 // EA2.1 // HLA-6.2 // MHC | 2901620 | chr6 | + | 30565243 | 30620086 | 1.04 |
| v-yes-1 Yamaguchi sarcoma viral related oncogene homolog | LYN | NM_002350 | FLJ26625 // JTK8 | 3098977 | chr8 | + | 56954505 | 57087291 | 1.04 |
| MYC-associated zinc finger protein (purine-binding transcription factor) // kinesin family member 22 | MAZ // KIF22 | NM 001042539 // NM_002383 // NM_007317 | PUR1 // Pur-1 // SAF-1 // SAF-2 // ZF87 // ZNF801 // Zif87 // KID // KNSL4 // OBP II OBP-1 II OBP-2 | 3655665 | chr16 | + | 29724956 | 29729977 | 1.04 |
| chromosome 3 open reading frame 58 | C3orf58 | NM_173552 | MGC33365 | 2646327 | chr3 | + | 145173603 | 145669744 | 1.04 |
| hect (homologous to the E6-AP (UBE3A) carboxyl terminus) domain and RCC1 (CHC1)-like domain (RLD) 1 | HERC1 | NM_003922 | p532 // p619 | 3628650 | chr15 | - | 61687879 | 61913158 | 1.04 |
| KIAA0652 | KlAA0652 | NM_014741 | FLJ20698 | 3329404 | chr11 | + | 46595671 | 46652934 | 1.04 |
| PX domain containing serine/threonine kinase | PXK | NM_017771 | FLJ20335 // MONaKA | 2626167 | chr3 | + | 58293657 | 58386884 | 1.04 |
| jumonji, AT rich interactive domain 2 | JARID2 // C20orf120 | NM_004973 | JMJ | 2896177 | chr6 | + | 15261674 | 15630231 | 1.04 |
| CGI-09 protein | CGI-09 | NM_015939 | MGC5029 | 3896524 | chr20 | - | 5865881 | 5879370 | 1.04 |
| syndecan binding protein (syntenin) | SDCBP | NM_001007067 // NM_001007068 // NM_001007069 // NM_001007070 // NM_005625 | MDA-9 // ST1 // SYCL // TACIP18 | 3099750 | chr8 | + | 59605208 | 59658960 | 1.04 |
| 2',3'-cyclic nucleotide 3' phosphodiesterase | CNP | NM_033133 | CNP1 | 3721548 | chr17 | + | 37372285 | 37383765 | 1.04 |
| family with sequence similarity 48, member A // family with sequence similarity 48, member B2 | FAM48A // FAM48B2 | NM_001014286 // NM_017569 // XM_293352 | C 13 // C 13orF19 // FP757 // P381P // bA421P11.4 // - | 3509910 | chr13 | - | 36480880 | 36556254 | 1.04 |
| ubiquitin-activating enzyme El-like 2 | UBE1L2 | NM_018227 | FLJ10808 // FLJ23367 | 2771718 | chr4 | - | 68130308 | 68249472 | 1.04 |
| solute carrier family 35, member B1 | SLC35B1 | NM_005827 | UGTREL1 | 3761959 | chr17 | - | 45133304 | 45141355 | 1.04 |
| pogo transposable element with ZNF domain | POGZ | NM_015100 // NM_145796 // NM_207171 | KIAA046 // MGC71543 // SUHW5 // ZNF635 | 2435044 | chr1 | - | 149641830 | 149753035 | 1.04 |
| forkhead box 01 A (rhabdomyosarcoma) | FOX01A | NM_002015 | FKH1 // FKHR // FOXO1 | 3510858 | chr13 | - | 40027817 | 40162098 | 1.04 |
| RAB14, member RAS family | RAB14 | NM_016322 | FBP // RAB-14 | 3223872 | chr9 | - | 122980236 | 123025093 | 1.04 |
| oncogene zinc finger protein 313 | ZNF313 | NM_018683 | RNF114 | 3888474 | chr20 | + | 47986321 | 48003818 | 1.04 |
| DnaJ (Hsp40) homolog, subfamily C, member 1 | DNAJC1 | NM_022365 | DNAJL1 // ERdj1 // HTJ1 // MGC 131954 | 3280902 | chr10 | - | 22078144 | 22332877 | 1.04 |
| integrin, beta 1 (fibronectin receptor, beta polypeptide, antigen CD29 includes MDF2, MSK12) | ITGB1 | NM_002211 // NM_033666 // NM_033667 // NM_033668 // NM_033669 // NM_133376 | CD29 // FNRB // GPIIA // MDF2 // MSK12 // VLAB | 3284188 | chr10 | - | 33211051 | 33321367 | 1.04 |
| NMDA receptor regulated 2 | NARG2 | NM_001018089 // NM_024611 | - | 3627363 | chr15 | - | 58500956 | 58558626 | 1.04 |
| mitochondrial ribosomal protein 57 | MRPS7 | NM_015971 | MRP-S // MRP-S7 // RP-S7 // | 3734760 | chr17 | + | 70769374 | 70774626 | 1.04 |
| TATA box binding protein | TBP | NM_003194 | GTF2D // GTF2D1 // MGC 117320 // MGC126054 // MGC126055 // SCA17 // TFIID | 2937984 | chr6 | + | 170705200 | 170723945 | 1.04 |
| polymerase (RNA) I polypeptide C, 30kDa | POLRIC | NM_004875 // NM_203290 | RPA39 // RPA40 // RPA5 // RPAC1 | 2908052 | chr6 | + | 43592769 | 43605071 | 1.04 |
| spectrin, alpha, non-erythrocytic 1 (alpha-fodrin) | SPTAN1 | NM_003127 | (ALPHA)II- SPECTRIN // FLJ44613 | 3190558 | chr9 | + | 130354704 | 130435758 | 1.04 |
| protein phosphatase 1, catalytic subunit, gamma isoform | PPP1 CC | NM_002710 | PPP1G | 3471374 | chr12 | - | 109642007 | 109665131 | 1.04 |
| CDC-like kinase 1 | CLK1 | NM_ 001024646 // NM_004071 | CLK // CLK/STY // STY | 2594497 | chr2 | - | 201425997 | 201437659 | 1.04 |
| transient receptor potential cation channel, subfamily C, member 4 associated protein | TRPC4AP | NM_015638 // NM_199368 | C20orf188 // TRRP4AP // | 3903708 | chr20 | - | 33053899 | 33144297 | 1.04 |
| actin, beta | ACTB | NM_001101 | PS1TP5BP1 | 3036924 | chr7 | - | 5533433 | 5537011 | 1.04 |
| polypyrimidine tract binding protein 1 | PTBP1 | NM_002819 // NM_031990 // NM_031991 // NM_175847 | HNRNPI // HNRPI // MGC10830 // MGC8461 // PTB // PTB-1 // PTB- T // PTB2 // PTB3 // PTB4 // pPTB | 3815165 | chr19 | + | 747768 | 763505 | 1.04 |
| chromosome 6 open reading frame 166 | C6orf166 | NM_018064 | FLJ10342 // dJ486L4.2 | 2963784 | chr6 | - | 88441297 | 88476721 | 1.04 |
| ring finger protein 40 | RNF40 | NM_014771 | BRE1B // DKFZp686K191 // KIAA0661 // MGC13051 // RBP95 // STARING | 3656555 | chr16 | + | 30681120 | 30695182 | 1.04 |
| coiled-coil domain containing 100 | CCDC100 | NM_53223 | DKFZp688I06246 // FLJ36090 // FLJ38327 | 2873168 | chr5 | - | 122642038 | 122832582 | 1.04 |
| ubiquitin specific peptidase 8 | USP8 | NM_005154 | FLJ34456 // HumORF8 // KIAA0055 // MGC129718 // UBPY | 3593652 | chr15 | + | 48503634 | 48580565 | 1.04 |
| splicing factors 1 | SF1 | NM_004630 // NM_2019961 // NM_201997 // NM_201998 | D11 5636 // ZFMI // ZNF162 | 3377044 | chr11 | - | 64287827 | 64302817 | 1.04 |
| family with sequence similarity 89, member B | FAM89B | NM_152832 | MTVR1 | 3335338 | chr11 | + | 65096490 | 65098230 | 1.04 |
| eukaryotic translation initiation factor 4A, isoform 2 | EIF4A2 | NM967 | BM-010 // DDX2B // EIF4A // ELF4 F | 2656738 | chr3 | + | 187961636 | 187990742 | 1.04 |
| leucine-rich PPR-motif containing | LRPPRC | NM_133259 | CLONE-23970 // GP130 // LRP130 // LSFC | 2550790 | chr2 | - | 43961504 | 44085128 | 1.04 |
| v-rel reticuloendotheliosis viral oncogene homolog A, nuclear factor of kappa light polypeptide gene enhancer in B-celts 3. p65 (avian) | RELA | NM_021975 | MGC131774 // NFKB3 | 3377789 | chr11 | - | 65176856 | 65204491 | 1.04 |
| chromodomain helicase DNA binding protein 4 | CHD4 | NM_001273 | DKFZp688E06161 // Mf-2b // Mi2- | 3442054 | chr12 | - | 6549521 | 6587067 | 1.04 |
| transferrin receptor (p90, CD71) | TFRC | NM_003234 | CD71 // TFR // TFR1 // TRFR | 2712632 | chr3 | - | 197238405 | 197334433 | 1.04 |
| GRP1 (general receptor for phosphoinositides 1)-associated scaffold protein | GRASP | NM_181717 | - | 3415193 | chr12 | + | 50686988 | 50695938 | 1.04 |
| small nuclear ribonucleoprotein 70kDa polypeptide (RNP antigen) | SNRP70 | NM_001009820 // NM_003089 | RNPU1Z // RPU1 // U170K // UIAP // U1RNP | 3838185 | chr19 | + | 54280358 | 54304714 | 1.04 |
| 1-acylglycercl-3-phosphate O-acyltransferase 3 | AGPAT3 | NM_001037553 // NM_020132 | LPAAT-GAMMAL // MGC4604 | 3923354 | chr21 | + | 44105141 | 44245644 | 1.04 |
| vacuolar protein sorting 4 homolog B (S. cerevisiae) | VPS4B | NM_004869 | MIG1 // SKD1 // VPS4-2 | 3811497 | chr18 | - | 59207407 | 59240734 | 1.04 |
| KIAA1128 | KIAA1128 | NM_018999 | FLJ14262 // FLJ25809 // Gcap14 // bA486022.1 | 3255402 | chr10 | + | 86020629 | 86268247 | 1.04 |
| c-src tyrosine kinase | CSK | NM_004383 | MGC117393 | 3801840 | chr15 | + | 72841734 | 72882558 | 1.04 |
| pleiotropic regulator 1 (PRL1 homolog, Arabidopsis) | PLRG1 | NM_002669 | MGC110980 // PRL1 | 2790570 | chr4 | - | 155675613 | 155691014 | 1.04 |
| WD repeat domain 1 | WDRI | NM_005112 // NM_017491 | AIP1 // NORI-1 | 2760371 | chr4 | - | 9685076 | 9772600 | 1.04 |
| FBJ murine osteosarcoma viral oncogene homolog B | FOSB | NM_006732 | DKFZp686C0818 // GOS3 // GOS3 // GOSB // | 3836266 | chr19 | + | 50624036 | 50671180 | 1.04 |
| c-Maf-inducing protein | CMIP | NM_030629 // NM_198390 | KIAA1694 | 3670772 | chr16 | + | 80030441 | 80323852 | 1.04 |
| aldolase A, fructose-bisphosphate | ALDOA | NM_000034 // NM_184041 // NM_184043 | ALDA // MGC10942 // MGC17716 // | 3655920 | chr16 | + | 29968869 | 29991009 | 1.04 |
| bolA homolog 1 (E. coli) | BOLA1 | NM_016074 | CGI-143 // MGC75015 // RP11-196G18.18 | 2357961 | chr1 | + | 148126062 | 148138968 | 1.04 |
| ring finger and SPRY domain containing 1 | RSPRY1 | NM_133368 | KIAA1972 | 3662612 | chr16 | + | 55777703 | 55831882 | 1.04 |
| centaurin, delta 2 | CENTD2 | NM_001040118 // NM_015242 // NM_139181 | ARAP1 // KIAA0782 | 3381241 | chr11 | - | 72073021 | 72141062 | 1.04 |
| NADH dehydrogenase (ubiquinone) 1 alpha subcomplex, 5, 13kDa | NDUFA5 | NM_005000 | B13 // 01-13KD-B // DKFZp781K1356 // FLJ12147 // NUFM // UOOR13 | 3070658 | chr7 | - | 122968075 | 122985216 | 1.04 |
| brix domain containing 2 | BXDC2 | NM_018321 | BRIX // FLJ11100 | 2806231 | chr5 | + | 34951248 | 34962845 | 1.04 |
| tumor necrosis factor receptor superfamily, member 25 // pleckstrin homology domain containing, family G (with RhoGef domain) member 5 | TNFRSF25 // PLEKHG5 | NM_001039664 // NM_003790 // NM_148965 // NM_148966 // NM_148967 // NM_148970 // NM_001042663 // NM_001 042664 // NM_001042665 // NM_020631 // NM_198681 | APO-3 // DDR3 // DR3 // LARD // TNFRSF12 // TR3 // TRAMP // WSL- 1 // WSL-LR // KIAA0720 // RP4- 650H14.3 | 2394699 | chr1 | - | 6443807 | 6448816 | 1.04 |
| nucleoporin like 1 | NUPL1 | NM_001008564 // NM_001008565 // NM_014089 | KIAA0410 // PRO2463 | 3482219 | chr13 | + | 24773258 | 24822202 | 1.04 |
| nuclear factor of kappa light polypeptide gene enhancer in B-cells inhibitor, epsilon | NFKBIE | NM_004556 | IKBE | 2955076 | chr6 | - | 44333884 | 44341478 | 1.04 |
| tripartite motif-containing 8 | TRIMB | NM_030912 | GERP // RNF27 | 3261820 | ehr10 | + | 104384137 | 104419190 | 1.04 |
| WD repeat and SOCS box-containing 1 | WSB1 | NM_015626 // NM_134265 | SWIP1 // WSB-1 | 3715109 | chr17 | + | 22644482 | 22684326 | 1.04 |
| SAPS domain family, member 3 | SAPS3 | NM_018312 | C11orf23 // DKFZp781E17107 // DKFZp781E2314 // DKFZp78102362 // FLJ11058 // FLJ43065 // MGC125711 // MGG125712 // PP6R3 // SAP190 // SAPL // SAPLa | 3337618 | chur11 | + | 67984785 | 68173381 | 1.04 |
| hypothetical protein FLJ21438 | FLJ21438 | XM_029084 | DKFZp667E013 // FLJ00087 | 3853453 | chr19 | - | 15423181 | 15436377 | 1.04 |
| CD14 molecule | CD14 | NM_000591 // NM_001040021 | - | 2878437 | chr5 | - | 139991507 | 139993422 | 1.04 |
| PRP3 pre-mRNA processing factor 3 homolog (S. cerevisiae) // KIAA0460 | PRPF3 // KIAA0460 | NM_004698 // NM_015203 | HPRP3 // HPRP3P // PRP3 // Prp3p // RP18 // FLJ32145 // HSPC099 | 2358171 | chr1 | + | 148560583 | 148592321 | 1.04 |
| signal transducer and activator of transcription | STAT5B | NM_012448 | STAT5 | 3757770 | chr17 | - | 37604727 | 37682109 | 1.04 |
| jumonji domain containing 1A | JMJD1A | NM_018433 | DKFZp686A24246 // DKFZp686P07111 // JHMD2A // JMJD1 // | 2492064 | chr2 | + | 86509150 | 86574548 | 1.04 |
| 1Q motif containing GTPase activating protein 1 | IQGAP1 | NM_003870 | HUMORFA01 // KlAA0051 // SAR1 // p195 | 3608113 | chr15 | + | 88732454 | 88846463 | 1.04 |
| ubiquitin-conjugating enzyme E2B (RAD6 homolog) | UBE2B | NM_003337 | E2-17kDa // HHR6B // HR6B // RAD6B // UBC2 | 2829275 | chr5 | + | 133734769 | 133788148 | 1.04 |
| splicing factor, arginine/serine-rich 10 (transformer 2 homolog, Drosophila) | SFRS10 | NM_004593 | DKFZp686F18120 // Htra2-beta // SRFS10 // TRA2- BETA // TRA2B | 2709062 | chr3 | - | 187048509 | 187160523 | 1.04 |
| poly(A) polymerase alpha | PAPOLA | NM 032632 | MGC5378 // PAP | 3550392 | chr14 | + | 96038038 | 96104627 | 1.04 |
| kelch-like 18 (Drosophila) | KLHL18 | NM_025010 | FLJ13703 // KWA0795 | 2621275 | chr3 | + | 47298888 | 47367590 | 1.04 |
| G protein-coupled receptor 132 | GPR132 | NM_013345 | G2A // MGC99642 | 3581404 | chr14 | - | 104586779 | 104622593 | 1.04 |
| phosphatidylinositol transfer protein, beta | PITPNB | NM_012399 | PI-TP-beta // PtdlnsTP // VIB1B | 3956290 | chr22 | - | 26577440 | 26645487 | 1.04 |
| Snf2-related CBP activator protein | SRCAP | NM_006662 | KIAA0309 | 3656418 | chr16 | + | 30616551 | 30663998 | 1.04 |
| engulfment and cell motility 1 | ELMO1 | NM_001039459 // NM_014800 // NM_130442 | CED-12 // CED12 // ELMO-1 // KlAA0281 // MGC 126406 | 3046197 | chr7 | - | 36860492 | 37455389 | 1.04 |
| metal response element binding transcription factor 2 | MTF2 | NM_007358 | M96 // PCL2 // RP5-976013.1 // dJ976013.2 | 2346934 | chr1 | + | 93147511 | 93377202 | 1.04 |
| hemopoietic cell kinase | HCK | NM_002110 | JTK9 | 3881651 | chr20 | + | 30086433 | 30153306 | 1.04 |
| SIN3 homolog A, transcription regulator (yeast) | SIN3A | NM_015477 | DKFZP434K2235 // FLJ90319 // KIAA0700 | 3633403 | chr15 | - | 73448786 | 73535167 | 1.04 |
| eukaryotic translation initiation factor 3, subunit 9 eta, 116kDa | EIF3S9 | NM_001037283 // NM_003751 | EIF3-ETA // EIF3- P110 // EIF3-P116 // MGC104664 // MGC131875 // PRT1 // eIF3b | 2987441 | chr7 | + | 2315929 | 2386896 | 1.04 |
| transmembrane 9 superfamily member 3 | TM9SF3 | NM_020123 | EP70-P-iso // RP11-34E5.1 // SMBP | 3301857 | chr10 | - | 98267858 | 98337079 | 1.04 |
| chromosome 1 open reading frame 50 | C1orf50 | NM_024097 | MGC955 | 2332767 | chr1 | + | 43005527 | 43036633 | 1.04 |
| chaperonin containing TCP1, subunit 6A (zeta 1) | CCT6A | NM_001009186 // NM_001762 | CCT-zeta // CCT- zeta-1 // CCT6 // Cctz // HTR3 // MGC126214 // MGC126215 // MoDP-2 // TCP-1- zeta // TCP20 // TCPZ // TTCP20 | 3003193 | chr7 | + | 56086894 | 56099148 | 1.04 |
| zinc finger protein 410 | ZNF410 | NM_021188 | APA-1 // APA1 | 3543884 | chr14 | + | 73423093 | 73468718 | 1.04 |
| KIAA1468 | KIAA1468 | NM_020854 | FLJ33841 // HsT3308 // HsT885 | 3791168 | chr18 | + | 58005478 | 58125326 | 1.04 |
| nucleolar protein NOP5/NOP58 | NOP5/NOP58 | NM_015934 | HSPC120 | 2523144 | chr2 | + | 202811346 | 202926967 | 1.04 |
| guanylate binding protein 5 | GBP5 | NM 052942 | GBP-5 | 2422035 | chr1 | - | 89496930 | 89511122 | 1.04 |
| 5'-3' exoribonuclease 2 | XRN2 | NM 012255 | - | 3879467 | chr20 | + | 21219705 | 21322172 | 1.04 |
| fragile X mental retardation, autosomal homolog 1 | FXR1 | NM 001013438 // NM_001013439 // NM_005087 | - | 2654394 | chr3 | + | 182113040 | 182179459 | 1.04 |
| oxoglutarate (alpha-ketoglutarate) dehydrogenase (lipoamide) | OGDH | NM-001003941 // NM_002541 | AKGDH // E1k // OGDC | 2999948 | chr7 | + | 44612716 | 44715181 | 1.04 |
| KIAA1967 | KIAA1967 | NM_021174 // NM_199205 | DBC-1 // DBC1 | 3089597 | chr8 | + | 22518038 | 22534618 | 1.04 |
| C-type lectin domain family 5, member A | CLEC5A | NM_013252 | CLECSF5 // MDL- 1 // MDL1 // MGC138304 | 3076868 | chr.7 | - | 141273626 | 141298905 | 1.04 |
| exportin 4 | XPO4 | NM_022459 | FLJ13046 // KIAA1721 | 3504434 | chr13 | - | 20251014 | 20375187 | 1.04 |
| RNA binding motif protein 6 | RBM6 | NM_005777 | 3G2 // DEF-3 // DEF3 // DKFZp686B0877 // FLJ36517 // HLC- 11 // NY-LU-12 // g16 | 2622359 | chr3 | + | 49942372 | 50089683 | 1.04 |
| nucleotide binding protein 1 (MinD homolog, E. coli) | NUBP1 | NM_002484 | MGC117406 // MGC130052 // MGC130053 // NBP // NBP1 | 3647956 | ehr16 | + | 10729448 | 10771777 | 1.04 |
| Yip1 domain family, member 3 // chromosome 6 open reading frame 154 | YIPF3 // C6orf154 | NM_015388 // NM_001012974 | C6orf109 // DKFZP566C243 // FinGER3 // KUP1 // dJ337H4.3 // FLJ44836 // MGC131686 // dJ337H4.2 | 2954646 | chr6 | - | 43587500 | 43592701 | 1.04 |
| chromosome 6 open reading frame 94 // LTV1 homolog (S. cerevisiae) | C6orf94 // LTV1 | XM_928657 // XM_941265 // NM_032860 | dJ468K18.5 // C6orf93 // FLJ14909 // dJ468K18.4 | 2929036 | chr6 | + | 144194149 | 144227146 | 1.04 |
| intercellular adhesion molecule 1 (CD54), human rhinovirus receptor | ICAM 1 | NM_000201 | BB2 // CD54 // P3.58 | 3820443 | chr19 | + | 10242765 | 10258289 | 1.04 |
| KIAA0368 | KIAA0368 | XM_001129450 // XM_001131778 // NM_001080398 | ECM29 // FLJ22036 // K1AA1962 // RP11- | 3220513 | chr9 | - | 113131941 | 113286475 | 1.04 |
| CD3d molecule, delta (CD3-TCR complex) | CD3D | NM_000732 // NM 001040651 | CD3-DELTA // T3D | 3393744 | chr11 | - | 117698596 | 117718659 | 1.04 |
| translocase of outer mitochondrial membrane 70 A (S. cerevisiae) | TOMM70A | NM_014820 | FLJ90470 | 2686371 | chr3 | - | 101565001 | 101614635 | 1.04 |
| homolog natural killer-tumor recognition sequence | NKTR | NM_001012651 // NM_005385 | DKFZp686F1754 // DKFZp686G0426 // DKFZp686J06106 // DKFZp686N24126 | 2619344 | chr3 | + | 42617105 | 42678166 | 1.04 |
| nucleolar protein 9 | NOL9 | NM_024654 | FLJ23323 // MGC131821 // MGC138483 | 2394784 | chr1 | - | 6504004 | 6537329 | 1.04 |
| proteasome (prosome, macropain) 26S subunit, ATPase, 4 | PSMC4 | NM_006503 // NM_153001 | MGC13687 // MGC23214 // MGC8570 // MIP224 // S6 // | 3833291 | chr19 | + | 45168772 | 45179181 | 1.04 |

## Claims

1. A method for assaying an action of a compound represented by formula (I), a pharmaceutically acceptable salt thereof, or a solvate of them to a mammal, which comprises detecting splicing defect caused by the compound represented by formula (I), the pharmaceutically acceptable salt thereof, or the solvate of them: 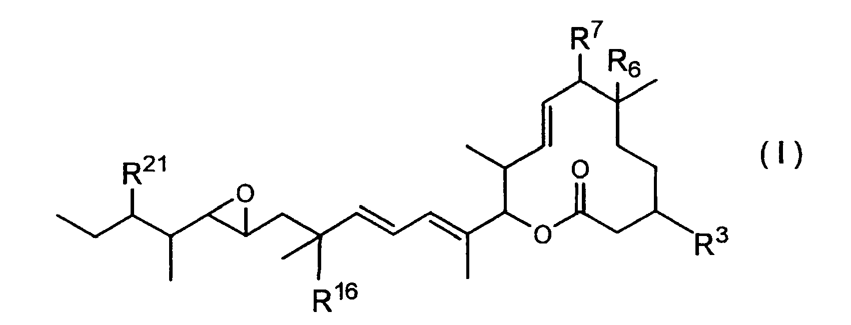 wherein R³, R⁶, R⁷ and R²¹, the same or different, each represents
1) a hydroxyl group or an oxo group formed together with the carbon atom to which it is bound, provided that R⁶ is limited to a hydroxyl group,
2) an optionally substituted C₁₋₂₂ alkoxy group,
3) an optionally substituted unsaturated C₂₋₂₂ alkoxy group,
4) an optionally substituted C₇₋₂₂ aralkyloxy group,
5) an optionally substituted 5- to 14-membered heteroaralkyloxy group,
6) RCO-O- wherein R represents
a) a hydrogen atom,
b) an optionally substituted C₁₋₂₂ alkyl group,
c) an optionally substituted unsaturated C₂₋₂₂ alkyl group,
d) an optionally substituted C₆₋₁₄ aryl group,
e) an optionally substituted 5- to 14-membered heteroaryl group,
f) an optionally substituted C₇₋₂₂ aralkyl group,
g) an optionally substituted 5- to 14-membered heteroaralkyl group,
h) an optionally substituted C₁₋₂₂ alkoxy group,
i) an optionally substituted unsaturated C₂₋₂₂ alkoxy group,
j) an optionally substituted C₆₋₁₄ aryloxy group or
k) an optionally substituted 5- to 14-membered heteroaryloxy group,
7) R^{S1} R^{S2}R^{S3}SiO- wherein R^{S1}, R^{S2}, and R^{S3}, the same or different, each represents
a) a C₁₋₆ alkyl group or
b) a C₆₋₁₄ aryl group,
8) a halogen atom,
9) R^{N1}R^{N2}N-R^{M}- wherein R^{M} represents
a) a single bond,
b) -CO-O-,
c) -SO₂-O-,
d) -CS-O-or
e) -CO-NR^{N3}- wherein R^{N3} represents a hydrogen atom or an optionally substituted C₁₋₆ alkyl group, provided that each of the leftmost bond in b) to e)is bound to the nitrogen atom; and R^{N1} and R^{N2}, the same or different from each other and each represents
a) a hydrogen atom,
b) an optionally substituted C₁₋₂₂ alkyl group,
c) an optionally substituted unsaturated C₂₋₂₂ alkyl group,
d) an optionally substituted aliphatic C₂₋₂₂ acyl group,
e) an optionally substituted aromatic C₇₋₁₅ acyl group,
f) an optionally substituted C₆₋₁₄ aryl group,
g) an optionally substituted 5- to 14-membered heteroaryl group,
h) an optionally substituted C₇₋₂₂ aralkyl group,
i) an optionally substituted C₁₋₂₂ alkylsulfonyl group,
j) an optionally substituted C₆₋₁₄ arylsulfonyl group,
k) an optionally substituted 3- to 14-membered non-aromatic heterocyclic group formed by R^{N1} and R^{N2} together with the nitrogen atom to which R^{N1} and R^{N2} are bound, and the non-aromatic heterocyclic group optionally has substituent(s),
l) an optionally substituted 5- to 14-membered heteroaralkyl group,
m) an optionally substituted C₃₋₁₄ cycloalkyl group or
n) an optionally substituted 3- to 14-membered non-aromatic heterocyclic group,
10) R^{N4}SO₂-O-wherein R^{N4} represents
a) an optionally substituted C₁₋₂₂ alkyl group,
b) an optionally substituted C₆₋₁₄ aryl group,
c) an optionally substituted C₁₋₂₂ alkoxy group,
d) an optionally substituted unsaturated C₂₋₂₂ alkoxy group,
e) an optionally substituted C₆₋₁₄ aryloxy group,
f) an optionally substituted 5- to 14-membered heteroaryloxy group,
g) an optionally substituted C₇₋₂₂ aralkyloxy group or
h) an optionally substituted 5- to 14-membered heteroaralkyloxy group,
11) (R^{N5}O)₂PO-O- wherein R^{N5} represents
a) an optionally substituted C₁₋₂₂ alkyl group,
b) an optionally substituted unsaturated C₂₋₂₂ alkyl group,
c) an optionally substituted C₆₋₁₄ aryl group,
d) an optionally substituted 5- to 14-membered heteroaryl group,
e) an optionally substituted C₇₋₂₂ aralkyl group or
f) an optionally substituted 5- to 14-membered heteroaralkyl group,
12) (R^{N1}R^{N2}N₂PO-O- wherein R^{N1} and R^{N2} have the same meanings as defined above or
13) (R^{N1}R^{N2}N)(R^{N5}O)PO-O- wherein R^{N1}, R^{N2} and R^{N5} have the same meanings as defined above, provided that a compound in which R³, R⁶, R⁷ and R²¹ are all hydroxyl groups, and a compound in which R³, R⁶ and R²¹ are all hydroxyl groups and R⁷ is an acetoxy group are excluded,
R¹⁶ represents a hydrogen atom or hydroxyl group.

2. The method according to claim 1, wherein the compound represented by formula (I) is selected from the group consisting of:
(8E,12E,14E)-7-(N-(2-(N',N'-Dimethylamino)ethyl)-N-methylcar bamoyloxy)-3,6,16, 21-tetra hydroxy-6,10,12,16, 20-pentamethyl -18,19-epoxytricosa-8,12,14-trien-11-olide;
(8E,12E,14E)-3,6,16,21-Tetrahyd roxy-6,10,12,16,20-pentameth yl-7-((4-methylhomopiperazin-1-yl)carbonyl)oxy-18,19-epoxytri cosa-8,12,14-trien-11-olide;
(8E,12E,14 E)-3,6,16, 21-Tetrahydroxy-6,10,12,16,20-pentameth yl-7-((4-methylpiperazin-1-yl)carbonyl)oxy-18,19-epoxytricosa-8,12,14-trien-11-olid e;
(8E,12E,14E)-7-((4-Butylpiperazin-1-yl)carbonyl)oxy-3,6,16,21-tetrahydroxy-6,10,12,16, 20-pentamethyl-18,19-epoxytricosa-8, 12,14-trien-11-olide;
(8E,12E,14E)-7-((4-Ethylpiperazin-1-yl)carbonyl)oxy-3,6,16,21-tetrahydroxy-6,10,12,16,20-pentamethyl-18,19-epoxytricosa-8, 12,14-trien-11-olide;
(8E,12E,14E)-3,6,16,21-Tetrahydroxy-6,10,12,16,20-pentameth yl-7-((4-propylpiperazin-1-yl)carbonyl)oxy-18,19-epoxytricosa-8,12,14-trien-11-olide;
(8E,12E,14E)-7-((4-Cyclohexylpiperazin-1-yl)carbonyl)oxy-3,6,1 6,21-tetrahydroxy-6,10,12,16,20-pentamethyl-18,19-epoxytrico sa-8,12,14-trien-11-olide;
(8E,12E,14E)-7-((4-(Cyclopropylmethyl)piperazin-1-yl)carbonyl) oxy-3, 6,16,
21-tetrahydroxy-6,10,12,16, 20-pentamethyl-18,19-epoxytricosa -8,12,14-trien-11-olide;
(8E,12E,14E)-3,6,16,21-Tetrahydroxy-6,10,12,16,20-pentameth yl-7-((4-propylhomopiperazin-1-yl)carbonyl)oxy-18,19-epoxytric osa-8,12,14-trien-11-olide;
(8E,12E,14E)-7-((4-(Cyclopropylmethyl)homopiperazin-1-yl)car bonyl)oxy-3,6,
16,21-tetrahydroxy-6,10,12,16,20-pentamethyl-18,19-epoxytric osa-8,12,14-trien-11-olide;
(8E,12E,14E)-7-((4-Cyclopentylpiperazin-1-yl)carbonyl)oxy-3,6, 16,21-tetrahydroxy-6,10,12,16,20-pentamethyl-18,19-epoxytric osa-8,12,14-trien-11-olide;
(8E,12E,14E)-3,6,16,21-Tetrahydroxy-7-((4-isopropylpiperazin-1-yl)carbonyl)oxy-6,10,12,16,20-pentamethyl-18,19-epoxytrico sa-8,12,14-trien-11-olide;
(8E,12E,14E)-7-((4-Cycloheptylpiperazin-1-yl)carbonyl)oxy-3,6, 16,21-Tetrahydroxy-6,10,12,16,20-pentamethyl-18,19-epoxytric osa-8,12,14-trien-11-olide;
(8E,12E,14E)-7-(N-(2-(N',N'-Diethylamino)ethyl)-N-methylcarba moyloxy)-3,6,16,21-tetrahydroxy-6,10,12,16,20-pentamethyl-1 8,19-epoxytricosa-8,12,14-trien-11-olide;
(8E,12E,14E)-3,6,16,21-Tetrahydroxy-7-((4-isobutylhomopipera zin-1-yl)carbonyl)oxy-6,10,1 2,16,20- pentamethyl-18,19-epoxyt ricosa-8,12,14-trien-11-olide;
(8E,12E,14E)-7-((4-Ethylhomopiperazin-1-yl)carbonyl)oxy-3,6,1 6,21-tetrahydroxy-6,10,12,16,20-pentamethyl-18,19-epoxytrico sa-8,12,14-trien-11-olide;
(8E,12E,14E)-7-((4-Butylhomopiperazin-1-yl)carbonyl)oxy-3,6,1 6,21-tetrahydroxy-6,10,12,16,20-pentamethyl-18,19-epoxytrico sa-8,12,14-trien-11-olide;
(8E,12E,14E)-3,16, 21-Trihydroxy-6-methoxy-6,10,12,16,20-pen tamethyl-7-((4-methylpiperazin-1-yl)carbonyl)oxy-18,19-epoxyt ricosa-8,12,14-trien-11-olide;
(8E,12E,14E)-3,16, 21-Trihydroxy-6-methoxy-6,10,12,16,20-pen tamethyl-7-((4-(piperidin-1-yl)piperidin-1-yl)carbonyl)oxy-18,1 9-epoxytricosa-8,12,14-trien-11-olide;
(8E,12E,14 E)-3,6, 7, 21-Tetrahydroxy-6,10,12,16, 20-pentamethyl -18,19-epoxytricosa-8,12,14-trien-11-olide;
(8E,12E,14E)-7-((4-(2,2-Dimethylpropyl)homopiperazin-1-yl)car bonyl)oxy-3,6,16,21-tetrahydroxy-6,10,12,16,20-pentamethyl-1 8,19-epoxytricosa-8,12,14-trien-11-olide; and
(8E,12E,14E)-3, 6,16-Trihydroxy-21-methoxy-6,10,12,16,20-pen tamethyl-7-((4-methylpiperazin-1-yl) carbonyl) oxy-18,19-epoxyt ricosa-8,12,14-trien-11-olide.

3. The method according to claim 1, wherein the detection of splicing defect comprises the steps of:
(a) measuring the expression level of pre-mRNA before and after administration of the compound represented by formula (I), the pharmaceutically acceptable salt thereof ,or the solvate of them to a mammal;
(b) comparing, based on the expression level measured in (a), the expression level of pre-mRNA before and after administration of the compound represented by formula (I), the pharmaceutically acceptable salt thereof, or the solvate of them, to determine that the compound represented by formula (I), the pharmaceutically acceptable salt thereof, or the solvate of them exerts an action to the mammal when the expression level of pre-mRNA after the administration increases.

4. The method according to claim 3, wherein the pre-mRNA of which expression level is measured is pre-mRNA of at least one gene selected from the genes listed in Table 1, Table 2, Table 3, Table 4, and Table 5, or a homologous gene thereof.

5. The method according to claim 4, wherein the gene(s) are selected from DNAJB1, BZW1, NUP54, RIOK3, CDKN1B, STK17B, EIF4A1, and ID1.

6. The method according to claim 3, wherein in step (a), the expression level of pre-mRNA in samples obtained from a subject before and after administration of the compound represented by formula (I), the pharmaceutically acceptable salt thereof, or the solvate of them, is measured.

7. The method according to claim 6, wherein the samples obtained from the subject are selected from hemocytes in peripheral blood, plasma, and serum.

8. A probe or primer for assaying an action of a compound represented by formula (I), a pharmaceutically acceptable salt thereof, or a solvate of them to a mammal, which consists of a polynucleotide capable of hybridizing with a polynucleotide consisting of a nucleotide sequence of at least one gene selected from the genes listed in Table 1, Table 2, Table 3, Table 4, and Table 5, and a homologous gene thereof, or a complementary sequence thereof.

9. The probe or primer according to claim 8, which is capable of detecting a genomic intron region or a part thereof in a gene listed in Table 1, Table 2, Table 3, Table 4 and Table 5, or which is capable of detecting a polynucleotide lacking a part of a genomic exon region in a gene listed in Table 1, Table 2, Table 3, Table 4 and Table 5.

10. A reagent or kit for assaying an action of a compound represented by formula (I), a pharmaceutically acceptable salt thereof, or a solvate of them to a mammal, which comprises the probe or the primer according to claim 8.

11. The method according to claim 1, wherein the detection of splicing defect comprises the steps of:
(f) measuring the expression level of an abnormal protein before and after administration of the compound represented by formula (I), the pharmaceutically acceptable salt thereof, or the solvate of them to a mammal;
(g) comparing, based on the expression level measured in (f), the expression level of the abnormal protein before and after administration of the compound represented by formula (I), the pharmaceutically acceptable salt thereof or the solvate of them, to determine that the compound represented by formula (I), the pharmaceutically acceptable salt thereof, or the solvate of them exerts an action to the mammal when the expression level of the abnormal protein after the administration increases.

12. The method according to claim 11, wherein the abnormal protein of which expression level is measured is a protein consisting of amino acids encoded by a polynucleotide of at least one gene selected from the genes listed in Table 1 and Table 3, or a homologous gene thereof where splicing defect has been caused in the polynucleotide, or a protein consisting of amino acids encoded by a polynucleotide of at least one gene selected from the genes listed in Table 2, Table 4 and Table 5 or a homologous gene thereof where splicing defect has been caused in the polynucleotide.

13. The method according to claim 11, wherein the abnormal protein of which expression level is measured is a protein consisting of amino acids encoded by a polynucleotide of at least one gene selected from DNAJB1, BZW1, NUP54, RIOK3, CDKN1B, STK17B, EIF4AI and ID1 where splicing defect has been caused in the polynucleotide.

14. The method according to claim 11, wherein in step (f), the expression level of the abnormal protein in the samples obtained from a subject before and after administration of the compound represented by formula (I) or the pharmaceutically acceptable salt thereof or the solvate of them, is measured.

15. The method according to claim 14, wherein the samples obtained from the subject are selected from hemocytes in peripheral blood, plasma, and serum.

16. An antibody against an abnormal protein consisting of amino acids encoded by a polynucleotide of at least one gene selected from the genes listed in Table 1, Table 2, Table 3, Table 4 and Table 5 where splicing defect has been caused in the polynucleotide, or a fragment thereof.

17. A reagent or kit for assaying an action of a compound represented by formula (I), a pharmaceutically acceptable salt thereof, or a solvate of them to a mammal, comprising the antibody or the fragment thereof according to claim 16.


SEQUENCE LISTING

<110> Eisai R&D Management Co., Ltd.

<120> Method for examining effects of antitumor agents using splicing ab
normality as index

<130> 172357PX

<150> US 60/904774
<151> 2007-03-05

<150> US 60/960403
<151> 2007-09-28

<160> 53

<170> PatentIn version 3.1

<210> 1
<211> 24
<212> DNA
<213> Artificial

<220>
<223> primer

<400> 1
atgtcaaacg tgcgagtgtc taac 24

<210> 2
<211> 24
<212> DNA
<213> Artificial

<220>
<223> primer

<400> 2
ttacgtttga cgtgttgtga ggcc 24

<210> 3
<211> 24
<212> DNA
<213> Artificial

<220>
<223>  primer

<400>  3
gccatgtcag aaccggctgg ggat                                              24


<210>  4
<211>  24
<212>  DNA
<213>  Artificial

<220>
<223>  primer

<400>  4
ttagggcttc ctcttggaga agat                                              24


<210>  5
<211>  30
<212>  DNA
<213>  Artificial

<220>
<223>  primer

<400>  5
cctcgatcct ccctttatcc agccctcact                                        30


<210>  6
<211>  30
<212>  DNA
<213>  Artificial

<220>
<223>  primer

<400>  6
gaaccaaaat cactttcccc aaggaaggag                                        30


<210>  7
<211>  27
<212>  DNA
<213>  Artificial

<220>
<223>  primer

<400>  7
aatgaggtcc ccacgtttct cgggtgt                                          27

<210>  8
<211>  29
<212>  DNA
<213>  Artificial

<220>
<223>  primer

<400>  8
gccaataagc aaagtgttga acacttcac                                        29

<210>  9
<211>  26
<212>  DNA
<213>  Artificial

<220>
<223>  primer

<400>  9
aagtgcttga tggcttgctc tgctac                                           26

<210>  10
<211>  27
<212>  DNA
<213>  Artificial

<220>
<223>  primer

<400>  10
acatggacag ctttgctgag tcggtcc                                          27

<210>  11
<211>  28
<212>  DNA
<213>  Artificial

<220>
<223>  primer

<400>  11
ttgctagacg actgttttcc aactccag                                          28

<210>  12
<211>  25
<212>  DNA
<213>  Artificial

<220>
<223>  primer

<400>  12
gctgaaggac catttattac tggag                                             25

<210>  13
<211>  25
<212>  DNA
<213>  Artificial

<220>
<223>  primer

<400>  13
ttcttgctgt gttctttctc ccaca                                             25

<210>  14
<211>  29
<212>  DNA
<213>  Artificial

<220>
<223>  primer

<400>  14
ctaatcaaac aggaaattca aaggaagag                                         29

<210>  15
<211>  27
<212>  DNA
<213>  Artificial

<220>
<223>  primer

<400>  15
cttgatttct cgtaacagat ctgcatc                                          27


<210>  16
<211>  27
<212>  DNA
<213>  Artificial

<220>
<223>  primer

<400>  16
actctcagca acaacaccag agctcta                                          27


<210>  17
<211>  27
<212>  DNA
<213>  Artificial

<220>
<223>  primer

<400>  17
tagctttcgt gccattgcca caacatc                                          27


<210>  18
<211>  23
<212>  DNA
<213>  Artificial

<220>
<223>  primer

<400>  18
ggcctgatgg gtcttatcta tgg                                              23


<210>  19
<211>  23
<212>  DNA
<213>  Artificial

<220>
<223>  primer

<400>  19
ttagatggaa gctggctcaa gag                                              23


<210>  20
<211>  23
<212>  DNA
<213>  Artificial

<220>
<223>  primer

<400>  20
gaactttgcc tcattctttt gca                                              23


<210>  21
<211>  26
<212>  DNA
<213>  Artificial

<220>
<223>  primer

<400>  21
ctgagctcca gtctcttgta tttctg                                           26


<210>  22
<211>  29
<212>  DNA
<213>  Artificial

<220>
<223>  primer

<400>  22
caaggtaacc acttctaaga ccataattc                                        29


<210>  23
<211>  28
<212>  DNA
<213>  Artificial

<220>
<223>  primer

<400>  23
cctgcttgaa gattacataa ctttttgt                                          28


<210>  24
<211>  29
<212>  DNA
<213>  Artificial

<220>
<223>  primer

<400>  24
tcaatggaga tagcaaaggt attataacc                                         29


<210>  25
<211>  30
<212>  DNA
<213>  Artificial

<220>
<223>  primer

<400>  25
agatttactt aggagcacat tatgagtgtt                                        30


<210>  26
<211>  21
<212>  DNA
<213>  Artificial

<220>
<223>  primer

<400>  26
atgtttatca acggtccgcc t                                                 21


<210>  27
<211>  21
<212>  DNA
<213>  Artificial

<220>
<223>  primer

<400>  27
catccccagt ggcttttaag g                                                21

<210>  28
<211>  18
<212>  DNA
<213>  Artificial

<220>
<223>  primer

<400>  28
tgcgcggaga cagcatag                                                    18

<210>  29
<211>  20
<212>  DNA
<213>  Artificial

<220>
<223>  primer

<400>  29
tgggttcaaa atgccaggtt                                                  20

<210>  30
<211>  21
<212>  DNA
<213>  Artificial

<220>
<223>  primer

<400>  30
ggcccttcag aaatgcttgt c                                                21

<210>  31
<211>  25
<212>  DNA
<213>  Artificial

<220>
<223>  primer

<400>  31
cccattacac aattcatgtg cttag                                          25


<210>  32
<211>  25
<212>  DNA
<213>  Artificial

<220>
<223>  primer

<400>  32
cagatcatct agaagcagct ggttt                                          25


<210>  33
<211>  26
<212>  DNA
<213>  Artificial

<220>
<223>  primer

<400>  33
acagaatctg gtgcctacta acaaaa                                         26


<210>  34
<211>  20
<212>  DNA
<213>  Artificial

<220>
<223>  primer

<400>  34
gagcagaagg aagcgtggtt                                                20


<210>  35
<211>  26
<212>  DNA
<213>  Artificial

<220>
<223>  primer

<400>  35
gacacatgaa tttccacaac agtaaa                                          26

<210>  36
<211>  28
<212>  DNA
<213>  Artificial

<220>
<223>  primer

<400>  36
acgtaatgac attttggtct gagtaact                                        28

<210>  37
<211>  23
<212>  DNA
<213>  Artificial

<220>
<223>  primer

<400>  37
aaatgctgct aaacatcctg gaa                                             23

<210>  38
<211>  25
<212>  DNA
<213>  Artificial

<220>
<223>  primer

<400>  38
tgaatcctga atactatgcc tcctt                                           25

<210>  39
<211>  28
<212>  DNA
<213>  Artificial

<220>
<223>  primer

<400>  39
tcccttcttc tcaagactac tcagtatg                                          28


<210>  40
<211>  27
<212>  DNA
<213>  Artificial

<220>
<223>  primer

<400>  40
accttcttct cacaagactt tttaagc                                           27


<210>  41
<211>  20
<212>  DNA
<213>  Artificial

<220>
<223>  primer

<400>  41
ccttctcagc caccatggaa                                                   20


<210>  42
<211>  19
<212>  DNA
<213>  Artificial

<220>
<223>  primer

<400>  42
ccaggtttcc cctgcagtt                                                    19


<210>  43
<211>  21
<212>  DNA
<213>  Artificial

<220>
<223>  primer

<400>  43
ccccctgact tagtcctcgt t                                                21


<210>  44
<211>  23
<212>  DNA
<213>  Artificial

<220>
<223>  primer

<400>  44
cccactgcct tgatctagtt gat                                              23


<210>  45
<211>  23
<212>  DNA
<213>  Artificial

<220>
<223>  primer

<400>  45
gtcaagcaat ctatggccta cca                                              23


<210>  46
<211>  23
<212>  DNA
<213>  Artificial

<220>
<223>  primer

<400>  46
tgctgtgaga ataatgggtt gtg                                              23


<210>  47
<211>  21
<212>  DNA
<213>  Artificial

<220>
<223>  primer

<400>  47
ggctggctta ggagcttcac t                                                21


<210>  48
<211>  21
<212>  DNA
<213>  Artificial

<220>
<223>  primer

<400>  48
acctgcggtt cccactttat t                                                21


<210>  49
<211>  25
<212>  DNA
<213>  Artificial

<220>
<223>  primer

<400>  49
accactccaa atgtctaagg tcact                                            25


<210>  50
<211>  23
<212>  DNA
<213>  Artificial

<220>
<223>  primer

<400>  50
ggcctgatgg gtcttatcta tgg                                              23


<210>  51
<211>  23
<212>  DNA
<213>  Artificial

<220>
<223>  primer

<400>  51
ttagatggaa gctggctcaa gag                                              23

<210>  52
<211>  23
<212>  DNA
<213>  Artificial

<220>
<223>  primer

<400>  52
gaactttgcc tcattctttt gca                                              23

<210>  53
<211>  26
<212>  DNA
<213>  Artificial

<220>
<223>  primer

<400>  53
ctgagctcca gtctcttgta tttctg                                           26